(19) Europäisches Patentamt / European Patent Office / Office européen des brevets

(11) **EP 3 819 283 A1**

(12) **EUROPEAN PATENT APPLICATION**
published in accordance with Art. 153(4) EPC

(43) Date of publication:
**12.05.2021 Bulletin 2021/19**

(21) Application number: **19830751.4**

(22) Date of filing: **28.06.2019**

(51) Int Cl.:
*C07B 61/00* (2006.01)   *C07C 67/14* (2006.01)
*C07C 69/80* (2006.01)   *C07C 69/82* (2006.01)
*C07C 231/02* (2006.01)   *C07C 233/67* (2006.01)
*C07C 233/77* (2006.01)   *C07C 233/81* (2006.01)
*C07C 235/84* (2006.01)   *C07C 273/18* (2006.01)
*C07C 275/40* (2006.01)   *C07D 207/448* (2006.01)
*C08F 2/00* (2006.01)   *C08G 18/32* (2006.01)
*C08G 18/34* (2006.01)   *C08G 63/16* (2006.01)
*C08G 63/78* (2006.01)   *C08G 69/26* (2006.01)
*C08G 73/10* (2006.01)

(86) International application number:
**PCT/JP2019/025811**

(87) International publication number:
**WO 2020/009016 (09.01.2020 Gazette 2020/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority:  **05.07.2018   JP 2018128473**

(71) Applicant: **Unitika Ltd.**
**Amagasaki-shi, Hyogo 660-0824 (JP)**

(72) Inventors:
• **FUKUBAYASHI, Yumeto**
  **Uji-shi, Kyoto 611-0021 (JP)**
• **NAKAI, Makoto**
  **Uji-shi, Kyoto 611-0021 (JP)**
• **YANAKA, Ayumi**
  **Uji-shi, Kyoto 611-0021 (JP)**

(74) Representative: **Vossius & Partner**
**Patentanwälte Rechtsanwälte mbB**
**Siebertstrasse 3**
**81675 München (DE)**

(54) **METHOD FOR PRODUCING ORGANIC COMPOUND**

(57)   The present invention provides a production method of an organic compound, in which a reaction is performed between functional groups without using any solvents. The present invention relates to a production method of an organic compound, in which a reaction is performed between functional groups by using a mechanochemical effect.

EP 3 819 283 A1

**Description**

TECHNICAL FIELD

The present invention relates to a production method of an organic compound using a mechanochemical effect.

BACKGROUND ART

High-molecular compounds obtained by sequential polymerization of polyamides, polyesters, polyimides, or the like are widely and industrially used. These high-molecular compounds are produced by various methods, but those having a high melting point and glass transition temperature, which are difficult to melt-polymerize, are generally polymerized in an organic solvent. However, when the reaction is performed in a solution, purification needs to be performed when the obtained compound is isolated, which complicates the process and is costly. In addition, there is a disadvantage that the yield of the compound is lowered in the process. Furthermore, in recent years, from the viewpoint of reducing the environmental load, there is an increasing demand for a clean process that does not use a solvent. Therefore, there has been a demand for the development of a production process for a high-molecular compound that can obtain a target product in good yield without using any solvents nor including complicated purification steps.

In recent years, a method utilizing a mechanochemical effect has attracted attention as a method for synthesizing a compound that does not use an organic solvent. The mechanochemical effect means that when a solid or particle is given various mechanical energy (compression, shear, impact, grinding, etc.), those substances become active and change their physicochemical properties. The effects that occur here are collectively referred to as a mechanochemical effect, and have been confirmed in many substances such as inorganic substances, organic substances, and metals.

Above all, in reactions between inorganic compounds or between metals, and reactions between inorganic compounds or metals and organic substances, there are restrictions such as a limited solvent for dissolving the inorganic compounds and metals, and consumption of enormous energy during a melting process. Therefore, many methods have been proposed to positively utilize the mechanochemical effect to perform the reactions.

On one hand, as a production method using the mechanochemical effect in the polymerization reaction of a high-molecular compound, for example, there are methods disclosed in Non Patent Literatures 1 to 4. However, these methods have been limited to high-molecular compounds produced by chain polymerization or living polymerization using radicals or ions.

On the other hand, a method of modifying a functional group in a high-molecular compound with another compound has been disclosed, and a technique in which a pulverized product of a cured product of a phenolic resin molding material and a specific phenol compound are mechanochemically reacted to surface fusion bond between particles of the components (Patent Literature 1), and a technique in which cellulose and an acylating agent are mechanochemically reacted to produce acylated cellulose (Patent Literature 2) are known.

CITATIONS LIST

PATENT LITERATURES

Patent Literature 1: JP 11-333836A
Patent Literature 2: JP 2004-191760 A

NON-PATENT LITERATURES

Non Patent Literature 1: Pharmaceutical Journal, 120 (12), p1337 (2000)
Non Patent Literature 2: Proceedings of the Society of Polymer Science, 49 (2), p181 (2000)
Non Patent Literature 3: Polymer-Plastic Technology and Engineering, 40 (2), p183 (2001)
Non Patent Literature 4: Recent Trends in Reactive Processing Technology in Polymer Materials (Sumibe Techno Research, 2003.3): Controlling Microstructures, Research and Development at the Nano Level Advances

SUMMARY OF INVENTION

PROBLEMS TO BE SOLVED BY THE INVENTION

An object of the present invention is to provide a production method of an organic compound, in which a reaction is performed between functional groups without using any solvents.

MEANS FOR SOLVING PROBLEMS

The present invention relates to a production method of an organic compound, in which a reaction is performed between functional groups by using a mechanochemical effect.
As a result of intensive studies on the above problems, the present inventors have found that the above problems can be solved by utilizing mechanical energy generated when a raw material compound used in the reaction is pulverized, to exhibit the mechanochemical effect. The present invention has been accomplished thereby.
That is, the gists of the present invention are as follows.

<1> A production method of an organic compound, wherein a reaction is performed between functional groups by using a mechanochemical effect.
<2> The production method of an organic compound of <1>, wherein the reaction is a condensation reaction, an addition reaction, or a composite reaction thereof.
<3> The production method of an organic compound of <1> or <2>, wherein the reaction is a reaction between two functional groups selected from the group consisting of a carboxyl group and a halide thereof, an acid anhydride group, an amino group, an isocyanate group, and a hydroxyl group.
<4> The production method of an organic compound of any one of <1> to <3>, wherein the reaction is one or more reactions selected from the group consisting of following reactions:

(A) a reaction in which an acid anhydride group and an amino group are allowed to react to form (a1) an amide group and a carboxyl group, (a2) an imide group, (a3) an isoimide group or (a4) a mixture thereof;
(B) a reaction in which an acid anhydride group and an isocyanate group are allowed to react to form an imide group;
(C) a reaction in which a carboxyl group or a halide thereof and an amino group or an isocyanate group are allowed to react to form an amide group;
(D) a reaction in which a carboxyl group or a halide thereof and a hydroxyl group are allowed to react to form an ester group;
(E) a reaction in which an isocyanate group and an amino group are allowed to react to form a urea group;
(F) a reaction in which an isocyanate group and a hydroxyl group are allowed to react to form a urethane group; and
(G) a reaction in which an acid anhydride group and a hydroxyl group are allowed to react to form an ester group and a carboxyl group.

<5> The production method of an organic compound of any one of <1> to <4>, wherein the reaction between functional groups occurs between two or more raw material compound molecules.
<6> The production method of an organic compound of <5>, wherein each of the raw material compounds is a compound having a molecular weight of 2000 or less.
<7> The production method of an organic compound of any one of <1> to <6>, wherein the reaction is promoted by heating.
<8> The production method of an organic compound of any one of <1> to <7>, wherein the organic compound is a high-molecular compound containing a repeating unit.
<9> The production method of an organic compound of <8>, wherein the reaction belongs to one or more reactions selected from the group consisting of a sequential polymerization reaction, a condensation polymerization reaction, and a polyaddition reaction.
<10> The production method of an organic compound of <8> or <9>, wherein the high-molecular compound is a polyamic acid-based compound, a polyimide-based compound, a polyamide-based compound, a polyamide-imide-based compound, a polyurethane-based compound, a polyurea-based compound, or a polyester-based compound.
<11> The production method of an organic compound of any one of <8> to <10>, wherein the reaction is performed in a presence of a terminal blocking agent.
<12> The production method of an organic compound of any one of <1> to <11>, wherein a polyamic acid-based compound, a polyimide-based compound, or a mixture thereof is produced as the organic compound by performing a reaction of a tetracarboxylic dianhydride component with a diamine component or a diisocyanate component, as the reaction.

<13> The production method of an organic compound of any one of <1> to <11>, wherein a polyamide-based compound is produced as the organic compound by performing a reaction of a dicarboxylic acid component or its acid halide component with a diamine component or a diisocyanate component, as the reaction.

<14> The production method of an organic compound of any one of <1> to <11>, wherein a polyamide-imide-based compound is produced as the organic compound by performing a reaction of a tricarboxylic acid anhydride component or its acid halide component with a diamine component or a diisocyanate component, as the reaction.

<15> The production method of an organic compound of any one of <1> to <11>, wherein a polyester-based compound is produced as the organic compound by performing a reaction of a dicarboxylic acid component or its acid halide component with a polyhydroxy component, as the reaction.

<16> The production method of an organic compound of any one of <1> to <11>, wherein a polyurea-based compound is produced as the organic compound by performing a reaction of a diisocyanate component with a diamine component as the reaction.

<17> The production method of an organic compound of any one of <1> to <11>, wherein a polyurethane-based compound is produced as the organic compound by performing a reaction of a diisocyanate component with a polyhydroxy component as the reaction.

<18> The production method of an organic compound of any one of <12> to <17>, wherein the components are used in an amount such that one component has a molar amount of 0.8 to 1.2 times that of the other component.

<19> The production method of an organic compound of any one of <1> to <7>, wherein the organic compound is a low-molecular compound containing no repeating unit.

<20> The production method of an organic compound of <19>, wherein a diimide dicarboxylic acid-based compound is produced as the organic compound by performing a reaction of a tricarboxylic acid anhydride component with a diamine component in a molar amount of 0.1 to 0.7 times that of the tricarboxylic acid anhydride component, as the reaction.

<21> The production method of an organic compound of <19>, wherein a diimide dicarboxylic acid-based compound is produced as the organic compound by performing a reaction of a tetracarboxylic dianhydride component with a monoaminomonocarboxylic acid component in a molar amount of 1.5 to 10.0 times that of the tetracarboxylic dianhydride component, as the reaction.

<22> The production method of an organic compound of <19>, wherein a diimide tricarboxylic acid-based compound is produced as the organic compound by performing a reaction of a tricarboxylic acid anhydride component with a diaminomonocarboxylic acid component in a molar amount of 0.1 to 0.7 times that of the tricarboxylic acid anhydride component, as the reaction.

<23> The production method of an organic compound of <19>, wherein a diimide tetracarboxylic acid-based compound is produced as the organic compound by performing a reaction of a tetracarboxylic dianhydride component with a monoaminodicarboxylic acid component in a molar amount of 1.5 to 10.0 times that of the tetracarboxylic dianhydride component, as the reaction.

<24> The production method of an organic compound of <19>, wherein a monoimide dicarboxylic acid-based compound is produced as the organic compound by performing a reaction of a tricarboxylic acid anhydride component with a monoaminomonocarboxylic acid component in a molar amount of 0.5 to 5.0 times that of the tricarboxylic acid anhydride component, as the reaction.

<25> The production method of an organic compound of <19>, wherein a monoimide tricarboxylic acid-based compound is produced as the organic compound by performing a reaction of a tricarboxylic acid anhydride component with a monoaminodicarboxylic acid component in a molar amount of 0.5 to 5.0 times that of the tricarboxylic acid anhydride component, as the reaction.

<26> The production method of an organic compound of <20>, wherein an amide group-containing diimide dicarboxylic acid-based compound is produced by using a diamine component containing an amide group as the diamine component.

<27> The production method of an organic compound of <19>, wherein an amide group-containing monoimide dicarboxylic acid-based compound is produced as the organic compound by performing a reaction of a tricarboxylic acid anhydride halide component with a monoaminomonocarboxylic acid component in a molar amount of 1.5 to 10.0 times that of the tricarboxylic acid anhydride halide component, as the reaction.

<28> The production method of an organic compound of <19>, wherein an amide group-containing monoimide tetracarboxylic acid-based compound is produced as the organic compound by performing a reaction of a tricarboxylic acid anhydride halide component with a monoaminodicarboxylic acid component in a molar amount of 1.5 to 10.0 times that of the tricarboxylic acid anhydride halide component, as the reaction.

<29> The production method of an organic compound of <19>, wherein an ester group-containing monoimide tricarboxylic acid-based compound is produced as the organic compound by performing a reaction of a tricarboxylic acid anhydride component with a monohydroxy monoamine component in a molar amount of 0.1 to 0.7 times that of the tricarboxylic acid anhydride component, as the reaction.

<30> The production method of an organic compound of <19>, wherein a diimide dihydroxy-based compound is produced as the organic compound by performing a reaction of a tetracarboxylic dianhydride component with a monohydroxy monoamine component in a molar amount of 1.5 to 10.0 times that of the tetracarboxylic dianhydride component, as the reaction.

<31> The production method of an organic compound of <19>, wherein a diamide dicarboxylic acid-based compound is produced as the organic compound by performing a reaction of a dicarboxylic acid halide component with a monoaminomonocarboxylic acid component in a molar amount of 1.5 to 10.0 times that of the dicarboxylic acid halide component, as the reaction.

<32> The production method of an organic compound of <19>, wherein a diamide tetracarboxylic acid-based compound is produced as the organic compound by performing a reaction of a dicarboxylic acid halide component with a monoaminodicarboxylic acid component in a molar amount of 1.5 to 10.0 times that of the dicarboxylic acid halide component, as the reaction.

<33> The production method of an organic compound of <19>, wherein a diester dicarboxylic acid-based compound is produced as the organic compound by performing a reaction of a dicarboxylic acid halide component with a monohydroxy monocarboxylic acid component in a molar amount of 1.5 to 10.0 times that of the dicarboxylic acid halide component, as the reaction.

<34> The production method of an organic compound of <19>, wherein a diester tetracarboxylic acid-based compound is produced as the organic compound by performing a reaction of a dicarboxylic acid halide component and a monohydroxy dicarboxylic acid component in a molar amount of 1.5 to 10.0 times that of the dicarboxylic acid halide component, as the reaction.

<35> The production method of an organic compound of <19>, wherein a curable imide-based compound is produced as the organic compound by performing a reaction of an unsaturated dicarboxylic acid anhydride component with a diamine component in a molar amount of 0.1 to 0.7 times that of the unsaturated dicarboxylic acid anhydride component, as the reaction.

<36> The production method of an organic compound of any one of <1> to <35>, wherein the reaction is performed in a presence of a catalyst.

<37> The production method of an organic compound of any one of <1> to <36>, wherein the reaction is performed in a presence of an auxiliary agent.

<38> The production method of an organic compound of any one of <1> to <37>, wherein after reaction by the mechanochemical effect, a heat treatment is performed, to thereby increase a reaction rate.

EFFECTS OF THE INVENTION

According to the present invention, organic compounds ranging from low-molecular compounds to high-molecular compounds can be produced without using any solvents.

MODES FOR CARRYING OUT THE INVENTION

<Production method of organic compound>

The production method of the present invention is a method for obtaining an organic compound by utilizing mechanical energy generated when a raw material compound used in the reaction is pulverized, to exhibit a mechanochemical effect.

In the present invention, the mechanochemical effect is an effect (or phenomenon) in which a raw material compound in a solid state under a reactive environment is pulverized by applying mechanical energy (compressive force, shearing force, impact force, grinding force, etc.) to the raw material compound, to thereby activate the pulverization interface thus formed. This causes a reaction between functional groups. The reaction between functional groups usually occurs between two or more raw material compound molecules. For example, the reaction between functional groups may occur between two raw material compound molecules having different chemical structures, or between two raw material compound molecules having the same chemical structure. The reaction between functional groups occur not only between a limited set of two raw material compound molecules, but also usually occur between other sets of two raw material compound molecules. A new reaction between functional groups may occur between a compound molecule formed by the reaction between functional groups, and the raw material compound molecule. The reaction between functional groups is usually a chemical reaction, which allows the functional group of each raw material compound molecule to form a bond group (particularly a covalent bond) between the two raw material compound molecules, to thereby form another compound molecule.

The reactive environment means an environment in which the raw material compound is placed for the reaction, that is, an environment in which mechanical energy is applied, and may be, for example, an environment in an apparatus. Being in a solid state under a reactive environment means being in a solid state under an environment where mechanical energy is applied (e.g., under a temperature and a pressure in an apparatus). The raw material compound in the solid state under the reactive environment may usually be in a solid state at normal temperature (25°C) and normal pressure (101.325 kPa). The raw material compound in the solid state under the reactive environment may be in a solid state at the start of applying mechanical energy. In the present invention, as the mechanical energy continues to be applied, the temperature and/or pressure increases, so that the raw material compound in the solid state under the reactive environment may still be changed to be in a liquid state (e.g., a molten state) during the reaction (or treatment), but from the viewpoint of improving the reaction rate, it is preferable that the raw material compound is continuously in the solid state during the reaction (or treatment).

The details of the mechanochemical effect are not clear, but it is considered to follow the following principle. When mechanical energy is applied to one or more raw material compounds in a solid state to cause pulverization, the pulverization interface is activated by absorption of the mechanical energy. It is considered that surface active energy of such a pulverization interface causes a chemical reaction between two raw material compound molecules. Pulverization means that when mechanical energy is applied to the raw material compound particles, the particles absorb the mechanical energy, so that cracks occur in the particles, and their surfaces are renewed. Renewing the surface means forming a pulverization interface as a new surface. In the mechanochemical effect, the state of the new surface formed by surface renewal is not particularly limited as long as the pulverization interface is activated due to the pulverization, and may be in a dry state or in a wet state. The wet state of the new surface due to the surface renewal is caused by the raw material compound in the liquid state different from the raw material compound in the solid state.

The mechanical energy is applied to a raw material mixture containing one or more raw material compounds that are in a solid state under the reactive environment. The state of the raw material mixture is not particularly limited as long as the raw material compound in the solid state is pulverized by applying the mechanical energy. For example, the raw material mixture may be in a dry state due to the fact that all the raw material compounds contained in the raw material mixture are in a solid state. Further, for example, the raw material mixture may be in a wet state due to the fact that at least one raw material compound contained in the raw material mixture is in a solid state and the remaining raw material compounds are in a liquid state. Specifically, for example, when the raw material mixture contains only one raw material compound, the one raw material compound is in a solid state. Further, for example, when the raw material mixture contains two raw material compounds, the two raw material compounds may be in a solid state, or one of the raw material compounds may be in a solid state and the other raw material compound may be in a liquid state.

In the present invention, the functional group is a monovalent group (atomic group) that can become a cause of reactivity in the molecular structure, and is used in a concept other than an unsaturated bond group (e.g., radical polymerizable group) such as a carbon-carbon double bond group or a carbon-carbon triple bond group. The functional group is a group containing a carbon atom and a hetero atom. The hetero atom is one or more atoms selected from the group consisting of an oxygen atom, a nitrogen atom, and a sulfur atom, particularly the group consisting of an oxygen atom and a nitrogen atom. The functional group may further contain a hydrogen atom. Usually two functional groups are subjected to the reaction, and a raw material compound molecule having one functional group and a raw material compound molecule having the other functional group may be different or identical in structure. The reaction allows a bond (particularly a covalent bond) to be formed between the two raw material compound molecules, thereby forming one molecule. Small molecules such as water, carbon dioxide, and/or alcohol may or may not be by-produced through the reaction between functional groups.

The reaction between functional groups may be a reaction between any functional groups (particularly monovalent functional groups) capable of chemically reacting with each other, and is, for example, a reaction between two functional groups selected from the group consisting of a carboxyl group and a halide (group) thereof, an acid anhydride group, an amino group, an isocyanate group, a hydroxyl group, and the like. The two functional groups are not particularly limited as long as a they causes a chemical reaction, and may be, for example, two functional groups having different chemical structures or two functional groups having the same chemical structure.

Examples of the reaction between functional groups include a condensation reaction, an addition reaction, or a composite reaction thereof, and the like.

The condensation reaction is a reaction in which a bond or link between raw material compound molecules is achieved with dissociation of small molecules such as water, carbon dioxide, and alcohol between the raw material compound molecules. Examples of the condensation reaction include a reaction in which an amide group is formed (amidation reaction), a reaction in which an imide group is formed (imidization reaction), and a reaction in which an ester group is formed (esterification reaction).

The addition reaction is an addition reaction performed between functional groups, and is a reaction in which a bond or link between raw material compound molecules is achieved without dissociation of small molecules between the raw material compound molecules. Examples of the addition reaction include a reaction in which a urea group is formed, a reaction in which a urethane group is formed, and a reaction in which a ring structure is opened (that is, a ring-opening reaction). The ring-opening reaction is a reaction in which, in a raw material compound having a ring structure (e.g., an acid anhydride group-containing compound, a cyclic amide compound, a cyclic ester compound, and an epoxy compound), a part of the ring structure is cleaved, and a bond or link between the cleaved site and a functional group of the other raw material compound is achieved. The ring-opening reaction results in the formation of, for example, an amide group, a carboxyl group, an ester group, and an ether group. In particular, in the ring-opening reaction of an acid anhydride group in an acid anhydride group-containing compound as the raw material compound, the acid anhydride group is opened, and a bond or a link with another raw material compound molecule (amino group or hydroxyl group) is achieved. As a result, for example, an amide group or an ester group, and a carboxyl group are simultaneously formed.

More specifically, the reaction between functional groups may be, for example, one or more reactions selected from the group consisting of the following reactions:

(A) A reaction in which an acid anhydride group and an amino group are allowed to react to form (a1) an amide group and a carboxyl group, (a2) an imide group, (a3) an isoimide group or (a4) a mixture thereof;
(B) a reaction in which an acid anhydride group and an isocyanate group are allowed to react to form an imide group;
(C) a reaction in which a carboxyl group or a halide (group) thereof and an amino group or an isocyanate group are allowed to react to form an amide group;
(D) a reaction in which a carboxyl group or a halide (group) thereof and a hydroxyl group are allowed to react to form an ester group;
(E) a reaction in which an isocyanate group and an amino group are allowed to react to form a urea group;
(F) a reaction in which an isocyanate group and a hydroxyl group are allowed to react to form a urethane group; and
(G) a reaction in which an acid anhydride group and a hydroxyl group are allowed to react to form an ester group and a carboxyl group.

The raw material compound contains one or more (e.g., 1 to 5), preferably 2 to 5, more preferably 2 to 3 functional groups selected from the group consisting of the above-mentioned functional groups per molecule. The molecular weight of the raw material compound is not particularly limited, and is preferably 2000 or less, particularly from 1500 to 30, more preferably from 1000 to 30, from the viewpoint of further improving the reaction rate. When a compound having a molecular weight larger than the above range is used as the raw material compound, the reaction rate may decrease, which is not preferable. The details of the reason are not clear, but the activation of the pulverization interface in the mechanochemical effect is based on the activity of the raw material compound molecule, and it is therefore considered that the larger molecular weight may attenuate such activity in the molecule. Alternatively, it is considered that when the molecular weight is large, the density of the functional group per molecule is low, so that the probability of contact between the activated functional groups may be reduced.

The reaction by the mechanochemical effect (that is, the application of mechanical energy) may be performed in one step or in multiple steps of two or more steps. A method of applying mechanical energy in one step can be referred to as a one-step mechanochemical method. A method of applying mechanical energy in multiple steps of two or more steps can be referred to as a multi-step mechanochemical method. For example, in the one-step mechanochemical method, the raw material compound is charged into an apparatus (e.g., a pulverizer, a mixer, or a stirrer) to be described later at a desired composition ratio, and then the mechanochemical reaction is terminated by a one-step pulverization treatment. Further, for example, in a two-step mechanochemical method, the raw material compound is charged into an apparatus to be described later at a desired composition ratio, and is then subjected to a first-step pulverization treatment of coarsely pulverizing, followed by further a second-step pulverization treatment of finely pulverizing.

From the viewpoint of further improving the reaction rate and operability, it is preferable to adopt the multi-step mechanochemical method (particularly a two-step mechanochemical method). Specifically, when a fine pulverization treatment is suddenly performed in the first step, adhesion and sticking of the sample to the apparatus occur, which may cause problems in operability such as a decrease in the yield of the obtained product, and unnecessary shutdown of the apparatus during the treatment. Therefore, from the viewpoint of further improving the reaction rate and operability, it is preferable to adopt the multi-step mechanochemical method.

In the multi-step mechanochemical method, the apparatus used in each step may be independently selected from the apparatuses described in detail later. In particular, in the two-step mechanochemical method, it is preferable that the apparatus used in the coarse pulverization treatment in the first step is different from the apparatus used in the fine pulverization treatment in the second step. Since the each apparatus has an appropriate target particle size (recommended target particle size after pulverization), efficient pulverization can be performed by using apparatuses having mutually different target particle sizes that are appropriate in the steps in the multi-step mechanochemical method. As a result, the reaction rate is further improved. From the viewpoint of further improving the reaction rate based on such efficient pulverization (that is, reduction in particle size), an apparatus in which the appropriate target particle size is smaller than that in the apparatus used in the immediately preceding step is preferably used as the apparatus used immediately after the step in the multi-step mechanochemical method. From the same viewpoint, for example, in the two-step mechanochemical method, it is preferable to perform pulverization using a high-speed bottom stirring type mixer in the first step and using a medium-stirring type mill in the second step.

In the present invention, the reaction between functional groups by the mechanochemical effect is achieved by subjecting a raw material mixture containing at least one or more raw material compounds in a solid state as described above to a pulverization treatment.

In the present invention, as described later, the type and molecular weight of the organic compound to be produced can be controlled by adjusting or selecting the reaction conditions (e.g., pulverization conditions) and/or the type and ratio of the raw material compound. The organic compound to be produced includes a high-molecular compound and a low-molecular compound.

[High-molecular compound]

The high-molecular compound is an organic compound containing a repeating unit. The organic compound containing a repeating unit means that, in the structural formula of the organic compound, one or more types of structural units that are continuously repeated twice or more times are included. A structural unit is a unit constituting a main chain and is different from a unit constituting a side chain or a substituent.

When a high-molecular compound is produced, the reaction between functional groups is a polymerization reaction. The polymerization reaction is a chemical reaction in which monomers (monomer) or polymers (polymer), particularly monomers, are allowed to react to be linked together, and a target polymer is then synthesized. The polymerization reaction is roughly classified into a sequential polymerization reaction and a chain polymerization reaction according to the difference in the reaction path. In the present invention, the polymerization reaction is a sequential polymerization reaction.

The sequential polymerization reaction is a polymerization reaction in which a monomer or a polymer, particularly functional groups of a monomer are allowed to react with each other and are gradually polymerized, and includes a condensation polymerization reaction and a polyaddition reaction.

The condensation polymerization reaction is a polymerization reaction which proceeds by repeating a condensation reaction between functional groups with the formation of small molecules such as water, alcohol, hydrogen halide, and/or carbon dioxide.

The polyaddition reaction is a polymerization reaction in which a covalent bond is formed by repeating an addition reaction between functional groups to form a polymer.

The sequential polymerization reaction is distinguished from an addition polymerization reaction of a vinyl compound or an olefin compound in which polymerization proceeds in a chain-reaction manner or a ring-opening polymerization reaction of a cyclic compound. In addition, the condensation polymerization reaction involves the formation of a covalent bond with by-production of small molecules such as water, alcohol, hydrogen halide, and/or carbon dioxide, whereas the polyaddition reaction does not produce any small molecule by-products.

On the other hand, the chain polymerization reaction is a polymerization reaction in which polymerization active species consecutively react by adding an initiator or the like to a monomer having no functional group to promote the reaction. It includes a radical polymerization reaction and an ion polymerization reaction. The radical polymerization reaction is a polymerization reaction used for polymerizing a vinyl compound using highly active neutral radical species as growing species. Further, the ionic polymerization reaction means that the end of the growing chain contains an ion such as an anion or a cation.

In the present invention, examples of the high-molecular compound that can be polymerized by the sequential polymerization reaction include a polyamic acid-based compound, a polyimide-based compound, a polyamide-based compound, a polyamide-imide-based compound, a polyurethane-based compound, a polyurea-based compound, and a polyester-based compound.

The polyamic acid-based compound is a precursor of a polyimide-based compound, which has an amide group and a carboxyl group in the molecular chain (particularly the repeating unit) and forms an imide group by performing a cyclization reaction. The polyamic acid-based compound can also be called polyamic acid.

The polyimide-based compound is a compound having an imide group in the molecular chain (particularly the repeating unit). The polyimide-based compound does not have an amide group in the molecular chain. The polyimide-based compound having an imidization ratio of not 100% is included in the polyamic acid-based compound. The polyimide-based compound includes a polyetherimide-based compound having an imide group and an ether group in the molecular chain (particularly the repeating unit).

The polyamide-based compound is a compound having an amide group in the molecular chain (particularly the repeating unit). The polyamide-based compound does not have an imide group in the molecular chain.

The polyamide-imide-based compound is a compound having an imide group and an amide group in the molecular chain (particularly the repeating unit).

The polyurethane-based compound is a compound having a urethane group in the molecular chain (particularly the repeating unit). The polyurethane-based compound may have an ester group in the molecular chain (particularly the repeating unit).

The polyurea-based compound is a compound having a urea group in the molecular chain (particularly the repeating unit).

The polyester-based compound is a compound having an ester group in the molecular chain (particularly the repeating unit). The polyester-based compound does not have a urethane group in the molecular chain.

The high-molecular compound also includes those containing two or more of the above-mentioned high-molecular compounds.

In the present invention, the type of the obtained high-molecular compound can be controlled by selecting the type of the raw material compound. The raw material compound for producing a high-molecular compound is usually a raw material compound having two or more (particularly two) functional groups selected from the group consisting of the above-mentioned functional groups per molecule. The molecular weight of the raw material compound is not particularly limited, and usually has a molecular weight within the above range.

In the production of a high-molecular compound, for example, when two components are used as the raw material compounds, the components are mutually used in approximately equal molar amounts, specifically, in an amount such that one component has a molar amount of 0.8 to 1.2 times, particularly 0.9 to 1.1 times, preferably 0.95 to 1.05 times that of the other component.


(Polyamic acid-based compound and polyimide-based compound) (Hereinafter, these compounds may be inclusively referred to as polyamic acid-based compound and the like).


A polyamic acid-based compound, a polyimide-based compound, or a mixture thereof can be produced by performing a reaction between functional groups by a mechanochemical effect, using a tetracarboxylic dianhydride component and a diamine component or a diisocyanate component as the raw material compounds. Here, the reaction between functional groups corresponds to the reactions (A) and (B) described above.

More specifically, for example, when a tetracarboxylic dianhydride component and a diamine component are used as the raw material compounds, a polyamic acid-based compound is mainly produced. Further, for example, when a tetracarboxylic dianhydride component and a diisocyanate component are used as the raw material compounds, a polyimide-based compound is mainly produced.

The tetracarboxylic dianhydride component capable of constituting the polyamic acid-based compound and the like includes an aromatic tetracarboxylic dianhydride component containing an aromatic ring, an alicyclic tetracarboxylic dianhydride component containing an aliphatic ring but not an aromatic ring, and an aliphatic tetracarboxylic dianhydride component that does not contain an aromatic ring or an alicyclic ring. The tetracarboxylic dianhydride component may contain an ether group and/or a thioether group, and/or one or more hydrogen atoms may be substituted with a halogen atom (e.g., fluorine atom, chlorine atom, and bromine atom). An ether group herein is an "-O-" group present between carbon atoms. A thioether group is a "-S-" group present between carbon atoms.

Examples of the aromatic tetracarboxylic dianhydride component include pyromellitic dianhydride, 3,3',4,4'-biphenyltetracarboxylic dianhydride, 2,3,3',4'-biphenyltetracarboxylic dianhydride, 3,3',4,4'-benzophenone tetracarboxylic dianhydride, 4,4'-oxydiphthalic anhydride, 3-fluoropyromellitic dianhydride, 3,6-difluoropyromellitic dianhydride, 3,6-bis(trifluoromethyl)pyromellitic dianhydride, 4,4'-(hexafluoroisopropylidene)diphthalic anhydride, 1,2,3,4-benzenetetracarboxylic dianhydride, 2,2',3,3'-benzophenone tetracarboxylic dianhydride, 3,3',4,4'-ethylene glycol dibenzoate tetracarboxylic dianhydride, 3,3",4,4"-terphenyltetracarboxylic dianhydride, 3,3',4,4-quaterphenyltetracarboxylic dianhydride, 3,3',4,4'-quinquephenyltetracarboxylic dianhydride, 2,2',3,3'-biphenyltetracarboxylic dianhydride, methylene-4,4'-diphthalic dianhydride, 1,1-ethynilidene-4,4'-diphthalic dianhydride, 2,2-propyridene-4,4'-diphthalic dianhydride, 1,2-ethylene-4,4'-diphthalic dianhydride, 1,3-trimethylene-4,4'-diphthalic dianhydride, 1,4-tetramethylene-4,4'-diphthalic dianhydride, 1,5-pentamethylene-4,4'-diphthalic dianhydride, 2,2-bis(3,4-dicarboxyphenyl)-1,1,1,3,3,3-hexafluoropropane dianhydride, difluoromethylene-4,4'-diphthalic dianhydride, 1,1,2,2-tetrafluoro-1,2-ethylene-4,4'-diphthalic dianhydride, 1,1,2,2,3,3-hexafluoro-1,3-trimethylene-4,4'-diphthalic dianhydride, 1,1,2,2,3,3,4,4-octafluoro-1,4-tetramethylene-4,4'-diphthalic dianhydride, 1,1,2,2,3,3,4, 4,5,5-decafluoro-1,5-pentamethylene-4,4'-diphthalic dianhydride, oxy-4,4'-diphthalic dianhydride, thio-4,4'-diphthalic anhydride, 3,3',4,4'-diphenylsulfone tetracarboxylic dianhydride, 1,3-bis(3,4-dicarboxyphenyl)-1,1,3,3-tetramethylsiloxane dianhydride, 1,3-bis(3,4-dicarboxyphenyl)benzene dianhydride, 1,4-bis(3,4-dicarboxyphenyl)benzene dianhydride, 1,3-bis(3,4-dicarboxyphenoxy)benzene dianhydride, 1,4-bis(3,4-dicarboxyphenoxy)benzene dianhydride, 1,3-bis[2-(3,4-dicarboxyphenyl)-2-propyl]benzene dianhydride, 1,4-bis[2-(3,4-dicarboxyphenyl)-2-propyl]benzene dianhydride, bis[3-(3,4-dicarboxyphenoxy)phenyl]methane dianhydride, bis[4-(3,4-dicarboxyphenoxy)phenyl]methane dianhydride, 2,2-bis[3-(3,4-dicarboxyphenoxy)phenyl]propane dianhydride, 2,2-bis[4-(3,4-dicarboxyphenoxy)phenyl]propane dianhydride, 2,2-bis[3-(3,4-dicarboxyphenoxy)phenyl]-1,1,1,3,3,3-hexafluoropropane dianhydride, 2,2-bis[4-(3,4-dicarboxyphenoxy)phenyl]propane dianhydride, bis(3,4-dicarboxyphenoxy)dimethylsilane dianhydride, 1,3-bis(3,4-dicarboxyphenoxy)-1,1,3,3-tetramethyldisiloxane dianhydride, 2,3,6,7-naphthalenetetracarboxylic dianhydride, 1,2,5,6-naphthalenetetracarboxylic dianhydride, 3,4,9,10-perylenetetracarboxylic dianhydride, 2,3,6,7-anthracenetetracarboxylic dianhydride, 1,2,7,8-phenanthrenetetracarboxylic dianhydride, 2,2'-difluoro-3,3',4,4'-biphenyltetracarboxylic dianhydride, 5,5'-difluoro-3,3',4,4'-biphenyltetracarboxylic dianhydride, 6,6'-difluoro-3,3',4,4'-biphenyltetracarboxylic dianhydride, 2,2',5,5',6,6'-hexafluoro-3,3',4,4'-biphenyltetracarboxylic dianhydride, 2,2'-bis(trifluoromethyl)-3,3',4,4'-biphenyltetracarboxylic dianhydride, 5,5'-bis(trifluoromethyl)-3,3',4,4'-biphenyltetracarboxylic dianhydride, 6,6'-bis(trifluoromethyl)-3,3',4,4'-biphenyltetracarboxylic dianhydride, 2,2',5,5'-tetrakis(trifluoromethyl)-3,3',4,4'-biphenyltetracarboxylic dianhydride, 2,2',6,6'-tetrakis(trifluoromethyl)-3,3',4,4'-biphenyltetracarboxylic dianhydride, 5,5',6,6'-tetrakis(trifluoromethyl)-3,3',4,4'-biphenyltetracarboxylic dianhydride,2,2',5,5',6,6'-hexakis(trifluoromethyl)-3,3',4,4'-biphenyltetracarboxylic dianhydride, 3,3'-difluorooxy-4,4'-diphthalic dianhydride, 5,5-difluorooxy-4,4'-diphthalic dianhydride, 6,6'-difluorooxy-4,4'-diphthalic dianhydride, 3,3',5,5',6,6'-hexafluorooxy-4,4'-diphthalic dianhydride, 3,3'-bis(trifluoromethyl) oxy-4,4'-diphthalic dianhydride, 5,5'-bis(trifluoromethyl)oxy-4,4'-diphthalic dianhydride, 6,6'-bis(trifluoromethyl)oxy-4,4'-diphthalic dianhydride, 3,3',5,5'-tetrakis(trifluoromethyl)oxy-4,4'-diphthalic dianhydride, 3,3',6,6'-tetrakis(trifluoromethyl)oxy-4,4'-diphthalic dianhydride, 5,5',6,6'-tetrakis(trifluoromethyl)oxy-4,4'-diphthalic dianhydride, 3,3',5,5',6,6'-hexakis(trifluoromethyl)oxy-4,4'-diphthalic dianhydride, 3,3'-difluorosulfonyl-4,4'-diphthalic dianhydride, 5,5'-difluorosulfonyl-4,4'-diphthalic dianhydride, 6,6'-difluorosulfonyl-4,4'-diphthalic dianhydride, 3,3',5,5',6,6'-hexafluorosulfonyl-4,4'-diphthalic dianhydride, 3,3'-bis(trifluoromethyl)sulfonyl-4,4'-diphthalic dianhydride, 5,5'-bis(trifluoromethyl)sulfonyl-4,4'-diphthalic dianhydride, 6,6'-bis(trifluoromethyl)sulfonyl-4,4'-diphthalic dianhydride, 3,3',5,5'-tetrakis(trifluoromethyl)sulfonyl-4,4'-diphthalic dianhydride, 3,3',6,6'-tetrakis(trifluoromethyl)sulfonyl-4,4'-diphthalic dianhydride, 5,5',6,6'-tetetrakis(trifluoromethyl)sulfonyl-4,4'-diphthalic dianhydride, 3,3',5,5',6,6'-hexakis(trifluoromethyl)sulfonyl-4,4'-diphthalic dianhydride, 3,3'-difluoro-2,2-perfluoropropylidene-4,4'-diphthalic dianhydride, 5,5'-difluoro-2,2-perfluoropropylidene-4,4'-diphthalic dianhydride, 6,6'-difluoro-2,2-perfluoropropylidene-4,4'-diphthalic dianhydride, 3,3',5,5',6,6'-hexafluoro-2,2-perfluoropropylidene-4,4'-diphthalic dianhydride,3,3'-bis(trifluoromethyl)-2,2-perfluoropropylidene-4,4'-diphthal dianhydride, 5,5'-bis(trifluoromethyl)-2,2-perfluoropropylidene-4,4'-diphthalic dianhydride, 6,6'-difluoro-2,2-perfluoropropylidene-4,4'-diphthalic dianhydride, 3,3',5,5'-tetrakis(trifluoromethyl)-2,2-perfluoropropylidene-4,4'-diphthalic dianhydride, 3,3',6,6'-tetrakis(trifluoromethyl)-2,2-perfluoropropylidene-4,4'-diphthalic dianhydride, 5,5',6,6'-tetrakis(trifluoromethyl)-2,2-perfluoropropylidene-4,4'-diphthalic dianhydride, 3,3',5,5',6,6'-hexakis(trifluoromethyl)-2,2-perfluoropropylidene-4,4'-diphthalic dianhydride, 9-phenyl-9-(trifluoromethyl)xanthene-2,3,6,7-tetracarboxylic dianhydride, 9,9-bis(trifluoromethyl)xanthene-2,3,6,7-tetracarboxylic dianhydride, bicyclo[2,2,2]octo-7-ene-2,3,5,6-tetracarboxylic dianhydride, 4,4'-(4,4'-isopropylidenediphenoxy)diphthalic anhydride, 9,9-bis[4-(3,4-dicarboxy)phenyl]fluorene dianhydride, and 9,9-bis[4-(2,3-dicarboxy)phenyl]fluorene dianhydride. One of these may be used alone, or two or more thereof may be used as a mixture.

Examples of the alicyclic tetracarboxylic dianhydride component include 1,2,3,4-cyclobutanetetracarboxylic dianhydride, cyclopentanetetracarboxylic dianhydride, cyclohexane-1,2,3,4-tetracarboxylic dianhydride, cyclohexane-l,2,4,5-tetracarboxylic dianhydride, 3,3',4,4'-bicyclohexyltetracarboxylic dianhydride, carbonyl-4,4'-bis(cyclohexane-1,2-dicarboxylic) dianhydride, methylene-4,4'-bis(cyclohexane-1,2-dicarboxylic) dianhydride, 1,2-ethylene-4,4'-bis(cyclohexane-1,2-dicarboxylic) dianhydride, 1, 1-ethynilidene-4,4'-bis(cyclohexane-1,2-dicarboxylic) dianhydride, 2,2-propylidene-4,4'-bis(cyclohexane-1,2-dicarboxylic) dianhydride, 1,1,1,3,3,3-hexafluoro-2,2-propylidene-4,4'-bis(cyclohexane-1,2-dicarboxylic) dianhydride, oxy-4,4'-bis(cyclohexane-1,2-dicarboxylic) dianhydride, thio-4,4'-bis(cyclohexane-1,2-dicarboxylic) dianhydride, and sulfonyl-4,4'-bis(cyclohexane-1,2-dicarboxylic) dianhydride. One of these may be used alone, or two or more thereof may be used as a mixture.

Examples of the aliphatic tetracarboxylic dianhydride component include 1,2,3,4-butanetetracarboxylic dianhydride and 1, 1,2,2-ethanetetracarboxylic dianhydride. One of these may be used alone, or two or more thereof may be used as a mixture.

From the viewpoint of heat resistance of the polyamic acid-based compound and the like (particularly the polyimide-based compound), the tetracarboxylic dianhydride component of the polyamic acid-based compound and the like preferably contains an aromatic tetracarboxylic dianhydride component.

From the viewpoint of further improving the heat resistance of the polyamic acid-based compound and the like (particularly the polyimide-based compound), the tetracarboxylic dianhydride component of the polyamic acid-based compound and the like preferably contains only an aromatic tetracarboxylic dianhydride component.

From the viewpoint of solubility of the polyamic acid-based compound and the like (particularly the polyimide-based compound), among the above-mentioned tetracarboxylic dianhydride components, it is preferable to use an aromatic tetracarboxylic dianhydride component or an alicyclic tetracarboxylic dianhydride component having an ether group, a thioether group, or a fluorine atom (or a fluorine atom-containing substituent), as the tetracarboxylic dianhydride component of the polyamic acid-based compound and the like.

From the viewpoint of further improving the solubility of the polyamic acid-based compound and the like (particularly the polyimide-based compound), among the above-mentioned tetracarboxylic dianhydride components, it is preferable to use an aromatic tetracarboxylic dianhydride component or an alicyclic tetracarboxylic dianhydride component having a fluorine atom (or a fluorine atom-containing substituent), as the tetracarboxylic dianhydride component of the polyamic acid-based compound and the like.

From the viewpoint of non-coloring properties of the polyamic acid-based compound and the like (particularly the polyimide-based compound), among the above-mentioned tetracarboxylic dianhydride components, the tetracarboxylic dianhydride component of the polyamic acid-based compound and the like preferably contains an aromatic tetracarboxylic dianhydride, an alicyclic tetracarboxylic dianhydride, or an aliphatic tetracarboxylic dianhydride having a fluorine atom (or a fluorine atom-containing substituent).

From the viewpoint of further improving the non-coloring properties of the polyamic acid-based compound and the like (particularly the polyimide-based compound), among the above-mentioned tetracarboxylic dianhydride components, the tetracarboxylic dianhydride component of the polyamic acid-based compound and the like preferably contains an aromatic tetracarboxylic dianhydride having a fluorine atom (or a fluorine atom-containing substituent).

From the viewpoint of versatility, among the above-mentioned tetracarboxylic dianhydride components, the tetracarboxylic dianhydride component of the polyamic acid-based compound and the like preferably contains one or more compounds selected from the group (hereinafter referred to as Group H1) consisting of pyromellitic dianhydride, 3,3',4,4'-biphenyltetracarboxylic dianhydride, 2,3,3',4'-biphenyltetracarboxylic dianhydride, 3,3',4,4'-benzophenone tetracarboxylic dianhydride, 4,4'-oxydiphthalic anhydride, 4,4'-(4,4'-isopropylidenediphenoxy)diphthalic anhydride, 3,3',4,4'-diphenylsulfone tetracarboxylic dianhydride, 4,4'-(hexafluoroisopropylidene)diphthalic anhydride, 1,2,3,4-cyclobutanetetracarboxylic dianhydride, cyclohexane-1,2,4,5-tetracarboxylic dianhydride, and 1,2,3,4-butanetetracarboxylic dianhydride.

From the viewpoint of further improving the versatility, among the above-mentioned tetracarboxylic dianhydride components, the tetracarboxylic dianhydride component of the polyamic acid-based compound and the like preferably contains only one or more compounds selected from the above-mentioned Group H1.

The diamine component capable of constituting the polyamic acid-based compound includes an aromatic diamine component containing an aromatic ring, an alicyclic diamine component containing an aliphatic ring but not an aromatic ring, and an aliphatic diamine component that does not contain an aromatic ring or an alicyclic ring. The diamine component may contain an ether group and/or a thioether group, and/or one or more hydrogen atoms may be substituted with a halogen atom (e.g., fluorine atom, chlorine atom, and bromine atom). The diamine component may have a side chain.

Examples of the aromatic diamine component include m-xylylenediamine, p-xylylenediamine, benzidine, m-phenylenediamine, o-phenylenediamine, p-phenylenediamine, 2,4-toluenediamine, 2,6-toluenediamine, m-aminobenzylamine, p-aminobenzylamine, bis(3-aminophenyl) sulfide, (3-aminophenyl) (4-aminophenyl) sulfide, bis(4-aminophenyl) sulfide, bis(3-aminophenyl) sulfoxide, (3-aminophenyl) (4-aminophenyl) sulfoxide, bis(3-aminophenyl) sulfone, (3-aminophenyl) (4-aminophenyl) sulfone, bis(4-aminophenyl)sulfone, 3,3'-diaminobenzophenone, 3,4'-diaminobenzophenone, 4,4'-diaminobenzophenone, 3,3'-diaminodiphenylmethane, 3,4'-diaminodiphenylmethane, 4,4'-diaminodiphenylmethane, 4,4'-diaminodiphenyl ether, 3,3'-diaminodiphenyl ether, 3,4'-diaminodiphenyl ether, 4,4'-diaminobenzanilide, 3,3'-dimethyl-3,4'-diaminobiphenyl, 2,2'-dimethyl-3,4'-diaminobiphenyl, 2,2'-bis(trifluoromethyl)-4,4'-diaminobiphenyl, bis[4-(3-aminophenoxy)phenyl]methane,bis[4-(4-aminophenoxy)phenyl]methane, 1,1-bis[4-(3-aminophenoxy)phenyl]ethane, 1,1-bis[4-(4-aminophenoxy)phenyl]ethane, 1,2-bis[4-(3-aminophenoxy)phenyl]ethane, 1,2-bis[4-(4-aminophenoxy)phenyl]ethane, 2,2-bis[4-(3-aminophenoxy)phenyl]propane, 2,2-bis[4-(3-aminophenoxy)phenyl]butane, 2,2-bis[3-(3-aminophenoxy)phenyl]-1,1,1,3,3,3-hexafluoropropane, 2,2-bis[4-(4-aminophenoxy)phenyl]-1,1,1,3,3,3-hexafluoropropane, 1,3-bis(3-aminophenoxy)benzene, 1,4-bis(3-aminophenoxy)benzene, 1,3-bis(4-aminophenoxy)benzene, 1,4-bis(4-aminophenoxy)benzene, 2,2-bis[4-(4-aminophenoxy)phenyl]propane, bis(4-aminophenyl)sulfone, 9,9-bis(4-aminophenyl) fluorene, meta-xylenediamine, 1,4'-bis(4-aminophenoxy)benzene, 4,4'-bis(3-aminophenoxy)biphenyl, 4,4'-bis(4-aminophenoxy)biphenyl, bis[4-(3-aminophenoxy)phenyl]ketone, bis[4-(4-aminophenoxy)phenyl]ketone, bis[4-(3-aminophenoxy)phenyl]sulfide, bis[4-(4-aminophenoxy)phenyl]sulfide, bis[4-(3-aminophenoxy)phenyl]sulfoxide, bis[4-(aminophenoxy)phenyl]sulfoxide, bis[4-(3-miinophenoxy)phenyl]sulfone, bis[4-(4-aminophenoxy)phenyl]sulfone, bis[4-(3-aminophenoxy)phenyl]ether, bis[4-(4-aminophenoxy)phenyl]ether, 1,4-bis[4-(3-aminophenoxy)benzoyl]benzene, 1,3-bis[4-(3-aminophenoxy)benzoyl]benzene, 4,4'-bis[3-(4-aminophenoxy)benzoyl]diphenyl ether, 4,4'-bis[3-(3-aminophenoxy)benzoyl]diphenyl ether, 4,4'-bis[4-(4-amino-α,α-dimethylbenzyl)phenoxy]benzophenone, 4,4'-bis[4-(4-amino-α,α-dimethylbenzyl)phenoxy]diphenylsulfone, bis[4-{4-(4-aminophenoxy)phenoxy}phenyl]sulfone, 1,4-bis[4-(4-aminophenoxy)-α,α-dimethylbenzyl]benzene, 1,3-bis[4-(4-aminophenoxy)-α,α-dimethylbenzyl]benzene, 1,3-bis(3-(4-aminophenoxy)phenoxy)benzene, 1,3-bis(3-(2-aminophenoxy)phenoxy)benzene, 1,3-bis(4-(2-aminophenoxy)phenoxy)benzene, 1,3-bis(2-(2-aminophenoxy)phenoxy)benzene, 1,3-bis(2-(3-aminophenoxy)phenoxy)benzene, 1,3-bis(2-(4-aminophenoxy)phenoxy)benzene, 1,4-bis(3-(3-aminophenoxy)phenoxy)benzene, 1,4-bis(3-(4-aminophenoxy)phenoxy)benzene, 1,4-bis(3-(2-aminophenoxy)phenoxy)benzene, 1,4-bis(4-(2-aminophenoxy)phenoxy)benzene, 1,4-bis(2-(2-aminophenoxy)phenoxy)benzene, 1,4-bis(2-(3-aminophenoxy)phenoxy)benzene, 1,4-bis(2-(4-aminophenoxy)phenoxy)benzene, 1,2-bis(3-(3-aminophenoxy)phenoxy)benzene, 1,2-bis(3-(4-aminophenoxy)phenoxy)benzene, 1,2-bis(3-(2-aminophenoxy)phenoxy)benzene, 1,2-bis(4-(4-aminophenoxy)phenoxy)benzene, 1,2-bis(4-(3-aminophenoxy)phenoxy)benzene, 1,2-bis(4-(2-aminophenoxy)phenoxy)benzene, 1,2-bis(2-(2-aminophenoxy)phenoxy)benzene, 1,2-bis(2-(3-aminophenoxy)phenoxy)benzene, 1,2-bis(2-(4-aminophenoxy)phenoxy)benzene, 1,3-bis(3-(3-aminophenoxy)phenoxy)-2-methylbenzene, 1,3-bis(3-(4-aminophenoxy)phenoxy)-4-methylbenzene, 1,3-bis(4-(3-ammophenoxy)phenoxy)-2-ethylbenzene, 1,3-bis(3-(2-aminophenoxy)phenoxy)phenoxy)-5-sec-butylbenzene, 1,3-bis(4-(3-aminophenoxy)phenoxy)-2,5-dimethylbenzene, 1,3-bis(4-(2-amino-6-methylphenoxy)phenoxy)benzene, 1,3-bis(2-(2-amino-6-ethylphenoxy)phenoxy)benzene, 1,3-bis(2-(3-aminophenoxy)-4-methylphenoxy)benzene, 1,3-bis(2-(4-aminophenoxy)-4-tert-butylphenoxy)benzene, 1,4-bis(3-(3-aminophenoxy)phenoxy)-2,5-di-tert-butylbenzene, 1,4-bis(3-(4-aminophenoxy)phenoxy)-2,3-dimethylbenzene, 1,4-bis(3-(2-amino-3-propylphenoxy)phenoxy)benzene, 1,2-bis(3-(3-aminophenoxy)phenoxy)-4-methylbenzene, 1,2-bis(3-(4-aminophenoxy)phenoxy)-3-n-butylbenzene, 1,2-bis(3-(2-amino-3-propylphenoxy)phenoxy)benzene, bis(3-aminopropyl)tetramethyldisiloxane, bis(3-aminophenoxymethyl)tetramethyldisiloxane, bis(3-aminophenoxymethyl)tetramethyldisiloxane, α,ω-bis(3-aminopropyl)polymethylphenylsiloxane, α,ω-bis(3-aminopropyl)poly(dimethylsiloxane-diphenylsiloxane)copolymer, and analogs of the above-mentioned diamines. One of these may be used alone, or two or more thereof may be used as a mixture.

Examples of the alicyclic diamine component include trans-1,4-cyclohexanediamine, cis-1,4-cyclohexanediamine, 4,4'-methylenebis(cyclohexylamine), 1,4-bis(aminomethyl)cyclohexane, and isophoronediamine. One of these may be used alone, or two or more thereof may be used as a mixture.

Examples of the aliphatic diamine component include hexamethylenediamine, heptamethylenediamine, octamethylenediamine, nonamethylenediamine, decamethylenediamine, 1,10-diaminodecane, 1,12-diaminododecane, 1,10-diamino-1,10-dimethyldecane, bis(10-aminodecamethylene)tetramethyldisiloxane, α,ω-bisaminopolydimethylsiloxane, α,ω-bis(3-aminopropyl)polydimethylsiloxane, 1,3-bis(3-aminopropyl)tetramethyldisiloxane, 1,3-bis(3-aminopropyl)-1,1,3,3-tetramethyldisiloxane, bis(10-aminodecamethylene)tetramethyldisiloxane, and dimer diamine. One of these may be used alone, or two or more thereof may be used as a mixture. Dimer diamine is a compound obtained by polymerizing unsaturated fatty acids such as oleic acid and linoleic acid to obtain dimer acid, and then reducing and aminating (reductively aminating) the dimer acid. Depending on the purpose of use, the degree of unsaturation may be lowered by a hydrogenation reaction. As the dimer diamine, commercially available products such as "PRIAMINE 1074, PRIAMINE 1075" (trade names, made by Croda Japan KK) and "Versamine 551, Versamine 552" (trade names, made by Cognis Japan Ltd.) can be used.

From the viewpoint of heat resistance of the polyamic acid-based compound and the like (particularly the polyimide-based compound), the diamine component of the polyamic acid-based compound preferably contains an aromatic diamine component and/or an alicyclic diamine component, more preferably an aromatic diamine component.

From the viewpoint of further improving the heat resistance of the polyamic acid-based compound and the like (particularly the polyimide-based compound), the diamine component of the polyamic acid-based compound preferably contains only an aromatic diamine component and/or an alicyclic diamine component, more preferably only an aromatic diamine component.

From the viewpoint of solubility of the polyamic acid-based compound and the like (particularly the polyimide-based compound), among the above-mentioned diamine components, it is preferable to use a diamine component having an ether group, a thioether group, a sulfonyl group, a sulfonic acid group, a methyl group, a methylene group, a phenyl group, a fluorene structure, a halogen atom (or a halogen atom-containing substituent), or a siloxane bond, as the diamine component of the polyamic acid-based compound.

From the viewpoint of further improving the solubility of the polyamic acid-based compound and the like (particularly the polyimide-based compound), among the above-mentioned diamine components, it is preferable to use only a diamine component having an ether group, a thioether group, a sulfonyl group, a sulfonic acid group, a methyl group, a methylene group, a phenyl group, a fluorene structure, a halogen atom (or a halogen atom-containing substituent), or a siloxane bond, as the diamine component of the polyamic acid-based compound.

From the viewpoint of heat resistance and non-coloring properties of the polyamic acid-based compound and the like (particularly the polyimide-based compound), among the above-mentioned diamine components, the diamine component of the polyamic acid-based compound preferably contains an aromatic diamine component, an alicyclic diamine component or an aliphatic diamine component having a fluorine atom (or a fluorine atom-containing substituent), more preferably an aromatic diamine component or an alicyclic diamine component having the fluorine atom (or the fluorine atom-containing substituent).

From the viewpoint of further improving the heat resistance and non-coloring properties of the polyamic acid-based compound and the like (particularly the polyimide-based compound), among the above-mentioned diamine components, the diamine component of the polyamic acid-based compound preferably contains an alicyclic diamine component and/or an aliphatic diamine component, more preferably only an alicyclic diamine component and/or an aliphatic diamine component.

From the viewpoint of versatility, among the above-mentioned diamine components, the diamine component of the polyamic acid-based compound preferably contains one or more compounds selected from the group (hereinafter referred to as Group H2) consisting of 4,4'-diaminodiphenyl ether, p-phenylenediamine, m-phenylenediamine, 3,4'-diaminodiphenyl ether, 4,4'-diaminodiphenylmethane, 2,2'-bis(trifluoromethyl)-4,4'-diaminobiphenyl, 2,2-bis[4-(4-aminophenoxy)phenyl]-1,1,1,3,3,3-hexafluoropropane, 1,4-bis(4-aminophenoxy)benzene, 1,3-bis(4-aminophenoxy)benzene, 4,4'-bis(4-aminophenoxy)biphenyl, 4,4'-bis(3-aminophenoxy)biphenyl, 2,2-bis[4-(4-aminophenoxy)phenyl]propane, bis(4-aminophenyl)sulfone, bis[4-(4-aminophenoxy)phenyl]sulfone, 9,9-bis(4-aminophenyl)fluorene, trans-1,4-cyclohexanediamine, cis-1,4-cyclohexanediamine, 4,4'-methylenebis(cyclohexylamine), 1,10-diaminodecane, 1,12-diaminododecane, m-xylylenediamine, p-xylylenediamine, 1,4-bis(aminomethyl)cyclohexane, and dimer diamine.

From the viewpoint of further improving the versatility, among the above-mentioned diamine components, the diamine component of the polyamic acid-based compound preferably contains only one or more compounds selected from the above-mentioned Group H2.

The diisocyanate component capable of constituting the polyimide-based compound includes an aromatic diisocyanate component containing an aromatic ring, an alicyclic diisocyanate component containing an aliphatic ring but not an aromatic ring, and an aliphatic diisocyanate component that does not contain an aromatic ring or an alicyclic ring. The diisocyanate component may contain an ether group and/or a thioether group, and/or one or more hydrogen atoms may be substituted with a halogen atom (e.g., fluorine atom, chlorine atom, and bromine atom).

Examples of the aromatic diisocyanate component include 4,4'-diphenylmethane diisocyanate, 3,3'-diphenylmethane diisocyanate, m-xylene diisocyanate, tetramethylxylene diisocyanate, 2,6-tolylene diisocyanate, 2,4-tolylene diisocyanate, 1,4-phenylene diisocyanate, and adducts, biurets, and isocyanurates thereof. One of these may be used alone, or two or more thereof may be used as a mixture.

Examples of the alicyclic diisocyanate component include 4,4'-dicyclohexylmethane diisocyanate, hydrogenated xylylene diisocyanate, isophorone diisocyanate, norbornene diisocyanate, and adducts, biurets, and isocyanurates thereof. One of these may be used alone, or two or more thereof may be used as a mixture.

Examples of the aliphatic diisocyanate component include hexamethylene diisocyanate, trimethylhexamethylene diisocyanate, 1,5-pentamethylene diisocyanate, dimer acid diisocyanate, and adducts, biurets, and isocyanurates thereof. One of these may be used alone, or two or more thereof may be used as a mixture.

From the viewpoint of heat resistance of the polyimide-based compound, the diisocyanate component of the polyimide-based compound preferably contains an aromatic diisocyanate component and/or an alicyclic diisocyanate component, more preferably an aromatic diisocyanate component.

From the viewpoint of further improving the heat resistance of the polyimide-based compound, the diisocyanate component of the polyimide-based compound preferably contains only an aromatic diisocyanate component and/or an alicyclic diisocyanate component, and more preferably only an aromatic diisocyanate component.

From the viewpoint of solubility of the polyimide-based compound, among the above-mentioned diisocyanate components, it is preferable to use an alicyclic diisocyanate component and/or an aliphatic diisocyanate component as the diisocyanate component of the polyimide-based compound.

From the viewpoint of further improving the solubility of the polyimide-based compound, among the above-mentioned diisocyanate components, it is preferable to use only an alicyclic diisocyanate component and/or an aliphatic diisocyanate component as the diisocyanate component of the polyimide-based compound.

From the viewpoint of non-coloring properties of the polyimide-based compound, among the above-mentioned diisocyanate components, the diisocyanate component of the polyimide-based compound preferably contains an alicyclic diisocyanate component and/or an aliphatic diisocyanate component, more preferably an alicyclic diisocyanate component or an aliphatic diisocyanate component.

From the viewpoint of further improving the non-coloring properties of the polyimide-based compound, among the above diisocyanate components, the diisocyanate component of the polyimide-based compound preferably contains only an alicyclic diisocyanate component and/or an aliphatic diisocyanate component, more preferably only an alicyclic diisocyanate component or an aliphatic diisocyanate component.

From the viewpoint of versatility, among the above diisocyanate components, the diisocyanate component of the polyimide-based compound preferably contains one or more compounds selected from the group (hereinafter referred to as Group H3) consisting of 4,4'-diphenylmethane diisocyanate, 2,6-tolylene diisocyanate, 2,4-tolylene diisocyanate, 1,5-diisocyanatonaphthalene, 4,4'-dicyclohexylmethane diisocyanate, isophorone diisocyanate, hexamethylene diisocyanate, trimethylhexamethylene diisocyanate, 1,5-pentamethylene diisocyanate, and adducts, biurets, and isocyanurates thereof.

From the viewpoint of further improving the versatility, among the above-mentioned diisocyanate components, the diisocyanate component of the polyimide-based compound preferably contains only one or more compounds selected from the above-mentioned Group H3.

In the production of the polyamic acid-based compound, the tetracarboxylic dianhydride component and the diamine component are usually used in approximately equal molar amounts. Specifically, the diamine component is used in an amount of usually 0.8 to 1.2 times the molar amount of the tetracarboxylic dianhydride component, particularly 0.9 to 1.1 times, and preferably 0.95 to 1.05 times.

In the production of the polyimide-based compound, the tetracarboxylic dianhydride component and the diisocyanate component are usually used in approximately equal molar amounts. Specifically, the diisocyanate component is used in an amount of usually 0.8 to 1.2 times the molar amount of the tetracarboxylic dianhydride component, particularly 0.9 to 1.1 times, and preferably 0.95 to 1.05 times.

(Polyamide-based compound)

A polyamide-based compound can be produced by performing a reaction between functional groups by a mechanochemical effect, using a dicarboxylic acid component or its acid halide component and a diamine component or a diisocyanate component as the raw material compounds. Here, the reaction between functional groups corresponds to the reaction (C) described above.

More specifically, from the viewpoint of further improving the reaction rate, it is preferable to produce a polyamide-based compound by using a combination of an acid halide component of a dicarboxylic acid component and a diamine component, or a combination of a dicarboxylic acid component and a diisocyanate component.

The dicarboxylic acid component capable of constituting the polyamide-based compound includes an aromatic dicarboxylic acid component containing an aromatic ring, an alicyclic dicarboxylic acid component containing an aliphatic ring but not an aromatic ring, and an aliphatic dicarboxylic acid component that does not contain an aromatic ring or an alicyclic ring. The dicarboxylic acid component may contain an ether group and/or a thioether group, and/or one or more hydrogen atoms may be substituted with a halogen atom (e.g., fluorine atom, chlorine atom, and bromine atom). The acid halide component of the dicarboxylic acid component is a compound in which the OH group of the carboxyl group is substituted with a halogen atom in the dicarboxylic acid component.

Examples of the aromatic dicarboxylic acid component include terephthalic acid, isophthalic acid, phthalic acid (orthophthalic acid), 2-chloroterephthalic acid, 2-methylterephthalic acid, 5-methylisophthalic acid, 5-sodium sulfoisophthalic acid, hexahydrotelephthalic acid, hexahydroisophthalic acid, 2,6-naphthalenedicarboxylic acid, 1,5-naphthalenedicarboxylic acid, 4,4'-biphenyldicarboxylic acid, diphenoxybutane-4,4'-dicarboxylic acid, diphenylethane-4,4'-dicarboxylic acid, phenyl ether-2,2'-dicarboxylic acid, diphenyl ether-2,3'-dicarboxylic acid, diphenyl ether-2,4'-dicarboxylic acid, diphenyl ether-3,3'-dicarboxylic acid, diphenyl ether-3,4'-dicarboxylic acid, diphenyl ether-4,4'-dicarboxylic acid, diphenoxy ether-3,3'-dicarboxylic acid, and diphenylethane-3,3'-dicarboxylic acid. One of these may be used alone, or two or more thereof may be used as a mixture.

Examples of the alicyclic dicarboxylic acid component include 1,4-cyclohexanedicarboxylic acid, 1,3-cyclohexanedicarboxylic acid, 1,2-cyclohexanedicarboxylic acid, and 2,5-norbomenedicarboxylic acid. One of these may be used alone, or two or more thereof may be used as a mixture.

Examples of the aliphatic dicarboxylic acid component include oxalic acid, malonic acid, succinic acid, glutaric acid, succinic acid, adipic acid, pimelic acid, suberic acid, azelaic acid, sebacic acid, dodecanedioic acid, dimer acid, and hydrogenated dimer acid, maleic anhydride, maleic acid, fumaric acid, itaconic acid, citraconic acid, and mesaconic acid. One of these may be used alone, or two or more thereof may be used as a mixture.

From the viewpoint of heat resistance of the polyamide-based compound, the dicarboxylic acid component of the polyamide-based compound preferably contains an aromatic dicarboxylic acid component and/or an alicyclic dicarboxylic acid component, more preferably an aromatic dicarboxylic acid component.

From the viewpoint of further improving the heat resistance of the polyamide-based compound, the dicarboxylic acid component of the polyamide-based compound preferably contains only an aromatic dicarboxylic acid component and/or an alicyclic dicarboxylic acid component, more preferably only an aromatic dicarboxylic acid component.

From the viewpoint of versatility, among the above-mentioned dicarboxylic acid components, the dicarboxylic acid component of the polyamide-based compound preferably contains one or more compounds selected from the group (hereinafter referred to as Group H4) consisting of terephthalic acid, isophthalic acid, 1,4-cyclohexanedicarboxylic acid, and 1,3-cyclohexanedicarboxylic acid.

From the viewpoint of further improving the versatility, among the above-mentioned dicarboxylic acid components, the dicarboxylic acid component of the polyamide-based compound preferably contains only one or more compounds selected from the above-mentioned Group H4.

The diamine component capable of constituting the polyamide-based compound is a diamine component similar to the diamine component capable of constituting the polyamic acid-based compound and the like, and specifically, includes an aromatic diamine component, an alicyclic diamine component, and an aliphatic diamine component that are similar to the diamine component capable of constituting the polyamic acid-based compound and the like.

From the viewpoint of heat resistance of the polyamide-based compound, the diamine component of the polyamide-based compound preferably contains an aromatic diamine component and/or an alicyclic diamine component, more preferably an aromatic diamine component.

From the viewpoint of further improving the heat resistance of the polyamide-based compound, the diamine component of the polyamide-based compound preferably contains only an aromatic diamine component and/or an alicyclic diamine component, more preferably only an aromatic diamine component.

From the viewpoint of solubility of the polyamide-based compound, among the above-mentioned diamine components, it is preferable to use a diamine component having an ether group, a thioether group, a sulfonyl group, a sulfonic acid group, a methyl group, a methylene group, a phenyl group, a fluorene structure, a halogen atom (or a halogen atom-containing substituent), or a siloxane bond, as the diamine component of the polyamide-based compound.

From the viewpoint of further improving the solubility of the polyamide-based compound, among the above-mentioned diamine components, it is preferable to use only a diamine component having an ether group, a thioether group, a sulfonyl group, a sulfonic acid group, a methyl group, a methylene group, a phenyl group, a fluorene structure, a halogen atom (or a halogen atom-containing substituent), or a siloxane bond, as the diamine component of the polyamide-based compound.

From the viewpoint of non-coloring properties of the polyamide-based compound, among the above diamine components, the diamine component of the polyamide-based compound preferably contains an aliphatic diamine component and/or an alicyclic diamine component.

From the viewpoint of further improving the non-coloring properties of the polyamide-based compound, among the above-mentioned diamine components, the diamine component of the polyamide-based compound preferably contains only an aliphatic diamine component and/or an alicyclic diamine component.

From the viewpoint of versatility, among the above-mentioned diamine components, the diamine component of the polyamide-based compound preferably contains one or more compounds selected from the group (hereinafter referred to as Group H5) consisting of 4,4'-diaminodiphenyl ether, p-phenylenediamine, m-phenylenediamine, 3,4'-diaminodiphenyl ether, 4,4'-diaminodiphenylmethane, 2,2'-bis(trifluoromethyl)-4,4'-diaminobiphenyl, 2,2-bis[4-(4-aminophenoxy)phenyl]-1,1,1,3,3,3-hexafluoropropane, 1,4-bis(4-aminophenoxy)benzene, 1,3-bis(4-aminophenoxy)benzene, 4,4'-bis(4-aminophenoxy)biphenyl, 4,4'-bis(3-aminophenoxy)biphenyl, 2,2-bis[4-(4-aminophenoxy)phenyl]propane, bis(4-aminophenyl)sulfone, bis[4-(4-aminophenoxy)phenyl]sulfone, 9,9-bis(4-aminophenyl)fluorene, trans-1,4-cyclohexanediamine, cis-1,4-cyclohexanediamine, 4,4'-methylenebis(cyclohexylamine), 1,10-diaminodecane, 1,12-diaminododecane, m-xylylenediamine, p-xylylenediamine, 1,4-bis(aminomethyl)cyclohexane, and dimer diamine.

From the viewpoint of further improving the versatility, among the above-mentioned diamine components, the diamine component of the polyamide-based compound preferably contains only one or more compounds selected from the above-mentioned Group H5.

The diisocyanate component capable of constituting the polyamide-based compound is a diisocyanate component similar to the diisocyanate component capable of constituting the polyimide-based compound and the like, and specifically, includes an aromatic diisocyanate component, an alicyclic diisocyanate component, and an aliphatic diisocyanate component that are similar to the diisocyanate component capable of constituting the polyimide-based compound and the like.

From the viewpoint of heat resistance of the polyamide-based compound, the diisocyanate component of the polyamide-based compound preferably contains an aromatic diisocyanate component and/or an alicyclic diisocyanate component, more preferably an aromatic diisocyanate component.

From the viewpoint of further improving the heat resistance of the polyamide-based compound, the diisocyanate component of the polyamide-based compound preferably contains only an aromatic diisocyanate component and/or an alicyclic diisocyanate component, more preferably only an aromatic diisocyanate component.

From the viewpoint of versatility, among the above-mentioned diisocyanate components, the diisocyanate component of the polyamide-based compound preferably contains one or more compounds selected from the group (hereinafter referred to as Group H6) consisting of 4,4'-diphenylmethane diisocyanate, 2,6-tolylene diisocyanate, 2,4-tolylene diisocyanate, 1,5-diisocyanatonaphthalene, 4,4'-dicyclohexylmethane diisocyanate, isophorone diisocyanate, hexamethylene diisocyanate, trimethylhexamethylene diisocyanate, 1,5-pentamethylene diisocyanate, and adducts, biurets, and isocyanurates thereof.

From the viewpoint of further improving the versatility, among the above-mentioned diisocyanate components, the diisocyanate component of the polyamide-based compound preferably contains only one or more compounds selected from the above-mentioned Group H6.

In the production of the polyamide-based compound, the dicarboxylic acid component or its acid halide component and the diamine component or the diisocyanate component are usually used in approximately equal molar amounts. Specifically, the diamine component or the diisocyanate component is used in an amount of usually 0.8 to 1.2 times the molar amount of the dicarboxylic acid component or its acid halide component, particularly 0.9 to 1.1 times, and preferably 0.95 to 1.05 times.

(Polyamide-imide-based compound)

A polyamide-imide-based compound can be produced by performing a reaction between functional groups by a mechanochemical effect, using a tricarboxylic acid anhydride component or its acid halide component and a diamine component or a diisocyanate component as the raw material compounds. Here, the reaction between functional groups corresponds to the reactions (B) and (C) described above.

More specifically, from the viewpoint of further improving the reaction rate, it is preferable to produce a polyamide-imide-based compound by using a combination of an acid halide component of a tricarboxylic acid anhydride component and a diamine component, or a combination of a tricarboxylic acid anhydride component and a diisocyanate component.

The tricarboxylic acid anhydride component capable of constituting the polyamide-imide-based compound includes an aromatic tricarboxylic acid anhydride component containing an aromatic ring, an alicyclic tricarboxylic acid anhydride component containing an aliphatic ring but not an aromatic ring, and an aliphatic tricarboxylic acid anhydride component that does not contain an aromatic ring or an alicyclic ring. The tricarboxylic acid anhydride component may contain an ether group and/or a thioether group, and/or one or more hydrogen atoms may be substituted with a halogen atom (e.g., fluorine atom, chlorine atom, and bromine atom). The acid halide component of the tricarboxylic acid anhydride component is a compound in which the OH group of the carboxyl group is substituted with a halogen atom in the tricarboxylic acid anhydride component.

Examples of the aromatic tricarboxylic acid anhydride component include trimellitic anhydride, hemimellitic anhydride, 1,2,4-naphthalene tricarboxylic anhydride, 1,4,5-naphthalene tricarboxylic anhydride, 2,3,6-naphthalene tricarboxylic anhydride, 1,2,8-naphthalene tricarboxylic anhydride, 3,4,4'-benzophenone tricarboxylic anhydride, 3,4,4'-biphenyl ether tricarboxylic anhydride, 3,4,4'-biphenyltricarboxylic anhydride, 2,3,2'-biphenyltricarboxylic anhydride, 3,4,4'-biphenyl methane tricarboxylic anhydride, and 3,4,4'-biphenyl sulfone tricarboxylic anhydride. One of these may be used alone, or two or more thereof may be used as a mixture.

Examples of the alicyclic tricarboxylic acid anhydride component include 1,2,4-cyclopentane tricarboxylic anhydride, 1,2,3-cyclohexanetricarboxylic anhydride, 1,2,4-cyclohexanetricarboxylic anhydride, and 1,3,5-cyclohexanetricarboxylic anhydride. One of these may be used alone, or two or more thereof may be used as a mixture.

Examples of the aliphatic tricarboxylic acid anhydride component include 3-carboxymethyl glutaric acid anhydride, 1,2,4-butane tricarboxylic acid-1,2-anhydride, and cis-propene-1,2,3-tricarboxylic acid-1,2-anhydride. One of these may be used alone, or two or more thereof may be used as a mixture.

From the viewpoint of heat resistance of the polyamide-imide-based compound, the tricarboxylic acid anhydride component of the polyamide-imide-based compound preferably contains an aromatic tricarboxylic acid anhydride component and/or an alicyclic tricarboxylic acid anhydride component, more preferably an aromatic tricarboxylic acid anhydride component.

From the viewpoint of further improving the heat resistance of the polyamide-imide-based compound, the tricarboxylic acid anhydride component of the polyamide-imide-based compound preferably contains only an aromatic tricarboxylic acid anhydride component and/or an alicyclic tricarboxylic acid anhydride component, more preferably only an aromatic tricarboxylic acid anhydride component.

From the viewpoint of solubility and non-coloring properties of the polyamide-imide-based compound, among the above-mentioned tricarboxylic acid anhydride components, it is preferable to use an alicyclic tricarboxylic acid anhydride component and/or an aliphatic tricarboxylic acid anhydride component as the tricarboxylic acid anhydride component of the polyamide-imide-based compound.

From the viewpoint of further improving the solubility and the non-coloring properties of the polyamide-imide-based compound, among the above-mentioned tricarboxylic acid anhydride components, it is preferable to use only an alicyclic tricarboxylic acid anhydride component and/or an aliphatic tricarboxylic acid anhydride component.

From the viewpoint of versatility, among the above-mentioned tricarboxylic acid anhydride components, the tricarboxylic acid anhydride component of the polyamide-imide-based compound preferably contains one or more compounds selected from the group (hereinafter referred to as Group H7) consisting of trimellitic anhydride and 1,2,4-cyclohexanetricarboxylic anhydride.

From the viewpoint of further improving the versatility, among the above-mentioned tricarboxylic acid anhydride components, the tricarboxylic acid anhydride component of the polyamide-imide-based compound preferably contains only one or more compounds selected from the above-mentioned Group H7.

The diamine component capable of constituting the polyamide-imide-based compound is a diamine component similar to the diamine component capable of constituting the polyamic acid-based compound and the like, and specifically, includes an aromatic diamine component, an alicyclic diamine component, and an aliphatic diamine component that are similar to the diamine component capable of constituting the polyamic acid-based compound and the like.

From the viewpoint of heat resistance of the polyamide-imide-based compound, the diamine component of the polyamide-imide-based compound preferably contains an aromatic diamine component and/or an alicyclic diamine component, more preferably an aromatic diamine component.

From the viewpoint of further improving the heat resistance of the polyamide-imide-based compound, the diamine component of the polyamide-imide-based compound preferably contains only an aromatic diamine component and/or an alicyclic diamine component, more preferably only an aromatic diamine component.

From the viewpoint of solubility of the polyamide-imide-based compound, among the above-mentioned diamine components, it is preferable to use a diamine component having an ether group, a thioether group, a sulfonyl group, a sulfonic acid group, a methyl group, a methylene group, a phenyl group, a fluorene structure, a halogen atom (or a halogen atom-containing substituent), or a siloxane bond, as the diamine component of the polyamide-imide-based compound.

From the viewpoint of further improving the solubility of the polyamide-imide-based compound, among the above-mentioned diamine components, it is preferable to use only a diamine component having an ether group, a thioether group, a sulfonyl group, a sulfonic acid group, a methyl group, a methylene group, a phenyl group, a fluorene structure, a halogen atom (or a halogen atom-containing substituent), or a siloxane bond, as the diamine component of the polyamide-imide-based compound.

From the viewpoint of non-coloring properties of the polyamide-imide-based compound, among the above diamine components, the diamine component of the polyamide-imide-based compound preferably contains an aromatic diamine component, an alicyclic diamine component or an aliphatic diamine component having a fluorine atom (or a fluorine atom-containing substituent).

From the viewpoint of further improving the non-coloring properties of the polyamide-imide-based compound, among the above-mentioned diamine components, the diamine component of the polyamide-imide-based compound preferably contains only an aromatic diamine component, an alicyclic diamine component, or an aliphatic diamine component having a fluorine atom (or a fluorine atom-containing substituent).

From the viewpoint of versatility, among the above-mentioned diamine components, the diamine component of the polyamide-imide-based compound preferably contains one or more compounds selected from the group (hereinafter referred to as Group H8) consisting of 4,4'-diaminodiphenyl ether, p-phenylenediamine, m-phenylenediamine, 3,4'-diaminodiphenyl ether, 4,4'-diaminodiphenylmethane, 2,2'-bis(trifluoromethyl)-4,4'-diaminobiphenyl, 2,2-bis[4-(4-aminophenoxy)phenyl]-1,1,1,3,3,3-hexafluoropropane, 1,4-bis(4-aminophenoxy)benzene, 1,3-bis(4-aminophenoxy)benzene, 4,4'-bis(4-aminophenoxy)biphenyl, 4,4'-bis(3-aminophenoxy)biphenyl, 2,2-bis[4-(4-aminophenoxy)phenyl]propane, bis(4-aminophenyl)sulfone, bis[4-(4-aminophenoxy)phenyl]sulfone, 9,9-bis(4-aminophenyl)fluorene, trans-1,4-cyclohexanediamine, cis-1,4-cyclohexanediamine, 4,4'-methylenebis(cyclohexylamine), 1,10-diaminodecane, 1,12-diaminododecane, m-xylylene diamine, p-xylylene diamine, 1,4-bis(aminomethyl)cyclohexane, and dimer diamine.

From the viewpoint of further improving the versatility, among the above-mentioned diamine components, the diamine component of the polyamide-imide-based compound preferably contains only one or more compounds selected from the above-mentioned Group H8.

The diisocyanate component capable of constituting the polyamide-imide-based compound is a diisocyanate component similar to the diisocyanate component capable of constituting the polyimide-based compound and the like, and specifically, includes an aromatic diisocyanate component, an alicyclic diisocyanate component, and an aliphatic diisocyanate component that are similar to the diisocyanate component capable of constituting the polyimide-based compound and the like.

From the viewpoint of heat resistance of the polyamide-imide-based compound, the diisocyanate component of the polyamide-imide-based compound preferably contains an aromatic diisocyanate component and/or an alicyclic diisocyanate component, more preferably an aromatic diisocyanate component.

From the viewpoint of further improving the heat resistance of the polyamide-imide-based compound, the diisocyanate component of the polyamide-imide-based compound preferably contains only an aromatic diisocyanate component and/or an alicyclic diisocyanate component, more preferably only an aromatic diisocyanate component.

From the viewpoint of versatility, among the above-mentioned diisocyanate components, the diisocyanate component of the polyamide-imide-based compound preferably contains one or more compounds selected from the group (hereinafter referred to as Group H9) consisting of 4,4'-diphenylmethane diisocyanate, 2,6-tolylene diisocyanate, 2,4-tolylene diisocyanate, 1,5-diisocyanatonaphthalene, 4,4'-dicyclohexylmethane diisocyanate, isophorone diisocyanate, hexamethylene diisocyanate, trimethylhexamethylene diisocyanate, 1,5-pentamethylene diisocyanate, and adducts, biurets, and isocyanurates thereof.

From the viewpoint of further improving the versatility, among the above-mentioned diisocyanate components, the diisocyanate component of the polyamide-imide-based compound preferably contains only one or more compounds selected from the above-mentioned Group H9.

In the production of the polyamide-imide-based compound, the tricarboxylic acid anhydride component or its acid halide component and the diamine component or the diisocyanate component are usually used in approximately equal molar amounts. Specifically, the diamine component or the diisocyanate component is used in an amount of usually 0.8 to 1.2 times the molar amount of the tricarboxylic acid anhydride component or its acid halide component, particularly 0.9 to 1.1 times, and preferably 0.95 to 1.05 times.

(Polyester-based compound)

A polyester-based compound can be produced by performing a reaction between functional groups by a mechanochemical effect, using a dicarboxylic acid component or its acid halide component and a polyhydroxy component as the raw material compounds. Here, the reaction between functional groups corresponds to the reaction (D) described above. The polyhydroxy component means a compound having two or more hydroxyl groups in one molecule, and includes a polyphenol component having two or more phenolic hydroxyl groups in one molecule.

More specifically, from the viewpoint of further improving the reaction rate, it is preferable to produce a polyester-based compound by using a combination of an acid halide component of a dicarboxylic acid component and a polyhydroxy component (e.g., a polyphenol component).

The dicarboxylic acid component capable of constituting the polyester-based compound is a dicarboxylic acid component similar to the dicarboxylic acid component capable of constituting the polyamide-based compound, and specifically, includes an aromatic dicarboxylic acid component, an alicyclic dicarboxylic acid component, and an aliphatic dicarboxylic acid component that are similar to the dicarboxylic acid component capable of constituting the polyamide-based compound. The acid halide component of the dicarboxylic acid component is a compound in which the OH group of the carboxyl group is substituted with a halogen atom in the dicarboxylic acid component.

From the viewpoint of heat resistance of the polyester-based compound, the dicarboxylic acid component of the polyester-based compound preferably contains an aromatic dicarboxylic acid component and/or an alicyclic dicarboxylic acid component, more preferably an aromatic dicarboxylic acid component.

From the viewpoint of further improving the heat resistance of the polyester-based compound, the dicarboxylic acid component of the polyester-based compound preferably contains only an aromatic dicarboxylic acid component and/or an alicyclic dicarboxylic acid component, more preferably only an aromatic dicarboxylic acid component.

From the viewpoint of versatility, among the above-mentioned dicarboxylic acid components, the dicarboxylic acid component of the polyester-based compound preferably contains one or more compounds selected from the group (hereinafter referred to as Group H10) consisting of terephthalic acid, isophthalic acid, 1,4-cyclohexanedicarboxylic acid, and 1,3-cyclohexanedicarboxylic acid.

From the viewpoint of further improving the versatility, among the above-mentioned dicarboxylic acid components, the dicarboxylic acid component of the polyester-based compound preferably contains only one or more compounds selected from the above-mentioned group H10.

The polyhydroxy component capable of constituting the polyester-based compound includes an aromatic polyhydroxy component containing an aromatic ring (e.g., a polyphenol component), an alicyclic polyhydroxy component containing an aliphatic ring but not an aromatic ring, and an aliphatic polyhydroxy component that does not contain an aromatic ring or an alicyclic ring. The aromatic polyhydroxy component may contain an ether group and/or a thioether group, and/or one or more hydrogen atoms may be substituted with a halogen atom (e.g., fluorine atom, chlorine atom, and bromine atom). The polyhydroxy component includes, for example, a dihydroxy component, a trihydroxy component, a tetrahydroxy component, and a polyvalent hydroxy component. The polyvalent hydroxy component is a hydroxy component having 5 or more hydroxyl groups in one molecule. The polyhydroxy component preferably contains a dihydroxy component, and more preferably contains only a dihydroxy component, from the viewpoint of obtaining a high-molecular compound having high melt fluidity and/or solubility in a solvent and excellent processability.

The aromatic polyhydroxy component includes, for example, an aromatic dihydroxy component, an aromatic trihydroxy component, and an aromatic tetrahydroxy component, and an aromatic polyvalent hydroxy component. One of these may be used alone, or two or more thereof may be used as a mixture. Among the aromatic polyhydroxy components, the aromatic dihydroxy component is preferable from the viewpoint of obtaining a high-molecular compound having high melt fluidity and/or solubility in a solvent and excellent processability. The aromatic polyhydroxy component is preferably a polyphenol component having two or more phenolic hydroxyl groups in one molecule. The aromatic dihydroxy component is preferably a diphenol component having two phenolic hydroxyl groups in one molecule. The aromatic trihydroxy component is preferably a triphenol component having three phenolic hydroxyl groups in one molecule. The aromatic tetrahydroxy component is preferably a tetraphenol component having four phenolic hydroxyl groups in one molecule. The aromatic polyvalent hydroxy component is preferably a polyvalent phenol component having 5 or more phenolic hydroxyl groups in one molecule.

Examples of the aromatic dihydroxy component (particularly the diphenol component) include 2,2'-bis(4-hydroxyphenyl)propane, 2,2-bis(4-hydroxy-3,5-dimethylphenyl)propane, 2,2-bis(4-hydroxy-3,5-dibromophenyl)propane, 2,2-bis(4-hydroxy-3,5-dichlorophenyl)propane, 4,4'-dihydroxydiphenyl sulfone, 4,4'-dihydroxydiphenyl ether, 4,4'-dihydroxydiphenyl sulfide, 4,4'-dihydroxydiphenyl ketone, 4,4'-dihydroxydiphenyl methane, 4,4'-biphenol, 3,3'-dimethyl-4,4'-biphenol, 3,3',5,5'-tetramethyl-4,4'-biphenol, 2,2',3,3',5,5'-hexamethyl-4,4'-biphenol, 3,3',5,5-tetra-tert-butyl-2,2'-biphenol, 2,2-bis(4-hydroxyphenyl)butane, 2,2-bis(4-hydroxyphenyl)pentane, 2,2-bis(4-hydroxyphenyl)hexane, 2,2-bis(4-hydroxyphenyl)-3-methylbutane, 2,2-bis(4-hydroxyphenyl)-4-methylpentane, l,l-bis(4-hydroxyphenyl)-2-ethylhexane, 2,2-bis(4-hydroxyphenyl)butane, 1,1-bis(4-hydroxyphenyl)-2-methylpropane, 2,2-bis(4-hydroxyphenyl)octane, 1,1-bis(4-hydroxyphenyl)-3-methylbutane, 3,3-bis(4-hydroxyphenyl)pentane, 1,1-bis(4-hydroxyphenyl)methane, 1,1-bis(4-hydroxyphenyl)ethane, 1,1-bis(4-methyl-2-hydroxyphenyl)methane, 1,1-bis(3,5-dimethyl-4-hydroxyphenyl)methane, 1,1-bis(4-hydroxyphenyl)cyclohexane, 2,2-bis(4-hydroxyphenyl)propane, 2,2-bis(3-methyl-4-hydroxyphenyl)propane, 1,1-bis(4-hydroxyphenyl)-1-phenylethane, 2,2-bis(3-phenyl-4-hydroxyphenyl)propane, 1,1-bis(3-methyl-4-hydroxyphenyl)methane, 1,1-bis(4-hydroxyphenyl)-1 -phenylmethane, 1,1-bis(3-methyl-4-hydroxyphenyl)cyclohexane, 2,2-bis(3-allyl-4-hydroxyphenyl)propane, 2,2-bis(3-isopropyl-4-hydroxyphenyl)propane, 2,2-bis(3-tert-butyl-4-hydroxyphenyl)propane, 2,2-bis(3-sec-butyl-4-hydroxyphenyl)propane, bisphenol fluorene, 1,1-bis(2-methyl-4-hydroxy-5-tert-butylphenyl)-2-methylpropane, 4,4'-[1,4-phenylene-bis(2-propylidene)-bis(3-methyl-4-hydroxyphenyl)], 1,1-bis(3-phenyl-4-hydroxyphenyl)cyclohexane, 4,4'-dihydroxyphenyl ether, bis(2-hydroxyphenyl)methane, 2,4'-methylenebisphenol, bis(3-methyl-4-hydroxyphenyl)methane, bis(4-hydroxyphenyl)propane, 1,1-bis(2-hydroxy-5-methylphenyl)ethane, bis(2-hydroxy-3,5-dimethylphenyl)methane, 1,1-bis(4-hydroxyphenyl)cyclopentane, 1,1-bis(3-methyl-4-hydroxyphenyl)cyclopentane, 3,3-bis(3-methyl-4-hydroxyphenyl)pentane, 3,3-bis(3,5-dimethyl)-4-hydroxyphenyl)pentane, 2,2-bis(2-hydroxy-3,5-dimethylphenyl)propane, 2,2-bis(4-hydroxyphenyl)nonane, 1,1-bis(3-methyl-4-hydroxyphenyl)-1-phenylethane, 1,1-bis(3,5-dimethyl-4-hydroxyphenyl)cyclohexane, 2,2-bis(4-hydroxyphenyl)decane, 1,1-bis(4-hydroxyphenyl)decane, bis(2-hydroxy-3-tert-butyl-5-methylphenyl)methane, bis(4-hydroxyphenyl)diphenylmethane, terpendiphenol, 1,1-bis(3-tert-butyl-4-hydroxyphenyl)cyclohexane, 1,1-bis(2-methyl-4-hydroxy-5-tert-butylphenyl)-2-methylpropane, 2,2-bis(3-cyclohexyl-4-hydroxyphenyl)propane, 1,1-bis(3,5-di-tert-butyl-4-hydroxyphenyl)methane, 1,1-bis(3,5-di-sec-butyl-4-hydroxyphenyl)methane, 1,1-bis(3-cyclohexyl-4-hydroxyphenyl)cyclohexane, 1,1-bis(2-hydroxy-3,5-di-tert-butylphenyl)ethane, bis(3-nonyl-4-hydroxyphenyl)methane, 2,2-bis(3,5-di-tert-butyl-4-hydroxyphenyl)propane, bis(2-hydroxy-3,5-di-tert-butyl-6-methylphenyl)methane, 1,1-bis(3-phenyl4-hydroxyphenyl)-1-phenylethane, bis(3-fluoro-4-hydroxyphenyl)methane, bis(2-hydroxy-5-fluorophenyl)methane, 2,2-bis(4-hydroxyphenyl)-1,1,1,3,3,3-hexafluoropropane, 2,2-bis(3-fluoro-4-hydroxyphenyl)propane, bis(3-fluoro-4-hydroxyphenyl)-phenylmethane, bis(3-fluoro-4-hydroxyphenyl)-(p-fluorophenyl)methane, bis(4-hydroxyphenyl)-(p-fluorophenyl)methane, 2,2-bis(3-chloro-4-hydroxy-5-methylphenyl)propane, 2,2-bis(3,5-dichloro-4-hydroxyphenyl)propane, 2,2-bis(3-chloro-4-hydroxyphenyl)propane, 1,1-bis(3,5-dibromo-4-hydroxyphenyl)methane, 2,2-bis(3,5-dibromo-4-hydroxyphenyl)propane, 2,2-bis(3-nitro-4-hydroxyphenyl)propane, bis(4-hydroxyphenyl)dimethylsilane, bis(3-methyl-4-hydroxyphenyl)ether, bis(3,5-dimethyl-4-hydroxyphenyl)ether, bis(2,3,5-trimethyl-4-hydroxyphenyl)-phenylmethane, 2,2-bis(4-hydroxyphenyl)dodecane, 2,2-bis(3-methyl-4-hydroxyphenyl)dodecane, 2,2-bis(3,5-dimethyl-4-hydroxyphenyl)dodecane, 1,1-bis(3-tert-butyl-4-hydroxyphenyl)-1-phenylethane, 1,1-bis(3,5-di-tert-butyl-4-hydroxyphenyl)-1-phenylethane, 1,1-bis(2-methyl-4-hydroxy-5-cyclohexylphenyl)-2-methylpropane, 1,1-bis(2-hydroxy-3,5-di-tert-butylphenyl)ethane, isatin bisphenol, isatin biscresol, bis(2-hydroxyphenyl)methane, 2,4'-methylenebisphenol, 1,2-bis(4-hydroxyphenyl)ethane, 2-(4-hydroxyphenyl)-2-(2-hydroxyphenyl)propane, bis(2-hydroxy-3-allylphenyl)methane, 1,1-bis(2-hydroxy-3,5-dimethylphenyl)-2-methylpropane, 1,1-bis(2-hydroxy-5-tert-butylphenyl)ethane, bis(2-hydroxy-5-phenylphenyl)methane, 1,1-bis(2-methyl-4-hydroxy-5-tert-butylphenyl)butane, bis(2-methyl-4-hydroxy-5-cyclohexylphenyl)methane, 2,2-bis(4-hydroxyphenyl)pentadecane, 2,2-bis(3-methyl-4-hydroxyphenyl)pentadecane, 2,2-bis(3,5-dimethyl-4-hydroxyphenyl)pentadecane, 1,2-bis(3,5-di-tert-butyl-4-hydroxyphenyl)ethane, bis(2-hydroxy-3,5-di-tert-butylphenyl)methane, 2,2-bis(3-styryl-4-hydroxyphenyl)propane, 1,1-bis(4-hydroxyphenyl))-1-(p-nitrophenyl)ethane, bis(3,5-difluoro-4-hydroxyphenyl)methane, bis(3,5-difluoro-4-hydroxyphenyl)phenylmethane, bis(3,5-difluoro-4-hydroxyphenyl)diphenylmethane, bis(3-fluoro-4-hydroxyphenyl)diphenylmethane, 2,2-bis(3-chloro-4-hydroxyphenyl)propane, 1,1-bis(4-hydroxyphenyl)-3,3,5-trimethyl-cyclohexane, 1,1-bis(4-hydroxyphenyl)-3,3,5,5-tetramethyl-cyclohexane, 1,1-bis(4-hydroxyphenyl)-3,3,4-trimethyl-cyclohexane, 1,1-bis(4-hydroxyphenyl)-3,3-dimethyl-5-ethyl-cyclohexane, 1,1-bis(4-hydroxyphenyl)-3,3,5-trimethyl-cyclopentane, 1,1-bis(3,5-dimethyl-4-hydroxyphenyl)-3,3,5-trimethyl-cyclohexane, 1,1-bis(3,5-diphenyl-4-hydroxyphenyl)-3,3,5-trimethyl-cyclohexane, 1,1-bis(3-methyl-4-hydroxyphenyl)-3,3,5-trimethyl-cyclohexane, 1,1-bis(3-phenyl-4-hydroxyphenyl)-3,3,5-trimethyl-cyclohexane, 1,1-bis(3,5-dichloro-4-hydroxyphenyl)-3,3,5-trimethyl-cyclohexane, 9,9-bis(4-hydroxyphenyl)fluorene, 9,9-bis(3-methyl-4-hydroxyphenyl)fluorene, 1,1-bis(3,5-dibromo-4-hydroxyphenyl)-3,3,5-trimethyl-cyclohexane, bis(4-hydroxyphenyl)sulfone, bis(2-hydroxyphenyl)sulfone, bis(3,5-dimethyl-4-hydroxyphenyl)sulfone, bis(3,5-diethyl-4-hydroxyphenyl)sulfone, bis(3-methyl-4-hydroxyphenyl)sulfone, bis(3-ethyl-4-hydroxyphenyl)sulfone, bis(4-hydroxyphenyl)sulfide, bis(3,5-dimethyl-4-hydroxyphenyl)sulfide, bis(3,5-diethyl-4-hydroxyphenyl)sulfide, bis(3-methyl-4-hydroxyphenyl)sulfide, bis(3-ethyl-4-hydroxyphenyl)sulfide, 2,4-dihydroxydiphenyl sulfone, 1,4-dihydroxybenzene, 1,3-dihydroxybenzene, and 1,2-dihydroxybenzene. One of these may be used alone, or two or more thereof may be used as a mixture.

Examples of the aromatic trihydroxy component (particularly the triphenol component) include 1,3,5-trihydroxybenzene, 1,3,5-tris(4-hydroxyphenyl)benzene, 1,3,3-tri(4-hydroxyphenyl)butane, 2-[bis(4-hydroxyphenyl)methyl]phenol, 4,4'-[1-[4-[1-(4-hydroxyphenyl)-1-methylethyl]phenyl]ethylidene]bisphenol, 4-[bis(4-hydroxyphenyl)methyl]-2-methoxyphenol, tris(3-methyl-4-hydroxyphenyl)methane, 4-[bis(3-methyl-4-hydroxyphenyl)methyl]-2-methoxyphenol, 4-[bis(3,5-dimethyl-4-hydroxyphenyl)methyl]-2-methoxyphenol, 1,1,1-tris(4-hydroxyphenyl)ethane, 1,1,1-tris(3-methyl-4-hydroxyphenyl)ethane, 1,1,1-tris(3,5-dimethyl-4-hydroxyphenyl)ethane, tris(3-methyl-4-hydroxyphenyl)methane, tris(3,5-dimethyl-4-hydroxyphenyl)methane, 2,6-bis[(2-hydroxy-5-methylphenyl)methyl]-4-methylphenol, 2-[bis(2-methyl-4-hydroxy-5-cyclohexylphenyl)methyl]-phenol, 4-methylphenyl-1,2,3-trihydroxybenzene, 4-[1-(4-hydroxyphenyl)-1-methylethyl]-1,3-dihydroxybenzene, 4-[bis(3-methyl-4-hydroxyphenyl)methyl]phenol, 2-[bis(2-methyl-4-hydroxyphenyl)methyl]phenol, 4-[bis(4-hydroxyphenyl)methyl]-2-ethoxyphenol, 2-[bis(2,3-dimethyl-4-hydroxyphenyl)methyl]phenol, 4-[bis(3,5-dimethyl-4-hydroxyphenyl)methyl]phenol, 3-[bis(3,5-dimethyl-4-hydroxyphenyl)methyl]phenol, 2-[bis(2-hydroxy-3,6-dimethylphenyl)methyl]phenol, 4-[bis(2-hydroxy-3,6-dimethylphenyl)methyl]phenol, 4-[bis(3,5-dimethyl-4-hydroxyphenyl)methyl]-2-methoxyphenol, 2-[bis(2,3,6-trimethyl-4-hydroxyphenyl)methyl]phenol, 2-[bis(2,3,5-trimethyl-4-hydroxyphenyl)methyl]phenol, 3-[bis(2,3,5-trimethyl-4-hydroxyphenyl)methyl] phenol, 4-[bis(2,3,5-trimethyl-4-hydroxyphenyl)methyl] phenol, 3-[bis(2-methyl-4-hydroxy-5-cyclohexylphenyl)methyl]phenol, 4-[bis(2-methyl-4-hydroxy-5-cyclohexylphenyl)methyl]phenol, 4-[bis(2-methyl-4-hydroxy-5-cyclohexylphenyl)methyl]-2-methoxyphenol, (2,4-dihydroxyphenyl) (4-hydroxyphenyl) ketone, 1,1,3-tris(2-methyl-4-hydroxy-5-t-butylphenyl)butane, 1,3,5-trimethyl-2,4,6-tris(3,5-di-t-butyl-4-hydroxybenzyl)benzene, 4,6-dimethyl-2,4,6-tris(4-hydroxyphenyl)heptene-2,4,6-dimethyl-2,4,6-tris(4-hydroxyphenyl)heptane, and 2,6-dimethyl-2,4,6-tris(4-hydroxyphenyl)hepten-3. One of these may be used alone, or two or more thereof may be used as a mixture.

Examples of the aromatic tetrahydroxy component (particularly the tetraphenol component) include 1,1,2,2-tetrakis(4-hydroxyphenyl)ethane, 1,1,2,2-tetra(3,5-dimethyl-4)-hydroxyphenyl)ethane, 2,3,4,4'-tetrahydroxybenzophenone, 4-[bis(3,5-dimethyl4-hydroxyphenyl)methyl]-1,2-dihydroxybenzene, 4-[bis(2-methyl-4-hydroxy-5-cyclohexylphenyl)methyl]-1,2-dihydroxybenzene, 4-[(4-hydroxyphenyl)methyl]-1,2,3-trihydroxybenzene, 4-[(3,5-dimethyl-4-hydroxyphenyl)methyl]-1,2,3-trihydroxybenzene, 1,4-bis[1-bis(3,4-dihydroxyphenyl)-1-methylethyl]benzene, 4-[bis(3-methyl-4-hydroxyphenyl)methyl]-1,2-dihydroxybenzene, 3,6-bis[(3,5-dimethyl-4-hydroxyphenyl)methyl]-1,2-dihydroxybenzene, 4-[bis(2,3,5-trimethyl-4-hydroxyphenyl)methyl]-1,2-dihydroxybenzene, 1,4-di[bis(4-hydroxyphenyl)methyl]benzene, 1,4-di[bis(3-methyl-4-hydroxyphenyl)methyl]benzene, 1,4-di[bis(3,5-dimethyl-4-hydroxyphenyl)methyl]benzene, and tetrakis{methylene-3-(3,5-di-t-butyl-4-hydroxyphenyl)propionate}methane. One of these may be used alone, or two or more thereof may be used as a mixture.

Examples of the aromatic polyvalent hydroxy component (particularly the polyhydric phenol component) include 1,4-bis[1-bis(2,3,4-trihydroxyphenyl)-1-methylethyl]benzene, 2,4-bis[(4-hydroxyphenyl)methyl]-1,3-dihydroxybenzene, 2-[bis(3-methyl-4-hydroxyphenyl)methyl]phenol, 4,6-[bis(3,5-dimethyl-4)-hydroxyphenyl)methyl]-1,2,3-trihydroxybenzene, and 2,4,6-tris[(4-hydroxyphenyl)methyl]-1,3-dihydroxybenzene. One of these may be used alone, or two or more thereof may be used as a mixture.

The alicyclic polyhydroxy component includes an alicyclic dihydroxy component and an alicyclic trihydroxy component. One of these may be used alone, or two or more thereof may be used as a mixture. Of the alicyclic polyhydroxy components, the alicyclic dihydroxy component is preferable from the viewpoint of obtaining a high-molecular compound having high melt fluidity and/or solubility in a solvent and excellent processability.

Examples of the alicyclic dihydroxy component include 1,4-cyclohexanedimethanol, 1,4-cyclohexanediethanol, and tricyclodecane dimethanol. One of these may be used alone, or two or more thereof may be used as a mixture.

Examples of the alicyclic trihydroxy component include 1,2,3-cyclohexanetriol, 1,3,5-cyclohexanetriol, and 1,3,5-cyclohexanetrimethanol. One of these may be used alone, or two or more thereof may be used as a mixture.

The aliphatic polyhydroxy component includes an aliphatic dihydroxy component, an aliphatic trihydroxy component, an aliphatic tetrahydroxy component, and an aliphatic polyvalent hydroxy component. One of these may be used alone, or two or more thereof may be used as a mixture. Among the aliphatic polyhydroxy components, the aliphatic dihydroxy component is preferable from the viewpoint of obtaining a high-molecular compound having a higher molecular weight.

Examples of the aliphatic dihydroxy component include low molecular weight polyols such as ethylene glycol, propylene glycol, 1,2-propanediol, 1,3-propanediol, 1,2-butanediol, dipropylene glycol, 1,3-butanediol, 1,4-butanediol, 2,3-butanediol, diethylene glycol, 1,5-pentanediol, 1,6-hexanediol, 2-ethyl-1,3-hexanediol, neopentyl glycol, triethylene glycol, and polyethylene glycol; polyether diols having a number average molecular weight of 2000 or less; and dimer diols obtained by converting the carboxyl group of dimer acid into a hydroxyl group, and alkylene oxide adducts and caprolactone adducts thereof. One of these may be used alone, or two or more thereof may be used as a mixture.

Examples of the aliphatic trihydroxy component include glycerin, trimethylolethane, trimethylolpropane, trimethylolbutane, 2-hydroxyalkylmethyl-1,4-butanediol, tris(2-hydroxyethyl)isocyanurate, and alkylene oxide adducts and caprolactone adducts thereof. One of these may be used alone, or two or more thereof may be used as a mixture.

Examples of the aliphatic tetrahydroxy component include pentaerythritol, ditrimethylolpropane, and alkylene oxide adducts and caprolactone adducts thereof. One of these may be used alone, or two or more thereof may be used as a mixture.

Examples of the aliphatic polyvalent hydroxy component include sugars such as xylose, arabinose, ribulose, glucose, fructose, mannose, galactose, erythritol, threitol, arabitol, ribitol, xylitol, sorbitol, mannitol, and sucrose, and alkylene oxide adducts and caprolactone adducts thereof. One of these may be used alone, or two or more thereof may be used as a mixture.

From the viewpoint of heat resistance of the polyester-based compound, the polyhydroxy component of the polyester-based compound preferably contains an aromatic dihydroxy component (particularly a diphenol component) and/or an alicyclic dihydroxy component, more preferably an aromatic hydroxy component (particularly a diphenol component).

From the viewpoint of further improving the heat resistance of the polyester-based compound, the polyhydroxy component of the polyester-based compound preferably contains only an aromatic dihydroxy component (particularly a diphenol component) and/or an alicyclic dihydroxy component, more preferably only an aromatic hydroxy component (particularly a diphenol component).

From the viewpoint of versatility, among the above-mentioned polyhydroxy components, the polyhydroxy component of the polyester-based compound preferably contains one or more compounds selected from the group (hereinafter referred to as Group H11) consisting of low molecular weight polyols such as 2,2-bis(4-hydroxyphenyl)propane, 1,1-bis(4-hydroxyphenyl)-1-phenylethane, 2,2-bis(4-hydroxyphenyl)hexafluoropropane, 2,2-bis(4-hydroxyphenyl)butane, bis(4-hydroxyphenyl)diphenylmethane, 2,2-bis(3-methyl)-4-hydroxyphenyl)propane, 1,1-bis(4-hydroxyphenyl)ethane, bis(4-hydroxyphenyl)methane, 2,2-bis(4-hydroxy-3 -isopropylphenyl)propane, bis(4-hydroxyphenyl) sulfone, 1,3-bis(2-(4-hydroxyphenyl)-2-propyl)benzene, 1,4-bis(2-(4-hydroxyphenyl)-2-propyl)benzene, 1,1-bis(4-hydroxyphenyl)-3,3,5-trimethylcyclohexane, 1,1-bis(4-hydroxyphenyl)cyclohexane, 1,4-cyclohexanedimethanol, 1,4-cyclohexanediethanol, ethylene glycol, propropylene glycol, 1,2-propanediol, 1,3-propanediol, 1,2-butanediol, dipropylene glycol, 1,3-butanediol, 1,4-butanediol, 2,3-butanediol, diethylene glycol, 1,5-pentanediol, 1,6-hexanediol, 2-ethyl-1,3-hexanediol, neopentyl glycol, triethylene glycol, and polyethylene glycol; polyether diols having a number average molecular weight of 2000 or less; dimer diols obtained by converting the carboxyl group of dimer acid into a hydroxyl group; trimethylolpropane, trimethylolethane, trimethylolbutane, 2-hydroxyalkylinethyl-1,4-butanediol, glycerin, pentaerythritol, and ditrimethylolpropane.

From the viewpoint of further improving the versatility, among the above-mentioned polyhydroxy components, the polyhydroxy component of the polyester-based compound preferably contains only one or more compounds selected from the above-mentioned Group H11.

In the production of the polyester-based compound, the dicarboxylic acid component or its acid halide component and the polyhydroxy component are usually used in approximately equal molar amounts. Specifically, the polyhydroxy component is used in an amount of usually 0.8 to 1.2 times the molar amount of the dicarboxylic acid component or its acid halide component, particularly 0.9 to 1.1 times, and preferably 0.95 to 1.05 times.

(Polyurea-based compound)

A polyurea-based compound can be produced by performing a reaction between functional groups by a mechanochemical effect, using a diisocyanate component and a diamine component as the raw material compounds. Here, the reaction between functional groups corresponds to the reaction (E) described above.

The diisocyanate component capable of constituting the polyurea-based compound is a diisocyanate component similar to the diisocyanate component capable of constituting the polyimide-based compound, and specifically, includes an aromatic diisocyanate component, an alicyclic diisocyanate component, and an aliphatic diisocyanate component that are similar to the diisocyanate component capable of constituting the polyimide-based compound.

From the viewpoint of heat resistance of the polyurea-based compound, the diisocyanate component of the polyurea-based compound preferably contains an aromatic diisocyanate component and/or an alicyclic diisocyanate component, more preferably an aromatic diisocyanate component.

From the viewpoint of further improving the heat resistance of the polyurea-based compound, the diisocyanate component of the polyurea-based compound preferably contains only an aromatic diisocyanate component and/or an alicyclic diisocyanate component, more preferably only an aromatic diisocyanate component.

From the viewpoint of versatility, among the above-mentioned diisocyanate components, the diisocyanate component of the polyurea-based compound preferably contains one or more compounds selected from the group (hereinafter referred to as Group H12) consisting of 4,4'-diphenylmethane diisocyanate, 2,6-tolylene diisocyanate, 2,4-tolylene diisocyanate, 1,5-diisocyanatonaphthalene, 4,4'-dicyclohexylmethane diisocyanate, isophorone diisocyanate, hexamethylene diisocyanate, trimethylhexamethylene diisocyanate, and 1,5-pentamethylene diisocyanate.

From the viewpoint of further improving the versatility, among the above-mentioned diisocyanate components, the diisocyanate component of the polyurea-based compound preferably contains only one or more compounds selected from the above-mentioned Group H12.

The diamine component capable of constituting the polyurea-based compound is a diamine component similar to the diamine component capable of constituting the polyamic acid-based compound and the like, and specifically, includes an aromatic diamine component, an alicyclic diamine component, and an aliphatic diamine component that are similar to the diamine component capable of constituting the polyamic acid-based compound and the like.

From the viewpoint of heat resistance of the polyurea-based compound, the diamine component of the polyurea-based compound preferably contains an aromatic diamine component and/or an alicyclic diamine component, more preferably an aromatic diamine component.

From the viewpoint of further improving the heat resistance of the polyurea-based compound, the diamine component of the polyurea-based compound preferably contains only an aromatic diamine component and/or an alicyclic diamine component, more preferably only an aromatic diamine component.

From the viewpoint of solubility of the polyurea-based compound, among the above-mentioned diamine components, it is preferable to use a diamine component having an ether group, a thioether group, a sulfonyl group, a sulfonic acid group, a methyl group, a methylene group, a phenyl group, a fluorene structure, a halogen atom (or a halogen atom-containing substituent), or a siloxane bond, as the diamine component of the polyurea-based compound.

From the viewpoint of further improving the solubility of the polyurea-based compound, among the above-mentioned diamine components, it is preferable to use only a diamine component having an ether group, a thioether group, a sulfonyl group, a sulfonic acid group, a methyl group, a methylene group, a phenyl group, a fluorene structure, a halogen atom (or a halogen atom-containing substituent), or a siloxane bond, as the diamine component of the polyurea-based compound.

From the viewpoint of imparting thermoplasticity to the polyurea-based compound, the diamine component of the polyurea-based compound preferably contains a diamine component having an ether group, a thioether group, a methyl group, a methylene group, an isopropylidene group, a ketone group, a sulfonyl group, a phenyl group, or a siloxane bond, among the above-mentioned diamine components.

From the viewpoint of more effectively imparting thermoplasticity to the polyurea-based compound, the diamine component of the polyurea-based compound preferably contains only a diamine component having an ether group, a thioether group, a methyl group, a methylene group, an isopropylidene group, a ketone group, a sulfonyl group, a phenyl group, or a siloxane bond, among the above-mentioned diamine components.

From the viewpoint of versatility, among the above-mentioned diamine components, the diamine component of the polyurea-based compound preferably contains one or more compounds selected from the group (hereinafter referred to as Group H13) consisting of 4,4'-diaminodiphenyl ether, p-phenylenediamine, m-phenylenediamine, 3,4'-diaminodiphenyl ether, 4,4'-diaminodiphenylmethane, 2,2'-bis(trifluoromethyl)-4,4'-diaminobiphenyl, 2,2-bis[4-(4-aminophenoxy)phenyl]-1, 1,1,3,3,3-hexafluoropropane, 1,4-bis(4-aminophenoxy)benzene, 1,3-bis(4-aminophenoxy)benzene, 4,4'-bis(4-aminophenoxy)biphenyl, 4,4'-bis(3-aminophenoxy)biphenyl, 2,2-bis[4-(4-aminophenoxy)phenyl]propane, bis(4-aminophenyl)sulfone, bis[4-(4-aminophenoxy)phenyl]sulfone, 9,9-bis(4-aminophenyl)fluorene, trans- 1,4-cyclohexanediamine, cis-1,4-cyclohexanediamine, 4,4'-methylenebis(cyclohexylamine), 1,10-diaminodecane, 1,12-diaminododecane, m-xylylene diamine, p-xylylene diamine, 1,4-bis(aminomethyl)cyclohexane, and dimer diamine.

From the viewpoint of further improving the versatility, among the above-mentioned diamine components, the diamine component of the polyurea-based compound preferably contains only one or more compounds selected from the above-mentioned Group H13.

In the production of the polyurea-based compound, the diisocyanate component and the diamine component are usually used in approximately equal molar amounts. Specifically, the diamine component is used in an amount of usually 0.8 to 1.2 times the molar amount of the diisocyanate component, particularly 0.9 to 1.1 times, and preferably 0.95 to 1.05 times.

(Polyurethane-based compound)

A polyurethane-based compound can be produced by performing a reaction between functional groups by a mechanochemical effect, using a diisocyanate component and a polyhydroxy component as the raw material compounds. Here, the reaction between functional groups corresponds to the reaction (F) described above.

The diisocyanate component capable of constituting the polyurethane-based compound is a diisocyanate component similar to the diisocyanate component capable of constituting the polyimide-based compound, and specifically, includes an aromatic diisocyanate component, an alicyclic diisocyanate component, and an aliphatic diisocyanate component that are similar to the diisocyanate component capable of constituting the polyimide-based compound.

From the viewpoint of heat resistance of the polyurethane-based compound, the diisocyanate component of the polyurethane-based compound preferably contains an aromatic diisocyanate component and/or an alicyclic diisocyanate component, more preferably an aromatic diisocyanate component.

From the viewpoint of further improving the heat resistance of the polyurethane-based compound, the diisocyanate component of the polyurethane-based compound preferably contains only an aromatic diisocyanate component and/or an alicyclic diisocyanate component, more preferably only an aromatic diisocyanate component.

From the viewpoint of versatility, among the above-mentioned diisocyanate components, the diisocyanate component of the polyurethane-based compound preferably contains one or more compounds selected from the group (hereinafter referred to as Group H14) consisting of 4,4'-diphenylmethane diisocyanate, 2,6-tolylene diisocyanate, 2,4-tolylene diisocyanate, 1,5-diisocyanatonaphthalene, 4,4'-dicyclohexylmethane diisocyanate, isophorone diisocyanate, hexamethylene diisocyanate, trimethylhexamethylene diisocyanate, and 1,5-pentamethylene diisocyanate.

From the viewpoint of further improving the versatility, among the above-mentioned diisocyanate components, the diisocyanate component of the polyurethane-based compound preferably contains only one or more compounds selected from the above-mentioned Group H14.

The polyhydroxy component capable of constituting the polyurethane-based compound is a polyhydroxy component similar to the polyhydroxy component capable of constituting the polyester-based compound, and specifically, includes an aromatic polyhydroxy component (particularly polyphenol component), an alicyclic polyhydroxy component, and an aliphatic polyhydroxy component that are similar to the polyhydroxy component capable of constituting the polyester-based compound.

From the viewpoint of heat resistance of the polyurethane-based compound, the polyhydroxy component of the polyurethane-based compound preferably contains an aromatic polyhydroxy component (particularly a polyphenol component) component and/or an alicyclic polyhydroxy component, more preferably an aromatic polyhydroxy component (particularly a polyphenol component).

From the viewpoint of further improving the heat resistance of the polyurethane-based compound, the polyhydroxy component of the polyurethane-based compound preferably contains only an aromatic polyhydroxy component (particularly a polyphenol component) and/or an alicyclic polyhydroxy component, more preferably only an aromatic polyhydroxy component (particularly a polyphenol component).

From the viewpoint of flexibility of the polyurethane-based compound, among the above-mentioned polyhydroxy components, it is preferable to use an aliphatic polyhydroxy component, as the polyhydroxy component of the polyurethane-based compound.

From the viewpoint of further improving the flexibility of the polyurethane-based compound, among the above-mentioned polyhydroxy components, it is preferable to use only an aliphatic polyhydroxy component, as the polyhydroxy component of the polyurethane-based compound.

From the viewpoint of versatility, among the above-mentioned polyhydroxy components, the polyhydroxy component of the polyurethane-based compound preferably contains one or more compounds selected from the group (hereinafter referred to as Group H15) consisting of low molecular weight polyols such as 2,2-bis(4-hydroxyphenyl)propane, 1,1-bis(4-hydroxyphenyl)-1-phenylethane, 2,2-bis(4-hydroxyphenyl)hexafluoropropane, 2,2-bis(4-hydroxyphenyl)butane, bis(4-hydroxyphenyl)diphenylmethane, 2,2-bis(3-methyl)-4-hydroxyphenyl)propane, 1,1-bis(4-hydroxyphenyl)ethane, bis(4-hydroxyphenyl)methane, 2,2-bis(4-hydroxy-3-isopropylphenyl)propane, bis(4-hydroxyphenyl) sulfone, 1,3-bis(2-(4-hydroxyphenyl)-2-propyl)benzene, 1,4-bis(2-(4-hydroxyphenyl)-2-propyl)benzene, 1,1 -bis(4-hydroxyphenyl)-3,3,5-trimethylcyclohexane, 1,1 -bis(4-hydroxyphenyl)cyclohexane, 1,4-cyclohexanedimethanol, 1,4-cyclohexanediethanol, ethylene glycol, propropylene glycol, 1,2-propanediol, 1,3-propanediol, 1,2-butanediol, dipropylene glycol, 1,3-butanediol, 1,4-butanediol, 2,3-butanediol, diethylene glycol, 1,5-pentanediol, 1,6-hexanediol, 2-ethyl-1,3-hexanediol, neopentyl glycol, triethylene glycol, and polyethylene glycol; polyether diols having a number average molecular weight of 2000 or less; dimer diols obtained by converting the carboxyl group of dimer acid into a hydroxyl group; trimethylolpropane, trimethylolethane, trimethylolbutane, 2-hydroxyalkyhnethyl-1,4-butanediol, glycerin, pentaerythritol, and ditrimethylolpropane.

From the viewpoint of further improving the versatility, among the above-mentioned polyhydroxy components, the polyhydroxy component of the polyurethane-based compound preferably contains only one or more compounds selected from the above-mentioned Group H15.

In the production of the polyurethane-based compound, the diisocyanate component and the polyhydroxy component are usually used in approximately equal molar amounts. Specifically, the polyhydroxy component is used in an amount of usually 0.8 to 1.2 times the molar amount of the diisocyanate component, particularly 0.9 to 1.1 times, and preferably 0.95 to 1.05 times.

[Production method of high-molecular compound]

In the production method of a high-molecular compound of the present invention, a reaction is performed between functional groups by a mechanochemical effect using a raw material mixture containing a given raw material compound. The given raw material compound is one or more, particularly two or more raw material compounds (monomer components) for producing each of the above-mentioned high-molecular compounds, and of these, at least one or more raw material compounds are in a solid state under the reactive environment as described above. Specifically, a reaction is performed between functional groups by using a mechanochemical effect by subjecting a raw material mixture containing such a raw material compound to a pulverization treatment. When a raw material compound in a liquid state under the reactive environment is used as the raw material compound, the raw material compound in the liquid state is preferably mixed or added before or while pulverizing at least one raw material compound in a solid state contained in the raw material mixture, from the viewpoint of further improving the reaction rate. At this time, from the viewpoint of further improving the reaction rate, the raw material compound in the liquid state is preferably added in an amount obtained by dividing a predetermined added amount into two or more portions in a plurality of times, more preferably added dropwise.

As the raw material compound (particularly the raw material compound in the solid state under the reactive environment), a compound having a particle shape of a maximum length of 0.001 to 20.0 mm, particularly 0.01 to 10.0 mm is usually used. A cumulative 50% particle size has been used as the maximum length. Specifically, when particles having a particle size of 0.5 mm or more are included, in accordance with JIS Z8815, the maximum length is a value measured as a cumulative 50% particle size from the particle size distribution obtained by a sieving test described in JIS Z8815. When particles with a particle size of 0.5 mm or more are not included, the maximum length is determined as a cumulative 50% particle size obtained by a laser diffraction/scattering method using a particle size distribution measuring apparatus.

The pulverization treatment for the production of a high-molecular compound may be achieved by any apparatus (e.g., so-called a pulverizer, a mixer, or a stirrer) as long as the apparatus can transfer mechanical energy to the raw material compound by compression, impact, shearing and/or grinding. For example, the pulverization treatment can be performed using an apparatus such as a jaw crusher, a gyratory crusher, a cone crusher, an impact (hammer) crusher, a roll crusher, a cutter mill, an autogenous mill, a stamp mill, a stone mill, a mortar, a mortar machine, a Muller type mill, an Eirich mill, a ring mill, a roller mill, a jet mill, a high-speed bottom stirring type mixer, high-speed rotary pulverizer (a hammer mill, a pin mill), a container drive type mill (a rolling mill, a vibration mill, a planetary mill), a medium stirring type mill (a bead mill), a high-speed flow type mixer, or a Henschel mixer. Among such apparatuses, examples of the typical apparatus include a high-speed bottom stirring type mixer, a high-speed rotary pulverizer, a container drive type mill, and a medium-stirring type mill.

A high-speed bottom stirring type mixer is generally an apparatus having a structure in which large high-speed rotary blades are arranged at the bottom of a cylindrical container, with two stages of upper and lower rotary blades. A high-speed rotary pulverizer is an apparatus in which a sample is allowed to collide against an impactor such as a hammer, a pin, or a bar on a rotating rotor, to thereby be pulverized. A container drive type mill (a rolling mill, a vibration mill, a planetary mill) is an apparatus in which a medium such as a ball is put in a rotating container, and the container is rotated to pulverize the raw material. A medium-stirring type mill is an apparatus in which balls or beads are used as a pulverizing medium to pulverize a sample by collision between the sample and the pulverizing medium.

The reaction conditions (that is, mixing/stirring/pulverization conditions) for producing a high-molecular compound are not particularly limited as long as the mechanochemical effect may be exhibited to obtain a desired high-molecular compound.

For example, when the above-mentioned maximum length of the raw material compound (particularly the raw material compound in the solid state under the reactive environment) having a particle shape is Rm ($\mu$m), pulverization treatment is performed until the average particle size of the raw material compound reaches $0.5 \times$ Rm or less, particularly $0.1 \times$ Rm or less.

Specifically, for example, when a medium-stirring type mill is used with a pulverizing chamber (or a tank) for pulverization treatment having a capacity of 4 to 6 L (particularly 5 L), a raw material mixture weighing 0.5 to 1.5 kg (particularly 1 kg), a pulverizing ball made of alumina, having a ball diameter of 10.0 mm, and an input weight of 6.0 kg, the rotation speed is usually 115 rpm or higher, particularly from 115 to 504 rpm, and the pulverization time is usually 1 minute or more, particularly from 1 to 60 minutes.

For example, when a planetary mil is used with a pulverizing chamber (or a tank) for pulverization treatment having a capacity of 0.08 to 0.5 L (particularly 0.25 L), a raw material mixture weighing 4 to 6 g (particularly 5 g), a pulverizing ball made of zirconia, having a ball diameter of 10.0 mm, and an input of 30 balls, the rotation speed is usually 100 rpm or higher, particularly from 100 to 600 rpm, and the pulverization time is usually 1 minute or more, particularly from 3 to 15 minutes.

Such a pulverization treatment and a subsequent cooling treatment (e.g., cooling-by-leaving treatment) of the pulverized product may be repeated twice or more, for example, 2 to 10 times. As a result, the mechanochemical effect is more effectively exhibited, the reaction rate is further improved, and the degree of polymerization of the obtained high-molecular compound is increased.

In the production method of a high-molecular compound, the molecular weight can be controlled by adjusting the reaction conditions (mixing/stirring/pulverization conditions). For example, the molecular weight increases as the pulverization conditions are strengthened within the range in which the raw material mixture does not melt. The molecular weight of the obtained high-molecular compound is not particularly limited and, for example, a high-molecular compound having an average degree of polymerization of 2 or more (e.g., 2 to 100), particularly 2 to 20 can be obtained.

In the present invention, the high-molecular compound obtained by the pulverization treatment preferably has an average particle size (D50) of 1000 $\mu$m or less, from 0.01 to 1000 $\mu$m, particularly from 0.1 to 100 $\mu$m.

In the production method of a high-molecular compound, a terminal blocking agent may be contained in the raw material mixture in order to adjust the molecular weight. Examples of the terminal blocking agent include a monofunctional acid anhydride compound, a monofunctional amine compound, a monofunctional carboxylic acid-based compound or a halide thereof, a monofunctional alcohol compound, a monofunctional phenol compound, a monofunctional isocyanate compound, and a monofunctional epoxy compound. Preferred terminal blocking agents are a monofunctional acid anhydride compound, a monofunctional amine compound, a monofunctional carboxylic acid-based compound or an acid halide thereof, a monofunctional alcohol compound, and a monofunctional phenol compound.

Examples of the monofunctional acid anhydride include, but are not limited to, the following: Phthalic anhydride, maleic anhydride, methylmaleic anhydride, 2,3-dimethylmaleic anhydride, trimellitic anhydride, 2,3-benzophenonedicarboxylic anhydride, 3,4-benzophenonedicarboxylic anhydride, 2,3-dicarboxyphenyl phenyl ether anhydride, 2,3-biphenyldicarboxylic anhydride, 3,4-biphenyldicarboxylic anhydride, 2,3-dicarboxyphenyl phenyl sulfone anhydride, 3,4-dicarboxyphenyl phenyl sulfone anhydride, 2,3-dicarboxyphenyl phenyl sulfide anhydride, 1,2-naphthalenedicarboxylic anhydride, 2,3-naphthalenedicarboxylic anhydride, 1,8-naphthalenedicarboxylic anhydride, 1,2-anthracenedicarboxylic anhydride, 2,3-anthracenedicarboxylic anhydride, 1,9-anthracenedicarboxylic anhydride, 5-norbornene-2,3-dicarboxylic anhydride, 4-ethynylphthalic anhydride, 4-(1-propinyl)phthalic anhydride, and 4-phenylethynyl phthalic anhydride. One of these may be used alone, or two or more thereof may be used as a mixture.

Examples of the monofunctional amine include, but are not limited to, the following: Aniline, o-toluidine, m-toluidine, p-toluidine, 2,3-xylidine, 2,4-xylidine, 2,5-xylidine, 2,6-xylidine, 3,4-xylidine, 3,5-xylidine, o-chloroaniline, m-chloroaniline, p-chloroaniline, o-nitroaniline, o-bromoaniline, m-bromoaniline, o-nitroaniline, m-nitroaniline, p-nitroaniline, o-aminophenol, m-aminophenol, p-aminophenol, o-anilidine, m-anilidine, p-anilidine, o-phenetidine, m-phenetidine, p-phenetidine, o-aminobenzaldehyde, m-aminobenzaldehyde, p-aminobenzaldehyde, o-aminobenzonitrile, m-aminobenzonitrile, p-aminobenzonitrile, 2-aminobiphenyl, 3-aminobiphenyl, 4-aminobiphenyl, 2-aminophenol phenyl ether, 3-aminophenol phenyl ether, 4-aminophenol phenyl ether, 2-aminobenzophenone, 3-aminobenzophenone, 4-aminobenzophenone, 2-aminophenol phenyl sulfide, 3-aminophenol phenyl sulfide, 4-aminophenol phenyl sulfide, 2-aminophenol phenyl sulfone, 3-aminophenol phenyl sulfone, 4-aminophenol phenyl sulfone, $\alpha$-naphthyiamine, $\beta$-naphthylamine, 1-amino-2-naphthol, 2-amino-1-naphthol, 4-amino-1-naphthol, 5-amino-1-nafthol, 5-amino-1-naphthol, 5-amino-2-naphthol, 7-amino-2-naphthol, 8-amino-2-naphthol, 1-aminoanthracene, 2-aminoanthracene, 9-aminoanthracene, 4-aminostyrene, 4-aminostilbene, 3-ethynylaniline, and 4-ethynylaniline. One of these may be used alone, or two or more thereof may be used as a mixture.

Examples of the monofunctional carboxylic acid include, but are not limited to, the following: Acetic acid, propionic acid, octanoic acid, cyclohexanecarboxylic acid, toluic acid, phenylacetic acid, p-methoxyphenylacetic acid, lauric acid, myristic acid, palmitic acid, stearic acid, oleic acid, linoleic acid, linolenic acid, benzoic acid, and p-tert-butyl benzoic acid. One of these may be used alone, or two or more thereof may be used as a mixture.

Examples of the monofunctional alcohol include, but are not limited to, the following: Methanol, ethanol, n-propanol, isopropanol, n-butanol, 2-butanol, pentanol, hexanol, dodecyl alcohol, stearyl alcohol, octyl alcohol, decyl alcohol, lauryl alcohol, myristyl alcohol, cetyl alcohol, 2-phenoxyethanol, benzyl alcohol, and phenethyl alcohol. One of these may be used alone, or two or more thereof may be used as a mixture.

Examples of the monofunctional phenol include, but are not limited to, the following: Phenol, o-cresol, m-cresol, p-cresol, p-tert-butylphenol, o-phenylphenol, m-phenylphenol, p-phenylphenol, o-methoxyphenol, m-methoxyphenol, p-methoxyphenol, 2,3,6-trimethylphenol, 2,3-xylenol, 2,4-xylenol, 2,5-xylenol, 2,6-xylenol, 3,4-xylenol, 3,5-xylenol, 2-phenyl-2-(4-hydroxyphenyl)propane, 2-phenyl-2-(2-hydroxyphenyl)propane, and 2-phenyl-2-(3-hydroxyphenyl)propane. One of these may be used alone, or two or more thereof may be used as a mixture.

In the present invention, in order to suppress the adhesion of particles to the inner walls of the mixing chamber, the stirring chamber, and the pulverizing chamber, to increase the pulverizing efficiency of the particles, and to increase the energy transfer efficiency to the particles, the raw material mixture may contain an auxiliary agent. As the auxiliary agent, water, alcohol, a watersoluble polymer, a synthetic polymer, inorganic particles, a surfactant, waxes or the like may be used. Examples thereof include hexane solutions such as water, methanol, ethanol, triethylamine, triethanolamine, and cetyl alcohol; lower alcohols such as propyl alcohol; glycols such as ethylene glycol, propylene glycol, neopentyl glycol, 1,3-butylene glycol, dipropylene glycol, 1,2-pentanediol, and polyethylene glycol; glycerols such as glycerin, diglycerin, and polyglycerin; cellulose derivatives such as methyl cellulose, hydroxymethyl cellulose, hydroxyethyl cellulose, hydroxypropyl cellulose, hydroxypropyl methylcellulose, and carboxymethyl cellulose; natural polymers such as sodium alginate, carrageenan, quince seed gum, agar, gelatin, xanthan gum, locust bean gum, pectin, and gellan gum; synthetic polymers such as polyvinyl alcohol, carboxyvinyl polymer, alkyl-added carboxyvinyl polymer, sodium polyacrylate, sodium polymethacrylate, glyceryl polyacrylate, and polyvinylpyrrolidone; inorganic powders such as carbon black, titanium oxide, black titanium oxide, cerium oxide, iron blue, ultramarine blue, red iron oxide, iron oxide, yellow iron oxide, black iron oxide, zinc oxide, aluminum oxide, silicic anhydride, magnesium oxide, zirconium oxide, magnesium carbonate, calcium carbonate, calcium sulfate, chromium oxide, chromium hydroxide, carbon black, aluminum silicate, magnesium silicate, aluminum magnesium silicate, mica, synthetic mica, sericite, talc, kaolin, silicon carbide, barium sulfate, bentonite, smectite, and boron nitride; glittering powders such as bismuth oxychloride, titanium oxide-coated mica, iron oxide-coated mica, iron oxide-coated mica titanium, organic pigment-coated mica titanium, and aluminum powder; organic powders such as nylon powder, polymethylmethacrylate powder, acrylonitrile-methacrylic acid copolymer powder, vinylidene chloride-methacrylic acid copolymer powder, polyethylene powder, polystyrene powder, (dimethicone/vinyl dimethicone) crosspolymer powder, (vinyl dimethicone/methicone silsesquioxane) crosspolymer powder, (diphenyl dimethicone/vinyl diphenyl dimethicone/silsesquioxane) crosspolymer powder, polymethylsilcesquioxane powder, polyurethane powder, wool powder, silk powder, and N-acyllysine; pigment powders such as organic tar pigments and lake pigments of organic pigments; composite powders such as fine particle titanium oxide-coated mica titanium, fine particle zinc oxide-coated mica titanium, barium sulfate-coated mica titanium, titanium oxide-containing silica, and zinc oxide-containing silica; nonionic surfactants such as glycerin fatty acid ester and its alkylene glycol adduct, polyglycerin fatty acid ester and its alkylene glycol adduct, propylene glycol fatty acid ester and its alkylene glycol adduct, sorbitan fatty acid ester and its alkylene glycol adduct, sorbitol fatty acid ester and its alkylene glycol adduct, polyalkylene glycol fatty acid ester, polyoxyalkylene-modified silicone, and polyoxyalkylene alkyl co-modified silicone; fatty acids such as stearic acid and lauric acid, and inorganic or organic salts thereof; anionic surfactants such as alkylbenzene sulfates, alkyl sulfonates, α-olefin sulfonates, dialkyl sulfosuccinates, α-sulfonated fatty acid salts, acylmethyltaurine salts, N-methyl-N-alkyltaurine salts, polyoxyethylene alkyl ether sulfates, polyoxyethylene alkylphenyl ether sulfates, alkyl phosphates, polyoxyethylene alkyl ether phosphates, polyoxyethylene alkylphenyl ether phosphates, and N-acyl-N-alkylamino acid salts; cationic surfactants such as alkylamine salts, polyamine and alkanoylamine fatty acid derivatives, alkylammonium salts, and alicyclic ammonium salts; amphoteric surfactants such as phospholipids and N,N-dimethyl-N-alkyl-N-carboxymethylammonium betaine; hydrocarbons such as paraffin wax, ceresin wax, ozokerite, microcrystalline wax, montan wax, Fisher-Tropsch wax, polyethylene wax, liquid paraffin, squalane, vaseline, polyisobutylene, and polybutene; natural waxes such as carnauba wax, bees wax, lanolin wax, and candelilla; esters such as glyceryl 2-ethylhexanoate, pentaerythritol rosinate, cetyl isooctanoate, isopropyl myristate, diglyceryl triisostearate, dipentaerythrite fatty acid ester, diisostearyl malate, and (phytosteryl/isostearyl/cetyl/stearyl/behenyl) dimer dilinoleate; fatty acids such as stealic acid, behenic acid, and 12-hydroxystearic acid; higher alcohols such as cetanol, stearyl alcohol, and behenyl alcohol; lanolin derivatives such as isopropyl lanolate and lanolin alcohol; amino acid derivatives such as di(cholesteryl/behenyl/octyldodecyl) N-lauroyl-L-glutamate; and fluorine oils such as perfluoropolyether, perfluorodecane, and perfluorooctane. Of these, one or two or more may be used.

In the production method of a high-molecular compound of the present invention, a catalyst may be contained in the raw material mixture in order to promote the reaction. As the catalyst, any catalyst (acid catalyst, base catalyst, metal catalyst, metal oxide catalyst, complex catalyst, sulfide, chloride, metal organic salt, mineral acid, etc.) useful for producing a high-molecular compound can be used. Specific examples of the catalyst include, for example, p-toluenesulfonic acid, dimethyl sulfate, diethyl sulfate, sulfuric acid, hydrochloric acid, oxalic acid, acetic acid, phosphoric acid, phosphorous acid, hypophosphorous acid or salts thereof, sodium hydroxide, potassium hydroxide, lithium hydroxide, magnesium hydroxide, calcium hydroxide, barium hydroxide, pyridine, ammonia, triethylamine, 2,4,6-tris(dimethylaminomethyl)phenol, N,N-dimethylethanolamine, aminoethanolamine, N-methyl-N,N-diethanolamine, isopropylamine, iminobispropylamine, ethylamine, diethylamine, 3-ethoxypropylamine, 3-diethylaminopropylamine, sec-butylamine, propylamine, methylaminopropylamine, 3-methoxypropylamine, monoethanolamine, morpholine, N-methylmorpholine, N-ethylmorpholine, imidazole compounds such as 1-methylimidazole, 2-methylimidazole, 1,2-dimethylimidazole, 2-ethyl-4-methylimidazole, 2-ethyl-2-phenylimidazole, and 1-cyanoethyl-2-ethyl-4-methylimidazole; lewis acid complexes including boron halides such as a boron trifluoride/piperidine complex, a boron trifluoride/monoethylamine complex, a boron trifluoride/triethanolamine complex, and a boron trichloride/octylamine complex; dicyandiamide derivatives; onium salts such as an ammonium salt and a phosphonium salt; N,N-dimethyl-N'-(3-chloro-4-methylphenyl)urea, N,N-dimethyl-N'-(4-chlorophenyl)urea, N,N-dimethyl-N'-(3,4-dichlorophenyl)urea, N,N-dimethyl-N'-(3,4-dichloromethylphenyl)urea, 2,4-(N',N-dimethylureide)toluene, 1,4-bis(N',N'-dimethylureide)benzene, quaternary ammonium salts such as tri-n-butylbenzylammonium halide, tetra-n-butylammonium halide, trimethylbenzylammonium halide, and triethylbenzylammonium halide; and quaternary phosphonium salts such as tri-n-butylbenzylphosphonium halide, tetra-n-butylphosphonium halide, trimethylbenzylphosphonium halide, and triethylbenzylphosphonium halide, oxides, acetates and others of magnesium, manganese, zinc, calcium, lithium, titanium, antimony, and germanium.

The degree of polymerization of the reaction product after the mechanochemical treatment usually varies depending on the type of the raw material compound and the treatment conditions (pulverization conditions). Therefore, the production method of a high-molecular compound of the present invention can include a heating step in the case where the degree of polymerization of the obtained high-molecular compound is low. The heating step may be performed during and/or after the pulverization treatment (i.e., mechanochemical treatment). The heating step makes it possible to promote the reaction between functional groups (particularly the polymerization reaction), thereby resulting in further increased molecular weight. In the case of heating during the pulverization treatment, it is necessary to heat at a temperature at which the raw material mixture and/or the high-molecular compound to be formed does not melt. Such a temperature is, for example, from 40 to 350°C.

In the case of heating after the pulverization treatment, the heating temperature needs to be lower than the decomposition temperature of the obtained high-molecular compound. The heating temperature may be, for example, from 90 to 400°C, particularly 120 to 400°C. The heating time is not particularly limited, and may be, for example, from 0.5 to 16 hours, particularly 0.5 to 8 hours. The heating may be performed in an inert gas stream such as nitrogen, or under pressure or reduced pressure. Further, the heating may be performed by standing or while stirring.

In the production method of a high-molecular compound, the heating after the pulverization treatment may be performed in one step or in multiple steps. The heating in multiple steps means that a heating step at different heating temperatures is continuously performed twice or more, preferably 2 to 3 times. When the heating is performed in multiple steps, the heating temperature in and after the second heating step is preferably higher than the heating temperature in the preceding heating step from the viewpoint of further improving the reaction rate and the degree of polymerization. For example, the heating temperature in the second heating step is preferably higher than the heating temperature in the first heating step. In addition, for example, the heating temperature in the third heating step is preferably higher than the heating temperature in the second heating step.

[Low-molecular compound]

The low-molecular compound is an organic compound containing no repeating unit. The organic compound containing no repeating unit means that, in the structural formula of the organic compound, no structural unit that is continuously repeated twice or more times is included. The low-molecular compound is an organic compound obtained by converting two or more, particularly two to five, preferably two to three raw material compound molecules into one molecule by a reaction between functional groups.

When a low-molecular compound is produced, the reaction between functional groups is a condensation reaction, an addition reaction, or a composite reaction thereof, and the like, as described above.

Examples of the low-molecular compound that can be produced by these reactions include a diimide dicarboxylic acid-based compound, a diimide tricarboxylic acid-based compound, a diimide tetracarboxylic acid-based compound, a monoimide dicarboxylic acid-based compound, a monoimide tricarboxylic acid-based compound, an amide group-containing diimide dicarboxylic acid-based compound, an amide group-containing monoimide dicarboxylic acid-based compound, an amide group-containing monoimide tetracarboxylic acid-based compound, an ester group-containing monoimide tricarboxylic acid-based compound, a diimide dihydroxy-based compound, a diamide dicarboxylic acid-based compound, a diamide tetracarboxylic acid-based compound, a diester dicarboxylic acid compound, a diester tetracarboxylic acid-based compound, and a curable diimide-based compound.

In the present invention, the type of the obtained low-molecular compound can be controlled by selecting the type and ratio of the raw material compound. The raw material compound for producing a low-molecular compound is usually a raw material compound having one or more, particularly two to three functional groups selected from the group consisting of the above-mentioned functional groups per molecule. The molecular weight of the raw material compound is not particularly limited, and usually has a molecular weight within the above range.

(Diimide dicarboxylic acid-based compound)

A diimide dicarboxylic acid-based compound can be produced as follows. Using a tricarboxylic acid anhydride component and a diamine component, or using a tetracarboxylic dianhydride component and a monoaminomonocarboxylic acid component as the raw material compounds, a reaction between functional groups is performed by a mechanochemical effect to thereby produce an amic acid-based compound, and the amic acid-based compound is then subjected to an imidization reaction. Here, the reaction between functional groups corresponds to the reaction (A) described above. The diimide dicarboxylic acid-based compound is a compound having two imide groups and two carboxyl groups in one molecule.

In the production of a diimide dicarboxylic acid-based compound using a tricarboxylic acid anhydride component and a diamine component, the diamine component is usually used in a molar amount of about 0.5 times, for example 0.1 to 0.7 times, preferably 0.3 to 0.7 times, more preferably 0.4 to 0.6 times, even more preferably 0.45 to 0.55 times that of the tricarboxylic acid anhydride component.

The tricarboxylic acid anhydride component capable of constituting the diimide dicarboxylic acid-based compound is a tricarboxylic acid anhydride component similar to the tricarboxylic acid anhydride component capable of constituting the polyamide-imide-based compound, and specifically, includes an aromatic tricarboxylic acid anhydride component, an alicyclic tricarboxylic acid anhydride component, and an aliphatic tricarboxylic acid anhydride component that are similar to the tricarboxylic acid anhydride component capable of constituting the polyamide-imide-based compound.

From the viewpoint of heat resistance of the diimide dicarboxylic acid-based compound or, for example, a high-molecular compound obtained using such compound, the tricarboxylic acid anhydride component of the diimide dicarboxylic acid-based compound preferably contains an aromatic tricarboxylic acid anhydride component and/or an alicyclic tricarboxylic acid anhydride component, more preferably an aromatic tricarboxylic acid anhydride component.

From the viewpoint of further improving the heat resistance of the diimide dicarboxylic acid-based compound or, for example, a high-molecular compound obtained using such compound, the tricarboxylic acid anhydride component of the diimide dicarboxylic acid-based compound preferably contains only an aromatic tricarboxylic acid anhydride component and/or an alicyclic tricarboxylic acid anhydride component, more preferably only an aromatic tricarboxylic acid anhydride component.

From the viewpoint of solubility of the diimide dicarboxylic acid-based compound, among the above-mentioned tricarboxylic acid anhydride components, it is preferable to use an alicyclic tricarboxylic acid anhydride component and/or an aliphatic tricarboxylic acid anhydride component as the tricarboxylic acid anhydride component of the diimide dicarboxylic acid-based compound.

From the viewpoint of further improving the solubility of the diimide dicarboxylic acid-based compound, among the above-mentioned tricarboxylic acid anhydride components, it is preferable to use only an alicyclic tricarboxylic acid anhydride component and/or an aliphatic tricarboxylic acid anhydride component as the tricarboxylic acid anhydride component of the diimide dicarboxylic acid-based compound.

From the viewpoint of non-coloring properties of the diimide dicarboxylic acid-based compound or, for example, a high-molecular compound obtained using such compound, among the above-mentioned tricarboxylic acid anhydride components, the tricarboxylic acid anhydride component of the diimide dicarboxylic acid-based compound preferably contains an alicyclic tricarboxylic acid anhydride component and/or an aliphatic tricarboxylic acid anhydride component, more preferably an alicyclic tricarboxylic acid anhydride component.

From the viewpoint of further improving the non-coloring properties of the diimide dicarboxylic acid-based compound or, for example, a high-molecular compound obtained using such compound, among the above-mentioned tricarboxylic acid anhydride components, the tricarboxylic acid anhydride component of the diimide dicarboxylic acid-based compound preferably contains only an alicyclic tricarboxylic acid anhydride component and/or an aliphatic tricarboxylic acid anhydride component, more preferably only an alicyclic tricarboxylic acid anhydride component.

From the viewpoint of versatility, among the above-mentioned tricarboxylic acid anhydride components, the tricarboxylic acid anhydride component of the diimide dicarboxylic acid-based compound preferably contains one or more compounds selected from the group (hereinafter referred to as Group L1) consisting of trimellitic anhydride and 1,2,4-cyclohexanetricarboxylic anhydride.

From the viewpoint of further improving the versatility, among the above-mentioned tricarboxylic acid anhydride components, the tricarboxylic acid anhydride component of the diimide dicarboxylic acid-based compound preferably contains only one or more compounds selected from the above-mentioned Group L1.

The diamine component capable of constituting the diimide dicarboxylic acid-based compound is a diamine component similar to the diamine component capable of constituting the polyamic acid-based compound and the like, and specifically, includes an aromatic diamine component, an alicyclic diamine component, and an aliphatic diamine component that are similar to the diamine component capable of constituting the polyamic acid-based compound and the like.

From the viewpoint of heat resistance of the diimide dicarboxylic acid-based compound or, for example, a high-molecular compound obtained using such compound, the diamine component of the diimide dicarboxylic acid-based compound preferably contains an aromatic diamine component and/or an alicyclic diamine component, more preferably an aromatic diamine component.

From the viewpoint of further improving the heat resistance of the diimide dicarboxylic acid-based compound or, for example, a high-molecular compound obtained using such compound, the diamine component of the diimide dicarboxylic acid-based compound preferably contains only an aromatic diamine component and/or an alicyclic diamine component, more preferably only an aromatic diamine component.

From the viewpoint of solubility of the diimide dicarboxylic acid-based compound, among the above-mentioned diamine components, it is preferable to use a diamine component having an ether group, a thioether group, a sulfonyl group, a sulfonic acid group, a methyl group, a methylene group, an isopropylidene group, a phenyl group, a fluorene structure, a halogen atom (or a halogen atom-containing substituent), or a siloxane bond, as the diamine component of the diimide dicarboxylic acid-based compound.

From the viewpoint of further improving the solubility of the diimide dicarboxylic acid-based compound, among the above-mentioned diamine components, it is preferable to use only a diamine component having an ether group, a thioether group, a sulfonyl group, a sulfonic acid group, a methyl group, a methylene group, an isopropylidene group, a phenyl group, a fluorene structure, a halogen atom (or a halogen atom-containing substituent), or a siloxane bond, as the diamine component of the diimide dicarboxylic acid-based compound.

From the viewpoint of non-coloring properties of the diimide dicarboxylic acid-based compound or, for example, a high-molecular compound obtained using such compound, among the above-mentioned diamine components, the diamine component of the diimide dicarboxylic acid-based compound preferably contains an alicyclic diamine component and/or an aliphatic diamine component.

From the viewpoint of further improving the non-coloring properties of the diimide dicarboxylic acid-based compound or, for example, a high-molecular compound obtained using such compound, among the above-mentioned diamine components, the diamine component of the diimide dicarboxylic acid-based compound preferably contains only an alicyclic diamine component and/or an aliphatic diamine component.

From the viewpoint of versatility, among the above-mentioned diamine components, the diamine component of the diimide dicarboxylic acid-based compound preferably contains one or more compounds selected from the group (hereinafter referred to as Group L2) consisting of 4,4'-diaminodiphenyl ether, p-phenylenediamine, m-phenylenediamine, 3,4'-diaminodiphenyl ether, 4,4'-diaminodiphenylmethane, 2,2'-bis(trifluoromethyl)-4,4'-diaminobiphenyl, 2,2-bis[4-(4-aminophenoxy)phenyl]-1,1,1,3,3,3-hexafluoropropane, 1,4-bis(4-aminophenoxy)benzene, 1,3-bis(4-aminophenoxy)benzene, 4,4'-bis(4-aminophenoxy)biphenyl, 4,4'-bis(3-aminophenoxy)biphenyl, 2,2-bis[4-(4-aminophenoxy)phenyl]propane, bis(4-aminophenyl)sulfone, bis[4-(4-aminophenoxy)phenyl]sulfone, 9,9-bis(4-aminophenyl)fluorene, trans-1,4-cyclohexanediamine, cis-1,4-cyclohexanediamine, 4,4'-methylenebis(cyclohexylamine), 1,10-diaminodecane, 1,12-diaminododecane, m-xylylene diamine, p-xylylene diamine, 1,4-bis(aminomethyl)cyclohexane, and dimer diamine.

From the viewpoint of further improving the versatility, among the above-mentioned diamine components, the diamine component of the diimide dicarboxylic acid-based compound preferably contains only one or more compounds selected from the above-mentioned Group L2.

In the production of a diimide dicarboxylic acid-based compound using a tetracarboxylic dianhydride component and a monoaminomonocarboxylic acid component, the monoaminomonocarboxylic acid component is usually used in an amount of about 2 times the molar amount of the tetracarboxylic dianhydride component, for example 1.5 to 10.0 times, preferably 1.8 to 2.2 times, more preferably 1.9 to 2.1 times, even more preferably 1.95 to 2.05 times.

The tetracarboxylic dianhydride component capable of constituting the diimide dicarboxylic acid-based compound is a tetracarboxylic dianhydride component similar to the tetracarboxylic dianhydride component capable of constituting the polyamic acid-based compound and the like, and specifically, includes an aromatic tetracarboxylic dianhydride component, an alicyclic tetracarboxylic dianhydride component, and an aliphatic tetracarboxylic dianhydride component that are similar to the tetracarboxylic dianhydride component capable of constituting the polyamic acid-based compound and the like.

From the viewpoint of heat resistance of the diimide dicarboxylic acid-based compound or, for example, a high-molecular compound obtained using such compound, the tetracarboxylic dianhydride component of the diimide dicarboxylic acid-based compound preferably contains an aromatic tetracarboxylic dianhydride component and/or an alicyclic tetracarboxylic dianhydride component.

From the viewpoint of further improving the heat resistance of the diimide dicarboxylic acid-based compound or, for example, a high-molecular compound obtained using such compound, the tetracarboxylic dianhydride component of the diimide dicarboxylic acid-based compound preferably contains only an aromatic tetracarboxylic dianhydride component and/or an alicyclic tetracarboxylic dianhydride component.

From the viewpoint of solubility of the diimide dicarboxylic acid-based compound, among the above-mentioned tetracarboxylic dianhydride components, it is preferable to use a tetracarboxylic dianhydride component having an ether group, a thioether group, a sulfonyl group, a ketone group, a methyl group, a methylene group, an isopropylidene group, a phenyl group, a fluorene structure, or a halogen atom (or a halogen atom-containing substituent), as the tetracarboxylic dianhydride component of the diimide dicarboxylic acid-based compound.

From the viewpoint of further improving the solubility of the diimide dicarboxylic acid-based compound, among the above-mentioned tetracarboxylic dianhydride components, it is preferable to use only a tetracarboxylic dianhydride component having an ether group, a thioether group, a sulfonyl group, a ketone group, a methyl group, a methylene group, an isopropylidene group, a phenyl group, a fluorene structure, or a halogen atom (or a halogen atom-containing substituent), as the tetracarboxylic dianhydride component of the diimide dicarboxylic acid-based compound.

From the viewpoint of heat resistance and non-coloring properties of the diimide dicarboxylic acid-based compound or, for example, a high-molecular compound obtained using such compound, among the above-mentioned tetracarboxylic dianhydride components, the tetracarboxylic dianhydride component of the diimide dicarboxylic acid-based compound preferably contains an aromatic tetracarboxylic dianhydride component and/or an alicyclic tetracarboxylic dianhydride component.

From the viewpoint of further improving the heat resistance and non-coloring properties of the diimide dicarboxylic acid-based compound or, for example, a high-molecular compound obtained using such compound, among the above-mentioned tetracarboxylic dianhydride components, the tetracarboxylic dianhydride component of the diimide dicarboxylic acid-based compound preferably contains only an aromatic tetracarboxylic dianhydride component and/or an alicyclic tetracarboxylic dianhydride component.

From the viewpoint of versatility, among the above-mentioned tetracarboxylic dianhydride components, the tetracarboxylic dianhydride component of the diimide dicarboxylic acid-based compound preferably contains one or more compounds selected from the group (hereinafter referred to as Group L3) consisting of pyromellitic dianhydride, 3,3',4,4'-biphenyltetracarboxylic dianhydride, 2,3,3',4'-biphenyltetracarboxylic dianhydride, 3,3',4,4'-benzophenone tetracarboxylic dianhydride, 4,4'-oxydiphthalic anhydride, 4,4'-(4,4'-isopropylidenediphenoxy)diphthalic anhydride, 3,3',4,4'-diphenylsulfone tetracarboxylic dianhydride, 4,4'-(hexafluoroisopropylidene)diphthalic anhydride, 1,2,3,4-cyclobutanetetracarboxylic dianhydride, cyclohexane-1,2,4,5-tetracarboxylic dianhydride, and 1,2,3,4-butanetetracarboxylic dianhydride.

From the viewpoint of further improving the versatility, among the above-mentioned tetracarboxylic dianhydride components, the tetracarboxylic dianhydride component of the diimide dicarboxylic acid-based compound preferably contains only one or more compounds selected from the above-mentioned Group L3.

The monoaminomonocarboxylic acid component capable of constituting the diimide dicarboxylic acid-based compound includes an aromatic monoaminomonocarboxylic acid component containing an aromatic ring, an alicyclic monoaminomonocarboxylic acid component containing an aliphatic ring but not an aromatic ring, and an aliphatic monoaminomonocarboxylic acid component that does not contain an aromatic ring or an alicyclic ring. The monoaminomonocarboxylic acid component may contain an ether group and/or a thioether group, and/or one or more hydrogen atoms may be substituted with a halogen atom (e.g., fluorine atom, chlorine atom, and bromine atom).

Examples of the aromatic monoaminomonocarboxylic acid component include phenylalanine, tryptophan, thyroxine, tyrosine, diiodotyrosine, 2-aminobenzoic acid, 3-aminobenzoic acid, 4-aminobenzoic acid, 2-amino-3-methylbenzoic acid, 2-amino-4-methylbenzoic acid, 2-amino-5-methylbenzoic acid, 2-amino-6-methylbenzoic acid, 3-amino-2-methylbenzoic acid, 3-amino-4-methylbenzoic acid, 4-amino-2-methylbenzoic acid, 4-amino-3-methylbenzoic acid, 5-amino-2-methylbenzoic acid, 2-amino-3,4-dimethylbenzoic acid, 2-amino-4,5-dimethylbenzoic acid, 2-amino-4-methoxybenzoic acid, 3-amino-4-methoxybenzoic acid, and 4-amino-2-methoxybenzoic acid. One of these may be used alone, or two or more thereof may be used as a mixture.

Examples of the alicyclic monoaminomonocarboxylic acid component include 4-aminocyclohexanecarboxylic acid, 3-aminocyclohexanecarboxylic acid, 1-aminocyclohexanecarboxylic acid, 2-cyclohexylglycine, 3-cyclohexylalanine, 2-aminocyclohexanecarboxylic acid, 4-(aminomethyl)cyclohexanecarboxylic acid, gabapetin, 1-aminocyclopentanecarboxylic acid, and 1-aminocyclobutanecarboxylic acid. One of these may be used alone, or two or more thereof may be used as a mixture.

Examples of the aliphatic monoaminomonocarboxylic acid component include glycine, alanine, valine, norvaline, $\alpha$-aminobutyric acid, $\gamma$-aminobutyric acid, $\beta$-alanine, serine, leucine, norleucine, isoleucine, threonine, proline, hydroxyproline, methionine, cystine, cysteine, 5-aminopentanoic acid, 6-aminocaproic acid, 7-aminoheptanoic acid, 9-nonanonic acid, 11-aminoundecanoic acid, 12-aminolauric acid, and 17-aminoheptadecanonic acid. One of these may be used alone, or two or more thereof may be used as a mixture.

From the viewpoint of heat resistance of the diimide dicarboxylic acid-based compound or, for example, a high-molecular compound obtained using such compound, the monoaminomonocarboxylic acid component of the diimide dicarboxylic acid-based compound preferably contains an aromatic monoaminomonocarboxylic acid component and/or an alicyclic monoaminomonocarboxylic acid component, more preferably an aromatic monoaminomonocarboxylic acid component.

From the viewpoint of heat resistance of the diimide dicarboxylic acid-based compound or, for example, a high-molecular compound obtained using such compound, the monoaminomonocarboxylic acid component of the diimide dicarboxylic acid-based compound preferably contains only an aromatic monoaminomonocarboxylic acid component and/or an alicyclic monoaminomonocarboxylic acid component, more preferably only an aromatic monoaminomonocarboxylic acid component.

From the viewpoint of versatility, among the above-mentioned monoaminomonocarboxylic acid components, the monoaminomonocarboxylic acid component of the diimide dicarboxylic acid-based compound preferably contains one or more compounds selected from the group (hereinafter referred to as Group L4) consisting of glycine, alanine, valine, norvaline, $\alpha$-aminobutyric acid, $\gamma$-aminobutyric acid, $\beta$-alanine, serine, leucine, isoleucine, threonine, proline, hydroxyproline, methionine, cysteine, 5-aminopentanoic acid, 6-aminocaproic acid, 7-aminoheptanoic acid, 9-nonanonic acid, 11-aminoundecanoic acid, 12-aminolauric acid, 17-aminoheptadecanonic acid, phenylalanine, tryptophan, 2-aminobenzoic acid, 3-aminobenzoic acid, 4-aminobenzoic acid, 2-amino-3-methylbenzoic acid, 2-amino-4-methylbenzoic acid, 2-amino-5-methylbenzoic acid, 2-amino-6-methylbenzoic acid, 3-amino-2-methylbenzoic acid, 3-amino-4-methylbenzoic acid, 4-amino-2-methylbenzoic acid, 4-amino-3-methylbenzoic acid, 5-amino-2-methylbenzoic acid, 2-amino-3,4-dimethylbenzoic acid, 2-amino-4,5-dimethylbenzoic acid, 2-amino-4-methoxybenzoic acid, 3-amino-4-methoxybenzoic acid, 4-amino-2-methoxybenzoic acid, 6-amino-2-naphthalenecarboxylic acid, and 3-amino-2-naphthalenecarboxylic acid.

From the viewpoint of further improving the versatility, among the above-mentioned monoaminomonocarboxylic acid components, the monoaminomonocarboxylic acid component of the diimide dicarboxylic acid-based compound preferably contains only one or more compounds selected from the above-mentioned Group L4.

(Diimide tricarboxylic acid-based compound)

A diimide tricarboxylic acid-based compound can be produced as follows. Using a tricarboxylic acid anhydride component and a diaminomonocarboxylic acid component as the raw material compounds, a reaction between functional groups is performed by a mechanochemical effect to thereby produce an amic acid-based compound, and the amic acid-based compound is then subjected to an imidization reaction. Here, the reaction between functional groups corresponds to the reaction (A) described above. The diimide tricarboxylic acid-based compound is a compound having two imide groups and three carboxyl groups in one molecule.

In the production of a diimide tricarboxylic acid-based compound using a tricarboxylic acid anhydride component and a diaminomonocarboxylic acid component, the diaminomonocarboxylic acid component is usually used in a molar amount of about 0.5 times, for example 0.1 to 0.7 times, preferably 0.3 to 0.7 times, more preferably 0.4 to 0.6 times, even more preferably 0.45 to 0.55 times that of the tricarboxylic acid anhydride component.

The tricarboxylic acid anhydride component capable of constituting the diimide tricarboxylic acid-based compound is a tricarboxylic acid anhydride component similar to the tricarboxylic acid anhydride component capable of constituting the polyamide-imide-based compound, and specifically, includes an aromatic tricarboxylic acid anhydride component, an alicyclic tricarboxylic acid anhydride component, and an aliphatic tricarboxylic acid anhydride component that are similar to the tricarboxylic acid anhydride component capable of constituting the polyamide-imide-based compound.

From the viewpoint of heat resistance of the diimide tricarboxylic acid-based compound or, for example, a high-molecular compound obtained using such compound, the tricarboxylic acid anhydride component of the diimide tricarboxylic acid-based compound preferably contains an aromatic tricarboxylic acid anhydride component and/or an alicyclic tricarboxylic acid anhydride component, more preferably an aromatic tricarboxylic acid anhydride component.

From the viewpoint of further improving the heat resistance of the diimide tricarboxylic acid-based compound or, for example, a high-molecular compound obtained using such compound, the tricarboxylic acid anhydride component of the diimide tricarboxylic acid-based compound preferably contains only an aromatic tricarboxylic acid anhydride component and/or an alicyclic tricarboxylic acid anhydride component, more preferably only an aromatic tricarboxylic acid anhydride component.

From the viewpoint of versatility, among the above-mentioned tricarboxylic acid anhydride components, the tricarboxylic acid anhydride component of the diimide tricarboxylic acid-based compound preferably contains one or more compounds selected from the group (hereinafter referred to as Group L5) consisting of trimellitic anhydride and 1,2,4-cyclohexanetricarboxylic anhydride.

From the viewpoint of further improving the versatility, among the above-mentioned tricarboxylic acid anhydride components, the tricarboxylic acid anhydride component of the diimide tricarboxylic acid-based compound preferably contains only one or more compounds selected from the above-mentioned Group L5.

The diaminomonocarboxylic acid component capable of constituting the diimide tricarboxylic acid-based compound includes an aromatic diaminomonocarboxylic acid component containing an aromatic ring, an alicyclic diaminomonocarboxylic acid component containing an aliphatic ring but not an aromatic ring, and an aliphatic diaminomonocarboxylic acid component that does not contain an aromatic ring or an alicyclic ring. The diaminomonocarboxylic acid component may contain an ether group and/or a thioether group, and/or one or more hydrogen atoms may be substituted with a halogen atom (e.g., fluorine atom, chlorine atom, and bromine atom).

Examples of the aromatic diaminomonocarboxylic acid component include 3,4-diaminobenzoic acid, 3,5-diaminobenzoic acid, 2,5-diaminobenzoic acid, and 3,5-bis(4-aminophenoxy)benzoic acid. One of these may be used alone, or two or more thereof may be used as a mixture.

Examples of the aliphatic diaminomonocarboxylic acid component include lysine, hydroxylysine, arginine, and histidine. One of these may be used alone, or two or more thereof may be used as a mixture.

From the viewpoint of heat resistance of the diimide tricarboxylic acid-based compound or, for example, a high-molecular compound obtained using such compound, the diaminomonocarboxylic acid component of the diimide tricarboxylic acid-based compound preferably contains an aromatic diaminomonocarboxylic acid component and/or an alicyclic diaminomonocarboxylic acid component, more preferably an aromatic diaminomonocarboxylic acid component.

From the viewpoint of further improving the heat resistance of the diimide tricarboxylic acid-based compound or, for example, a high-molecular compound obtained using such compound, the diaminomonocarboxylic acid component of the diimide tricarboxylic acid-based compound preferably only contains an aromatic diaminomonocarboxylic acid component and/or an alicyclic diaminomonocarboxylic acid component, more preferably only an aromatic diaminomonocarboxylic acid component.

From the viewpoint of solubility of the diimide tricarboxylic acid-based compound, among the above-mentioned diaminomonocarboxylic acid components, it is preferable to use an aliphatic diaminomonocarboxylic acid component as the diaminomonocarboxylic acid component of the diimide tricarboxylic acid-based compound.

From the viewpoint of further improving the solubility of the diimide tricarboxylic acid-based compound, among the above-mentioned diaminomonocarboxylic acid components, it is preferable to use only an aliphatic diaminomonocarboxylic acid component as the diaminomonocarboxylic acid component of the diimide tricarboxylic acid-based compound.

From the viewpoint of versatility, among the above-mentioned diaminomonocarboxylic acid components, the diaminomonocarboxylic acid component of the diimide tricarboxylic acid-based compound preferably contains one or more compounds selected from the group (hereinafter referred to as Group L6) consisting of 3,4-diaminobenzoic acid, 3,5-diaminobenzoic acid, 2,5-diaminobenzoic acid, 3,5-bis(4-aminophenoxy)benzoic acid, lysine, hydroxylysine, arginine, and histidine.

From the viewpoint of further improving the versatility, among the above-mentioned diaminomonocarboxylic acid components, the diaminomonocarboxylic acid component of the diimide tricarboxylic acid-based compound preferably contains only one or more compounds selected from the above-mentioned Group L6.

(Diimide tetracarboxylic acid-based compound)

A diimide tetracarboxylic acid-based compound can be produced as follows. Using a tetracarboxylic dianhydride component and a monoaminodicarboxylic acid component as the raw material compounds, a reaction between functional groups is performed by a mechanochemical effect to thereby produce an amic acid-based compound, and the amic acid-based compound is then subjected to an imidization reaction. Here, the reaction between functional groups corresponds to the reaction (A) described above. The diimide tetracarboxylic acid-based compound is a compound having two imide groups and four carboxyl groups in one molecule.

In the production of a diimide tetracarboxylic acid-based compound using a tetracarboxylic dianhydride component and a monoaminodicarboxylic acid component, the monoaminodicarboxylic acid component is usually used in an amount of about 2 times the molar amount of the tetracarboxylic dianhydride component, for example 1.5 to 10.0 times, preferably 1.8 to 2.2 times, more preferably 1.9 to 2.1 times, even more preferably 1.95 to 2.05 times.

The tetracarboxylic dianhydride component capable of constituting the diimide tetracarboxylic acid-based compound is a tetracarboxylic dianhydride component similar to the tetracarboxylic dianhydride component capable of constituting the polyamic acid-based compound and the like, and specifically, includes an aromatic tetracarboxylic dianhydride component, an alicyclic tetracarboxylic dianhydride component, and an aliphatic tetracarboxylic dianhydride component that are similar to the tetracarboxylic dianhydride component capable of constituting the polyamic acid-based compound and the like.

From the viewpoint of heat resistance of the diimide tetracarboxylic acid-based compound or, for example, a high-molecular compound obtained using such compound, the tetracarboxylic dianhydride component of the diimide tetracarboxylic acid-based compound preferably contains an aromatic tetracarboxylic dianhydride component.

From the viewpoint of further improving the heat resistance of the diimide tetracarboxylic acid-based compound or, for example, a high-molecular compound obtained using such compound, the tetracarboxylic dianhydride component of the diimide tetracarboxylic acid-based compound preferably contains only an aromatic tetracarboxylic dianhydride component.

From the viewpoint of solubility of the diimide tetracarboxylic acid-based compound, among the above-mentioned tetracarboxylic dianhydride components, it is preferable to use a tetracarboxylic dianhydride component having an ether group, a thioether group, a sulfonyl group, a ketone group, a methyl group, a methylene group, an isopropylidene group, a phenyl group, a fluorene structure, or a halogen atom (or a halogen atom-containing substituent), as the tetracarboxylic dianhydride component of the diimide tetracarboxylic acid-based compound.

From the viewpoint of further improving the solubility of the diimide tetracarboxylic acid-based compound, among the above-mentioned tetracarboxylic dianhydride components, it is preferable to use only a tetracarboxylic dianhydride component having an ether group, a thioether group, a sulfonyl group, a ketone group, a methyl group, a methylene group, a phenyl group, an isopropylidene group, a fluorene structure, or a halogen atom (or a halogen atom-containing substituent), as the tetracarboxylic dianhydride component of the diimide tetracarboxylic acid-based compound.

From the viewpoint of heat resistance and non-coloring properties of the diimide tetracarboxylic acid-based compound or, for example, a high-molecular compound obtained using such compound, among the above-mentioned tetracarboxylic dianhydride components, the tetracarboxylic dianhydride component of the diimide tetracarboxylic acid-based compound preferably contains an aromatic tetracarboxylic dianhydride component and/or an alicyclic tetracarboxylic dianhydride component.

From the viewpoint of heat resistance and non-coloring properties of the diimide tetracarboxylic acid-based compound or, for example, a high-molecular compound obtained using such compound, among the above-mentioned tetracarboxylic dianhydride components, the tetracarboxylic dianhydride component of the diimide tetracarboxylic acid-based compound preferably contains only an aromatic tetracarboxylic dianhydride component and/or an alicyclic tetracarboxylic dianhydride component.

From the viewpoint of versatility, among the above-mentioned tetracarboxylic dianhydride components, the tetracarboxylic dianhydride component of the diimide tetracarboxylic acid-based compound preferably contains one or more compounds selected from the group (hereinafter referred to as Group L7) consisting of pyromellitic dianhydride, 3,3',4,4'-biphenyltetracarboxylic dianhydride, 2,3,3',4'-biphenyltetracarboxylic dianhydride, 3,3',4,4'-benzophenone tetracarboxylic dianhydride, 4,4'-oxydiphthalic anhydride, 4,4'-(4,4'-isopropylidenediphenoxy)diphthalic anhydride, 3,3',4,4'-diphenylsulfone tetracarboxylic dianhydride, 4,4'-(hexafluoroisopropylidene)diphthalic anhydride, 1,2,3,4-cyclobutanetetracarboxylic dianhydride, cyclohexane-1,2,4,5-tetracarboxylic dianhydride, and 1,2,3,4-butanetetracarboxylic dianhydride.

From the viewpoint of further improving the versatility, among the above-mentioned tetracarboxylic dianhydride components, the tetracarboxylic dianhydride component of the diimide tetracarboxylic acid-based compound preferably contains only one or more compounds selected from the above-mentioned Group L7.

The monoaminodicarboxylic acid component capable of constituting the diimide tetracarboxylic acid-based compound includes an aromatic monoaminodicarboxylic acid component containing an aromatic ring, an alicyclic monoaminodicarboxylic acid component containing an aliphatic ring but not an aromatic ring, and an aliphatic monoaminodicarboxylic acid component that does not contain an aromatic ring or an alicyclic ring. The monoaminodicarboxylic acid component may contain an ether group and/or a thioether group, and/or one or more hydrogen atoms may be substituted with a halogen atom (e.g., fluorine atom, chlorine atom, and bromine atom).

Examples of the aromatic monoaminodicarboxylic acid component include 2-aminoterephthalic acid, 2-aminoisophthalic acid, 4-aminoisophthalic acid, 5-aminoisophthalic acid, 3-aminophthalic acid, 4-aminophthalic acid, 3-amino-1,2-dicarboxynaphthalene, 4-amino-1,2-dicarboxynaphthalene, 5-amino-1,2-dicarboxynaphthalene, 6-amino-1,2-dicarboxynaphthalene, 7-amino-1,2-dicarboxynaphthalene, 8-amino-1,2-dicarboxynaphthalene, 1-amino-2,3-dicarboxynaphthalene, 4-amino-2,3-dicarboxynaphthalene, 5-amino-2,3-dicarboxynaphthalene, 6-amino-2,3-dicarboxynaphthalene, 7-amino-2,3-dicarboxynaphthalene, and 8-amino-2,3-dicarboxynaphthalene. One of these may be used alone, or two or more thereof may be used as a mixture.

Examples of the aliphatic monoaminodicarboxylic acid component include glutamic acid, aspartic acid, 2-aminopimelic acid, $\alpha$-amino-$\gamma$-oxypimelic acid, 2-aminosuberic acid, 2-aminoadipic acid, $\alpha$-amino-$\gamma$-oxyadipic acid, $\alpha$-aminosebacic acid, carbocysteine, aminomalonic acid, $\alpha$-amino-$\alpha$-methylsuccinic acid, $\beta$-oxyglutamic acid, $\gamma$-oxyglutamic acid, $\gamma$-methylglutamic acid, $\gamma$-methyleneglutamic acid, and $\gamma$-methyl-$\gamma$-oxyglutamic acid. One of these may be used alone, or two or more thereof may be used as a mixture.

From the viewpoint of heat resistance of the diimide tetracarboxylic acid-based compound or, for example, a high-molecular compound obtained using such compound, the monoaminodicarboxylic acid component of the diimide tetracarboxylic acid-based compound preferably contains an aromatic monoaminodicarboxylic acid component and/or an alicyclic monoaminodicarboxylic acid component, more preferably an aromatic monoaminodicarboxylic acid component.

From the viewpoint of further improving the heat resistance of the diimide tetracarboxylic acid-based compound or, for example, a high-molecular compound obtained using such compound, the monoaminodicarboxylic acid component of the diimide tetracarboxylic acid-based compound preferably contains only an aromatic monoaminodicarboxylic acid component and/or an alicyclic monoaminodicarboxylic acid component, more preferably only an aromatic monoaminodicarboxylic acid component.

From the viewpoint of versatility, among the above-mentioned monoaminodicarboxylic acid components, the monoaminodicarboxylic acid component of the diimide tetracarboxylic acid-based compound preferably contains one or more compounds selected from the group (hereinafter referred to as Group L8) consisting of 2-aminoterephthalic acid, 2-aminoisophthalic acid, 4-aminoisophthalic acid, 5-aminoisophthalic acid, 3-aminophthalic acid, 4-aminophthalic acid, glutamic acid, aspartic acid, 2-aminopimelic acid, 2-aminosberic acid, 2-aminoadipic acid, $\alpha$-aminosebacic acid, and aminomalonic acid.

From the viewpoint of further improving the versatility, among the above-mentioned monoaminodicarboxylic acid components, the monoaminodicarboxylic acid component of the diimide tetracarboxylic acid-based compound preferably contains only one or more compounds selected from the above-mentioned Group L8.

(Monoimide dicarboxylic acid-based compound)

A monoimide dicarboxylic acid-based compound can be produced as follows. Using a tricarboxylic acid anhydride component and a monoaminomonocarboxylic acid component as the raw material compounds, a reaction between functional groups is performed by a mechanochemical effect to thereby produce an amic acid-based compound, and the amic acid-based compound is then subjected to an imidization reaction. Here, the reaction between functional groups corresponds to the reaction (A) described above. The monoimide dicarboxylic acid-based compound is a compound having one imide group and two carboxyl groups in one molecule.

In the production of a monoimide dicarboxylic acid-based compound using a tricarboxylic acid anhydride component and a monoaminomonocarboxylic acid component, the monoaminomonocarboxylic acid component is usually used in an amount of about 1 time the molar amount of the tricarboxylic acid anhydride component, for example 0.5 to 5.0 times, preferably 0.8 to 1.2 times, more preferably 0.9 to 1.1 times, even more preferably 0.95 to 1.05 times.

The tricarboxylic acid anhydride component capable of constituting the monoimide dicarboxylic acid-based compound is a tricarboxylic acid anhydride component similar to the tricarboxylic acid anhydride component capable of constituting the polyamide-imide-based compound, and specifically, includes an aromatic tricarboxylic acid anhydride component, an alicyclic tricarboxylic acid anhydride component, and an aliphatic tricarboxylic acid anhydride component that are similar to the tricarboxylic acid anhydride component capable of constituting the polyamide-imide-based compound.

From the viewpoint of heat resistance of the monoimide dicarboxylic acid-based compound or, for example, a high-molecular compound obtained using such compound, the tricarboxylic acid anhydride component of the monoimide dicarboxylic acid-based compound preferably contains an aromatic tricarboxylic acid anhydride component and/or an alicyclic tricarboxylic acid anhydride component, more preferably an aromatic tricarboxylic acid anhydride component.

From the viewpoint of further improving the heat resistance of the monoimide dicarboxylic acid-based compound or, for example, a high-molecular compound obtained using such compound, the tricarboxylic acid anhydride component of the monoimide dicarboxylic acid-based compound preferably contains only an aromatic tricarboxylic acid anhydride component and/or an alicyclic tricarboxylic acid anhydride component, more preferably only an aromatic tricarboxylic acid anhydride component.

From the viewpoint of solubility and non-coloring properties of the monoimide dicarboxylic acid-based compound, among the above-mentioned tricarboxylic acid anhydride components, it is preferable to use an alicyclic tricarboxylic acid anhydride component and/or an aliphatic tricarboxylic acid anhydride component as the tricarboxylic acid anhydride component of the monoimide dicarboxylic acid-based compound.

From the viewpoint of further improving the solubility and non-coloring properties of the monoimide dicarboxylic acid-based compound, among the above-mentioned tricarboxylic acid anhydride components, it is preferable to use only an alicyclic tricarboxylic acid anhydride component and/or an aliphatic tricarboxylic acid anhydride component as the tricarboxylic acid anhydride component of the monoimide dicarboxylic acid-based compound.

From the viewpoint of versatility, among the above-mentioned tricarboxylic acid anhydride components, the tricarboxylic acid anhydride component of the monoimide dicarboxylic acid-based compound preferably contains one or more compounds selected from the group (hereinafter referred to as Group L9) consisting of trimellitic anhydride and 1,2,4-cyclohexanetricarboxylic anhydride.

From the viewpoint of further improving the versatility, among the above-mentioned tricarboxylic acid anhydride components, the tricarboxylic acid anhydride component of the monoimide dicarboxylic acid-based compound preferably contains only one or more compounds selected from the above-mentioned Group L9.

The monoaminomonocarboxylic acid component capable of constituting the monoimide dicarboxylic acid-based compound is a monoaminomonocarboxylic acid component similar to the monoaminomonocarboxylic acid component capable of constituting the diimide dicarboxylic acid-based compound, and specifically, includes an aromatic monoaminomonocarboxylic acid component, an alicyclic monoaminomonocarboxylic acid component, and an aliphatic monoaminomonocarboxylic acid component that are similar to the monoaminomonocarboxylic acid component capable of constituting the diimide dicarboxylic acid-based compound.

From the viewpoint of heat resistance of the monoimide dicarboxylic acid-based compound or, for example, a high-molecular compound obtained using such compound, the monoaminomonocarboxylic acid component of the monoimide dicarboxylic acid-based compound preferably contains an aromatic monoaminomonocarboxylic acid component and/or an alicyclic monoaminomonocarboxylic acid component, more preferably an aromatic monoaminomonocarboxylic acid component.

From the viewpoint of further improving the heat resistance of the monoimide dicarboxylic acid-based compound or, for example, a high-molecular compound obtained using such compound, the monoaminomonocarboxylic acid component of the monoimide dicarboxylic acid-based compound preferably contains only an aromatic monoaminomonocarboxylic acid component and/or an alicyclic monoaminomonocarboxylic acid component, more preferably only an aromatic monoaminomonocarboxylic acid component.

From the viewpoint of solubility and non-coloring properties of the monoimide dicarboxylic acid-based compound, among the above-mentioned monoaminomonocarboxylic acid components, it is preferable to use an alicyclic monoaminomonocarboxylic acid component and/or an aliphatic monoaminomonocarboxylic acid component as the monoaminomonocarboxylic acid component of the monoimide dicarboxylic acid-based compound.

From the viewpoint of further improving the solubility and non-coloring properties of the monoimide dicarboxylic acid-based compound, among the above-mentioned monoaminomonocarboxylic acid components, it is preferable to use only an alicyclic monoaminomonocarboxylic acid component and/or an aliphatic monoaminomonocarboxylic acid component as the monoaminomonocarboxylic acid component of the monoimide dicarboxylic acid-based compound.

From the viewpoint of versatility, among the above-mentioned monoaminomonocarboxylic acid components, the monoaminomonocarboxylic acid component of the monoimide dicarboxylic acid-based compound preferably contains one or more compounds selected from the group (hereinafter referred to as Group L10) consisting of glycine, alanine, valine, norvaline, α-aminobutyric acid, γ-aminobutyric acid, β-alanine, serine, leucine, isoleucine, threonine, proline, hydroxyproline, methionine, cysteine, 5-aminopentanoic acid, 6-aminocaproic acid, 7-aminoheptanoic acid, 9-nonanonic acid, 11-aminoundecanoic acid, 12-aminolauric acid, 17-aminoheptadecanonic acid, phenylalanine, tryptophan, 2-aminobenzoic acid, 3-aminobenzoic acid, 4-aminobenzoic acid, 2-amino-3-methylbenzoic acid, 2-amino-4-methylbenzoic acid, 2-amino-5-methylbenzoic acid, 2-amino-6-methylbenzoic acid, 3-amino-2-methylbenzoic acid, 3-amino-4-methylbenzoic acid, 4-amino-2-methylbenzoic acid, 4-amino-3-methylbenzoic acid, 5-amino-2-methylbenzoic acid, 2-amino-3,4-dimethylbenzoic acid, 2-amino-4,5-dimethylbenzoic acid, 2-amino-4-methoxybenzoic acid, 3-amino-4-methoxybenzoic acid, 4-amino-2-methoxybenzoic acid, 6-amino-2-naphthalenecarboxylic acid, and 3-amino-2-naphthalenecarboxylic acid.

From the viewpoint of further improving the versatility, among the above-mentioned monoaminomonocarboxylic acid components, the monoaminomonocarboxylic acid component of the monoimide dicarboxylic acid-based compound preferably contains only one or more compounds selected from the above-mentioned Group L10.

(Monoimide tricarboxylic acid-based compound)

A monoimide tricarboxylic acid-based compound can be produced as follows. Using a tricarboxylic acid anhydride component and a monoaminodicarboxylic acid component as the raw material compounds, a reaction between functional groups is performed by a mechanochemical effect to thereby produce an amic acid-based compound, and the amic acid-based compound is then subjected to an imidization reaction. Here, the reaction between functional groups corresponds to the reaction (A) described above. The monoimide tricarboxylic acid-based compound is a compound having one imide group and three carboxyl groups in one molecule.

In the production of a monoimide tricarboxylic acid-based compound using a tricarboxylic acid anhydride component and a monoaminodicarboxylic acid component, the monoaminodicarboxylic acid component is usually used in an amount of about 1 time the molar amount of the tricarboxylic acid anhydride component, for example 0.5 to 5.0 times, preferably 0.8 to 1.2 times, more preferably 0.9 to 1.1 times, even more preferably 0.95 to 1.05 times.

The tricarboxylic acid anhydride component capable of constituting the monoimide tricarboxylic acid-based compound is a tricarboxylic acid anhydride component similar to the tricarboxylic acid anhydride component capable of constituting the polyamide-imide-based compound, and specifically, includes an aromatic tricarboxylic acid anhydride component, an alicyclic tricarboxylic acid anhydride component, and an aliphatic tricarboxylic acid anhydride component that are similar to the tricarboxylic acid anhydride component capable of constituting the polyamide-imide-based compound.

From the viewpoint of heat resistance of the monoimide tricarboxylic acid-based compound or, for example, a high-molecular compound obtained using such compound, the tricarboxylic acid anhydride component of the monoimide tricarboxylic acid-based compound preferably contains an aromatic tricarboxylic acid anhydride component and/or an alicyclic tricarboxylic acid anhydride component, more preferably an aromatic tricarboxylic acid anhydride component.

From the viewpoint of further improving the heat resistance of the monoimide tricarboxylic acid-based compound or, for example, a high-molecular compound obtained using such compound, the tricarboxylic acid anhydride component of the monoimide tricarboxylic acid-based compound preferably contains only an aromatic tricarboxylic acid anhydride component and/or an alicyclic tricarboxylic acid anhydride component, more preferably only an aromatic tricarboxylic acid anhydride component.

From the viewpoint of solubility and non-coloring properties of the monoimide tricarboxylic acid-based compound, among the above-mentioned tricarboxylic acid anhydride components, it is preferable to use an alicyclic tricarboxylic acid anhydride component and/or an aliphatic tricarboxylic acid anhydride component as the tricarboxylic acid anhydride component of the monoimide tricarboxylic acid-based compound.

From the viewpoint of further improving the solubility and non-coloring properties of the monoimide tricarboxylic acid-based compound, among the above-mentioned tricarboxylic acid anhydride components, it is preferable to use only an alicyclic tricarboxylic acid anhydride component and/or an aliphatic tricarboxylic acid anhydride component as the tricarboxylic acid anhydride component of the monoimide tricarboxylic acid-based compound.

From the viewpoint of versatility, among the above-mentioned tricarboxylic acid anhydride components, the tricarboxylic acid anhydride component of the monoimide tricarboxylic acid-based compound preferably contains one or more compounds selected from the group (hereinafter referred to as Group L11) consisting of trimellitic anhydride and 1,2,4-cyclohexanetricarboxylic anhydride.

From the viewpoint of further improving the versatility, among the above-mentioned tricarboxylic acid anhydride components, the tricarboxylic acid anhydride component of the monoimide tricarboxylic acid-based compound preferably contains only one or more compounds selected from the above-mentioned Group L11.

The monoaminodicarboxylic acid component capable of constituting the monoimide tricarboxylic acid-based compound is a monoaminodicarboxylic acid component similar to the monoaminodicarboxylic acid component capable of constituting the diimide tetracarboxylic acid-based compound, and specifically, includes an aromatic monoaminodicarboxylic acid component, an alicyclic monoaminodicarboxylic acid component, and an aliphatic monoaminodicarboxylic acid component that are similar to the monoaminodicarboxylic acid component capable of constituting the diimide tetracarboxylic acid-based compound.

From the viewpoint of heat resistance of the monoimide tricarboxylic acid-based compound or, for example, a high-molecular compound obtained using such compound, the monoaminodicarboxylic acid component of the monoimide tricarboxylic acid-based compound preferably contains an aromatic monoaminodicarboxylic acid component and/or an alicyclic monoaminodicarboxylic acid component, more preferably an aromatic monoaminodicarboxylic acid component.

From the viewpoint of further improving the heat resistance of the monoimide tricarboxylic acid-based compound or, for example, a high-molecular compound obtained using such compound, the monoaminodicarboxylic acid component of the monoimide tricarboxylic acid-based compound preferably contains only an aromatic monoaminodicarboxylic acid component and/or an alicyclic monoaminodicarboxylic acid component, more preferably only an aromatic monoaminodicarboxylic acid component.

From the viewpoint of versatility, among the above-mentioned monoaminodicarboxylic acid components, the monoaminodicarboxylic acid component of the monoimide tricarboxylic acid-based compound preferably contains one or more compounds selected from the group (hereinafter referred to as Group L12) consisting of 2-aminoterephthalic acid, 2-aminoisophthalic acid, 4-aminoisophthalic acid, 5-aminoisophthalic acid, 3-aminophthalic acid, 4-aminophthalic acid, glutamic acid, aspartic acid, 2-aminopimelic acid, 2-aminosberic acid, 2-aminoadipic acid, $\alpha$-aminosebacic acid, and aminomalonic acid

From the viewpoint of further improving the versatility, among the above-mentioned monoaminodicarboxylic acid components, the monoaminodicarboxylic acid component of the monoimide tricarboxylic acid-based compound preferably contains only one or more compounds selected from the above-mentioned Group L12.

(Amide group-containing diimide dicarboxylic acid-based compound)

An amide group-containing diimide dicarboxylic acid-based compound can be produced as follows. In the production method of the diimide dicarboxylic acid-based compound as described above, when using a tricarboxylic acid anhydride component and a diamine component as the raw material compounds, a reaction between functional groups is performed by a mechanochemical effect to thereby produce a diimide dicarboxylic acid-based compound, a diamine component containing an amide group as the diamine component is used to produce an amic acid-based compound, and the amic acid-based compound is then subjected to an imidization reaction. When the diimide dicarboxylic acid-based compound contains an amide group, for example, the solubility, the fluidity at the time of melting, the heat resistance, and the mechanical properties of the high-molecular compound obtained by using such diimide dicarboxylic acid-based compound can be improved. Here, the reaction between functional groups corresponds to the reaction (A) described above. The amide group-containing diimide dicarboxylic acid-based compound is a compound having one or more amide groups, two imide groups, and two carboxyl groups in one molecule.

In the production of an amide group-containing diimide dicarboxylic acid-based compound using a tricarboxylic acid anhydride component and an amide group-containing diamine component, the amide group-containing diamine component is usually used in a molar amount of about 0.5 times, for example 0.1 to 0.7 times, preferably 0.3 to 0.7 times, more preferably 0.4 to 0.6 times, even more preferably 0.45 to 0.55 times that of the tricarboxylic acid anhydride component.

The tricarboxylic acid anhydride component capable of constituting the amide group-containing diimide dicarboxylic acid-based compound is a tricarboxylic acid anhydride component similar to the tricarboxylic acid anhydride component capable of constituting the polyamide-imide-based compound, and specifically, includes an aromatic tricarboxylic acid anhydride component, an alicyclic tricarboxylic acid anhydride component, and an aliphatic tricarboxylic acid anhydride component that are similar to the tricarboxylic acid anhydride component capable of constituting the polyamide-imide-based compound.

From the viewpoint of heat resistance of the amide group-containing diimide dicarboxylic acid-based compound or, for example, a high-molecular compound obtained using such compound, the tricarboxylic acid anhydride component of the amide group-containing diimide dicarboxylic acid-based compound preferably contains an aromatic tricarboxylic acid anhydride component and/or an alicyclic tricarboxylic acid anhydride component, more preferably an aromatic tricarboxylic acid anhydride component.

From the viewpoint of further improving the heat resistance of the amide group-containing diimide dicarboxylic acid-based compound or, for example, a high-molecular compound obtained using such compound, the tricarboxylic acid anhydride component of the amide group-containing diimide dicarboxylic acid-based compound preferably contains only an aromatic tricarboxylic acid anhydride component and/or an alicyclic tricarboxylic acid anhydride component, more preferably only an aromatic tricarboxylic acid anhydride component.

From the viewpoint of solubility and non-coloring properties of the amide group-containing diimide dicarboxylic acid-based compound, among the above-mentioned tricarboxylic acid anhydride components, it is preferable to use an alicyclic tricarboxylic acid anhydride component and/or an aliphatic tricarboxylic acid anhydride component as the tricarboxylic acid anhydride component of the amide group-containing diimide dicarboxylic acid-based compound.

From the viewpoint of further improving the solubility and non-coloring properties of the monoimide tricarboxylic acid-based compound, among the above-mentioned tricarboxylic acid anhydride components, it is preferable to use only an alicyclic tricarboxylic acid anhydride component and/or an aliphatic tricarboxylic acid anhydride component as the tricarboxylic acid anhydride component of the amide group-containing diimide dicarboxylic acid-based compound.

From the viewpoint of versatility, among the above-mentioned tricarboxylic acid anhydride components, the tricarboxylic acid anhydride component of the amide group-containing diimide dicarboxylic acid-based compound preferably contains one or more compounds selected from the group (hereinafter referred to as Group L13) consisting of trimellitic anhydride and 1,2,4-cyclohexanetricarboxylic anhydride.

From the viewpoint of further improving the versatility, among the above-mentioned tricarboxylic acid anhydride components, the tricarboxylic acid anhydride component of the amide group-containing diimide dicarboxylic acid-based compound preferably contains only one or more compounds selected from the above-mentioned Group L13.

The amide group-containing diamine component capable of constituting an amide group-containing diimide dicarboxylic acid-based compound includes an amide group-containing aromatic diamine component containing an amide group and an aromatic ring, an amide group-diamine component containing an aliphatic ring but not an aromatic ring, and an amide group-containing aliphatic diamine component that does not contain an aromatic ring or an alicyclic ring. The amide group-containing diamine component may contain an ether group and/or a thioether group, and/or one or more hydrogen atoms may be substituted with a halogen atom (e.g., fluorine atom, chlorine atom, and bromine atom).

Examples of the amide group-containing aromatic diamine component include 4,4'-diaminobenzanilide. One of these may be used alone, or two or more thereof may be used as a mixture.

From the viewpoint of heat resistance of the amide group-containing diimide dicarboxylic acid-based compound or, for example, a high-molecular compound obtained using such compound, the amide group-containing diamine component of the amide group-containing diimide dicarboxylic acid-based compound preferably contains an amide group-containing aromatic diamine component and/or an amide group-containing alicyclic diamine component, more preferably an amide group-containing aromatic diamine component.

From the viewpoint of further improving the heat resistance of the amide group-containing diimide dicarboxylic acid-based compound or, for example, a high-molecular compound obtained using such compound, the amide group-containing diamine component of the amide group-containing diimide dicarboxylic acid-based compound preferably contains only an amide group-containing aromatic diamine component and/or an amide group-containing alicyclic diamine component, more preferably only an amide group-containing aromatic diamine component.

From the viewpoint of versatility, among the above-mentioned amide group-containing diamine components, the amide group-containing diamine component of the amide group-containing diimide dicarboxylic acid-based compound preferably contains one or more compounds selected from the group (hereinafter referred to as Group L14) consisting of 4,4'-diaminobenzanilide.

From the viewpoint of further improving the versatility, among the above-mentioned amide group-containing diamine components, the amide group-containing diamine component of the amide group-containing diimide dicarboxylic acid-based compound preferably contains only one or more compounds selected from the above-mentioned Group L14.

(Amide group-containing monoimide dicarboxylic acid-based compound)

A amide group-containing monoimide dicarboxylic acid-based compound can be produced as follows. Using a tricarboxylic acid anhydride halide component and a monoaminomonocarboxylic acid component as the raw material compounds, a reaction between functional groups is performed by a mechanochemical effect to thereby produce an amic acid-based compound, and the amic acid-based compound is then subjected to an imidization reaction. When the monoimide dicarboxylic acid-based compound contains an amide group, for example, the solubility, the fluidity at the time of melting, the heat resistance, and the mechanical properties of the high-molecular compound obtained by using such monoimide dicarboxylic acid-based compound can be improved. Here, the reaction between functional groups corresponds to the reactions (A) and (C) described above. The amide group-containing monoimide dicarboxylic acid-based compound is a compound having one or more amide groups, one imide group, and two carboxyl groups in one molecule.

In the production of an amide group-containing monoimide dicarboxylic acid-based compound using a tricarboxylic acid anhydride halide component and a monoaminomonocarboxylic acid component, the monoaminomonocarboxylic acid component is usually used in an amount of about 2 time the molar amount of the tricarboxylic acid anhydride halide component, for example 1.5 to 10.0 times, preferably 1.8 to 2.2 times, more preferably 1.9 to 2.1 times, even more preferably 1.95 to 2.05 times.

The tricarboxylic acid anhydride halide component capable of constituting the amide group-containing monoimide dicarboxylic acid-based compound is an acid halide of a tricarboxylic acid anhydride component similar to the tricarboxylic acid anhydride component capable of constituting the polyamide-imide-based compound, and specifically, includes acid halides of an aromatic tricarboxylic acid anhydride component, an alicyclic tricarboxylic acid anhydride component, and an aliphatic tricarboxylic acid anhydride component that are similar to the tricarboxylic acid anhydride component capable of constituting the polyamide-imide-based compound.

From the viewpoint of heat resistance of the amide group-containing monoimide dicarboxylic acid-based compound or, for example, a high-molecular compound obtained using such compound, the tricarboxylic acid anhydride halide component of the amide group-containing monoimide dicarboxylic acid-based compound preferably contains an acid halide of an aromatic tricarboxylic acid anhydride component and/or an acid halide of an alicyclic tricarboxylic acid anhydride component, more preferably an acid halide of an aromatic tricarboxylic acid anhydride component.

From the viewpoint of further improving the heat resistance of the amide group-containing monoimide dicarboxylic acid-based compound or, for example, a high-molecular compound obtained using such compound, the tricarboxylic acid anhydride halide component of the amide group-containing monoimide dicarboxylic acid-based compound preferably contains only an acid halide of an aromatic tricarboxylic acid anhydride component and/or an acid halide of an alicyclic tricarboxylic acid anhydride component, more preferably only an acid halide of an aromatic tricarboxylic acid anhydride component.

From the viewpoint of versatility, among the above-mentioned tricarboxylic acid anhydride halide components, the tricarboxylic acid anhydride halide component of the amide group-containing monoimide dicarboxylic acid-based compound preferably contains one or more compounds selected from the group (hereinafter referred to as Group L15) consisting of trimellitic anhydride chloride.

From the viewpoint of further improving the versatility, among the above-mentioned tricarboxylic acid anhydride halide components, the tricarboxylic acid anhydride halide component of the amide group-containing monoimide dicarboxylic acid-based compound preferably contains only one or more compounds selected from the above-mentioned Group L15.

The monoaminomonocarboxylic acid component capable of constituting the amide group-containing monoimide dicarboxylic acid-based compound is a monoaminomonocarboxylic acid component similar to the monoaminomonocarboxylic acid component capable of constituting the diimide dicarboxylic acid-based compound, and specifically, includes an aromatic monoaminomonocarboxylic acid component, an alicyclic monoaminomonocarboxylic acid component, and an aliphatic monoaminomonocarboxylic acid component that are similar to the monoaminomonocarboxylic acid component capable of constituting the diimide dicarboxylic acid-based compound.

From the viewpoint of heat resistance of the amide group-containing monoimide dicarboxylic acid-based compound or, for example, a high-molecular compound obtained using such compound, the monoaminomonocarboxylic acid component of the amide group-containing monoimide dicarboxylic acid-based compound preferably contains an aromatic monoaminomonocarboxylic acid component and/or an alicyclic monoaminomonocarboxylic acid component, more preferably an aromatic monoaminomonocarboxylic acid component.

From the viewpoint of further improving the heat resistance of the amide group-containing monoimide dicarboxylic acid-based compound or, for example, a high-molecular compound obtained using such compound, the monoaminomonocarboxylic acid component of the amide group-containing monoimide dicarboxylic acid-based compound preferably contains only an aromatic monoaminomonocarboxylic acid component and/or an alicyclic monoaminomonocarboxylic acid component, more preferably only an aromatic monoaminomonocarboxylic acid component.

From the viewpoint of versatility, among the above-mentioned monoaminomonocarboxylic acid components, the monoaminomonocarboxylic acid component of the amide group-containing monoimide dicarboxylic acid-based compound preferably contains one or more compounds selected from the group (hereinafter referred to as Group L16) consisting of glycine, alanine, valine, norvaline, $\alpha$-aminobutyric acid, $\gamma$-aminobutyric acid, $\beta$-alanine, serine, leucine, isoleucine, threonine, proline, hydroxyproline, methionine, cysteine, 5-aminopentanoic acid, 6-aminocaproic acid, 7-aminoheptanoic acid, 9-nonanonic acid, 11-aminoundecanoic acid, 12-aminolauric acid, 17-aminoheptadecanonic acid, phenylalanine, tryptophan, 2-aminobenzoic acid, 3-aminobenzoic acid, 4-aminobenzoic acid, 2-amino-3-methylbenzoic acid, 2-amino-4-methylbenzoic acid, 2-amino-5-methylbenzoic acid, 2-amino-6-methylbenzoic acid, 3-amino-2-methylbenzoic acid, 3-amino-4-methylbenzoic acid, 4-amino-2-methylbenzoic acid, 4-amino-3-methylbenzoic acid, 5-amino-2-methylbenzoic acid, 2-amino-3,4-dimethylbenzoic acid, 2-amino-4,5-dimethylbenzoic acid, 2-amino-4-methoxybenzoic acid, 3-amino-4-methoxybenzoic acid, 4-amino-2-methoxybenzoic acid, 6-amino-2-naphthalenecarboxylic acid, and 3-amino-2-naphthalenecarboxylic acid.

From the viewpoint of further improving the versatility, among the above-mentioned monoaminomonocarboxylic acid components, the monoaminomonocarboxylic acid component of the amide group-containing monoimide dicarboxylic acid-based compound preferably contains only one or more compounds selected from the above-mentioned Group L16.

(Amide group-containing monoimide tetracarboxylic acid-based compound)

A amide group-containing monoimide tetracarboxylic acid-based compound can be produced as follows. Using a tricarboxylic acid anhydride halide component and a monoaminodicarboxylic acid component as the raw material compounds, a reaction between functional groups is performed by a mechanochemical effect to thereby produce an amic acid-based compound, and the amic acid-based compound is then subjected to an imidization reaction. When the monoimide tetracarboxylic acid-based compound contains an amide group, for example, the solubility, the fluidity at the time of melting, the heat resistance, and the mechanical properties of the high-molecular compound obtained by using such monoimide dicarboxylic acid-based compound can be improved. Here, the reaction between functional groups corresponds to the reactions (A) and (C) described above. The amide group-containing monoimide tetracarboxylic acid-based compound is a compound having one or more amide groups, one imide group, and four carboxyl groups in one molecule.

In the production of an amide group-containing monoimide tetracarboxylic acid-based compound using a tricarboxylic acid anhydride halide component and a monoaminodicarboxylic acid component, the monoaminodicarboxylic acid component is usually used in an amount of about 2 time the molar amount of the tricarboxylic acid anhydride halide component, for example 1.5 to 10.0 times, preferably 1.8 to 2.2 times, more preferably 1.9 to 2.1 times, even more preferably 1.95 to 2.05 times.

The tricarboxylic acid anhydride halide component capable of constituting the amide group-containing monoimide tetracarboxylic acid-based compound is an acid halide of a tricarboxylic acid anhydride component similar to the tricarboxylic acid anhydride component capable of constituting the polyamide-imide-based compound, and specifically, includes acid halides of an aromatic tricarboxylic acid anhydride component, an alicyclic tricarboxylic acid anhydride component, and an aliphatic tricarboxylic acid anhydride component that are similar to the tricarboxylic acid anhydride component capable of constituting the polyamide-imide-based compound.

From the viewpoint of heat resistance of the amide group-containing monoimide tetracarboxylic acid-based compound or, for example, a high-molecular compound obtained using such compound, the tricarboxylic acid anhydride halide component of the amide group-containing monoimide tetracarboxylic acid-based compound preferably contains an acid halide of an aromatic tricarboxylic acid anhydride component and/or an acid halide of an alicyclic tricarboxylic acid anhydride component, more preferably an acid halide of an aromatic tricarboxylic acid anhydride component.

From the viewpoint of further improving the heat resistance of the amide group-containing monoimide tetracarboxylic acid-based compound or, for example, a high-molecular compound obtained using such compound, the tricarboxylic acid anhydride halide component of the amide group-containing monoimide tetracarboxylic acid-based compound preferably contains only an acid halide of an aromatic tricarboxylic acid anhydride component and/or an acid halide of an alicyclic tricarboxylic acid anhydride component, more preferably only an acid halide of an aromatic tricarboxylic acid anhydride component.

From the viewpoint of versatility, among the above-mentioned tricarboxylic acid anhydride halide components, the tricarboxylic acid anhydride halide component of the amide group-containing monoimide tetracarboxylic acid-based compound preferably contains one or more compounds selected from the group (hereinafter referred to as Group L17) consisting of trimellitic anhydride chloride.

From the viewpoint of further improving the versatility, among the above-mentioned tricarboxylic acid anhydride halide components, the tricarboxylic acid anhydride halide component of the amide group-containing monoimide tetracarboxylic acid-based compound preferably contains only one or more compounds selected from the above-mentioned Group L17.

The monoaminodicarboxylic acid component capable of constituting the amide group-containing monoimide tetracarboxylic acid-based compound is a monoaminodicarboxylic acid component similar to the monoaminodicarboxylic acid component capable of constituting the diimide tetracarboxylic acid-based compound, and specifically, includes an aromatic monoaminodicarboxylic acid component, an alicyclic monoaminodicarboxylic acid component, and an aliphatic monoaminodicarboxylic acid component that are similar to the monoaminodicarboxylic acid component capable of constituting the diimide tetracarboxylic acid-based compound.

From the viewpoint of heat resistance of the amide group-containing monoimide tetracarboxylic acid-based compound or, for example, a high-molecular compound obtained using such compound, the monoaminodicarboxylic acid component of the amide group-containing monoimide tetracarboxylic acid-based compound preferably contains an aromatic monoaminodicarboxylic acid component and/or an alicyclic monoaminodicarboxylic acid component, more preferably an aromatic monoaminodicarboxylic acid component.

From the viewpoint of further improving the heat resistance of the amide group-containing monoimide tetracarboxylic acid-based compound or, for example, a high-molecular compound obtained using such compound, the monoaminodicarboxylic acid component of the amide group-containing monoimide tetracarboxylic acid-based compound preferably contains only an aromatic monoaminodicarboxylic acid component and/or an alicyclic monoaminodicarboxylic acid component, more preferably only an aromatic monoaminodicarboxylic acid component.

From the viewpoint of versatility, among the above-mentioned monoaminodicarboxylic acid components, the monoaminodicarboxylic acid component of the amide group-containing monoimide tetracarboxylic acid-based compound preferably contains one or more compounds selected from the group (hereinafter referred to as Group L18) consisting of 2-aminoterephthalic acid, 2-aminoisophthalic acid, 4-aminoisophthalic acid, 5-aminoisophthalic acid, 3-aminophthalic acid, 4-aminophthalic acid, glutamic acid, aspartic acid, 2-aminopimelic acid, 2-aminosberic acid, 2-aminoadipic acid, α-aminosebacic acid, and aminomalonic acid.

From the viewpoint of further improving the versatility, among the above-mentioned monoaminodicarboxylic acid components, the monoaminodicarboxylic acid component of the amide group-containing monoimide tetracarboxylic acid-based compound preferably contains only one or more compounds selected from the above-mentioned Group L18.

(Ester group-containing monoimide tricarboxylic acid-based compound)

An ester group-containing monoimide tricarboxylic acid-based compound can be produced as follows. Using a tricarboxylic acid anhydride component and a monohydroxy monoamine component as the raw material compounds, a reaction between functional groups is performed by a mechanochemical effect to thereby produce an amic acid-based compound, and the amic acid-based compound is then subjected to an imidization reaction. When the monoimide tricarboxylic acid-based compound contains an ester group, for example, the solubility, the fluidity at the time of melting, the heat resistance, the low water absorption properties, and the mechanical properties of the high-molecular compound obtained by using such monoimide tricarboxylic acid-based compound can be improved. Here, the reaction between functional groups corresponds to the reactions (A) and (G) described above. The ester group-containing monoimide tricarboxylic acid-based compound is a compound having one or more ester groups, one imide group, and three carboxyl groups in one molecule.

In the production of an ester group-containing monoimide tricarboxylic acid-based compound using a tricarboxylic acid anhydride component and a monohydroxy monoamine component, the monohydroxy monoamine component is usually used in a molar amount of about 0.5 times, for example 0.1 to 0.7 times, preferably 0.3 to 0.7 times, more preferably 0.4 to 0.6 times, even more preferably 0.45 to 0.55 times that of the tricarboxylic acid anhydride component.

The tricarboxylic acid anhydride component capable of constituting the ester group-containing monoimide tricarboxylic acid-based compound is a tricarboxylic acid anhydride component similar to the tricarboxylic acid anhydride component capable of constituting the polyamide-imide-based compound, and specifically, includes an aromatic tricarboxylic acid anhydride component, an alicyclic tricarboxylic acid anhydride component, and an aliphatic tricarboxylic acid anhydride component that are similar to the tricarboxylic acid anhydride component capable of constituting the polyamide-imide-based compound.

From the viewpoint of heat resistance of the ester group-containing monoimide tricarboxylic acid-based compound or, for example, a high-molecular compound obtained using such compound, the tricarboxylic acid anhydride component of the ester group-containing monoimide tricarboxylic acid-based compound preferably contains an aromatic tricarboxylic acid anhydride component and/or an alicyclic tricarboxylic acid anhydride component, more preferably an aromatic tricarboxylic acid anhydride component.

From the viewpoint of further improving the heat resistance of the ester group-containing monoimide tricarboxylic acid-based compound or, for example, a high-molecular compound obtained using such compound, the tricarboxylic acid anhydride component of the ester group-containing monoimide tricarboxylic acid-based compound preferably contains only an aromatic tricarboxylic acid anhydride component and/or an alicyclic tricarboxylic acid anhydride component, more preferably only an aromatic tricarboxylic acid anhydride component.

From the viewpoint of versatility, among the above-mentioned tricarboxylic acid anhydride components, the tricarboxylic acid anhydride component of the ester group-containing monoimide tricarboxylic acid-based compound preferably contains one or more compounds selected from the group (hereinafter referred to as Group L19) consisting of trimellitic anhydride and 1,2,4-cyclohexanetricarboxylic anhydride.

From the viewpoint of further improving the versatility, among the above-mentioned tricarboxylic acid anhydride components, the tricarboxylic acid anhydride component of the ester group-containing monoimide tricarboxylic acid-based compound preferably contains only one or more compounds selected from the above-mentioned Group L19.

The monohydroxy monoamine component capable of constituting the ester group-containing monoimide tricarboxylic acid-based compound includes an aromatic monohydroxy monoamine component containing an aromatic ring, an alicyclic monohydroxy monoamine component containing an aliphatic ring but not an aromatic ring, and an aliphatic monohydroxy monoamine component that does not contain an aromatic ring or an alicyclic ring. The monohydroxy monoamine component may contain an ether group and/or a thioether group, and/or one or more hydrogen atoms may be substituted with a halogen atom (e.g., fluorine atom, chlorine atom, and bromine atom).

Examples of the aromatic monohydroxy monoamine component include 2-aminophenol, 3-aminophenol, 4-aminophenol, 2-amino-4-methylphenol, 2-amino-5-methylphenol, 3-amino-2-methylphenol, 3-amino-4-methylphenol, 4-amino-2-methylphenol, 5-amino-2-methylphenol, 4-amino-2-methoxyphenol, 3-hydroxy-4-methoxyaniline, 4-amino-3,5-xylenol, 2-aminobenzylalcohol, 3-aminobenzylalcohol, 4-aminobenzylalcohol, 2-(4-aminophenyl)ethanol, 2-amino-4-phenylphenol, and 4-amino-2,6-diphenylphenol. One of these may be used alone, or two or more thereof may be used as a mixture.

Examples of the alicyclic monohydroxy monoamine component include 1-amino-1-cyclopentanemethanol, 2-aminocyclohexanol, 4-aminocyclohexanol, 4-(2-aminoethyl)cyclohexanol, 1-aminomethyl-1-cyclohexanol, 3-aminomethyl-3,5,5-trimethylcyclohexanol, and 4-aminocyclohexaneethanol. One of these may be used alone, or two or more thereof may be used as a mixture.

Examples of the aliphatic monohydroxy monoamine component include ethanolamine, 2-amino-1-propanol, 3-amino-1-propanol, 3-amino-2,2-dimethyl-1-propanol, 2-amino-2-methyl-1-propanol, 2-amino-1-butanol, 3-amino-1-butanol, 4-amino-1-butanol, 4-amino-2-methyl-1-butanol, 5-amino-1-pentanol, 6-amino-1-hexanol, 3-pyrrolidinol, 2-pyrrolidinemethanol, 2-piperidinemethanol, 3-piperidinemethanol, 3-hydroxypiperidine, 4-hydroxypiperidine, piperazinethanol, 4-amino-1-piperazinethanol, 8-amino-1-octanol, 10-amino-1-decanol, and 12-amino-1-dodecanol. One of these may be used alone, or two or more thereof may be used as a mixture.

From the viewpoint of heat resistance of the ester group-containing monoimide tricarboxylic acid-based compound or, for example, a high-molecular compound obtained using such compound, the monohydroxy monoamine component of the ester group-containing monoimide tricarboxylic acid-based compound preferably contains an aromatic monohydroxy monoamine component and/or an alicyclic monohydroxy monoamine component, more preferably an aromatic monohydroxy monoamine component.

From the viewpoint of further improving the heat resistance of the ester group-containing monoimide tricarboxylic acid-based compound or, for example, a high-molecular compound obtained using such compound, the monohydroxy monoamine component of the ester group-containing monoimide tricarboxylic acid-based compound preferably contains only an aromatic monohydroxy monoamine component and/or an alicyclic monohydroxy monoamine component, more preferably only an aromatic monohydroxy monoamine component.

From the viewpoint of versatility, among the above-mentioned monohydroxy monoamine components, the monohydroxy monoamine component of the ester group-containing monoimide tricarboxylic acid-based compound preferably contains one or more compounds selected from the group (hereinafter referred to as Group L20) consisting of ethanolamine, 2-amino-1-propanol, 3-amino-1-propanol, 3-amino-2,2-dimethyl-1-propanol, 2-amino-1-butanol, 3-amino-1-butanol, 4-amino-1-butanol, 4-amino-2-methyl-1-butanol, 5-amino-1-pentanol, 6-amino-1-hexanol, 8-amino-1-octanol, 10-amino-1-decanol, 12-amino-1-dodecanol, 2-aminocyclohexanol, 4-aminocyclohexanol, 2-aminophenol, 3-aminophenol, 4-aminophenol, 2-amino-4-methylphenol, 2-amino-5-methylphenol, 3-amino-2-methylphenol, 3-amino-4-methylphenol, 4-amino-2-methylphenol, 5-amino-2-methylphenol, 4-amino-2-methoxyphenol, 3-hydroxy-4-methoxyaniline, 4-amino-3,5-xylenol, 2-aminobenzylalcohol, 3-aminobenzylalcohol, 4-aminobenzylalcohol, 2-(4-aminophenyl)ethanol, 2-amino-4-phenylphenol, and 4-amino-2,6-diphenylphenol.

From the viewpoint of further improving the versatility, among the above-mentioned monohydroxy monoamine components, the monohydroxy monoamine component of the ester group-containing monoimide tricarboxylic acid-based compound preferably contains only one or more compounds selected from the above-mentioned Group L20.

(Diimide dihydroxy-based compound)

A diimide dihydroxy-based compound (e.g., diimide diphenol-based compound) can be produced as follows. Using a tetracarboxylic dianhydride component and a monohydroxy monoamine component as the raw material compounds, a reaction between functional groups is performed by a mechanochemical effect to thereby produce an amic acid-based compound, and the amic acid-based compound is then subjected to an imidization reaction. Here, the reaction between functional groups corresponds to the reaction (A) described above. The diimide dihydroxy-based compound is a compound having two imide groups and two hydroxyl groups in one molecule, and preferably includes a diimide diphenol-based compound having two imide groups and two phenolic hydroxyl groups in one molecule.

In the production of a diimide dihydroxy-based compound (e.g., diimide diphenol-based compound) using a tetracarboxylic dianhydride component and a monohydroxy monoamine component, the monohydroxy monoamine component is usually used in an amount of about 2 times the molar amount of the tetracarboxylic dianhydride component, for example 1.5 to 10.0 times, preferably 1.8 to 2.2 times, preferably 1.9 to 2.1 times, more preferably 1.95 to 2.05 times.

The tetracarboxylic dianhydride component capable of constituting the diimide dihydroxy-based compound is a tetracarboxylic dianhydride component similar to the tetracarboxylic dianhydride component capable of constituting the polyamic acid-based compound and the like, and specifically, includes an aromatic tetracarboxylic dianhydride component, an alicyclic tetracarboxylic dianhydride component, and an aliphatic tetracarboxylic dianhydride component that are similar to the tetracarboxylic dianhydride component capable of constituting the polyamic acid-based compound and the like.

From the viewpoint of heat resistance of the diimide dihydroxy-based compound or, for example, a high-molecular compound obtained using such compound, the tetracarboxylic dianhydride component of the diimide dihydroxy-based compound preferably contains an aromatic tetracarboxylic dianhydride component.

From the viewpoint of further improving the heat resistance of the diimide dihydroxy-based compound or, for example, a high-molecular compound obtained using such compound, the tetracarboxylic dianhydride component of the diimide dihydroxy-based compound preferably contains only an aromatic tetracarboxylic dianhydride component.

From the viewpoint of solubility of the diimide dihydroxy-based compound, among the above-mentioned tetracarboxylic dianhydride components, it is preferable to use a tetracarboxylic dianhydride component having an ether group, a thioether group, a sulfonyl group, a ketone group, a methyl group, a methylene group, an isopropylidene group, a phenyl group, a fluorene structure, or a halogen atom (or a halogen atom-containing substituent), as the tetracarboxylic dianhydride component of the diimide dihydroxy-based compound.

From the viewpoint of further improving the solubility of the diimide dihydroxy-based compound, among the above-mentioned tetracarboxylic dianhydride components, it is preferable to use only a tetracarboxylic dianhydride component having an ether group, a thioether group, a sulfonyl group, a ketone group, a methyl group, a methylene group, an isopropylidene group, a phenyl group, a fluorene structure, or a halogen atom (or a halogen atom-containing substituent), as the tetracarboxylic dianhydride component of the diimide dihydroxy-based compound.

From the viewpoint of heat resistance and non-coloring properties of the diimide dihydroxy-based compound or, for example, a high-molecular compound obtained using such compound, among the above-mentioned tetracarboxylic dianhydride components, the tetracarboxylic dianhydride component of the diimide dihydroxy-based compound preferably contains an aromatic tetracarboxylic dianhydride component and/or an alicyclic tetracarboxylic dianhydride component.

From the viewpoint of further improving the heat resistance and non-coloring properties of the diimide dihydroxy-based compound or, for example, a high-molecular compound obtained using such compound, among the above-mentioned tetracarboxylic dianhydride components, the tetracarboxylic dianhydride component of the diimide dihydroxy-based compound preferably contains only an aromatic tetracarboxylic dianhydride component and/or an alicyclic tetracarboxylic dianhydride component.

From the viewpoint of versatility, among the above-mentioned tetracarboxylic dianhydride components, the tetracarboxylic dianhydride component of the diimide dihydroxy-based compound preferably contains one or more compounds selected from the group (hereinafter referred to as Group L21) consisting of pyromellitic dianhydride, 3,3',4,4'-biphenyltetracarboxylic dianhydride, 2,3,3',4'-biphenyltetracarboxylic dianhydride, 3,3',4,4'-benzophenone tetracarboxylic dianhydride, 4,4'-oxydiphthalic anhydride, 4,4'-(4,4'-isopropylidenediphenoxy)diphthalic anhydride, 3,3',4,4'-diphenylsulfone tetracarboxylic dianhydride, 4,4'-(hexafluoroisopropylidene)diphthalic anhydride, 1,2,3,4-cyclobutanetetracarboxylic dianhydride, cyclohexane-1,2,4,5-tetracarboxylic dianhydride, and 1,2,3,4-butanetetracarboxylic dianhydride.

From the viewpoint of further improving the versatility, among the above-mentioned tetracarboxylic dianhydride components, the tetracarboxylic dianhydride component of the diimide dihydroxy-based compound preferably contains only one or more compounds selected from the above-mentioned Group L21.

The monohydroxy monoamine component capable of constituting the diimide dihydroxy-based compound is a monohydroxy monoamine component similar to the monohydroxy monoamine component capable of constituting the ester group-containing monoimide tricarboxylic acid-based compound, and specifically, includes an aromatic monohydroxy monoamine component, an alicyclic monohydroxy monoamine component, and an aliphatic monohydroxy monoamine component that are similar to the monohydroxy monoamine component capable of constituting the ester group-containing monoimide tricarboxylic acid-based compound.

From the viewpoint of heat resistance of the diimide dihydroxy-based compound or, for example, a high-molecular compound obtained using such compound, the monohydroxy monoamine component of the diimide dihydroxy-based compound preferably contains an aromatic monohydroxy monoamine component and/or an alicyclic monohydroxy monoamine component, more preferably an aromatic monohydroxy monoamine component.

From the viewpoint of further improving the heat resistance of the diimide dihydroxy-based compound or, for example, a high-molecular compound obtained using such compound, the monohydroxy monoamine component of the diimide dihydroxy-based compound preferably contains only an aromatic monohydroxy monoamine component and/or an alicyclic monohydroxy monoamine component, more preferably only an aromatic monohydroxy monoamine component.

From the viewpoint of solubility and non-coloring properties of the diimide dihydroxy-based compound, among the above-mentioned monohydroxy monoamine components, it is preferable to use an alicyclic monohydroxy monoamine component and/or an aliphatic monohydroxy monoamine component as the monohydroxy monoamine component of the diimide dihydroxy-based compound.

From the viewpoint of further improving the solubility and non-coloring properties of the diimide dihydroxy-based compound, among the above-mentioned monohydroxy monoamine components, it is preferable to use only an alicyclic monohydroxy monoamine component and/or an aliphatic monohydroxy monoamine component as the monohydroxy monoamine component of the diimide dihydroxy-based compound.

From the viewpoint of versatility, among the above-mentioned monohydroxy monoamine components, the monohydroxy monoamine component of the diimide dihydroxy-based compound preferably contains one or more compounds selected from the group (hereinafter referred to as Group L22) consisting of ethanolamine, 2-amino-1-propanol, 3-amino-1-propanol, 3-amino-2,2-dimethyl-1-propanol, 2-amino-1-butanol, 3-amino-1-butanol, 4-amino-1-butanol, 4-amino-2-methyl-1-butanol, 5-amino-1-pentanol, 6-amino-1-hexanol, 8-amino-1-octanol, 10-amino-1-decanol, 12-amino-1-dodecanol, 2-aminocyclohexanol, 4-aminocyclohexanol, 2-aminophenol, 3-aminophenol, 4-aminophenol, 2-amino-4-methylphenol, 2-amino-5-methylphenol, 3-amino-2-methylphenol, 3-amino-4-methylphenol, 4-amino-2-methylphenol, 5-amino-2-methylphenol, 4-amino-2-methoxyphenol, 3-hydroxy-4-methoxyaniline, 4-amino-3,5-xylenol, 2-aminobenzylalcohol, 3-aminobenzylalcohol, 4-aminobenzylalcohol, 2-(4-aminophenyl)ethanol, 2-amino-4-phenylphenol, and 4-amino-2,6-diphenylphenol.

From the viewpoint of further improving the versatility, among the above-mentioned monohydroxy monoamine components, the monohydroxy monoamine component of the diimide dihydroxy-based compound preferably contains only one or more compounds selected from the above-mentioned Group L22.

(Diamide dicarboxylic acid-based compound)

A diamide dicarboxylic acid-based compound can be produced by performing a reaction between functional groups by a mechanochemical effect, using a dicarboxylic acid halide component and a monoaminomonocarboxylic acid component as the raw material compounds. Here, the reaction between functional groups corresponds to the reaction (C) described above. The diamide dicarboxylic acid-based compound is a compound having two amide groups and two carboxyl groups in one molecule.

In the production of a diamide dicarboxylic acid-based compound using a dicarboxylic acid halide component and a monoaminomonocarboxylic acid component, the monoaminomonocarboxylic acid component is usually used in an amount of about 2 time the molar amount of the dicarboxylic acid halide component, for example 1.5 to 10.0 times, preferably 1.8 to 2.2 times, preferably 1.9 to 2.1 times, more preferably 1.95 to 2.05 times.

The dicarboxylic acid halide component capable of constituting the diamide dicarboxylic acid-based compound is an acid halide of a dicarboxylic acid component similar to the dicarboxylic acid component capable of constituting the polyamide-based compound, and specifically, includes acid halides of an aromatic dicarboxylic acid component, an alicyclic dicarboxylic acid component, and an aliphatic dicarboxylic acid component that are similar to the dicarboxylic acid component capable of constituting the polyamide-based compound.

From the viewpoint of heat resistance of the diamide dicarboxylic acid-based compound or, for example, a high-molecular compound obtained using such compound, the dicarboxylic acid halide component of the diamide dicarboxylic acid-based compound preferably contains an acid halide of an aromatic dicarboxylic acid component and/or an acid halide of an alicyclic dicarboxylic acid component, more preferably an acid halide of an aromatic dicarboxylic acid component.

From the viewpoint of further improving the heat resistance of the diamide dicarboxylic acid-based compound or, for example, a high-molecular compound obtained using such compound, the dicarboxylic acid halide component of the diamide dicarboxylic acid-based compound preferably contains only an acid halide of an aromatic dicarboxylic acid component and/or an acid halide of an alicyclic dicarboxylic acid component, more preferably only an acid halide of an aromatic dicarboxylic acid component.

From the viewpoint of versatility, among the above-mentioned dicarboxylic acid halide components, the dicarboxylic acid halide component of the diamide dicarboxylic acid-based compound preferably contains one or more compounds selected from the group (hereinafter referred to as Group L23) consisting of terephthalic acid chloride, isophthalic acid chloride, 1,4-cyclohexanedicarboxylic acid chloride, and 1,3-cyclohexanedicarboxylic acid chloride.

From the viewpoint of further improving the versatility, among the above-mentioned dicarboxylic acid halide components, the dicarboxylic acid halide component of the diamide dicarboxylic acid-based compound preferably contains only one or more compounds selected from the above-mentioned Group L23.

The monoaminomonocarboxylic acid component capable of constituting the diamide dicarboxylic acid-based compound is a monoaminomonocarboxylic acid component similar to the monoaminomonocarboxylic acid component capable of constituting the diimide dicarboxylic acid-based compound, and specifically, includes an aromatic monoaminomonocarboxylic acid component, an alicyclic monoaminomonocarboxylic acid component, and an aliphatic monoaminomonocarboxylic acid component that are similar to the monoaminomonocarboxylic acid component capable of constituting the diimide dicarboxylic acid-based compound.

From the viewpoint of heat resistance of the diamide dicarboxylic acid-based compound or, for example, a high-molecular compound obtained using such compound, the monoaminomonocarboxylic acid component of the diamide dicarboxylic acid-based compound preferably contains an aromatic monoaminomonocarboxylic acid component and/or an alicyclic monoaminomonocarboxylic acid component, more preferably an aromatic monoaminomonocarboxylic acid component.

From the viewpoint of further improving the heat resistance of the diamide dicarboxylic acid-based compound or, for example, a high-molecular compound obtained using such compound, the monoaminomonocarboxylic acid component of the diamide dicarboxylic acid-based compound preferably contains only an aromatic monoaminomonocarboxylic acid component and/or an alicyclic monoaminomonocarboxylic acid component, more preferably only an aromatic monoaminomonocarboxylic acid component.

From the viewpoint of versatility, among the above-mentioned monoaminomonocarboxylic acid components, the monoaminomonocarboxylic acid component of the diamide dicarboxylic acid-based compound preferably contains one or more compounds selected from the group (hereinafter referred to as Group L24) consisting of glycine, alanine, valine, norvaline, α-aminobutyric acid, γ-aminobutyric acid, β-alanine, serine, leucine, isoleucine, threonine, proline, hydroxyproline, methionine, cysteine, 5-aminopentanoic acid, 6-aminocaproic acid, 7-aminoheptanoic acid, 9-nonanonic acid, 11-aminoundecanoic acid, 12-aminolauric acid, 17-aminoheptadecanonic acid, phenylalanine, tryptophan, 2-aminobenzoic acid, 3-aminobenzoic acid, 4-aminobenzoic acid, 2-amino-3-methylbenzoic acid, 2-amino-4-methylbenzoic acid, 2-amino-5-methylbenzoic acid, 2-amino-6-methylbenzoic acid, 3-amino-2-methylbenzoic acid, 3-amino-4-methylbenzoic acid, 4-amino-2-methylbenzoic acid, 4-amino-3-methylbenzoic acid, 5-amino-2-methylbenzoic acid, 2-amino-3,4-dimethylbenzoic acid, 2-amino-4,5-dimethylbenzoic acid, 2-amino-4-methoxybenzoic acid, 3-amino-4-methoxybenzoic acid, 4-amino-2-methoxybenzoic acid, 6-amino-2-naphthalenecarboxylic acid, and 3-amino-2-naphthalenecarboxylic acid.

From the viewpoint of further improving the versatility, among the above-mentioned monoaminomonocarboxylic acid components, the monoaminomonocarboxylic acid component of the diamide dicarboxylic acid-based compound preferably contains only one or more compounds selected from the above-mentioned Group L24.

(Diamide tetracarboxylic acid-based compound)

A diamide tetracarboxylic acid-based compound can be produced by performing a reaction between functional groups by a mechanochemical effect, using a dicarboxylic acid halide component and a monoaminodicarboxylic acid component as the raw material compounds. Here, the reaction between functional groups corresponds to the reaction (C) described above. The diamide tetracarboxylic acid-based compound is a compound having two amide groups and four carboxyl groups in one molecule.

In the production of a diamide tetracarboxylic acid-based compound using a dicarboxylic acid halide component and a monoaminodicarboxylic acid component, the monoaminodicarboxylic acid component is usually used in an amount of about 2 time the molar amount of the dicarboxylic acid halide component, for example 1.5 to 10.0 times, preferably 1.8 to 2.2 times, more preferably 1.9 to 2.1 times, even more preferably 1.95 to 2.05 times.

The dicarboxylic acid halide component capable of constituting the diamide tetracarboxylic acid-based compound is an acid halide of a dicarboxylic acid component similar to the dicarboxylic acid component capable of constituting the polyamide-based compound, and specifically, includes acid halides of an aromatic dicarboxylic acid component, an alicyclic dicarboxylic acid component, and an aliphatic dicarboxylic acid component that are similar to the dicarboxylic acid component capable of constituting the polyamide-based compound.

From the viewpoint of heat resistance of the diamide tetracarboxylic acid-based compound or, for example, a high-molecular compound obtained using such compound, the dicarboxylic acid halide component of the diamide tetracarboxylic acid-based compound preferably contains an acid halide of an aromatic dicarboxylic acid component and/or an acid halide of an alicyclic dicarboxylic acid component, more preferably an acid halide of an aromatic dicarboxylic acid component.

From the viewpoint of further improving the heat resistance of the diamide tetracarboxylic acid-based compound or, for example, a high-molecular compound obtained using such compound, the dicarboxylic acid halide component of the diamide tetracarboxylic acid-based compound preferably contains only an acid halide of an aromatic dicarboxylic acid component and/or an acid halide of an alicyclic dicarboxylic acid component, more preferably only an acid halide of an aromatic dicarboxylic acid component.

From the viewpoint of versatility, among the above-mentioned dicarboxylic acid halide components, the dicarboxylic acid halide component of the diamide tetracarboxylic acid-based compound preferably contains one or more compounds selected from the group (hereinafter referred to as Group L25) consisting of terephthalic acid chloride, isophthalic acid chloride, 1,4-cyclohexanedicarboxylic acid chloride, and 1,3-cyclohexanedicarboxylic acid chloride.

From the viewpoint of further improving the versatility, among the above-mentioned dicarboxylic acid halide components, the dicarboxylic acid halide component of the diamide tetracarboxylic acid-based compound preferably contains only one or more compounds selected from the above-mentioned Group L25.

The monoaminodicarboxylic acid component capable of constituting the diamide tetracarboxylic acid-based compound is a monoaminodicarboxylic acid component similar to the monoaminodicarboxylic acid component capable of constituting the diimide tetracarboxylic acid-based compound, and specifically, includes an aromatic monoaminodicarboxylic acid component, an alicyclic monoaminodicarboxylic acid component, and an aliphatic monoaminodicarboxylic acid component that are similar to the monoaminodicarboxylic acid component capable of constituting the diimide tetracarboxylic acid-based compound.

From the viewpoint of heat resistance of the diamide tetracarboxylic acid-based compound or, for example, a high-molecular compound obtained using such compound, the monoaminodicarboxylic acid component of the diamide tetracarboxylic acid-based compound preferably contains an aromatic monoaminodicarboxylic acid component and/or an alicyclic monoaminodicarboxylic acid component, more preferably an aromatic monoaminodicarboxylic acid component.

From the viewpoint of further improving the heat resistance of the diamide tetracarboxylic acid-based compound or, for example, a high-molecular compound obtained using such compound, the monoaminodicarboxylic acid component of the diamide tetracarboxylic acid-based compound preferably contains only an aromatic monoaminodicarboxylic acid component and/or an alicyclic monoaminodicarboxylic acid component, more preferably only an aromatic monoaminodicarboxylic acid component.

From the viewpoint of versatility, among the above-mentioned monoaminodicarboxylic acid components, the monoaminodicarboxylic acid component of the diamide tetracarboxylic acid-based compound preferably contains one or more compounds selected from the group (hereinafter referred to as Group L26) consisting of 2-aminoterephthalic acid, 2-aminoisophthalic acid, 4-aminoisophthalic acid, 5-aminoisophthalic acid, 3-aminophthalic acid, 4-aminophthalic acid, glutamic acid, aspartic acid, 2-aminopimelic acid, 2-aminosberic acid, 2-aminoadipic acid, α-aminosebacic acid, and aminomalonic acid.

From the viewpoint of further improving the versatility, among the above-mentioned monoaminodicarboxylic acid components, the monoaminodicarboxylic acid component of the diamide tetracarboxylic acid-based compound preferably contains only one or more compounds selected from the above-mentioned Group L26.

(Diester dicarboxylic acid-based compound)

A diester dicarboxylic acid-based compound can be produced by performing a reaction between functional groups by a mechanochemical effect, using a dicarboxylic acid halide component and a monohydroxy monocarboxylic acid component as the raw material compounds. Here, the reaction between functional groups corresponds to the reaction (D) described above. The diester dicarboxylic acid-based compound is a compound having two ester groups and two carboxyl groups in one molecule.

In the production of a diester dicarboxylic acid-based compound using a dicarboxylic acid halide component and a monohydroxy monocarboxylic acid component, the monohydroxy monocarboxylic acid component is usually used in an amount of about 2 time the molar amount of the dicarboxylic acid halide component, for example 1.5 to 10.0 times, preferably 1.8 to 2.2 times, preferably 1.9 to 2.1 times, more preferably 1.95 to 2.05 times.

The dicarboxylic acid halide component capable of constituting the diester dicarboxylic acid-based compound is an acid halide of a dicarboxylic acid component similar to the dicarboxylic acid component capable of constituting the polyamide-based compound, and specifically, includes acid halides of an aromatic dicarboxylic acid component, an alicyclic dicarboxylic acid component, and an aliphatic dicarboxylic acid component that are similar to the dicarboxylic acid component capable of constituting the polyamide-based compound.

From the viewpoint of heat resistance of the diester dicarboxylic acid-based compound or, for example, a high-molecular compound obtained using such compound, the dicarboxylic acid halide component of the diester dicarboxylic acid-based compound preferably contains an acid halide of an aromatic dicarboxylic acid component and/or an acid halide of an alicyclic dicarboxylic acid component, more preferably an acid halide of an aromatic dicarboxylic acid component.

From the viewpoint of further improving the heat resistance of the diester dicarboxylic acid-based compound or, for example, a high-molecular compound obtained using such compound, the dicarboxylic acid halide component of the diester dicarboxylic acid-based compound preferably contains only an acid halide of an aromatic dicarboxylic acid component and/or an acid halide of an alicyclic dicarboxylic acid component, more preferably only an acid halide of an aromatic dicarboxylic acid component.

From the viewpoint of versatility, among the above-mentioned dicarboxylic acid halide components, the dicarboxylic acid halide component of the diester dicarboxylic acid-based compound preferably contains one or more compounds selected from the group (hereinafter referred to as Group L27) consisting of terephthalic acid chloride, isophthalic acid chloride, 1,4-cyclohexanedicarboxylic acid chloride, and 1,3-cyclohexanedicarboxylic acid chloride.

From the viewpoint of further improving the versatility, among the above-mentioned dicarboxylic acid halide components, the dicarboxylic acid halide component of the diester dicarboxylic acid-based compound preferably contains only one or more compounds selected from the above-mentioned Group L27.

The monohydroxy monocarboxylic acid component capable of constituting the diester dicarboxylic acid-based compound includes an aromatic monohydroxy monocarboxylic acid component containing an aromatic ring, an alicyclic monohydroxy monocarboxylic acid component containing an aliphatic ring but not an aromatic ring, and an aliphatic monohydroxy monocarboxylic acid component that does not contain an aromatic ring or an alicyclic ring. The monohydroxy monocarboxylic acid component may contain an ether group and/or a thioether group, and/or one or more hydrogen atoms may be substituted with a halogen atom (e.g., fluorine atom, chlorine atom, and bromine atom).

Examples of the aromatic monohydroxy monocarboxylic acid component include 2-hydroxybenzoic acid, 3-hydroxybenzoic acid, 4-hydroxybenzoic acid, 4-hydroxy-2-methylbenzoic acid, 4-hydroxy-3-methylbenzoic acid, 4-hydroxy-3,5,-dimethylbenzoic acid, 2-hydroxy-4-methoxybenzoic acid, 3-hydroxy-4-methoxybenzoic acid, 4-hydroxy-3-methoxybenzoic acid, 4-hydroxy-3,5-dimethoxybenzoic acid, 4-hydroxy-2,6-dimethoxybenzoic acid, 3,5-di-tert-butyl-4-hydroxybenzoic acid, 3-hydroxy-2-naphthoic acid, 5-hydroxy-1-naphthoic acid, 5-hydroxy-2-naphthoic acid, 6-hydroxy-2-naphthoic acid, 7-hydroxy-2-naphthoic acid, 4-(4-hydroxyphenyl)benzoic acid, 4-(3-hydroxyphenyl)benzoic acid, 3-(4-hydroxyphenyl)benzoic acid, 3-chloro-4-hydroxybenzoic acid, 3,5-dichloro-4-hydroxybenzoic acid, 2,5-dichloro-4-hydroxybenzoic acid, 3-phenyl-4-hydroxybenzoic acid, 2-phenyl-4-hydroxybenzoic acid, 3-phenoxy-4-hydroxybenzoic acid, 6-hydroxy-5-chloro-2-naphthoic acid, 6-hydroxy-5-methyl-2-naphthoic acid, 7-hydroxy-5-methoxy-2-naphthoic acid, 4-carboxy-4'-hydroxybiphenyl, and 3-bromo-4-hydroxybenzoic acid. One of these may be used alone, or two or more thereof may be used as a mixture.

Examples of the alicyclic monohydroxy monocarboxylic acid component include 1-hydroxy-1-cyclopropanecarboxylic acid, 2-hydroxycyclohexanecarboxylic acid, 3-hydroxycyclohexanecarboxylic acid, 4-hydroxycyclohexanecarboxylic acid, 3-(hydroxymethyl)cyclohexanecarboxylic acid, 4-(hydroxymethyl)cyclohexanecarboxylic acid, 3-hydroxy-1-adamantanecarboxylic acid, and 3-hydroxy-1-adamantane acetic acid. One of these may be used alone, or two or more thereof may be used as a mixture.

Examples of the aliphatic monohydroxy monocarboxylic acid component include lactic acid, hydroxyacetic acid, 2-hydroxybutanoic acid, 2-hydroxypentanoic acid, 2-hydroxyhexanoic acid, 2-hydroxyheptanoic acid, 2-hydroxyoctanoic acid, 2-hydroxy-2-methylpropanoic acid, 2-hydroxy-2-methylbutanoic acid, 2-hydroxy-2-ethylbutanoic acid, 2-hydroxy-2-methylpentanoic acid, 2-hydroxy-2-ethylpentanoic acid, 2-hydroxy-2-propylpentanoic acid, 2-hydroxy-2-butylpentanoic acid, 2-hydroxy-2-methylhexanoic acid, 2-hydroxy-2-ethylhexanoic acid, 2-hydroxy-2-propylhexanoic acid, 2-hydroxy-2-butylhexanoic acid, 2-hydroxy-2-pentylhexanoic acid, 2-hydroxy-2-methylheptanoic acid, 2-hydroxy-2-methylheptanoic acid, 2-hydroxy-2-ethylheptanoic acid, 2-hydroxy-2-propylheptanoic acid, 2-hydroxy-2-butylheptanoic acid, 2-hydroxy-2-pentylheptanoic acid, 2-hydroxy-2-hexylheptanoic acid, 2-hydroxy-2-methyloctanoic acid, 2-hydroxy-2-ethyloctanoic acid, 2-hydroxy-2-propyloctanoic acid, 2-hydroxy-2-butyloctanoic acid, 2-hydroxy-2-pentyloctanoic acid, 2-hydroxy-2-hexyloctanoic acid, 2-hydroxy-2-heptyloctanoic acid, 3-hydroxypropanoic acid, 3-hydroxybutanoic acid, 3-hydroxypentanoic acid, 3-hydroxyhexanoic acid, 3-hydroxyheptanoic acid, 3-hydroxyoctanoic acid, 3-hydroxy-3-methylbutanoic acid, 3-hydroxy-3-methylpentanoic acid, 3-hydroxy-3-ethylpentanoic acid, 3-hydroxy-3-methylhexanoic acid, 3-hydroxy-3-ethylhexanoic acid, 3-hydroxy-3-propylhexanoic acid, 3-hydroxy-3-methylheptanoic acid, 3-hydroxy-3-ethylheptanoic acid, 3-hydroxy-3-propylheptanoic acid, 3-hydroxy-3-butylheptanoic acid, 3-hydroxy-3-methyloctanoic acid, 3-hydroxy-3-ethyloctanoic acid, 3-hydroxy-3-propyloctanoic acid, 3-hydroxy-3-butyloctanoic acid, 3-hydroxy-3-pentyloctanoic acid, 4-hydroxybutanoic acid, 4-hydroxypentanoic acid, 4-hydroxyhexanoic acid, 4-hydroxyheptanoic acid, 4-hydroxyoctanoic acid, 4-hydroxy-4-methylpentanoic acid, 4-hydroxy-4-methylhexanoic acid, 4-hydroxy-4-ethylhexanoic acid, 4-hydroxy-4-methylheptanoic acid, 4-hydroxy-4-ethylheptanoic acid, 4-hydroxy-4-propylheptanoic acid, 4-hydroxy-4-methyloctanoic acid, 4-hydroxy-4-ethyloctanoic acid, 4-hydroxy-4-propyloctanoic acid, 4-hydroxy-4-butyloctanoic acid, 5-hydroxypentanoic acid, 5-hydroxyhexanoic acid, 5-hydroxyheptanoic acid, 5-hydroxyoctanoic acid, 5-hydroxy-5-methylhexanoic acid, 5-hydroxy-5-methylheptanoic acid, 5-hydroxy-5-ethylheptanoic acid, 5-hydroxy-5-methyloctanoic acid, 5-hydroxy-5-ethyloctanoic acid, 5-hydroxy-5-propyloctanoic acid, 6-hydroxyhexanoic acid, 6-hydroxyheptanoic acid, 6-hydroxyoctanoic acid, 6-hydroxy-6-methylheptanoic acid, 6-hydroxy-6-methyloctanoic acid, 6-hydroxy-6-ethyloctanoic acid, 7-hydroxyheptanoic acid, 7-hydroxyoctanoic acid, 7-hydroxy-7-methyloctanoic acid, 8-hydroxyoctanoic acid, 9-hydroxynonanoic acid, 10-hydroxydecanoic acid, 11-hydroxyundecanoic acid, 10-hydroxydodecanoic acid, 12-hydroxydodecanic acid, 13-hydroxytridecanoic acid, 10-hydroxytetradecanoic acid, 12-hydroxytetradecanoic acid, 14-hydroxytetradecanoic acid, 15-hydroxypentanoic acid, 10-hydroxyhexadecanoic acid, 12-hydroxyhexadecanoic acid, 16-hydroxyhexadecanoic acid, 17-hydroxyheptadecanoic acid, 10-hydroxyoctadecanoicacid, 12-hydroxyoctadecanoic acid, 18-hydroxyoctadecanoic acid, 19-hydroxynonadecanoic acid, 12-hydroxyicosanoic acid, 14-hydroxyicosanoic acid, 20-hydroxyicosanoic acid, 21-hydroxyhenicosanoic acid, 22-hydroxydocosanoic acid, 23-hydroxytricosanoic acid, 2-hydroxyethoxyacetic acid, and 2-hydroxypropoxyacetic acid. One of these may be used alone, or two or more thereof may be used as a mixture.

From the viewpoint of heat resistance of the diester dicarboxylic acid-based compound or, for example, a high-molecular compound obtained using such compound, the monohydroxy monocarboxylic acid component of the diester dicarboxylic acid-based compound preferably contains an aromatic monohydroxy monocarboxylic acid component and/or an alicyclic monohydroxy monocarboxylic acid component, more preferably an aromatic monohydroxy monocarboxylic acid component.

From the viewpoint of further improving the heat resistance of the diester dicarboxylic acid-based compound or, for example, a high-molecular compound obtained using such compound, the monohydroxy monocarboxylic acid component of the diester dicarboxylic acid-based compound preferably contains only an aromatic monohydroxy monocarboxylic acid component and/or an alicyclic monohydroxy monocarboxylic acid component, more preferably only an aromatic monohydroxy monocarboxylic acid component.

From the viewpoint of versatility, among the above-mentioned monohydroxy monocarboxylic acid components, the monohydroxy monocarboxylic acid component of the diester dicarboxylic acid-based compound preferably contains one or more compounds selected from the group (hereinafter referred to as Group L28) consisting of 2-hydroxybenzoic acid, 3-hydroxybenzoic acid, 4-hydroxybenzoic acid, 4-hydroxy-2-methylbenzoic acid, 4-hydroxy-3-methylbenzoic acid, 4-hydroxy-3,5,-dimethylbenzoic acid, 2-hydroxy-4-methoxybenzoic acid, 3-hydroxy-4-methoxybenzoic acid, 4-hydroxy-3-methoxybenzoic acid, 4-hydroxy-3,5-dimethoxybenzoic acid, 4-hydroxy-2,6-dimethoxybenzoic acid, 3,5-di-tert-butyl-4-hydroxybenzoic acid, 3-hydroxy-2-naphthoic acid, 5-hydroxy-1-naphthoic acid, 5-hydroxy-2-naphthoic acid, 6-hydroxy-2-naphthoic acid, 7-hydroxy-2-naphthoic acid, 4-(4-hydroxyphenyl)benzoic acid, 4-(3-hydroxyphenyl)benzoic acid, 3-(4-hydroxyphenyl)benzoic acid, 3-phenyl-4-hydroxybenzoic acid, 2-phenyl-4-hydroxybenzoic acid, 3-phenoxy-4-hydroxybenzoic acid, 6-hydroxy-5-chloro-2-naphthoic acid, 6-hydroxy-5-methyl-2-naphthoic acid, 7-hydroxy-5-methoxy-2-naphthoic acid, 4-carboxy-4'-hydroxybiphenyl, 2-hydroxycyclohexanecarboxylic acid, 3-hydroxycyclohexanecarboxylic acid, 4-hydroxycyclohexanecarboxylic acid, and aliphatic monohydroxy monocarboxylic acid.

From the viewpoint of further improving the versatility, among the above-mentioned monohydroxy monocarboxylic acid components, the monohydroxy monocarboxylic acid component of the diester dicarboxylic acid-based compound preferably contains only one or more compounds selected from the above-mentioned Group L28.

(Diester tetracarboxylic acid-based compound)

A diester tetracarboxylic acid-based compound can be produced by performing a reaction between functional groups by a mechanochemical effect, using a dicarboxylic acid halide component and a monohydroxy dicarboxylic acid component as the raw material compounds. Here, the reaction between functional groups corresponds to the reaction (D) described above. The diester tetracarboxylic acid-based compound is a compound having two ester groups and four carboxyl groups in one molecule.

In the production of a diester tetracarboxylic acid-based compound using a dicarboxylic acid halide component and a monohydroxy dicarboxylic acid component, the monohydroxy dicarboxylic acid component is usually used in an amount of about 2 time the molar amount of the dicarboxylic acid halide component, for example 1.5 to 10.0 times, preferably 1.8 to 2.2 times, more preferably 1.9 to 2.1 times, even more preferably 1.95 to 2.05 times.

The dicarboxylic acid halide component capable of constituting the diester tetracarboxylic acid-based compound is an acid halide of a dicarboxylic acid component similar to the dicarboxylic acid component capable of constituting the polyamide-based compound, and specifically, includes acid halides of an aromatic dicarboxylic acid component, an alicyclic dicarboxylic acid component, and an aliphatic dicarboxylic acid component that are similar to the dicarboxylic acid component capable of constituting the polyamide-based compound.

From the viewpoint of heat resistance of the diester tetracarboxylic acid-based compound or, for example, a high-molecular compound obtained using such compound, the dicarboxylic acid halide component of the diester tetracarboxylic acid-based compound preferably contains an acid halide of an aromatic dicarboxylic acid component and/or an acid halide of an alicyclic dicarboxylic acid component, more preferably an acid halide of an aromatic dicarboxylic acid component.

From the viewpoint of further improving the heat resistance of the diester tetracarboxylic acid-based compound or, for example, a high-molecular compound obtained using such compound, the dicarboxylic acid halide component of the diester tetracarboxylic acid-based compound preferably contains only an acid halide of an aromatic dicarboxylic acid component and/or an acid halide of an alicyclic dicarboxylic acid component, more preferably only an acid halide of an aromatic dicarboxylic acid component.

From the viewpoint of versatility, among the above-mentioned dicarboxylic acid halide components, the dicarboxylic acid halide component of the diester tetracarboxylic acid-based compound preferably contains one or more compounds selected from the group (hereinafter referred to as Group L29) consisting of terephthalic acid chloride, isophthalic acid chloride, 1,4-cyclohexanedicarboxylic acid chloride, and 1,3-cyclohexanedicarboxylic acid chloride.

From the viewpoint of further improving the versatility, among the above-mentioned dicarboxylic acid halide components, the dicarboxylic acid halide component of the diester tetracarboxylic acid-based compound preferably contains only one or more compounds selected from the above-mentioned Group L29.

The monohydroxy dicarboxylic acid component capable of constituting the diester tetracarboxylic acid-based compound includes an aromatic monohydroxy dicarboxylic acid component containing an aromatic ring, an alicyclic monohydroxy dicarboxylic acid component containing an aliphatic ring but not an aromatic ring, and an aliphatic monohydroxy dicarboxylic acid component that does not contain an aromatic ring or an alicyclic ring. The monohydroxy dicarboxylic acid component may contain an ether group and/or a thioether group, and/or one or more hydrogen atoms may be substituted with a halogen atom (e.g., fluorine atom, chlorine atom, and bromine atom).

Examples of the aromatic monohydroxy dicarboxylic acid component include 2-hydroxyterephthalic acid, 4-hydroxyisophthalic acid, 5-hydroxyisophthalic acid, and 4-hydroxyphthalic acid. One of these may be used alone, or two or more thereof may be used as a mixture.

Examples of the aliphatic monohydroxy dicarboxylic acid component include 2-hydroxymalonic acid, malic acid, isomalic acid, 1-hydroxypropane-1,1-dicarboxylic acid, 1-hydroxybutane-1,1-dicarboxylic acid, 1-hydroxy-2-methylpropane-1,1-dicarboxylic acid, 2-hydroxyethane-1,1-dicarboxylic acid, 2-hydroxy-3-methylpropane-1,1-dicarboxylic acid, 1-(hydroxymethyl)propane-1,1-dicarboxylic acid, $\alpha$-methylmalic acid, $\alpha$-hydroxy-$\alpha$'-methylsuccinic acid, $\alpha$-hydroxy-$\alpha$',$\alpha$'-dimethylsuccinic acid, $\alpha$-hydroxy-$\alpha$,$\alpha$'-dimethylsuccinic acid, $\alpha$-hydroxy-$\alpha$'-ethylsuccinic acid, $\alpha$-hydroxy-$\alpha$'-methyl-$\alpha$-

ethylsuccinic acid, trimethylmalic acid, $\alpha$-hydroxyglutaric acid, $\beta$-hydroxyglutaric acid, $\beta$-hydroxy-$\beta$-methylglutaric acid, $\beta$-hydroxy-$\alpha,\alpha$-dimethylglutaric acid, $\alpha$-hydroxysuberic acid, and $\alpha$-hydroxysebacic acid. One of these may be used alone, or two or more thereof may be used as a mixture.

From the viewpoint of heat resistance of the diester tetracarboxylic acid-based compound or, for example, a high-molecular compound obtained using such compound, the monohydroxy dicarboxylic acid component of the diester tetracarboxylic acid-based compound preferably contains an aromatic monohydroxy dicarboxylic acid component and/or an alicyclic monohydroxy dicarboxylic acid component, more preferably an aromatic monohydroxy monocarboxylic acid component.

From the viewpoint of further improving the heat resistance of the diester tetracarboxylic acid-based compound or, for example, a high-molecular compound obtained using such compound, the monohydroxy dicarboxylic acid component of the diester tetracarboxylic acid-based compound preferably contains only an aromatic monohydroxy dicarboxylic acid component and/or an alicyclic monohydroxy dicarboxylic acid component, more preferably only an aromatic monohydroxy monocarboxylic acid component.

From the viewpoint of versatility, among the above-mentioned monohydroxy dicarboxylic acid components, the monohydroxy dicarboxylic acid component of the diester tetracarboxylic acid-based compound preferably contains one or more compounds selected from the group (hereinafter referred to as Group L30) consisting of 2-hydroxyterephthalic acid, 4-hydroxyisophthalic acid, 5-hydroxyisophthalic acid, and 4-hydroxyphthalic acid.

From the viewpoint of further improving the versatility, among the above-mentioned monohydroxy dicarboxylic acid components, the monohydroxy dicarboxylic acid component of the diester tetracarboxylic acid-based compound preferably contains only one or more compounds selected from the above-mentioned Group L30.

(Curable imide-based compound)

A curable imide-based compound can be produced as follows. Using an unsaturated dicarboxylic acid anhydride component and a diamine component as the raw material compounds, a reaction between functional groups is performed by a mechanochemical effect to thereby produce an amic acid-based compound, and the amic acid-based compound is then subjected to an imidization reaction. Here, the reaction between functional groups corresponds to the reaction (A) described above. The curable imide-based compound is a thermosetting compound or a photocurable compound containing one or more (particularly one to four) imide groups and one or more curable unsaturated bonds (double bond and/or triple bond) in one molecule.

In the production of a curable imide-based compound using an unsaturated dicarboxylic acid anhydride component and a diamine component, the diamine component is usually used in a molar amount of about 0.5 times, for example 0.1 to 0.7 times, preferably 0.3 to 0.7 times, more preferably 0.4 to 0.6 times, even more preferably 0.45 to 0.55 times that of the unsaturated dicarboxylic acid anhydride component.

The unsaturated dicarboxylic acid anhydride component capable of constituting a curable imide-based compound is not particularly limited as long as it is a compound having one or more (particularly one) addition reaction (polymerization) or radical reaction (polymerization) double bonds and/or triple bonds and one or more (particularly one) acid anhydride groups in one molecule.

The unsaturated dicarboxylic acid anhydride component includes an aromatic unsaturated dicarboxylic acid anhydride component containing an aromatic ring, an alicyclic unsaturated dicarboxylic acid anhydride component containing an aliphatic ring but not an aromatic ring, and an aliphatic unsaturated dicarboxylic acid anhydride component that does not contain an aromatic ring or an alicyclic ring. The unsaturated dicarboxylic acid anhydride component may contain an ether group and/or a thioether group, and/or one or more hydrogen atoms may be substituted with a halogen atom (e.g., fluorine atom, chlorine atom, and bromine atom).

Examples of the aromatic unsaturated dicarboxylic acid anhydride component include 4-phenylethynyl phthalic anhydride, 4-(1-propynyl)phthalic anhydride, and 4-ethynylphthalic anhydride. One of these may be used alone, or two or more thereof may be used as a mixture.

Examples of the alicyclic unsaturated dicarboxylic acid anhydride component include 5-norbornene-2,3-dicarboxylic anhydride, 1,2,3,6-tetrahydrophthalic anhydride, 3,4,5,6-tetrahydrophthalic anhydride, and 2,5-norbomadiene-2,3-dicarboxylic anhydride. One of these may be used alone, or two or more thereof may be used as a mixture.

Examples of the aliphatic unsaturated dicarboxylic acid anhydride component include maleic anhydride, methylmaleic anhydride, 2,3-dimethylmaleic anhydride, 2-phenylmaleic anhydride, 2-(diphenyl)maleic anhydride, 2-(1-hydroxyhexyl)maleic anhydride, 2-(4-methylphenyl)maleic anhydride, 2-[2-(hexyloxy)ethyl]maleic anhydride, 2,5-dihydro-2,5-dioxo-3-furanacetic acid, and 2,5-dihydro-2,5-dioxofuran-3-carboxylic acid methyl ester. One of these may be used alone, or two or more thereof may be used as a mixture.

From the viewpoint of heat resistance of the curable imide-based compound or, for example, a high-molecular compound obtained using such compound, the unsaturated dicarboxylic acid anhydride component of the curable imide-based compound preferably contains an aromatic unsaturated dicarboxylic acid anhydride component and/or an alicyclic unsaturated dicarboxylic acid anhydride component, more preferably an aromatic unsaturated dicarboxylic acid anhydride component.

From the viewpoint of further improving the heat resistance of the curable imide-based compound or, for example, a high-molecular compound obtained using such compound, the unsaturated dicarboxylic acid anhydride component of the curable imide-based compound preferably contains only an aromatic unsaturated dicarboxylic acid anhydride component and/or an alicyclic unsaturated dicarboxylic acid anhydride component, more preferably only an aromatic unsaturated dicarboxylic acid anhydride component.

From the viewpoint of versatility, among the above-mentioned unsaturated dicarboxylic acid anhydride components, the unsaturated dicarboxylic acid anhydride component of the curable imide-based compound preferably contains one or more compounds selected from the group (hereinafter referred to as Group L31) consisting of 4-phenylethynyl phthalic anhydride, 4-(1-propynyl)phthalic anhydride, 4-ethynylphthalic anhydride, 5-norbornene-2,3-dicarboxylic anhydride, maleic anhydride, and methylmaleic anhydride.

From the viewpoint of further improving the versatility, the unsaturated dicarboxylic acid anhydride component of the curable imide-based compound preferably contains only one or more compounds selected from the above-mentioned Group L31.

The diamine component capable of constituting the curable imide-based compound is a diamine component similar to the diamine component capable of constituting the polyamic acid-based compound and the like, and specifically, includes an aromatic diamine component, an alicyclic diamine component, and an aliphatic diamine component that are similar to the diamine component capable of constituting the polyamic acid-based compound and the like.

From the viewpoint of heat resistance of the curable imide-based compound or, for example, a high-molecular compound obtained using such compound, the diamine component of the curable imide-based compound preferably contains an aromatic diamine component and/or an alicyclic diamine component, more preferably an aromatic diamine component.

From the viewpoint of further improving the heat resistance of the curable imide-based compound or, for example, a high-molecular compound obtained using such compound, the diamine component of the curable imide-based compound preferably contains only an aromatic diamine component and/or an alicyclic diamine component, more preferably only an aromatic diamine component.

From the viewpoint of solubility of the curable imide-based compound, among the above-mentioned diamine components, it is preferable to use a diamine component having an ether group, a thioether group, a sulfonyl group, a sulfonic acid group, a methyl group, a methylene group, an isopropylidene group, a phenyl group, a fluorene structure, a halogen atom (or a halogen atom-containing substituent), or a siloxane bond, as the diamine component of the curable imide-based compound.

From the viewpoint of further improving the solubility of the curable imide-based compound, among the above-mentioned diamine components, it is preferable to use only a diamine component having an ether group, a thioether group, a sulfonyl group, a sulfonic acid group, a methyl group, a methylene group, an isopropylidene group, a phenyl group, a fluorene structure, a halogen atom (or a halogen atom-containing substituent), or a siloxane bond, as the diamine component of the curable imide-based compound.

From the viewpoint of non-coloring properties and solubility of the curable imide-based compound or, for example, a high-molecular compound obtained using such compound, among the above-mentioned diamine components, the diamine component of the curable imide-based compound preferably contains an alicyclic diamine component and/or an aliphatic diamine component.

From the viewpoint of further improving the non-coloring properties and solubility of the curable imide-based compound or, for example, a high-molecular compound obtained using such compound, among the above-mentioned diamine components, the diamine component of the curable imide-based compound preferably contains only an alicyclic diamine component and/or an aliphatic diamine component.

From the viewpoint of versatility, among the above-mentioned diamine components, the diamine component of the curable imide-based compound preferably contains one or more compounds selected from the group (hereinafter referred to as Group L32) consisting of 4,4'-diaminodiphenyl ether, p-phenylenediamine, m-phenylenediamine, 3,4'-diaminodiphenyl ether, 4,4'-diaminodiphenylmethane, 2,2'-bis(trifluoromethyl)-4,4'-diaminobiphenyl, 2,2-bis[4-(4-aminophenoxy)phenyl]-1,1,1,3,3,3-hexafluoropropane, 1,4-bis(4-aminophenoxy)benzene, 1,3-bis(4-aminophenoxy)benzene, 4,4'-bis(4-aminophenoxy)biphenyl, 4,4'-bis(3-aminophenoxy)biphenyl, 2,2-bis[4-(4-aminophenoxy)phenyl]propane, bis(4-aminophenyl)sulfone, bis[4-(4-aminophenoxy)phenyl]sulfone, 9,9-bis(4-aminophenyl)fluorene, trans-1,4-cyclohexanediamine, cis-1,4-cyclohexanediamine, 4,4'-methylenebis(cyclohexylamine), 1,10-diaminodecane, 1,12-diaminododecane, m-xylylene diamine, p-xylylene diamine, 1,4-bis(aminomethyl)cyclohexane, and dimer diamine.

From the viewpoint of further improving the versatility, among the above-mentioned diamine components, the diamine component of the curable imide-based compound preferably contains only one or more compounds selected from the above-mentioned Group L32.

[Production method of low-molecular compound]

In the production method of a low-molecular compound of the present invention, a reaction is performed between functional groups by a mechanochemical effect using a raw material mixture containing a given raw material compound. The given raw material compound is two or more raw material compounds (monomer components) for producing each of the above-mentioned low-molecular compounds, and of these, at least one or more raw material compounds are in a solid state under the reactive environment as described above. Specifically, a reaction is performed between functional groups by using a mechanochemical effect by subjecting a raw material mixture containing such a raw material compound to a pulverization treatment. When a raw material compound in a liquid state under the reactive environment is used as the raw material compound, the raw material compound in the liquid state is preferably mixed or added before or while pulverizing at least one raw material compound in a solid state contained in the raw material mixture, from the viewpoint of further improving the reaction rate. At this time, from the viewpoint of further improving the reaction rate, the raw material compound in the liquid state is preferably added in an amount obtained by dividing a predetermined added amount into two or more portions in a plurality of times, more preferably added dropwise.

As the raw material compound (particularly the raw material compound in the solid state under the reactive environment), a compound having a particle shape of a maximum length of 0.001 to 20.0 mm, particularly 0.01 to 10.0 mm is usually used. A cumulative 50% particle size has been used as the maximum length. Specifically, when particles having a particle size of 0.5 mm or more are included, in accordance with JIS Z8815, the maximum length is a value measured as a cumulative 50% particle size from the particle size distribution obtained by a sieving test described in JIS Z8815. When particles with a particle size of 0.5 mm or more are not included, the maximum length is determined as a cumulative 50% particle size obtained by a laser diffraction/scattering method using a particle size distribution measuring apparatus.

The pulverization treatment for the production of a low-molecular compound may be achieved by any apparatus (e.g., so-called a pulverizer, a mixer, or a stirrer) as long as the apparatus can transfer mechanical energy to the raw material compound by compression, impact, shearing and/or grinding. For example, the pulverization treatment can be performed using the same apparatus as the one exemplified in the description of the production method of a high-molecular compound. Examples of the typical apparatus for producing a low-molecular compound include a high-speed bottom stirring type mixer, a high-speed rotary pulverizer, a container drive type mill, and a medium-stirring type mill that are same as those exemplified in the description of the production method of a high-molecular compound.

The reaction conditions (that is, mixing/stirring/pulverization conditions) for producing a low-molecular compound are not particularly limited as long as the mechanochemical effect may be exhibited to obtain a desired low-molecular compound.

For example, when the above-mentioned maximum length of the raw material compound (particularly the raw material compound in the solid state under the reactive environment) having a particle shape is Rm ($\mu$m), pulverization treatment is performed until the average particle size of the raw material compound reaches $0.5 \times$ Rm or less, particularly $0.1 \times$ Rm or less.

Specifically, for example, when a medium-stirring type mill is used with a pulverizing chamber (or a tank) for pulverization treatment having a capacity of 4 to 6 L (particularly 5 L), a raw material mixture weighing 0.5 to 1.5 kg (particularly 1 kg), a pulverizing ball made of alumina, having a ball diameter of 10.0 mm, and an input weight of 6.0 kg, the rotation speed is usually 115 rpm or higher, particularly from 115 to 504 rpm, and the pulverization time is usually 0.5 minutes or more, particularly from 0.5 to 60 minutes.

Specifically, for example, when a high-speed bottom stirring type mixer is used with a mixing bath (or a tank) for pulverization treatment having a capacity of 9 to 150 L (particularly 20 L) and a raw material mixture weighing 4 to 6 kg (particularly 5 kg), the rotation speed is usually 100 rpm or higher, particularly from 500 to 5000 rpm, and the pulverization time is usually 1 minute or more, particularly from 1 to 60 minutes.

Specifically, for example, when a high-speed rotary pulverizer is used with a pulverizing chamber (or a tank) for pulverization treatment having a capacity of 75 to 200 mL (particularly 150 mL) and a raw material mixture weighing 50 to 250 g (particularly 100 g), the rotation speed is usually 3000 rpm or higher, particularly from 3000 to 14000 rpm, and the pulverization time is usually 1 minute or more, particularly from 2 to 10 minutes.

Such a pulverization treatment and a subsequent cooling treatment (e.g., cooling-by-leaving treatment) of the pulverized product may be repeated twice or more, for example, 2 to 10 times. As a result, the mechanochemical effect is more effectively exhibited, the reaction rate is further improved.

In the production method of a low-molecular compound, the reaction rate can be improved by adjusting the reaction conditions (pulverization conditions). For example, the reaction rate increases as the pulverization conditions are strengthened within the range in which the raw material mixture does not melt.

In the present invention, the low-molecular compound obtained by the pulverization treatment preferably has an average particle size (D90) of 1000 $\mu$m or less, from 0.01 to 1000 $\mu$m, particularly from 0.1 to 100 $\mu$m.

In the present invention, in order to suppress the adhesion of particles to the inner walls of the mixing chamber, the stirring chamber, and the pulverizing chamber, to increase the pulverizing efficiency of the particles, and to increase the energy transfer efficiency to the particles, the raw material mixture may contain an auxiliary agent. As the auxiliary agent, the same auxiliary agents as those exemplified in the description of the production method of a high-molecular compound may be used.

In the production method of a low-molecular compound of the present invention, a catalyst may be contained in the raw material mixture in order to promote the reaction. As the catalyst, any catalyst (acid catalyst, base catalyst, metal catalyst, metal oxide catalyst, complex catalyst, sulfide, chloride, metal organic salt, mineral acid, etc.) useful for producing a low-molecular compound can be used. Specific examples of the catalyst include, for example, p-toluenesulfonic acid, dimethyl sulfate, diethyl sulfate, sulfuric acid, hydrochloric acid, oxalic acid, acetic acid, phosphoric acid, phosphorous acid, hypophosphorous acid or salts thereof, sodium hydroxide, potassium hydroxide, lithium hydroxide, magnesium hydroxide, calcium hydroxide, barium hydroxide, pyridine, ammonia, triethylamine, 2,4,6-tris(dimethylaminomethyl)phenol, N,N-dimethylethanolamine, aminoethanolamine, N-methyl-N,N-diethanolamine, isopropylamine, iminobispropylamine, ethylamine, diethylamine, 3-ethoxypropylamine, 3-diethylaminopropylamine, sec-butylamine, propylamine, methylaminopropylamine, 3-methoxypropylamine, monoethanolamine, morpholine, N-methylmorpholine, N-ethylmorpholine, imidazole compounds such as 1-methylimidazole, 2-methylimidazole, 1,2-dimethylimidazole, 2-ethyl-4-methylimidazole, 2-ethyl-2-phenylimidazole, and 1-cyanoethyl-2-ethyl-4-methylimidazole; lewis acid complexes including boron halides such as a boron trifluoride/piperidine complex, a boron trifluoride/monoethylamine complex, a boron trifluoride/triethanolamine complex, and a boron trichloride/octylamine complex; dicyandiamide derivatives; onium salts such as an ammonium salt and a phosphonium salt; N,N-dimethyl-N'-(3-chloro-4-methylphenyl)urea, N,N-dimethyl-N'-(4-chlorophenyl)urea, N,N-dimethyl-N'-(3,4-dichlorophenyl)urea, N,N-dimethyl-N'-(3,4-dichloromethylphenyl)urea, 2,4-(N',N'-dimethylureide)toluene, 1,4-bis(N',N-dimethylureide)benzene, quaternary ammonium salts such as tri-n-butylbenzylammonium halide, tetra-n-butylammonium halide, trimethylbenzylammonium halide, and triethylbenzylammonium halide; and quaternary phosphonium salts such as tri-n-butylbenzylphosphonium halide, tetra-n-butylphosphonium halide, trimethylbenzylphosphonium halide, and triethylbenzylphosphonium halide, oxides, acetates and others of magnesium, manganese, zinc, calcium, lithium, titanium, antimony, and germanium.

The production method of a low-molecular compound of the present invention can include a heating step. This makes it possible to promote the reaction between functional groups, thereby resulting in further increased reaction rate. The heating step may be performed during and/or after the pulverization treatment (i.e., mechanochemical treatment). In the case of heating during the pulverization treatment, it is necessary to heat at a temperature at which the raw material mixture and/or the low-molecular compound to be formed does not melt. Such a temperature is, for example, from 40 to 350°C.

In the case of heating after the pulverization treatment, the heating temperature needs to be lower than the decomposition temperature of the obtained low-molecular compound. The heating temperature may be, for example, from 90 to 400°C, particularly 90 to 350°C. The heating time is not particularly limited, and may be, for example, from 0.5 to 16 hours, particularly 0.5 to 8 hours. The heating may be performed in an inert gas stream such as nitrogen, or under pressure or reduced pressure. Further, the heating may be performed by standing or while stirring.

In the production method of a low-molecular compound, the heating after the pulverization treatment may be performed in one step or in multiple steps. The heating in multiple steps means that a heating step at different heating temperatures is continuously performed twice or more, preferably 2 to 3 times. When the heating is performed in multiple steps, the heating temperature in and after the second heating step is preferably higher than the heating temperature in the preceding heating step from the viewpoint of further improving the reaction rate. For example, the heating temperature in the second heating step is preferably higher than the heating temperature in the first heating step. In addition, for example, the heating temperature in the third heating step is preferably higher than the heating temperature in the second heating step.

EXAMPLES

While in the following, the present invention will be specifically described with reference to Examples, the present invention is not limited thereto. The measurement in the production method of the organic compound of the present invention was carried out by the following methods.

1. Reaction rate

1-1. Reaction rate of acid dianhydride and diamine in high-molecular compound

The reaction rate was determined by $^1$H-NMR measurement on polyimide resin precursor powders obtained by examples of Example A1 series (Tables 1A to 1E and Table 2). Approximately 10 mg of the polyimide resin precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.

In the obtained $^1$H-NMR spectrum, a sum of integral values of peaks derived from a structure a1 in which both amino groups were amidated among diamine-derived peaks was taken as A1, a sum of integral values of peaks derived from a structure a2 in which one amino group was amidated was taken as A2, a sum of integral values of peaks derived from a structure a3 in which both amino groups were not amidated was taken as A3, and the reaction rate of the diamine was then calculated as the following equation. For example, the peaks derived from the structure a1 were detected near 7.03 ppm (multiplet), 7.17 ppm (multiplet), 7.70 ppm (multiplet), and 8.32 ppm (multiplet); the peaks derived from the structure a2 were detected near 6.68 ppm (multiplet), 6.78 ppm (multiplet), and 6.89 ppm (multiplet); and the peaks derived from the structure a3 were detected near 6.51 ppm (doublet) and 6.61 ppm (doublet).

$$\text{Reaction rate of diamine (\%)} = (A1 + A2/2)/(A1 + A2 + A3) \times 100$$

When each peak was not able to be assigned to the above-mentioned three structures due to overlapping peaks or the like, a sum of integral values of peaks derived from a proton b1 which was close to the amide bond formed by the reaction in the diamine structure was taken as B1, a sum of integral values of peaks derived from a proton b2 which was close to the unreacted amino group was taken as B2, and the reaction rate of the diamine was then calculated as the following equation.

$$\text{Reaction rate of diamine (\%)} = B1/(B1 + B2) \times 100$$

1-2. Reaction rate of acid dianhydride and diisocyanate in high-molecular compound

Since the polyimide-based compounds obtained by examples of Example A2 series (Table 2) were insoluble in a solvent, the reaction rate was not able to be evaluated. However, from the fact that most of the sample is insolubilized in the solvent after mechanochemical treatment, and from the results of IR measurement as described later, it is clear that the reaction is qualitatively proceeding. The sample is a pulverized product obtained after mechanochemical treatment and before heat treatment.

1-3. Reaction rate of acid halide and diamine or diisocyanate in high-molecular compound

The reaction rate was determined by $^1$H-NMR measurement on polyamide resin powders obtained by Examples A3-3 and A3-4 of Example A3 series (Table 4). Approximately 10 mg of the polyamide resin powder was dissolved in approximately 1 mL of a mixed solution of deuterated trifluoroacetic acid/heavy water (= 98.0/2.0 wt%) and ultrasonically treated for 30 minutes, followed by $^1$H-NMR measurement.

In the obtained $^1$H-NMR spectrum, a sum of integral values of peaks derived from a structure a1 in which both amino groups were amidated among diamine-derived peaks was taken as A1, a sum of integral values of peaks derived from a structure a2 in which one amino group was amidated was taken as A2, a sum of integral values of peaks derived from a structure a3 in which both amino groups were not amidated was taken as A3, and the reaction rate of the diamine was then calculated as the following equation.

$$\text{Reaction rate of diamine (\%)} = (A1 + A2/2)/(A1 + A2 + A3) \times 100$$

Since the polyamide resin powders obtained by Examples A3-1, A3-2, A3-5, and A3-6 of Example A3 series (Table 4) were insoluble in a solvent, the reaction rate was not able to be evaluated. However, from the fact that most of the sample is insolubilized in the solvent after mechanochemical treatment, and from the results of IR measurement as described later, it is clear that the reaction is qualitatively proceeding. The sample is a pulverized product obtained after mechanochemical treatment and before heat treatment.

1-4. Reaction rate of trimellitic anhydride or halide thereof and diamine or diisocyanate in high-molecular compound

The reaction rate was determined by $^1$H-NMR measurement on polyamide-imide resin powders obtained by examples of Example A4 series (Table 5). Approximately 10 mg of the polyamide-imide resin powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement. The reaction rate was measured on a pulverized product obtained after mechanochemical treatment and before heat treatment, and on a pulverized product obtained after the heat treatment.

For Examples A4-1 and A4-2, in the obtained $^1$H-NMR spectrum, a sum of integral values of peaks derived from a structure a1 in which both amino groups were amidated among diamine-derived peaks was taken as A1, a sum of integral values of peaks derived from a structure a2 in which one amino group was amidated was taken as A2, a sum of integral values of peaks derived from a structure a3 in which both amino groups were not amidated was taken as A3, and the reaction rate of the diamine was then calculated as the following equation.

$$\text{Reaction rate of diamine (\%)} = (A1 + A2/2)/(A1 + A2 + A3) \times 100$$

For Examples A4-3 and A4-4, in the obtained $^1$H-NMR spectrum, a sum of integral values of peaks derived from a structure c1 in which isocyanate groups were amidated among isocyanate-derived peaks was taken as C1, an integral value of peaks derived from an imidized structure c2 was taken as C2, an integral value of peaks derived from a structure c3 of unreacted isocyanate group and an amino group produced therefrom was taken as C3, and the reaction rate of the isocyanate was then calculated as the following equation.

$$\text{Reaction rate of isocyanate (\%)} = (C1 + C2)/(C1 + C2 + C3) \times 100$$

1-5. Reaction rate of acid halide and diol in high-molecular compound

The reaction rate was determined by $^1$H-NMR measurement on polyester resin powders obtained by examples of Example A5 series (Table 6). Approximately 10 mg of the polyester resin powder was dissolved in approximately 1 mL of a mixed solution of deuterated tetrachloroethane/trifluoroacetic acid (= 99.0/1.0 wt%) and ultrasonically treated for 30 minutes, followed by $^1$H-NMR measurement. The reaction rate was measured on a pulverized product obtained after mechanochemical treatment and before heat treatment, and on a pulverized product obtained after the heat treatment.

In the obtained $^1$H-NMR spectrum, a sum of integral values of peaks derived from a structure d1 in which both hydroxyl groups were esterified among diol-derived peaks was taken as D1, a sum of integral values of peaks derived from a structure d2 in which one hydroxyl group was esterified was taken as D2, a sum of integral values of peaks derived from a structure d3 in which both hydroxyl groups were not esterified was taken as D3, and the reaction rate of the diamine was then calculated as the following equation.

$$\text{Reaction rate of diol (\%)} = (D1 + D2/2)/(D1 + D2 + D3) \times 100$$

1-6. Reaction rate of diisocyanate and diamine in high-molecular compound

Since the polyurea-based compounds obtained by examples of Example A6 series (Table 7) were insoluble in a solvent, the reaction rate was not able to be evaluated. However, from the fact that most of the sample is insolubilized in the solvent after mechanochemical treatment, and from the results of IR measurement as described later, it is clear that the reaction is qualitatively proceeding. The sample is a pulverized product obtained after mechanochemical treatment and before heat treatment.

1-7. Reaction rate of acid monoanhydride and diamine in low-molecular compound

The reaction rate was determined by $^1$H-NMR measurement on diimide dicarboxylic acid precursor powders obtained by examples of Examples B1 to B3 series (Tables 8 to 11) and B9-1 of B9 series (Table 17). Approximately 10 mg of the diimide dicarboxylic acid precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.

In the obtained $^1$H-NMR spectrum, a sum of integral values of peaks derived from a structure e1 in which both amino groups were amidated among diamine-derived peaks was taken as E1, a sum of integral values of peaks derived from a structure e2 in which one amino group was amidated was taken as E2, a sum of integral values of peaks derived from a structure e3 in which both amino groups were not amidated was taken as E3, and the reaction rate of the amine was then calculated as the following equation.

$$\text{Reaction rate of amine (\%)} = (E1 + E2/2)/(E1 + E2 + E3) \times 100$$

When each peak was not able to be assigned to the above-mentioned three structures due to overlapping peaks or the like, a sum of integral values of peaks derived from a proton f1 which was close to the amide bond formed by the reaction in the diamine structure was taken as F1, a sum of integral values of peaks derived from a proton f2 which was close to the unreacted amino group was taken as F2, and the reaction rate of the amine was then calculated as the following equation.

$$\text{Reaction rate of amine (\%)} = F1/(F1 + F2) \times 100$$

1-8. Reaction rate of acid dianhydride and monoaminomonocarboxylic acid in low-molecular compound

The reaction rate was determined by $^1$H-NMR measurement on diimide dicarboxylic acid precursor powders obtained by examples of Example B4 series (Table 12). Approximately 10 mg of the diimide dicarboxylic acid precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.

In the obtained $^1$H-NMR spectrum, a sum of integral values of peaks derived from a structure g1 in which the amino group was amidated among monoaminomonocarboxylic acid-derived peaks was taken as G1, a sum of integral values of peaks derived from a structure g2 in which the amino group was not amidated was taken as G2, and the reaction rate of the amine was then calculated as the following equation.

$$\text{Reaction rate of amine (\%)} = G1/(G1 + G2) \times 100$$

1-9. Reaction rate of acid monoanhydride and diaminomonocarboxylic acid in low-molecular compound

The reaction rate was determined by $^1$H-NMR measurement on diimide tricarboxylic acid precursor powders obtained by examples of Example B5 series (Table 13). Approximately 10 mg of the diimide tricarboxylic acid precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.

In the obtained $^1$H-NMR spectrum, a sum of integral values of peaks derived from a structure h1 in which both amino groups were amidated among diaminomonocarboxylic acid-derived peaks was taken as H1, a sum of integral values of peaks derived from a structure h2 in which one amino group was amidated was taken as H2, a sum of integral values of peaks derived from a structure h3 in which both amino groups were not amidated was taken as H3, and the reaction rate of the amine was then calculated as the following equation.

$$\text{Reaction rate of amine (\%)} = (H1 + H2/2)/(H1 + H2 + H3) \times 100$$

When each peak was not able to be assigned to the above-mentioned three structures due to overlapping peaks or the like, a sum of integral values of peaks derived from a proton i1 which was close to the amide bond formed by the reaction in the diaminomonocarboxylic acid structure was taken as I1, a sum of integral values of peaks derived from a proton i2 which was close to the unreacted amino group was taken as 12, and the reaction rate of the amine was then calculated as the following equation.

$$\text{Reaction rate of amine (\%)} = I1/(I1 + I2) \times 100$$

1-10. Reaction rate of acid dianhydride and monoaminodicarboxylic acid in low-molecular compound

The reaction rate was determined by $^1$H-NMR measurement on diimide tetracarboxylic acid precursor powders obtained by examples of Example B6 series (Table 14). Approximately 10 mg of the diimide tetracarboxylic acid precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.

In the obtained $^1$H-NMR spectrum, a sum of integral values of peaks derived from a structure j1 in which the amino group was amidated among monoaminodicarboxylic acid-derived peaks was taken as J1, a sum of integral values of peaks derived from a structure j2 in which the amino group was not amidated was taken as J2, and a reaction rate of amine was then calculated as the following equation.

$$\text{Reaction rate of amine (\%)} = \text{J1}/(\text{J1} + \text{J2}) \times 100$$

1-11. Reaction rate of acid monoanhydride and monoaminomonocarboxylic acid in low-molecular compound

The reaction rate was determined by $^1$H-NMR measurement on monoimide dicarboxylic acid precursor powders obtained by examples of Example B7 series (Table 15). Approximately 10 mg of the monoimide dicarboxylic acid precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.
In the obtained $^1$H-NMR spectrum, a sum of integral values of peaks derived from a structure k1 in which the amino group was amidated among monoaminomonocarboxylic acid-derived peaks was taken as K1, a sum of integral values of peaks derived from a structure k2 in which the amino group was not amidated was taken as K2, and a reaction rate of amine was then calculated as the following equation.

$$\text{Reaction rate of amine (\%)} = \text{K1}/(\text{K1} + \text{K2}) \times 100$$

1-12. Reaction rate of acid monoanhydride and monoaminodicarboxylic acid in low-molecular compound
The reaction rate was determined by $^1$H-NMR measurement on monoimide tricarboxylic acid precursor powders obtained by examples of Example B8 series (Table 16). Approximately 10 mg of the monoimide tricarboxylic acid precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.
In the obtained $^1$H-NMR spectrum, a sum of integral values of peaks derived from a structure 11 in which the amino group was amidated among monoaminodicarboxylic acid-derived peaks was taken as L1, a sum of integral values of peaks derived from a structure 12 in which the amino group was not amidated was taken as L2, and a reaction rate of amine was then calculated as the following equation.

$$\text{Reaction rate of amine (\%)} = \text{L1}/(\text{L1} + \text{L2}) \times 100$$

1-13. Reaction rate of acid monoanhydride-chloride and monoaminomonocarboxylic acid or monoaminodicarboxylic acid in low-molecular compound

The reaction rate was determined by $^1$H-NMR measurement on amide group-containing monoimide dicarboxylic acid precursor powders or amide group-containing monoimide tetracarboxylic acid precursor powders obtained by Examples B9-2 to B9-6 of Example B9 series (Table 17). Approximately 10 mg of the amide group-containing monoimide dicarboxylic acid precursor powder or the amide group-containing monoimide tetracarboxylic acid precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.
In the obtained $^1$H-NMR spectrum, a sum of integral values of peaks derived from a structure m1 in which the amino group was amidated among monoaminomonocarboxylic acid- or monoaminodicarboxylic acid-derived peaks was taken as M1, a sum of integral values of peaks derived from a structure m2 in which the amino group was not amidated was taken as M2, and a reaction rate of amine was then calculated as the following equation.

$$\text{Reaction rate of amine (\%)} = \text{M1}/(\text{M1} + \text{M2}) \times 100$$

1-14. Reaction rate of acid monoanhydride and monohydroxy monoamine in low-molecular compound
The reaction rate was determined by $^1$H-NMR measurement on ester group-containing monoimide tricarboxylic acid precursor powders obtained by examples of Example B10 series (Table 18). Approximately 10 mg of the ester group-containing monoimide tricarboxylic acid precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.
In the obtained $^1$H-NMR spectrum, a sum of integral values of peaks derived from a structure n1 in which the amino group was amidated among monohydroxy monoamino acid-derived peaks was taken as N1, a sum of integral values of peaks derived from a structure n2 in which the amino group was not amidated was taken as N2, and a reaction rate of amine was then calculated as the following equation.

$$\text{Reaction rate of amine reaction rate (\%)} = N1/(N1 + N2) \times 100$$

1-15. Reaction rate of acid dianhydride and monohydroxy monoamine in low-molecular compound

The reaction rate was determined by $^1$H-NMR measurement on diimide diphenol precursor powders or diimide dihydroxy precursor powders obtained by examples of Example B11 series (Table 19). Approximately 10 mg of the diimide diphenol precursor powder or the diimide dihydroxy precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.

In the obtained $^1$H-NMR spectrum, a sum of integral values of peaks derived from a structure o1 in which the amino group was amidated among monohydroxy monoamino acid-derived peaks was taken as Ol, a sum of integral values of peaks derived from a structure o2 in which the amino group was not amidated was taken as O2, and a reaction rate of amine was then calculated as the following equation.

$$\text{Reaction rate of amine reaction rate (\%)} = O1/(O1 + O2) \times 100$$

1-16. Reaction rate of dicarboxylic acid chloride and monoaminomonocarboxylic acid or monoaminodicarboxylic acid in low-molecular compound

The reaction rate was determined by $^1$H-NMR measurement on diamide dicarboxylic acid precursor powders or diamide tetracarboxylic acid precursor powders obtained by examples of Example B12 series (Table 20). Approximately 10 mg of the diamide dicarboxylic acid precursor powder or the diamide tetracarboxylic acid precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.

In the obtained $^1$H-NMR spectrum, a sum of integral values of peaks derived from a structure p1 in which the amino group was amidated among monoaminomonocarboxylic acid- or monoaminodicarboxylic acid-derived peaks was taken as P1, a sum of integral values of peaks derived from a structure p2 in which the amino group was not amidated was taken as P2, and a reaction rate of amine was then calculated as the following equation.

$$\text{Reaction rate of amine (\%)} = P1/(P1 + P2) \times 100$$

1-17. Reaction rate of dicarboxylic acid chloride and monohydroxy monocarboxylic acid or monohydroxy dicarboxylic acid in low-molecular compound

The reaction rate was determined by $^1$H-NMR measurement on diester dicarboxylic acid precursor powders or diester tetracarboxylic acid precursor powders obtained by examples of Example B13 series (Table 21). Approximately 10 mg of the diester dicarboxylic acid precursor powder or the diester tetracarboxylic acid precursor powder was dissolved in approximately 1 mL of a mixed solution of deuterated dimethyl sulfoxide/deuterated trifluoroacetic acid (= 96.5/3.5 wt%) and ultrasonically treated for 30 minutes, followed by $^1$H-NMR measurement.

In the obtained $^1$H-NMR spectrum, a sum of integral values of peaks derived from a structure q1 in which a hydroxyl group was esterified among monohydroxy monocarboxylic acid- or monohydroxy dicarboxylic acid-derived peaks was taken as Q1, a sum of integral values of peaks derived from a structure q2 in which a hydroxyl group was not esterified was taken as Q2, and the reaction rate of the hydroxyl group was then calculated as the following equation.

$$\text{Reaction rate of hydroxyl group (\%)} = Q1/(Q1 + Q2) \times 100$$

1-18. Reaction rate of unsaturated acid monoanhydride and diamine in low-molecular compound

The reaction rate was determined by $^1$H-NMR measurement on curable imide-based compound precursor powders obtained by examples of Example B14 (Table 22). Approximately 10 mg of the curable imide-based compound precursor powder was dissolved in approximately 1 mL of a mixed solution of deuterated dimethyl sulfoxide/heavy water/deuterated trifluoroacetic acid (= 91.5/5.0/3.5 wt%) and ultrasonically treated for 30 minutes, followed by $^1$H-NMR measurement.

In the obtained $^1$H-NMR spectrum, a sum of integral values of peaks derived from a structure r1 in which both amino groups were amidated among diamine-derived peaks was taken as R1, a sum of integral values of peaks derived from a structure r2 in which one amino group was amidated was taken as R2, a sum of integral values of peaks derived from a structure r3 in which both amino groups were not amidated was taken as R3, and the reaction rate of the amine was then calculated as the following equation.

$$\text{Reaction rate of amine (\%)} = (R1 + R2/2)/(R1 + R2 + R3) \times 100$$

When each peak was not able to be assigned to the above-mentioned three structures due to overlapping peaks or the like, a sum of integral values of peaks derived from a proton s1 which was close to the amide bond formed by the reaction in the diamine structure was taken as S1, a sum of integral values of peaks derived from a proton s2 which was close to the unreacted amino group was taken as S2, and the reaction rate of the amine was then calculated as the following equation.

$$\text{Reaction rate of amine (\%)} = S1/(S1 + S2) \times 100$$

2. Average degree of polymerization of high-molecular compounds

For Example A1 series (Examples A1-1 to A1-95), the average degree of polymerization was calculated from the amine reaction rate obtained by the above-mentioned method by the following equation.

$$\text{Average degree of polymerization (n)} = [\text{Amine reaction rate}/(100 - \text{amine reaction rate})] \times 2 + 2$$

3. Measurement of average particle size in high-molecular compounds and low-molecular compounds

The average particle size was measured by a laser diffraction method and determined as the value of a particle size (median size) at a cumulative 50% in the obtained cumulative distribution. Specifically, 0.1 to 1.0 g of the powder obtained after the mechanochemical treatment was measured with a laser diffraction type particle size distribution measuring apparatus (Mastersizer 3000, manufactured by Malvern Panalytical Ltd.) to obtain a particle size distribution. The value of a particle size (median size) at a cumulative 50% in the obtained cumulative distribution was determined as the average particle size.

[Experimental Example A: Production method of high-molecular compound]

(Production method of polyamic acid-based compounds (polyimide resin precursor and polyimide resin), solid/solid mechanochemical method)

Example A1-1

To a pulverizing chamber of a medium-stirring type mill (tank capacity: 5.0 L), 6.0 kg of alumina balls were added, and 521 parts by mass of pyromellitic dianhydride and 479 parts by mass of 4,4'-diaminodiphenyl ether were then added thereto. The mixture was pulverized at 504 rpm for 3 minutes in a nitrogen atmosphere to perform a mechanochemical reaction anda polyimide resin precursor (polyamic acid) powder was obtained.

The reaction rate of the amino group derived from the 4,4'-diaminodiphenyl ether and the acid anhydride group of the pyromellitic anhydride in the obtained pulverized product was measured and found to be 78.2%, and the average degree of polymerization was 9.2. The average particle size of the obtained pulverized product was 48.2 $\mu$m.

In the NMR measurement of Example A1-1, in the $^1$H-NMR spectrum, peaks derived from the reaction product in which both amino groups were amidated were detected near 7.03 ppm (multiplet), 7.17 ppm (multiplet), 7.70 ppm (multiplet), and 8.32 ppm (multiplet); peaks derived from the reaction product in which one amino group was amidated were detected near 6.68 ppm (multiplet), 6.78 ppm (multiplet), and 6.89 ppm (multiplet); and peaks derived from unreacted amine were detected near 6.51 ppm (doublet) and 6.61 ppm (doublet).

The obtained pulverized product was heated at 300°C for 2 hours under a nitrogen stream, and IR measurement was performed on the obtained powder. As a result, absorption derived from the imide group was observed near 1750 to 1800 cm$^{-1}$, and it was able to be confirmed that a polyimide resin was formed.

Examples A1-2 to A1-80

A polyimide resin precursor powder was obtained by performing the same operation as in Example A1-1, except that the composition of the diamine was changed.

In the NMR measurement of each example, as in Example A1-1, peaks derived from the reaction product in which both amino groups were amidated, peaks derived from the reaction product in which one amino group was amidated, and peaks derived from unreacted amine were detected, respectively, in the $^1$H-NMR spectrum.

The obtained pulverized product was heated at 300°C for 2 hours under a nitrogen stream, and IR measurement was performed on the obtained powder. As a result, absorption derived from the imide group was observed near 1750 to 1800 cm$^{-1}$, and it was able to be confirmed that a polyimide resin was formed.

(Production method of polyamic acid-based compounds (polyimide resin precursor and polyimide resin), solid/liquid mechanochemical method)

Example A1-81

To a mixing chamber equipped with a double helical type stirring blade, 616 parts by mass of pyromellitic dianhydride was added, and the mixture was stirred at 40°C. Using a tube pump, 384 parts by mass of m-xylylene diamine was added at a rate of 2.13 parts by mass/min to obtain a two-component mixed powder. The reaction rate of the amino group derived from m-xylylene diamine and the acid anhydride group of pyromellitic anhydride in the obtained powder was measured and found to be 37.2%.

Next, 1.0 kg of the obtained mixed powder was added to a pulverizing chamber of a medium-stirring type mill (tank capacity: 5.0 L), and 6.0 kg of alumina balls were added thereto. The mixture was pulverized at 504 rpm for 3 minutes in a nitrogen atmosphere to perform a mechanochemical reaction and a polyimide resin precursor powder was obtained.

The reaction rate of the amino group derived from m-xylylene diamine and the acid anhydride group of pyromellitic anhydride in the obtained pulverized product was measured and found to be 65.7%, and the average degree of polymerization was 5.8. The average particle size of the obtained pulverized product was 46.1 μm.

In the NMR measurement of the example, as in Example A1-1, peaks derived from the reaction product in which both amino groups were amidated, peaks derived from the reaction product in which one amino group was amidated, and peaks derived from unreacted amine were detected, respectively, in the $^1$H-NMR spectrum.

The obtained pulverized product was heated at 300°C for 2 hours under a nitrogen stream, and IR measurement was performed on the obtained powder. As a result, absorption derived from the imide group was observed near 1750 to 1800 cm$^{-1}$, and it was able to be confirmed that a polyimide resin was formed.

Examples A1-82 to A1-95

A polyimide resin precursor powder was obtained by performing the same operation as in Example A1-81, except that the composition of the diamine was changed.

In the NMR measurement of each example, as in Example A1-1, peaks derived from the reaction product in which both amino groups were amidated, peaks derived from the reaction product in which one amino group was amidated, and peaks derived from unreacted amine were detected, respectively, in the $^1$H-NMR spectrum.

The obtained pulverized product was heated at 300°C for 2 hours under a nitrogen stream, and IR measurement was performed on the obtained powder. As a result, absorption derived from the imide group was observed near 1750 to 1800 cm$^{-1}$, and it was able to be confirmed that a polyimide resin was formed.

(Production method of polyimide-based compound)

Example A2-1

Thirty zirconia balls having a diameter of 10 mm were added to a pulverizing chamber of a planetary mill (capacity: 0.25 L), and a total of 5.0 g, including 466 parts by mass of pyromellitic dianhydride and 534 parts by mass of diphenylmethane diisocyanate, were added thereto. The cycle, in which the mixture was pulverized in air at 600 rpm for 5 minutes and then 5 minute-cooling was performed, was repeated 6 times to perform a mechanochemical reaction. The average particle size of the obtained pulverized product was 44.1 μm.

IR measurement was performed on the obtained pulverized product. As a result, absorption derived from the imide group was observed near 1750 to 1800 cm$^{-1}$, and it was able to be confirmed that a polyimide-based compound was formed. In addition, absorption derived from the isocyanate group was observed near 2200 to 2300 cm$^{-1}$, and it was confirmed that the isocyanate group remained.

Therefore, by heating the obtained pulverized product under the conditions of 150°C for 1 hour and then 250°C for 1 hour in a nitrogen atmosphere, it was confirmed that the absorption derived from the isocyanate group was reduced, while the absorption derived from the imide group was increased, and the formation of the polyimide-based compound and the molecular weight were increased.

Example A2-2

A pulverized product (polyimide-based compound) was obtained by performing the same operation as in Example A2-1, except that the composition of the diisocyanate was changed.

Before and after heating, IR measurement was performed on the obtained pulverized product by the same method as in Example A2-1, and the following items were confirmed:

- Before heating, it was able to be confirmed that the polyimide-based compound was formed by the absorption derived from the imide group; the absorption derived from the isocyanate group was observed, and it was confirmed that the isocyanate group remained; and
- After heating, it was able to be confirmed that the absorption derived from the isocyanate group was reduced, while the absorption derived from the imide group was increased, and the formation of the polyimide-based compound and the molecular weight were increased.

(Production method of polyamide-based compound)

Example A3-1

Thirty zirconia balls having a diameter of 10 mm were added to a pulverizing chamber of a planetary mill (capacity: 0.25 L), and a total of 5.0 g, including 652 parts by mass of terephthalic acid chloride and 348 parts by mass of para-phenylenediamine, were added thereto. The cycle, in which the mixture was pulverized in air at 600 rpm for 5 minutes and then 5 minute-cooling was performed, was repeated 6 times to perform a mechanochemical reaction. The average particle size of the obtained pulverized product was 56.5 $\mu$m.

IR measurement was performed on the obtained pulverized product. As a result, absorption derived from the amide group was observed near 1515 to 1650 cm$^{-1}$, and it was able to be confirmed that a polyamide-based compound was formed. In addition, absorption derived from the acid halide was observed near 1785 to 1815 cm$^{-1}$, and it was confirmed that the acid chloride remained.

Therefore, by heating the obtained pulverized product under the conditions of 100°C for 1 hour, then 150°C for 1 hour, and 250°C for 1 hour in a nitrogen atmosphere, it was confirmed that the absorption derived from the acid halide was reduced, while the absorption derived from the amide group was increased, and the formation of the polyamide-based compound and the molecular weight were increased.

Examples A3-2 to A3-4

A polyamide-based compound was obtained by performing the same operation as in Example A3-1, except that the composition of the diamine was changed.

Before and after heating, IR measurement was performed on the obtained pulverized product by the same method as in Example A3-1, and the following items were confirmed:

- Before heating, it was able to be confirmed that the polyamide-based compound was formed by the absorption derived from the amide group; the absorption derived from the acid halide was observed, and it was confirmed that the acid chloride remained; and
- After heating, it was able to be confirmed that the absorption derived from the acid halide was reduced, while the absorption derived from the amide group was increased, and the formation of the polyamide-based compound and the molecular weight were increased.

Example A3-5

Thirty zirconia balls having a diameter of 10 mm were added to a pulverizing chamber of a planetary mill (capacity: 0.25 L), and a total of 5.0 g, including 399 parts by mass of terephthalic acid and 601 parts by mass of diphenylmethane diisocyanate, were added thereto. The cycle, in which the mixture was pulverized in air at 600 rpm for 5 minutes and then 5 minute-cooling was performed, was repeated 6 times to perform a mechanochemical reaction. The average particle size of the obtained pulverized product was 44.2 $\mu$m.

IR measurement was performed on the obtained pulverized product. As a result, absorption derived from the amide group was observed near 1515 to 1650 cm$^{-1}$, and it was able to be confirmed that a polyamide-based compound was formed. In addition, absorption derived from the isocyanate group was observed near 2200 to 2300 cm$^{-1}$, and it was confirmed that the isocyanate group remained.

Therefore, by heating the obtained pulverized product under the conditions of 150°C for 1 hour and then 250°C for 1 hour in a nitrogen atmosphere, it was confirmed that the absorption derived from the isocyanate group was reduced, while the absorption derived from the amide group was increased, and the formation of the polyamide-based compound and the molecular weight were increased.

Example A3-6

A pulverized product (polyamide-based compound) was obtained by performing the same operation as in Example A3-5, except that the composition of the diisocyanate was changed.

Before and after heating, IR measurement was performed on the obtained pulverized product by the same method as in Example A3-1, and the following items were confirmed:

- Before heating, it was able to be confirmed that the polyamide-based compound was formed by the absorption derived from the amide group; the absorption derived from the isocyanate group was observed, and it was confirmed that the isocyanate group remained; and
- After heating, it was able to be confirmed that the absorption derived from the isocyanate group was reduced, while the absorption derived from the amide group was increased, and the formation of the polyamide-based compound and the molecular weight were increased.

(Production method of polyamide-imide-based compound)

Example A4-1

Thirty zirconia balls having a diameter of 10 mm were added to a pulverizing chamber of a planetary mill (capacity: 0.25 L), and a total of 5.0 g, including 513 parts by mass of trimellitic anhydride chloride and 487 parts by mass of 4,4'-diaminodiphenyl ether, were added thereto. The cycle, in which the mixture was pulverized in air at 600 rpm for 5 minutes and then 5 minute-cooling was performed, was repeated 6 times to perform a mechanochemical reaction. The average particle size of the obtained pulverized product was 42.1 $\mu$m.

The reaction rate of the amino group derived from the 4,4'-diaminodiphenyl ether, and the acid chloride and the acid anhydride group of the trimellitic anhydride chloride in the obtained pulverized product was measured by NMR and found to be 73.3%.

Therefore, the obtained pulverized product was heated under the conditions of 100°C for 1 hour, then 200°C for 1 hour, and further then 300°C for 1 hour in a nitrogen atmosphere. The reaction rate of the treated product thus obtained was determined by NMR and found to be 95.7%.

Example A4-2

A polyamide-imide-based compound was obtained by performing the same operation as in Example A4-1, except that the composition of the diamine was changed.

The reaction rate of the amino group derived from 3,4'-diaminodiphenyl ether, and the acid chloride and the acid anhydride group of the trimellitic anhydride chloride in the obtained pulverized product was measured and found to be 74.5%.

Therefore, the obtained pulverized product was heated under the conditions of 100°C for 1 hour, then 200°C for 1 hour, and further then 300°C for 1 hour in a nitrogen atmosphere. The reaction rate of the treated product thus obtained was determined by NMR and found to be 94.3%.

Example A4-3

Thirty zirconia balls having a diameter of 10 mm were added to a pulverizing chamber of a planetary mill (capacity: 0.25 L), and a total of 5.0 g, including 434 parts by mass of trimellitic anhydride and 566 parts by mass of diphenylmethane diisocyanate, were added thereto. The cycle, in which the mixture was pulverized in air at 600 rpm for 5 minutes and then 5 minute-cooling was performed, was repeated 6 times to perform a mechanochemical reaction. The average particle size of the obtained pulverized product was 45.3 $\mu$m.

The reaction rate of the isocyanate group derived from the diphenylmethane diisocyanate, and the carboxyl group and the acid anhydride group of the trimellitic anhydride in the obtained pulverized product was measured and found to be 36.2%.

Therefore, the obtained pulverized product was heated under the conditions of 100°C for 1 hour, then 200°C for 1 hour, and further then 300°C for 1 hour in a nitrogen atmosphere. The reaction rate of the treated product thus obtained was determined by NMR and found to be 92.1 %.

Example A4-4

A polyamide-imide-based compound was obtained by performing the same operation as in Example A4-3, except that the composition of the diisocyanate was changed.

The reaction rate of the isocyanate group derived from 1,5-isocyanatonaphthalene, and the carboxyl group and the acid anhydride group of the trimellitic anhydride in the obtained pulverized product was measured and found to be 38.8%.

Therefore, the obtained pulverized product was heated under the conditions of 100°C for 1 hour, then 200°C for 1 hour, and further then 300°C for 1 hour in a nitrogen atmosphere. The reaction rate of the treated product thus obtained was determined by NMR and found to be 92.3%.

(Production method of polyester-based compound)

Example A5-1

Thirty zirconia balls having a diameter of 10 mm were added to a pulverizing chamber of a planetary mill (capacity: 0.25 L), and a total of 5.0 g, including 471 parts by mass of terephthalic acid chloride and 529 parts by mass of 2,2'-bis(4-hydroxyphenyl)propane, were added thereto. The cycle, in which the mixture was pulverized in air at 600 rpm for 5 minutes and then 5 minute-cooling was performed, was repeated 6 times to perform a mechanochemical reaction. The average particle size of the obtained pulverized product was 33.1 $\mu$m.

The reaction rate of the hydroxyl group derived from the 2,2'-bis(4-hydroxyphenyl)propane and the acid chloride of the terephthalic acid chloride in the obtained pulverized product was measured and found to be 70.1%.

Therefore, the obtained pulverized product was heated under the conditions of 100°C for 1 hour, then 200°C for 1 hour, and 250°C for 1 hour in a nitrogen atmosphere. The reaction rate of the treated product thus obtained was determined by NMR and found to be 94.5%.

Example A5-2

A polyamide-imide-based compound was obtained by performing the same operation as in Example A5-1, except that the composition of the acid chloride was changed.

The reaction rate of the hydroxyl group derived from the 2,2'-bis(4-hydroxyphenyl)propane, and the acid chloride of the isophthalic acid chloride in the obtained pulverized product was measured and found to be 71.3%.

Therefore, the obtained pulverized product was heated under the conditions of 100°C for 1 hour, then 200°C for 1 hour, and 250°C for 1 hour in a nitrogen atmosphere. The reaction rate of the treated product thus obtained was determined by NMR and found to be 93.8%.

(Production method of polyurea-based compound)

Example A6-1

Thirty zirconia balls having a diameter of 10 mm were added to a pulverizing chamber of a planetary mill (capacity: 0.25 L), and a total of 5.0 g, including 556 parts by mass of diphenylmethane diisocyanate and 444 parts by mass of 4,4'-bisdiaminodiphenyl ether, were added thereto. The cycle, in which the mixture was pulverized in air at 600 rpm for 5 minutes and then 5 minute-cooling was performed, was repeated 6 times to perform a mechanochemical reaction. The average particle size of the obtained pulverized product was 53.4 $\mu$m.

IR measurement was performed on the obtained pulverized product. As a result, absorption derived from a urea bond was observed near 1500 to 1700 cm$^{-1}$, and it was able to be confirmed that a polyurea-based compound was formed. In addition, absorption derived from the isocyanate group was observed near 2200 to 2300 cm$^{-1}$, and it was

confirmed that the isocyanate group remained.

Therefore, by heating the obtained pulverized product under the conditions of 150°C for 1 hour and then 250°C for 1 hour in a nitrogen atmosphere, it was confirmed that the absorption derived from the isocyanate group was reduced, while the absorption derived from the urea bond was increased, and the formation of the polyurea-based compound and the molecular weight were increased.

Example A6-2

A pulverized product (polyurea-based compound) was obtained by performing the same operation as in Example 110, except that the composition of the diisocyanate and diamine was changed.
Before and after heating, IR measurement was performed on the obtained pulverized product by the same method as in Example A6-1, and the following items were confirmed:

- Before heating, it was able to be confirmed that the polyurea-based compound was formed by the absorption derived from the urea bond; the absorption derived from the isocyanate group was observed, and it was confirmed that the isocyanate group remained; and
- After heating, it was able to be confirmed that the absorption derived from the isocyanate group was reduced, while the absorption derived from the urea bond was increased, and the formation of the polyurea-based compound and the molecular weight were increased.

[Experimental Example B: Production method of low-molecular compound]

(Production method of diimide dicarboxylic acid-based compound)

Example B1-1

To a pulverizing chamber of a medium-stirring type mill (tank capacity: 5.0 L), 6.0 kg of alumina balls were added, and 657 parts by mass of trimellitic anhydride and 343 parts by mass of 4,4'-diaminodiphenyl ether were then added thereto. The mixture was pulverized at 504 rpm for 1 minute in a nitrogen atmosphere to perform a mechanochemical reaction and a diimide dicarboxylic acid precursor powder was obtained.
The reaction rate of the amino group derived from the 4,4'-diaminodiphenyl ether and the acid anhydride group of the trimellitic anhydride in the obtained pulverized product was measured and found to be 72.1%.
In the NMR measurements of Example B1-1 and Examples B-2 to B1-8 to be described later, in the $^1$H-NMR spectrum, peaks derived from the reaction product in which both amino groups were amidated were detected near 6.98 ppm (doublet); peaks derived from the reaction product in which one amino group was amidated were detected near 7.00 ppm (doublet), 7.07 ppm (doublet), and 7.35 ppm (doublet); and peaks derived from unreacted amine were detected near 7.13 ppm (doublet) and 4.49 ppm (doublet).
The obtained pulverized product was heated at 300°C for 2 hours under a nitrogen stream, and the reaction rate of the amino group in the obtained powder was determined and found to be 98.2%. Further, IR measurement was performed on the obtained powder. As a result, absorption derived from the imide group was observed near 1750 to 1800 cm$^{-1}$, and it was able to be confirmed that a diimide dicarboxylic acid was formed.

Examples B1-2 to B1-1

A pulverized product (diimide dicarboxylic acid) was obtained by performing the same operation as in Example B1-1, except that the rotation speed of the apparatus or the pulverization time was changed.
In the NMR measurement of each example, as in Example B1-1, peaks derived from the reaction product in which both amino groups were amidated, peaks derived from the reaction product in which one amino group was amidated, and peaks derived from unreacted amine were detected, respectively, in the $^1$H-NMR spectrum.
After heating, IR measurement was performed on the obtained pulverized product by the same method as in Example B1-1, and the following items were confirmed:

- After heating, absorption derived from the imide group was observed, and it was able to be confirmed that diimide dicarboxylic acid was formed.

Example B2-1

A total of 5.0 kg, including 657 parts by mass of trimellitic anhydride and 343 parts by mass of 4,4'-diaminodiphenyl ether, were added to a pulverizing chamber of a high-speed bottom stirring type mixer (capacity: 20 L). The mixture was stirred at 362 rpm for 3 minutes in a nitrogen atmosphere to perform a mechanochemical reaction and a diimide dicarboxylic acid precursor powder was obtained.

The reaction rate of the amino group derived from the 4,4'-diaminodiphenyl ether and the acid anhydride group of the trimellitic anhydride in the obtained pulverized product was measured and found to be 41.9%.

The obtained pulverized product was heated at 300°C for 2 hours under a nitrogen stream, and the reaction rate of the amino group in the obtained powder was determined and found to be 97.1%. Further, IR measurement was performed on the obtained powder. As a result, absorption derived from the imide group was observed near 1750 to 1800 cm$^{-1}$, and it was able to be confirmed that a diimide dicarboxylic acid was formed.

Examples B2-2 to B2-10

A pulverized product (diimide dicarboxylic acid) was obtained by performing the same operation as in Example B2-1, except that the rotation speed of the apparatus was changed.

After heating, IR measurement was performed on the obtained pulverized product by the same method as in Example B2-1, and the following items were confirmed:

- After heating, absorption derived from the imide group was observed, and it was able to be confirmed that diimide dicarboxylic acid was formed.

Example B3-1

A total of 5.0 kg, including 780 parts by mass of trimellitic anhydride and 220 parts by mass of p-phenylenediamine, were added to a pulverizing chamber of a high-speed bottom stirring type mixer (capacity: 20 L). The mixture was stirred at 1452 rpm for 10 minutes in a nitrogen atmosphere to perform a mechanochemical reaction and a diimide dicarboxylic acid precursor powder was obtained.

The reaction rate of the amino group derived from the p-phenylenediamine and the acid anhydride group of the trimellitic anhydride in the obtained pulverized product was measured and found to be 57.5%.

The obtained pulverized product was heated at 300°C for 2 hours under a nitrogen stream, and the reaction rate of the amino group in the obtained powder was determined and found to be 97.2%. Further, IR measurement was performed on the obtained powder. As a result, absorption derived from the imide group was observed near 1750 to 1800 cm$^{-1}$, and it was able to be confirmed that a diimide dicarboxylic acid was formed.

Examples B3-2 to B3-15

A pulverized product (diimide dicarboxylic acid) was obtained by performing the same operation as in Example B3-1, except that the composition of the diamine was changed.

After heating, IR measurement was performed on the obtained pulverized product by the same method as in Example B3-1, and the following items were confirmed:

- After heating, absorption derived from the imide group was observed, and it was able to be confirmed that diimide dicarboxylic acid was formed.

Example B3-16

To a mixing chamber equipped with a double helical type stirring blade, 738 parts by mass of trimellitic anhydride was added, and the mixture was stirred at 40°C. Using a tube pump, 262 parts by mass of m-xylylene diamine was added at a rate of 2.13 parts by mass/min to obtain a two-component mixed powder. The reaction rate of the amino group derived from m-xylylene diamine and the acid anhydride group of trimellitic anhydride in the obtained powder was measured and found to be 30.8%.

Next, 5.0 kg of the above-mentioned two-component mixed powder was added to the pulverizing chamber of the high-speed bottom stirring type mixer (capacity: 20 L). The mixture was stirred at 1452 rpm for 10 minutes in a nitrogen atmosphere to perform a mechanochemical reaction and a diimide dicarboxylic acid precursor powder was obtained.

The reaction rate of the amino group derived from the p-phenylenediamine and the acid anhydride group of the trimellitic anhydride in the obtained pulverized product was measured and found to be 55.7%.

The obtained pulverized product was heated at 300°C for 2 hours under a nitrogen stream, and the reaction rate of the amino group in the obtained powder was determined and found to be 98.9%. Further, IR measurement was performed on the obtained powder. As a result, absorption derived from the imide group was observed near 1750 to 1800 cm$^{-1}$, and it was able to be confirmed that a diimide dicarboxylic acid was formed.

Examples B3-17 to B3-18

A pulverized product (diimide dicarboxylic acid) was obtained by performing the same operation as in Example 3-16, except that the composition of the diamine was changed.

After heating, IR measurement was performed on the obtained pulverized product by the same method as in Example B3-16, and the following items were confirmed:

-    After heating, absorption derived from the imide group was observed, and it was able to be confirmed that diimide dicarboxylic acid was formed.

Example B4-1

A total of 100 g, including 444 parts by mass of pyromellitic dianhydride and 556 parts by mass of 4-aminobenzoic acid, were added to a pulverizing chamber of a high-speed rotary pulverizer (capacity: 150 mL). The mixture was stirred at 14,000 rpm for 5 minutes in an air atmosphere to perform a mechanochemical reaction and a diimide dicarboxylic acid precursor powder was obtained.

The reaction rate of the amino group derived from the 4-aminobenzoic acid and the acid anhydride group of the pyromellitic dianhydride in the obtained pulverized product was measured and found to be 64.3%.

The obtained pulverized product was heated at 300°C for 2 hours under a nitrogen stream, and the reaction rate of the amino group in the obtained powder was determined and found to be 99.2%. Further, IR measurement was performed on the obtained powder. As a result, absorption derived from the imide group was observed near 1750 to 1800 cm$^{-1}$, and it was able to be confirmed that a diimide dicarboxylic acid was formed.

Examples B4-2 to B4-6

A pulverized product (diimide dicarboxylic acid) was obtained by performing the same operation as in Example B4-1, except that the composition of the acid anhydride was changed.

After heating, IR measurement was performed on the obtained pulverized product by the same method as in Example B4-1, and the following items were confirmed:

-    After heating, absorption derived from the imide group was observed, and it was able to be confirmed that diimide dicarboxylic acid was formed.

(Production method of diimide tricarboxylic acid)

Example B5-1

A total of 100 g, including 716 parts by mass of trimellitic anhydride and 284 parts by mass of 3,4-diaminobenzoic acid, were added to a pulverizing chamber of a high-speed rotary pulverizer (capacity: 150 mL). The mixture was stirred at 14,000 rpm for 5 minutes in an air atmosphere to perform a mechanochemical reaction and a diimide tricarboxylic acid precursor powder was obtained.

The reaction rate of the amino group derived from the 3,4-diaminobenzoic acid and the acid anhydride group of the trimellitic anhydride in the obtained pulverized product was measured and found to be 62.3%.

The obtained pulverized product was heated at 300°C for 2 hours under a nitrogen stream, and the reaction rate of the amino group in the obtained powder was determined and found to be 98.2%. Further, IR measurement was performed on the obtained powder. As a result, absorption derived from the imide group was observed near 1750 to 1800 cm$^{-1}$, and it was able to be confirmed that a diimide tricarboxylic acid was formed.

Examples B5-2 to B5-4

A pulverized product (diimide tricarboxylic acid) was obtained by performing the same operation as in Example B5-1, except that the composition of the amine was changed.

After heating, IR measurement was performed on the obtained pulverized product by the same method as in Example B5-1, and the following items were confirmed:

- After heating, absorption derived from the imide group was observed, and it was able to be confirmed that diimide tricarboxylic acid was formed.

(Production method of diimide tetracarboxylic acid)

Example B6-1

A total of 100 g, including 377 parts by mass of pyromellitic dianhydride and 623 parts by mass of 2-aminoterephthalic acid, were added to a pulverizing chamber of a high-speed rotary pulverizer (capacity: 150 mL). The mixture was stirred at 14,000 rpm for 5 minutes in an air atmosphere to perform a mechanochemical reaction and a diimide tetracarboxylic acid precursor powder was obtained.

The reaction rate of the amino group derived from the 2-aminoterephthalic acid and the acid anhydride group of the pyromellitic dianhydride in the obtained pulverized product was measured and found to be 63.3%.

The obtained pulverized product was heated at 300°C for 2 hours under a nitrogen stream, and the reaction rate of the amino group in the obtained powder was determined and found to be 98.3%. Further, IR measurement was performed on the obtained powder. As a result, absorption derived from the imide group was observed near 1750 to 1800 cm$^{-1}$, and it was able to be confirmed that a diimide tetracarboxylic acid was formed.

Examples B6-2 to B6-24

A pulverized product (diimide tetracarboxylic acid) was obtained by performing the same operation as in Example B6-1, except that the composition of the acid anhydride and amine was changed.

After heating, IR measurement was performed on the obtained pulverized product by the same method as in Example B6-1, and the following items were confirmed:

- After heating, absorption derived from the imide group was observed, and it was able to be confirmed that diimide tetracarboxylic acid was formed.

(Production method of monoimide dicarboxylic acid)

Example B7-1

A total of 100 g, including 584 parts by mass of trimellitic anhydride and 416 parts by mass of 2-aminobenzoic acid, were added to a pulverizing chamber of a high-speed rotary pulverizer (capacity: 150 mL). The mixture was stirred at 14,000 rpm for 5 minutes in an air atmosphere to perform a mechanochemical reaction and a monoimide dicarboxylic acid precursor powder was obtained.

The reaction rate of the amino group derived from the 2-aminobenzoic acid and the acid anhydride group of the trimellitic anhydride in the obtained pulverized product was measured and found to be 65.5%.

The obtained pulverized product was heated at 300°C for 2 hours under a nitrogen stream, and the reaction rate of the amino group in the obtained powder was determined and found to be 98.2%. Further, IR measurement was performed on the obtained powder. As a result, absorption derived from the imide group was observed near 1750 to 1800 cm$^{-1}$, and it was able to be confirmed that a monoimide dicarboxylic acid was formed.

Examples B7-2 to B7-17

A pulverized product (monoimide dicarboxylic acid) was obtained by performing the same operation as in Example B7-1, except that the composition of the amine was changed.

After heating, IR measurement was performed on the obtained pulverized product by the same method as in Example B7-1, and the following items were confirmed:

- After heating, absorption derived from the imide group was observed, and it was able to be confirmed that monoimide dicarboxylic acid was formed.

(Production method of monoimide tricarboxylic acid)

Example B8-1

A total of 100 g, including 515 parts by mass of trimellitic anhydride and 485 parts by mass of 2-aminoterephthalic acid, were added to a pulverizing chamber of a high-speed rotary pulverizer (capacity: 150 mL). The mixture was stirred at 14,000 rpm for 5 minutes in an air atmosphere to perform a mechanochemical reaction and a monoimide tricarboxylic acid precursor powder was obtained.

The reaction rate of the amino group derived from the 2-aminoterephthalic acid and the acid anhydride group of the trimellitic anhydride in the obtained pulverized product was measured and found to be 69.7%.

The obtained pulverized product was heated at 300°C for 2 hours under a nitrogen stream, and the reaction rate of the amino group in the obtained powder was determined and found to be 99.4%. Further, IR measurement was performed on the obtained pulverized product. As a result, absorption derived from the imide group was observed near 1750 to 1800 cm$^{-1}$, and it was able to be confirmed that a monoimide tricarboxylic acid was formed.

Examples B8-2 to B8-4

A pulverized product (monoimide tricarboxylic acid) was obtained by performing the same operation as in Example B8-1, except that the composition of the amine was changed.

After heating, IR measurement was performed on the obtained pulverized product by the same method as in Example B8-1, and the following items were confirmed:

- After heating, absorption derived from the imide group was observed, and it was able to be confirmed that monoimide tricarboxylic acid was formed.

(Production method of amide group-containing imide-based compounds (amide group-containing diimide dicarboxylic acid-based compound, amide group-containing monoimide dicarboxylic acid-based compound, and amide group-containing monoimide tetracarboxylic acid-based compound))

Example B9-1

A total of 100 g, including 628 parts by mass of trimellitic anhydride and 372 parts by mass of 4,4'-diaminobenzanilide, were added to a pulverizing chamber of a high-speed rotary pulverizer (capacity: 150 mL). The mixture was stirred at 14,000 rpm for 5 minutes in an air atmosphere to perform a mechanochemical reaction and an amide group-containing imide-based compound precursor powder (amide group-containing diimide dicarboxylic acid-based compound precursor powder) was obtained.

The reaction rate of the amino group derived from the 4,4'-diaminobenzanilide and the acid anhydride group of the trimellitic anhydride in the obtained pulverized product was measured and found to be 63.2%.

The obtained pulverized product was heated at 300°C for 2 hours under a nitrogen stream, and the reaction rate of the amino group in the obtained powder was determined and found to be 98.4%. Further, IR measurement was performed on the obtained powder. As a result, absorption derived from the amide group was observed near 1515 to 1650 cm$^{-1}$, absorption derived from the imide group was observed near 1750 to 1800 cm$^{-1}$, and it was able to be confirmed that an amide group-containing imide-based compound (amide group-containing diimide dicarboxylic acid-based compound) was formed.

Example B9-2

A total of 100 g, including 435 parts by mass of trimellitic anhydride chloride and 565 parts by mass of 2-aminobenzoic acid, were added to a pulverizing chamber of a high-speed rotary pulverizer (capacity: 150 mL). The mixture was stirred at 14,000 rpm for 5 minutes in an air atmosphere to perform a mechanochemical reaction and an amide group-containing imide-based compound precursor powder (amide group-containing monoimide dicarboxylic acid-based compound precursor powder) was obtained.

The reaction rate of the amino group derived from the 2-aminobenzoic acid, and the acid chloride group and acid anhydride group of the trimellitic anhydride chloride in the obtained pulverized product was measured and found to be 71.3%.

The obtained pulverized product was heated at 300°C for 2 hours under a nitrogen stream, and the reaction rate of the amino group in the obtained powder was determined and found to be 98.7%. Further, IR measurement was performed on the obtained powder. As a result, absorption derived from the amide group was observed near 1515 to 1650 cm$^{-1}$, absorption derived from the imide group was observed near 1750 to 1800 cm$^{-1}$, and it was able to be confirmed that an amide group-containing imide-based compound (amide group-containing monoimide dicarboxylic acid-based compound) was formed.

Examples B9-3 to B9-6

An amide group-containing imide-based compound was obtained by performing the same operation as in Example B9-1, except that the composition of the amine was changed.

In Examples B9-3 and 9-4, by the same method as in Example B9-1, through the formation of an amide group-containing imide-based compound precursor powder (amide group-containing monoimide dicarboxylic acid-based compound precursor powder), it was confirmed that an amide group-containing monoimide dicarboxylic acid-based compound was formed.

In Examples B9-5 and B9-6, by the same method as in Example B9-1, through the formation of an amide group-containing imide-based compound precursor powder (amide group-containing monoimide tetracarboxylic acid-based compound precursor powder), it was confirmed that an amide group-containing monoimide tetracarboxylic acid-based compound was formed.

In Examples B9-3 to B9-6, after heating, IR measurement was performed on the obtained pulverized product by the same method as in Example B9-1, and the following items were confirmed:

- After heating, absorption derived from the amide group and the imide group was observed, and it was able to be confirmed that a given amide group-containing imide-based compound was formed.

(Production method of ester group-containing imide-based compound (ester group-containing monoimide tricarboxylic acid-based compound))

Example B10-1

A total of 100 g, including 778 parts by mass of trimellitic anhydride and 222 parts by mass of 2-aminophenol, were added to a pulverizing chamber of a high-speed rotary pulverizer (capacity: 150 mL). The mixture was stirred at 14,000 rpm for 5 minutes in an air atmosphere to perform a mechanochemical reaction and an ester group-containing imide-based compound precursor powder (ester group-containing monoimide tricarboxylic acid-based compound precursor powder) was obtained.

The reaction rate of the amino group and the hydroxyl group derived from the 2-aminophenol and the acid anhydride group of the trimellitic anhydride in the obtained pulverized product was measured and found to be 43.3%.

The obtained pulverized product was heated at 200°C for 1 hour, and further at an increased temperature of 270°C for 1 hour, under a nitrogen stream, and the reaction rate of the amino group and the hydroxyl group in the obtained powder was determined and found to be 97.3%. Further, IR measurement was performed on the obtained powder. As a result, absorption derived from the ester group was observed near 1715 to 1730 $cm^{-1}$, absorption derived from the imide group was observed near 1750 to 1800 $cm^{-1}$, and it was able to be confirmed that an ester group-containing imide-based compound (ester group-containing monoimide tricarboxylic acid-based compound) was formed.

Examples B10-2 to B10-16

An ester group-containing imide-based compound (ester group-containing monoimide tricarboxylic acid-based compound) was obtained by performing the same operation as in Example B10-1, except that the composition of the amine was changed.

In these examples, by the same method as in Example B10-1, through the formation of an ester group-containing imide-based compound precursor powder (ester group-containing monoimide tricarboxylic acid-based compound precursor powder), it was confirmed that an ester group-containing monoimide tricarboxylic acid-based compound was formed.

In each example, after heating, IR measurement was performed on the obtained pulverized product by the same method as in Example B10-1, and the following items were confirmed:

- After heating, absorption derived from the ester group and the imide group was observed, and it was able to be confirmed that a given ester group-containing imide-based compound was formed.

In particular, in the NMR measurement (before heating) of Example B10-3, in the $^1$H-NMR spectrum, peaks derived from the reaction product in which the amino group was amidated near 6.72 ppm (doublet) and 7.44 ppm (doublet), and peaks derived from unreacted amine were detected near 6.44 ppm (doublet).

(Production method of hydroxyl group-containing imide-based compound (diimide dihydroxy-based compound (particularly diimide diphenol-based compound)))

Example B11-1

A total of 100 g, including 501 parts by mass of pyromellitic dianhydride and 499 parts by mass of 3-aminophenol, were added to a pulverizing chamber of a high-speed rotary pulverizer (capacity: 150 mL). The mixture was stirred at 14,000 rpm for 5 minutes in an air atmosphere to perform a mechanochemical reaction and a hydroxyl group-containing imide-based compound precursor powder (diimide diphenol-based compound precursor powder) was obtained.

The reaction rate of the amino group derived from the 3-aminophenol and the acid anhydride group of the pyromellitic dianhydride in the obtained pulverized product was measured and found to be 66.4%.

The obtained pulverized product was heated at 200°C for 1 hour, and further at an increased temperature of 270°C for 1 hour, under a nitrogen stream, and the reaction rate of the amino group in the obtained powder was determined and found to be 99.0%. In addition, peaks derived from the hydroxyl group was detected near 5.0 to 5.5 ppm in the $^1$H-NMR spectrum. Further, IR measurement was performed on the obtained powder. As a result, absorption derived from the imide group was observed near 1750 to 1800 cm$^{-1}$, and it was able to be confirmed that a hydroxyl group-containing imide-based compound (diimide diphenol-based compound) was formed.

Examples B11-2 to B11-24

A hydroxyl group-containing imide-based compound (diimide dihydroxy-based compound (particularly diimide diphenol-based compound)) was obtained by performing the same operation as in Example B11-1, except that the composition of the acid anhydride and the composition of the amine were changed.

In these examples, by the same method as in Example B11-1, through the formation of a hydroxyl group-containing imide-based compound precursor powder (diimide diphenol-based compound precursor powder), it was confirmed that a diimide diphenol-based compound was formed.

In each example, after heating, $^1$H-NMR measurement and IR measurement were performed on the obtained pulverized product by the same method as in Example B11-1, and the following items were confirmed:

- After heating, peaks derived from the hydroxyl group were observed in the $^1$H-NMR spectrum.
- After heating, absorption derived from the imide group was observed in the IR spectrum, and it was able to be confirmed that a given hydroxyl group-containing imide-based compound was formed.

(Production method of amide group-containing carboxylic acid-based compounds (diamide dicarboxylic acid-based compound and diamide tetracarboxylic acid-based compound))

Example B12-1

A total of 100 g, including 426 parts by mass of terephthalic acid chloride and 574 parts by mass of 3-aminobenzoic acid, were added to a pulverizing chamber of a high-speed rotary pulverizer (capacity: 150 mL). The mixture was stirred at 14,000 rpm for 5 minutes in an air atmosphere to perform a mechanochemical reaction and an amide group-containing carboxylic acid-based compound powder (diamide dicarboxylic acid-based compound powder) was obtained.

The reaction rate of the amino group derived from the 2-aminobenzoic acid and the acid chloride group of the terephthalic acid chloride in the obtained pulverized product was measured and found to be 70.1%.

The obtained pulverized product was heated under the conditions of 100°C for 1 hour, then 150°C for 1 hour, and 250°C for 1 hour in a nitrogen atmosphere. The reaction rate of the amino group in the obtained powder was determined and found to be 99.0%. IR measurement was performed on the obtained powder. As a result, absorption derived from the amide group was observed near 1515 to 1650 cm$^{-1}$, and it was able to be confirmed that an amide group-containing carboxylic acid-based compound (diamide dicarboxylic acid-based compound) was formed.

Examples B12-2 to B12-10

An amide group-containing carboxylic acid-based compound (diamide dicarboxylic acid-based compound or diamide tetracarboxylic acid-based compound) was obtained by performing the same operation as in Example B12-1, except that the composition of the acid chloride and the composition of the amine were changed.

In Examples B12-2 to B12-3 and B12-6 to B12-8, by the same method as in Example B12-1, through the formation of an amide group-containing carboxylic acid-based compound precursor powder (diamide dicarboxylic acid-based compound precursor powder), it was confirmed that a diamide dicarboxylic acid-based compound was formed.

In Examples B12-4 to B12-5 and B12-9 to B12-10, by the same method as in Example B12-1, through the formation of an amide group-containing carboxylic acid-based compound precursor powder (diamide tetracarboxylic acid-based compound precursor powder), it was confirmed that a diamide tetracarboxylic acid-based compound was formed.

In each example, after heating, IR measurement was performed on the obtained pulverized product by the same method as in Example B12-1, and the following items were confirmed:

- After heating, absorption derived from the amide group was observed, and it was able to be confirmed that a given amide group-containing carboxylic acid-based compound was formed.

(Production method of ester group-containing carboxylic acid-based compounds (diester dicarboxylic acid-based compound and diester tetracarboxylic acid-based compound))

Example B13-1

A total of 100 g, including 424 parts by mass of terephthalic acid chloride and 576 parts by mass of 4-hydroxybenzoic acid, were added to a pulverizing chamber of a high-speed rotary pulverizer (capacity: 150 mL). The mixture was stirred at 14,000 rpm for 5 minutes in an air atmosphere to perform a mechanochemical reaction and an ester group-containing carboxylic acid-based compound powder (diester dicarboxylic acid-based compound powder) was obtained.

The reaction rate of the hydroxyl group derived from the 4-hydroxybenzoic acid and the acid chloride group of the terephthalic acid chloride in the obtained pulverized product was measured and found to be 66.2%.

The obtained pulverized product was heated under the conditions of 100°C for 1 hour, then 150°C for 1 hour, and 250°C for 1 hour in a nitrogen atmosphere. The reaction rate of the hydroxyl group in the obtained powder was determined and found to be 97.8%. IR measurement was performed on the obtained powder. As a result, absorption derived from the ester group was observed near 1715 to 1730 cm$^{-1}$, and it was able to be confirmed that an ester group-containing carboxylic acid-based compound (diester dicarboxylic acid-based compound) was formed.

Examples B13-2 to B13-22

An ester group-containing carboxylic acid-based compound (diester dicarboxylic acid-based compound or diester tetracarboxylic acid-based compound) was obtained by performing the same operation as in Example B13-1, except that the composition of the acid chloride and the composition of the hydroxy were changed.

In Examples B13-2 to B13-9 and B13-12 to B13-20, by the same method as in Example B13-1, through the formation of an ester group-containing carboxylic acid-based compound precursor powder (diester dicarboxylic acid-based compound precursor powder), it was confirmed that a diester dicarboxylic acid-based compound was formed.

In Examples B13-10 to B13-11 and B13-21 to B13-22, by the same method as in Example B13-1, through the formation of an ester group-containing carboxylic acid-based compound precursor powder (diester tetracarboxylic acid-based compound precursor powder), it was confirmed that a diester tetracarboxylic acid-based compound was formed.

In each example, after heating, IR measurement was performed on the obtained pulverized product by the same method as in Example B13-1, and the following items were confirmed:

- After heating, absorption derived from the ester group was observed, and it was able to be confirmed that a given ester group-containing carboxylic acid-based compound was formed.

(Production method of curable imide-based compound)

Example B14-1

A total of 100 g, including 821 parts by mass of 4-phenylethynyl phthalic anhydride and 179 parts by mass of p-phenylenediamine, were added to a pulverizing chamber of a high-speed rotary pulverizer (capacity: 150 mL). The mixture was stirred at 14,000 rpm for 5 minutes in an air atmosphere to perform a mechanochemical reaction and a curable diimide-based compound precursor powder was obtained.

The reaction rate of the amino group derived from the p-phenylenediamine and the acid anhydride group of the 4-phenylethynyl phthalic anhydride in the obtained pulverized product was measured and found to be 64.3%.

The obtained pulverized product was heated under the conditions of 170°C for 1 hour, then 210°C for 1 hour, and 270°C for 1 hour in a nitrogen atmosphere. The reaction rate of the amino group in the obtained powder was determined and found to be 98.0%. IR measurement was performed on the obtained powder. As a result, absorption derived from the imide group was observed near 1750 to 1800 cm$^{-1}$, absorption derived from the ethynyl group was observed near 2100 to 2260 cm$^{-1}$, and it was able to be confirmed that a curable diimide-based compound was formed.

Examples B14-2 to B14-8

A curable diimide-based compound was obtained by performing the same operation as in Example B14-1, except that the composition of the acid anhydride and the composition of the amine were changed.

In Examples B14-2 to B14-8, by the same method as in Example B14-1, through the formation of a curable diimide-based compound precursor powder, it was confirmed that a curable diimide-based compound was formed.

In each example, after heating, IR measurement was performed on the obtained pulverized product by the same method as in Example B14-1, and the following items were confirmed:

- After heating, absorption derived from the unsaturated bond and the imide group was observed, and it was able to be confirmed that a curable diimide-based compound was formed.

[Experimental Example C: Production method of high-molecular compound (2-step mechanochemical method)]

(Production method of polyamic acid-based compounds (polyimide resin precursor and polyimide resin))

Example C1

First, a first-step mechanochemical treatment was performed. Specifically, a total of 5.0 kg, including 521 parts by mass of pyromellitic dianhydride and 479 parts by mass of 4,4'-diaminodiphenyl ether, were added to a high-speed bottom stirring type mixer (capacity: 20 L). The mixture was pulverized at 5600 rpm for 3 minutes in a nitrogen atmosphere to perform the first-step mechanochemical treatment and a polyimide resin precursor powder was obtained.

Next, a second-step mechanochemical treatment was performed. Specifically, 1.0 kg of the polyimide precursor powder obtained by the first-step mechanochemical treatment and 6.0 kg of alumina balls were added into a medium-stirring type mill chamber (capacity 5.0 L). The mixture was pulverized at 504 rpm for 30 minutes in a nitrogen atmosphere to perform the second-step mechanochemical treatment and a polyimide resin precursor powder was obtained.

The reaction rate of the amino group derived from 4,4'-diaminodiphenyl ether and the acid anhydride group of pyromellitic anhydride in the obtained final pulverized product was measured and found to be 90.1%, and the average degree of polymerization was 10.3. The average particle size of the obtained pulverized product was 14.2 $\mu$m. IR measurement was performed on the obtained powder. As a result, imidization did not proceed, and the imidization ratio was 0.0%.

In the NMR measurement of the example, as in Example A1-1, peaks derived from the reaction product in which both amino groups were amidated, peaks derived from the reaction product in which one amino group was amidated, and peaks derived from unreacted amine were detected, respectively, in the $^1$H-NMR spectrum.

The obtained pulverized product was heated at 300°C for 2 hours under a nitrogen stream, and IR measurement was performed on the obtained powder. As a result, absorption derived from the imide group was observed near 1750 to 1800 cm$^{-1}$, and it was able to be confirmed that a polyimide resin was formed.

Examples C2 to C4

A polyimide resin precursor powder was obtained by performing the same two-step mechanochemical operation as in Example C1, except that the composition of the acid component composition and the mixing ratio were changed.

The reaction rate of the amino group derived from 4,4'-diaminodiphenyl ether and the acid anhydride group of each acid component in the obtained final pulverized product, the average degree of polymerization, and the average particle size of the obtained pulverized product were measured.

IR measurement was performed on the obtained powder of each example. As a result, imidization did not proceed, and the imidization ratio was 0.0%.

In the NMR measurement of each example, as in Example A1-1, peaks derived from the reaction product in which both amino groups were amidated, peaks derived from the reaction product in which one amino group was amidated, and peaks derived from unreacted amine were detected, respectively, in the $^1$H-NMR spectrum.

The obtained pulverized product in each example was heated at 300°C for 2 hours under a nitrogen stream, and IR measurement was performed. As a result, absorption derived from the imide group was observed near 1750 to 1800 cm$^{-1}$, and it was able to be confirmed that a polyimide resin was formed.

[Experimental Example D: Production method of low-molecular compound (2-step mechanochemical method)]

(Production method of diimide dicarboxylic acid-based compound)

Example D1

First, a first-step mechanochemical treatment was performed. Specifically, a total of 5.0 kg, including 657 parts by mass of trimellitic anhydride and 343 parts by mass of 4,4-diaminodiphenyl ether, were added to a high-speed bottom stirring type mixer (capacity: 20 L). The mixture was pulverized at 5600 rpm for 3 minutes in a nitrogen atmosphere to perform the first-step mechanochemical reaction and a diimide dicarboxylic acid precursor powder was obtained.

Next, a second-step mechanochemical treatment was performed. Specifically, 1.0 kg of the diimide dicarboxylic acid precursor powder obtained by the first-step mechanochemical treatment and 6.0 kg of alumina balls were added into a medium-stirring type mill chamber (capacity 5.0 L). The mixture was pulverized at 504 rpm for 30 minutes in a nitrogen atmosphere to perform the second-step mechanochemical treatment and a diimide dicarboxylic acid precursor powder was obtained.

The reaction rate of the amino group derived from the 4,4'-diaminodiphenyl ether and the acid anhydride group of the trimellitic anhydride in the obtained final pulverized product was measured and found to be 92.3%. The average particle size of the obtained pulverized product was 13.3 $\mu$m. IR measurement was performed on the obtained powder. As a result, imidization did not proceed, and the imidization ratio was 0.0%.

The obtained pulverized product was heated at 300°C for 2 hours under a nitrogen stream, and the reaction rate of the amino group in the obtained powder was determined and found to be 99.1%. Further, IR measurement was performed on the obtained powder. As a result, absorption derived from the imide group was observed near 1750 to 1800 cm$^{-1}$, and it was able to be confirmed that a diimide dicarboxylic acid was formed.

(Production method of ester group-containing imide-based compound (ester group-containing monoimide tricarboxylic acid-based compound))

Example D2

First, a first-step mechanochemical treatment was performed. Specifically, a total of 5.0 kg, including 778 parts by mass of trimellitic anhydride and 222 parts by mass of 4-aminophenol, were added to a high-speed bottom stirring type mixer (capacity: 20 L). The mixture was pulverized at 5600 rpm for 3 minutes in a nitrogen atmosphere to perform the first-step mechanochemical treatment and an ester group-containing imide-based compound precursor powder (ester group-containing monoimide tricarboxylic acid-based compound precursor powder) was obtained.

Next, a second-step mechanochemical treatment was performed. Specifically, 1.0 kg of the ester group-containing imide-based compound precursor powder obtained by the first-step mechanochemical treatment and 6.0 kg of alumina balls were added into a medium-stirring type mill chamber (capacity 5.0 L). The mixture was pulverized at 504 rpm for 30 minutes in a nitrogen atmosphere to perform the second-step mechanochemical treatment and an ester group-containing imide-based compound precursor powder was obtained.

The reaction rate of the amino group and the hydroxyl group derived from the 4-aminophenol and the acid anhydride group of the trimellitic anhydride in the obtained final pulverized product was measured and found to be 87.5%. The average particle size of the obtained pulverized product was 15.6 $\mu$m. IR measurement was performed on the obtained powder. As a result, imidization did not proceed, and the imidization ratio was 0.0%.

The obtained pulverized product was heated at 200°C for 1 hour, and further at an increased temperature of 270°C for 1 hour, under a nitrogen stream, and the reaction rate of the amino group and the hydroxyl group in the obtained powder was determined and found to be 98.7%. Further, IR measurement was performed on the obtained powder. As a result, absorption derived from the ester group was observed near 1715 to 1730 cm$^{-1}$, absorption derived from the imide group was observed near 1750 to 1800 cm$^{-1}$, and it was able to be confirmed that an ester group-containing imide-based compound (ester group-containing monoimide tricarboxylic acid-based compound) was formed.

(Production method of hydroxyl group-containing imide-based compound (diimide dihydroxy-based compound (particularly diimide diphenol-based compound)))

Example D3

First, a first-step mechanochemical treatment was performed. Specifically, a total of 5.0 kg, including 597 parts by mass of 3,3',4,4'-benzophenone tetracarboxylic dianhydride and 403 parts by mass of 4-aminophenol, were added to a high-speed bottom stirring type mixer (capacity: 20 L). The mixture was pulverized at 5600 rpm for 3 minutes in a nitrogen atmosphere to perform the first-step mechanochemical treatment and a hydroxyl group-containing imide-based compound precursor powder (diimide diphenol-based compound precursor powder) was obtained.

Next, a second-step mechanochemical treatment was performed. Specifically, 1.0 kg of the hydroxyl group-containing imide-based compound precursor powder (diimide diphenol-based compound precursor powder) obtained by the first-step mechanochemical treatment and 6.0 kg of alumina balls were added into a medium-stirring type mill chamber (capacity 5.0 L). The mixture was pulverized at 504 rpm for 30 minutes in a nitrogen atmosphere to perform the second-step mechanochemical treatment and a hydroxyl group-containing imide-based compound precursor powder (diimide diphenol-based compound precursor powder) was obtained.

The reaction rate of the amino group derived from the 4-aminophenol and the acid anhydride group of the 3,3',4,4'-benzophenone tetracarboxylic dianhydride in the obtained final pulverized product was measured and found to be 93.4%. The average particle size of the obtained pulverized product was 12.5 $\mu$m. IR measurement was performed on the obtained powder. As a result, imidization did not proceed, and the imidization ratio was 0.0%.

The obtained pulverized product was heated at 200°C for 1 hour, and further at an increased temperature of 270°C for 1 hour, under a nitrogen stream, and the reaction rate of the amino group in the obtained powder was determined and found to be 98.9%. In addition, NMR measurement was performed on the obtained powder. As a result, peaks derived from the hydroxyl group were detected near 5.0 to 5.5 ppm in the $^1$H-NMR spectrum. Further, IR measurement was performed on the obtained powder. As a result, absorption derived from the imide group was observed near 1750 to 1800 cm[-1], and it was able to be confirmed that a hydroxyl group-containing imide-based compound (diimide diphenol-based compound) was formed.

[Table 1A]

| Polyamic acid-based compound (solid/solid reaction) | | | | | | | |
|---|---|---|---|---|---|---|---|
| Examples | Raw materials | | Mixing ratio (part by mass) | | Evaluation | | |
| | Acid component | Amine component | Acid component | Amine component | Reaction rate (%) | Average degree of polymerization | Average particle size (μm) |
| A1-1 | Pyromellitic dianhydride | 4,4'-Diaminodiphenyl ether | 521 | 479 | 78.2 | 9.2 | 48.2 |
| A1-2 | | p-Phenylenediamine | 669 | 331 | 67.5 | 6.2 | 49.4 |
| A1-3 | | m-Phenylenediamine | 669 | 331 | 70.4 | 6.8 | 46.5 |
| A1-4 | | 3,4'-Diaminodiphenyl ether | 521 | 479 | 79.6 | 9.8 | 50.1 |
| A1-5 | | 1,4-Bis(4-aminophenoxy)benzene | 427 | 573 | 56.1 | 4.6 | 49.5 |
| A1-6 | | 1,3-Bis(4-aminophenoxy)benzene | 427 | 573 | 63.6 | 5.5 | 49.9 |
| A1-7 | | 4,4'-Bis(4-aminophenoxy)biphenyl | 372 | 628 | 69.7 | 6.6 | 50.3 |
| A1-8 | | 4,4'-Bis(3-aminophenoxy)biphenyl | 372 | 628 | 72.1 | 7.2 | 48.6 |
| A1-9 | | 2,2-Bis[4-(4-aminophenoxy)phenyl]propane | 347 | 653 | 66.7 | 6.0 | 42.3 |
| A1-10 | | Bis(4-aminophenyl)sulfone | 468 | 532 | 62.1 | 5.3 | 48.1 |
| A1-11 | | Bis[4-(4-aminophenoxy)phenyl]sulfone | 335 | 665 | 69.7 | 6.6 | 47.7 |
| A1-12 | | 9,9-Bis(4-aminophenyl)fluorene | 385 | 615 | 68.8 | 6.4 | 52.2 |
| A1-13 | | trans-1,4-Cyclohexanediamine | 656 | 344 | 38.0 | 3.2 | 42.5 |
| A1-14 | | 4,4'-Methylenebis(cyclohexylamine) | 509 | 491 | 33.7 | 3.0 | 45.4 |
| A1-15 | | 1,10-Diaminodecane | 559 | 441 | 50.5 | 4.0 | 50.3 |
| A1-16 | | 1,12-Diaminododecane | 521 | 479 | 47.2 | 3.8 | 49.9 |

73

[Table 1B]

| Polyamic acid-based compound (solid/solid reaction) | | | | | | | |
|---|---|---|---|---|---|---|---|
| Examples | Raw materials | | Mixing ratio (part by mass) | | Evaluation | | |
| | Acid component | Amine component | Acid component | Amine component | Reaction rate (%) | Average degree of polymerization | Average particle size ($\mu$m) |
| A1-17 | 3,3',4,4'-Biphenyltetracarboxy lic dianhydride | p-Phenylenediamine | 731 | 269 | 62.3 | 5.3 | 46.2 |
| A1-18 | | m-Phenylenediamine | 731 | 269 | 71.3 | 7.0 | 47.3 |
| A1-19 | | 4,4'-Diaminodiphenyl ether | 595 | 405 | 72.8 | 7.4 | 50.2 |
| A1-20 | | 3,4'-Diaminodiphenyl ether | 595 | 405 | 78.9 | 9.5 | 50.5 |
| A1-21 | | 1,4-Bis(4-aminophenoxy) benzene | 502 | 498 | 55.2 | 4.5 | 48.7 |
| A1-22 | | 1,3 -Bis(4-aminophenoxy) benzene | 502 | 498 | 62.6 | 5.3 | 44.3 |
| A1-23 | | 4,4'-Bis(4-aminophenoxy) biphenyl | 444 | 556 | 70.5 | 6.8 | 47.2 |
| A1-24 | | 4,4'-Bis(3-aminophenoxy) biphenyl | 444 | 556 | 73.1 | 7.4 | 48.9 |
| A1-25 | | 2,2-Bis[4-(4-aminophenoxy) phenyl]propane | 417 | 583 | 69.3 | 6.5 | 50.9 |
| A1-26 | | Bis(4-aminophenyl)sulfone | 542 | 458 | 66.3 | 5.9 | 48.1 |
| A1-27 | | Bis[4-(4-aminophenoxy)phenyl] sulfone | 405 | 595 | 69.7 | 6.6 | 43.1 |
| A1-28 | | 9,9-Bis(4-aminophenyl)fluorene | 458 | 542 | 67.9 | 6.2 | 49.4 |
| A1-29 | | trans-1,4-Cyclohexanediamine | 720 | 280 | 35.1 | 3.1 | 53.9 |
| A1-30 | | 4,4'-Methylenebis (cyclohexylamine) | 583 | 417 | 32.2 | 2.9 | 54.8 |
| A1-31 | | 1,10-Diaminodecane | 631 | 369 | 51.1 | 4.1 | 51.2 |
| A1-32 | | 1,12-Diaminododecane | 595 | 405 | 47.4 | 3.8 | 54.1 |

[Table 1C]

| | Raw materials | | Mixing ratio (part by mass) | | Evaluation | | |
|---|---|---|---|---|---|---|---|
| Examples | Acid component | Amine component | Acid component | Amine component | Reaction rate (%) | Average degree of polymerization | Average particle size ($\mu$m) |
| A1-33 | 2,3,3',4'-Biphenyltetracarboxy lic dianhydride | p-Phenylenediamine | 731 | 269 | 58.2 | 4.8 | 45.2 |
| A1-34 | | m-Phenylenediamine | 731 | 269 | 72.4 | 7.2 | 48.3 |
| A1-35 | | 4,4'-Diaminodiphenyl ether | 595 | 405 | 73.8 | 7.6 | 50.4 |
| A1-36 | | 3,4'-Diaminodiphenyl ether | 595 | 405 | 76.5 | 8.5 | 50.0 |
| A1-37 | | 1,4-Bis(4-aminophenoxy) benzene | 502 | 498 | 56.8 | 4.6 | 47.7 |
| A1-38 | | 1,3-Bis(4-aminophenoxy) benzene | 502 | 498 | 61.8 | 5.2 | 45.3 |
| A1-39 | | 4,4'-Bis(4-aminophenoxy) biphenyl | 444 | 556 | 71.1 | 6.9 | 48.2 |
| A1-40 | | 4,4'-Bis(3-aminophenoxy) biphenyl | 444 | 556 | 74.2 | 7.8 | 49.0 |
| A1-41 | | 2,2-Bis[4-(4-aminophenoxy) phenyl]propane | 417 | 583 | 68.8 | 6.4 | 51.0 |
| A1-42 | | Bis(4-aminophenyl)sulfone | 542 | 458 | 67.9 | 6.2 | 48.1 |
| A1-43 | | Bis[4-(4-aminophenoxy)phenyl] sulfone | 405 | 595 | 69.6 | 6.6 | 43.3 |
| A1-44 | | 9,9-Bis(4-aminophenyl)fluorene | 458 | 542 | 67.9 | 6.2 | 47.2 |
| A1-45 | | trans-1,4-Cyclohexanediamine | 720 | 280 | 36.1 | 3.1 | 53.3 |
| A1-46 | | 4,4'-Methylenebis (cyclohexylamine) | 583 | 417 | 32.3 | 3.0 | 59.1 |
| A1-47 | | 1,10-Diaminodecane | 631 | 369 | 60.1 | 5.0 | 49.2 |
| A1-48 | | 1,12-Diaminododecane | 595 | 405 | 49.2 | 3.9 | 48.1 |

Polyamic acid-based compound (solid/solid reaction)

[Table 1D]

| Polyamic acid-based compound (solid/solid reaction) | | | | | | | |
|---|---|---|---|---|---|---|---|
| Examples | Raw materials | | Mixing ratio (part by mass) | | Evaluation | | |
| | Acid component | Amine component | Acid component | Amine component | Reaction rate (%) | Average degree of polymerization | Average particle size (μm) |
| A1-49 | 3,3',4,4'-Benzophenone tetracarboxylic dianhydride | p-Phenylenediamine | 749 | 251 | 61.1 | 5.1 | 47.7 |
| A1-50 | | m-Phenylenediamine | 749 | 251 | 70.3 | 6.7 | 48.9 |
| A1-51 | | 4,4'-Diaminodiphenyl ether | 617 | 383 | 72.2 | 7.2 | 46.2 |
| A1-52 | | 3,4'-Diaminodiphenyl ether | 617 | 383 | 76.3 | 8.4 | 43.8 |
| A1-53 | | 1,4-Bis(4-aminophenoxy) benzene | 524 | 476 | 54.1 | 4.4 | 47.1 |
| A1-54 | | 1,3-Bis(4-aminophenoxy) benzene | 524 | 476 | 62.6 | 5.3 | 45.4 |
| A1-55 | | 4,4'-Bis(4-aminophenoxy) biphenyl | 467 | 533 | 71.5 | 7.0 | 52.3 |
| A1-56 | | 4,4'-Bis(3-aminophenoxy) biphenyl | 467 | 533 | 74.3 | 7.8 | 49.8 |
| A1-57 | | 2,2-Bis[4-(4-aminophenoxy) phenyl]propane | 440 | 560 | 69.9 | 6.6 | 50.1 |
| A1-58 | | Bis(4-aminophenyl)sulfone | 565 | 435 | 66.5 | 6.0 | 52.8 |
| A1-59 | | Bis[4-(4-aminophenoxy) phenyl]sulfone | 427 | 573 | 69.7 | 6.6 | 53.9 |
| A1-60 | | 9,9-Bis(4-aminophenyl) fluorene | 480 | 520 | 68.1 | 6.3 | 48.7 |
| A1-61 | | trans-1,4-Cyclohexanediamine | 738 | 262 | 33.2 | 3.0 | 54.0 |
| A1-62 | | 4,4'-Methylenebis (cyclohexylamine) | 605 | 395 | 35.2 | 3.1 | 54.8 |
| A1-63 | | 1,10-Diaminodecane | 652 | 348 | 52.1 | 4.2 | 53.5 |
| A1-64 | | 1,12-Diaminododecane | 617 | 383 | 48.4 | 3.9 | 58.0 |

[Table 1E]

| Polyamic acid-based compound (solid/solid reaction) | | | | | | | |
|---|---|---|---|---|---|---|---|
| Examples | Raw materials | | Mixing ratio (part by mass) | | Evaluation | | |
| | Acid component | Amine component | Acid component | Amine component | Reaction rate (%) | Average degree of polymerization | Average particle size (μm) |
| A1-65 | 4,4'-Oxydiphthalic anhydride | p-Phenylenediamine | 742 | 258 | 60.3 | 5.0 | 48.1 |
| A1-66 | | m-Phenylenediamine | 742 | 258 | 69.3 | 6.5 | 49.2 |
| A1-67 | | 4,4'-Diaminodiphenyl ether | 608 | 392 | 71.1 | 6.9 | 47.3 |
| A1-68 | | 3,4'-Diaminodiphenyl ether | 608 | 392 | 77.3 | 8.8 | 42.2 |
| A1-69 | | 1,4-Bis(4-aminophenoxy)benzene | 515 | 485 | 53.1 | 4.3 | 45.5 |
| A1-70 | | 1,3-Bis(4-aminophenoxy)benzene | 515 | 485 | 63.6 | 5.5 | 46.1 |
| A1-71 | | 4,4'-Bis(4-aminophenoxy) biphenyl | 457 | 543 | 70.5 | 6.8 | 51.3 |
| A1-72 | | 4,4'-Bis(3-aminophenoxy) biphenyl | 457 | 543 | 73.3 | 7.5 | 50.1 |
| A1-73 | | 2,2-Bis[4-(4-aminophenoxy) phenyl]propane | 430 | 570 | 68.9 | 6.4 | 42.1 |
| A1-74 | | Bis(4-aminophenyl)sulfone | 555 | 445 | 65.9 | 5.9 | 43.5 |
| A1-75 | | Bis[4-(4-aminophenoxy)phenyl] sulfone | 418 | 582 | 68.7 | 6.4 | 53.3 |
| A1-76 | | 9,9-Bis(4-aminophenyl)fluorene | 471 | 529 | 69.2 | 6.5 | 47.7 |
| A1-77 | | trans-1,4-Cyclohexanediamine | 731 | 269 | 36.4 | 3.1 | 52.4 |
| A1-78 | | 4,4'-Methylenebis (cyclohexylamine) | 596 | 404 | 37.4 | 3.2 | 55.3 |
| A1-79 | | 1,10-Diaminodecane | 643 | 357 | 53.1 | 4.3 | 52.1 |
| A1-80 | | 1,12-Diaminododecane | 608 | 392 | 47.2 | 3.8 | 57.0 |

[Table 2]

| | Raw materials | | Mixing ratio (part by mass) | | Evaluation | | |
|---|---|---|---|---|---|---|---|
| Polyamic acid-based compound (solid/liquid reaction) | | | | | | | |
| Examples | Acid component | Amine component | Acid component | Amine component | Reaction rate (%) | Average degree of polymerization | Average particle size (μm) |
| A1-81 | Pyromellitic dianhydride | Meta-xylylenediamine | 616 | 384 | 65.7 | 5.8 | 46.1 |
| A1-82 | | 1,4-Bis(aminomethyl) cyclohexane | 605 | 395 | 57.5 | 4.7 | 43.3 |
| A1-83 | | PRIAMINE 1075 (Croda Japan KK) | 287 | 713 | 80.2 | 10.1 | 48.8 |
| A1-84 | 3,3',4,4'-Biphenyltetracarboxy lic dianhydride | 1,4-Bis(aminomethyl) cyclohexane | 674 | 326 | 56.5 | 4.6 | 52.1 |
| A1-85 | | Meta-xylylenediamine | 684 | 316 | 66.0 | 5.9 | 44.4 |
| A1-86 | | PRIAMINE 1075 (Croda Japan KK) | 352 | 648 | 79.9 | 10.0 | 47.3 |
| A1-87 | 2,3,3',4'-Biphenyltetracarboxy lic dianhydride | 1,4-Bis(aminomethyl) cyclohexane | 674 | 326 | 52.1 | 4.2 | 42.5 |
| A1-88 | | Meta-xylylenediamine | 684 | 316 | 65.6 | 5.8 | 50.9 |
| A1-89 | | PRIAMINE 1075 (Croda Japan KK) | 352 | 648 | 79.0 | 9.5 | 42.9 |
| A1-90 | 3,3',4,4'-Benzophenone tetracarboxylic dianhydride | 1,4-Bis(aminomethyl) cyclohexane | 694 | 306 | 50.1 | 4.0 | 49.5 |
| A1-91 | | Meta-xylylenediamine | 703 | 297 | 62.7 | 5.4 | 53.4 |
| A1-92 | | PRIAMINE 1075 (Croda Japan KK) | 373 | 627 | 78.2 | 9.2 | 40.3 |
| A1-93 | 4,4'-Oxydiphthalic anhydride | 1,4-Bis(aminomethyl) cyclohexane | 686 | 314 | 56.6 | 4.6 | 50.1 |
| A1-94 | | Meta-xylylenediamine | 695 | 305 | 66.7 | 6.0 | 48.2 |
| A1-95 | | PRIAMINE 1075 (Croda Japan KK) | 364 | 636 | 77.2 | 8.8 | 41.2 |

[Table 3]

| Polyimide-based compound (solid/solid reaction) | | | | | | |
|---|---|---|---|---|---|---|
| Examples | Raw materials | | Mixing ratio (part by mass) | | Evaluation | |
| | Acid component | Diisocyanate component | Acid component | Diisocyanate component | Reaction rate (%) | Average particle size ($\mu$m) |
| A2-1 | Pyromellitic dianhydride | Diphenylmethane diisocyanate | 466 | 534 | Solvent insoluble | 44.1 |
| A2-2 | | 1,5-Diisocyanato naphthalene | 509 | 491 | Solvent insoluble | 42.3 |

[Table 4]

| Polyamide-based compound (solid/solid reaction) | | | | | | |
|---|---|---|---|---|---|---|
| Examples | Raw materials | | Mixing ratio (part by mass) | | Evaluation | |
| | Acid component | Diamine and isocyanate components | Acid component | Diamine and diisocyanate components | (%) Reaction rate (%) | Average particle size ($\mu$m) |
| A3-1 | Terephthalic acid chloride | Para-phenylenediamine | 652 | 348 | Solvent insoluble | 56.5 |
| A3-2 | | Meta-phenylenediamine | 652 | 348 | Solvent insoluble | 52.3 |
| A3-3 | | 1,10-Diaminodecane | 541 | 459 | 72.8 | 58.9 |
| A3-4 | | 1,12-Diaminododecane | 503 | 497 | 70.3 | 60.1 |
| A3-5 | Terephthalic acid | Diphenylmethane diisocyanate | 399 | 601 | Solvent insoluble | 44.2 |
| A3-6 | | 1,5-Diisocyanato naphthalene | 441 | 559 | Solvent insoluble | 43.1 |

[Table 5]

| Polyamide-imide-based compound (solid/solid reaction) | | | | | | | |
| --- | --- | --- | --- | --- | --- | --- | --- |
| Examples | Raw materials | | Mixing ratio (part by mass) | | Evaluation | | |
| | Acid component | Diamine component | Acid component | Diamine component | Reaction rate after mechanochemical treatment (%) | Average particle size (μm) | Reaction rate after heat treatment (%) |
| A4-1 | Trimellitic anhydride chloride | 4,4'-Diaminodiphenyl ether | 513 | 487 | 73.3 | 42.1 | 95.7 |
| A4-2 | | 3,4'-Diaminodiphenyl ether | 513 | 487 | 74.5 | 40.2 | 94.3 |
| A4-3 | Trimellitic anhydride | Diphenylmethane diisocyanate | 434 | 566 | 36.2 | 45.3 | 92.1 |
| A4-4 | | 1,5-Diisocyanato naphthalene | 478 | 522 | 38.8 | 45.1 | 92.3 |

EP 3 819 283 A1

[Table 6]

| Polyester-based compound (solid/solid reaction) | | | | | | | |
|---|---|---|---|---|---|---|---|
| Examples | Raw materials | | Mixing ratio (part by mass) | | Evaluation | | |
| | Acid component | Bisphenol component | Acid component | Bisphenol component | Reaction rate after mechanochemical treatment (%) | Average particle size ($\mu$m) | Reaction rate after heat treatment (%) |
| A5-1 | Terephthalic acid chloride | 2,2'-Bis(4-hydroxyphenyl) propane | 471 | 529 | 70.1 | 33.1 | 94.5 |
| A5-2 | Isophthalic acid chloride | 2,2'-Bis(4-hydroxyphenyl) propane | 471 | 529 | 71.3 | 37.8 | 93.8 |

[Table 7]

| Polyurea-based compound (solid/solid reaction) | | | | | | |
|---|---|---|---|---|---|---|
| | Raw materials | | Mixing ratio (part by mass) | | Evaluation | |
| Examples | Isocyanate component | Diamine component | Acid component | Diamine component | Reaction rate (%) | Average particle size (μm) |
| A6-1 | Diphenylmethane diisocyanate | 4,4'-Diaminodiphenyl ether | 556 | 444 | Solvent insoluble | 53.4 |
| A6-2 | 1,5-Diisocyanato naphthalene | 3,4'-Diaminodiphenyl ether | 512 | 488 | Solvent insoluble | 54.1 |

[Table 8]

| Diimide dicarboxylic acid-based compound (acid monoanhydride/diamine) (solid/solid reaction) | | | | | | | | | | |
|---|---|---|---|---|---|---|---|---|---|---|
| Examples | Pulverization conditions | | | Raw materials | | Mixing ratio (part by mass) | | Reaction rate (%) | | Average particle size ($\mu$m) |
| | Apparatus | Rotation speed (rpm) | Time (minutes) | Acid component | Amine component | Acid component | Amine component | Reaction rate after mechanochemical treatment (%) | After heat treatment | |
| B1-1 | Mediumstirring type mill | 504 | 1 | Trimellitic anhydride | 4,4'-Diaminodiphenyl ether | 657 | 343 | 72.1 | 98.2 | 165.7 |
| B1-2 | | | 3 | | | | | 72.6 | 98.3 | 82.8 |
| B1-3 | | | 5 | | | | | 76.1 | 98.5 | 57.9 |
| B1-4 | | | 10 | | | | | 77.9 | 98.2 | 49.2 |
| B1-5 | | | 15 | | | | | 83.3 | 98.9 | 48.5 |
| B1-6 | | | 30 | | | | | 84.3 | 98.5 | 50.1 |
| B1-7 | | | 45 | | | | | 84.8 | 98.4 | 47.9 |
| B1-8 | | | 60 | | | | | 83.0 | 98.9 | 48.4 |
| B1-9 | Mediumstirring type mill | 115 | 1 | Trimellitic anhydride | 4,4'-Diaminodiphenyl ether | 657 | 343 | 37.7 | 97.5 | 207.0 |
| B1-10 | | 223 | | | | | | 64.3 | 98.1 | 183.3 |
| B1-11 | | 307 | | | | | | 70.7 | 98.5 | 191.2 |
| B1-12 | | 401 | | | | | | 77.8 | 98.2 | 179.7 |
| B1-13 | | 504 | | | | | | 83.0 | 98.2 | 161.2 |

[Table 9]

| Diimide dicarboxylic acid-based compound (solid/solid reaction) | | | | | | | | | | |
|---|---|---|---|---|---|---|---|---|---|---|
| Examples | Pulverization conditions | | | Raw materials | | Mixing ratio (part by mass) | | Reaction rate (%) | | Average particle size ($\mu$m) |
| | Apparatus | Rotation speed (rpm) | Time (minutes) | Acid component | Amine component | Acid component | Amine component | After mechanochemical treatment | After heat treatment | |
| B2-1 | High-speed bottom stirring type mixer | 362 | 3 | Trimellitic anhydride | 4,4'-Diaminodiphenyl ether | 657 | 343 | 41.9 | 97.1 | 134.6 |
| B2-2 | | 543 | | | | | | 64.6 | 97.2 | 93.9 |
| B2-3 | | 726 | | | | | | 72.8 | 98.5 | 67.8 |
| B2-4 | | 909 | | | | | | 60.4 | 98.4 | 59.1 |
| B2-5 | | 1090 | | | | | | 72.7 | 98.4 | 48.4 |
| B2-6 | | 1274 | | | | | | 69.9 | 98.9 | 48.5 |
| B2-7 | | 1452 | | | | | | 57.0 | 98.2 | 47.3 |
| B2-8 | | 2800 | | | | | | 63.3 | 99.0 | 47.5 |
| B2-9 | | 4200 | | | | | | 68.8 | 98.9 | 46.4 |
| B2-10 | | 5600 | | | | | | 65.3 | 99.2 | 42.8 |

[Table 10]

| Diimide dicarboxylic acid-based compound (solid/solid reaction) | | | | | | | | | | |
|---|---|---|---|---|---|---|---|---|---|---|
| Examples | Pulverization conditions | | | Raw materials | | Mixing ratio (part by mass) | | Reaction rate (%) | | Average particle size (μm) |
| | Apparatus | Rotation speed (rpm) | Time (minutes) | Acid component | Amine component | Acid component | Amine component | After mechanochemical treatment | After heat treatment | |
| B3-1 | High-speed bottom stirring type mixer | 1452 | 10 | Trimellitic anhydride | p-Phenylenediamine | 780 | 220 | 57.5 | 97.2 | 57.7 |
| B3-2 | | | | | m-Phenylenediamine | 780 | 220 | 60.4 | 98.5 | 50.9 |
| B3-3 | | | | | 3,4'-Diaminodiphenyl ether | 657 | 343 | 69.6 | 98.4 | 55.0 |
| B3-4 | | | | | 1,4-Bis(4-aminophenoxy)benzene | 567 | 433 | 46.1 | 98.4 | 61.6 |
| B3-5 | | | | | 1,3-Bis(4-aminophenoxy)benzene | 567 | 433 | 53.6 | 98.9 | 58.0 |
| B3-6 | | | | | 4,4'-Bis(4-aminophenoxy)biphenyl | 510 | 490 | 59.7 | 98.2 | 50.4 |
| B3-7 | | | | | 4,4'-Bis(3-aminophenoxy)biphenyl | 510 | 490 | 58.6 | 99.0 | 61.7 |
| B3-8 | | | | | 2,2-Bis[4-(4-aminophenoxy)phenyl]propane | 483 | 517 | 56.7 | 99.0 | 57.1 |
| B3-9 | | | | | Bis(4-aminophenyl)sulfone | 607 | 393 | 52.1 | 98.9 | 53.8 |
| B3-10 | | | | | Bis[4-(4-aminophenoxy)phenyl]sulfone | 470 | 530 | 59.7 | 99.2 | 57.6 |

| | Pulverization conditions | | | Raw materials | | Mixing ratio (part by mass) | | Reaction rate (%) | | Average particle size (μm) |
|---|---|---|---|---|---|---|---|---|---|---|
| **Diimide dicarboxylic acid-based compound (solid/solid reaction)** | | | | | | | | | | |
| Examples | Apparatus | Rotation speed (rpm) | Time (minutes) | Acid component | Amine component | Acid component | Amine component | After mechanochemical treatment | After heat treatment | |
| B3-11 | | | | | 9,9-Bis(4-aminophenyl)fluorene | 524 | 476 | 60.7 | 99.2 | 56.2 |
| B3-12 | | | | | trans-1,4-Cyclohexanediamine | 770 | 230 | 28.0 | 99.0 | 63.7 |
| B3-13 | | | | | 4,4'-Methylenebis(cyclohexylamine) | 646 | 354 | 23.7 | 98.9 | 51.0 |
| B3-14 | | | | | 1,10-Diaminodecane | 690 | 310 | 40.5 | 99.2 | 56.9 |
| B3-15 | | | | | 1,12-Diaminododecane | 657 | 343 | 37.2 | 99.1 | 61.8 |

EP 3 819 283 A1

[Table 11]

| Diimide dicarboxylic acid-based compound (solid/liquid reaction) | | | | | | | | | | |
|---|---|---|---|---|---|---|---|---|---|---|
| Examples | Pulverization conditions | | | Raw materials | | Mixing ratio (part by mass) | | Reaction rate (%) | | Average particle size ($\mu$m) |
| | Apparatus | Rotation speed (rpm) | Time (minutes) | Acid component | Amine component | Acid component | Amine component | After mechanochemical treatment | After heat treatment | |
| B3-16 | High-speed bottom stirring type mixer | 1452 | 10 | Trimellitic anhydride | Metaxylylenediamine | 738 | 262 | 55.7 | 98.9 | 51.3 |
| B3-17 | | | | | 1,4-Bis(aminomethyl) cyclohexane | 729 | 271 | 47.5 | 97.6 | 52.7 |
| B3-18 | | | | | PRIAMINE 1075 (Croda Japan KK) | 414 | 586 | 70.2 | 98.9 | 51.9 |

[Table 12]

| Examples | Raw materials | | | Mixing ratio (part by mass) | | Reaction rate (%) | | Average particle size ($\mu$m) |
|---|---|---|---|---|---|---|---|---|
| | Acid component | | Amine component | Acid component | Amine component | After mechanochemical treatment | After heat treatment | |
| | Diimide dicarboxylic acid-based compound (acid dianhydride/aminocarboxylic acid) (solid/solid reaction) | | | | | | | |
| B4-1 | Pyromellitic dianhydride | | 4-Aminobenzoic acid | 444 | 556 | 64.3 | 99.2 | 46.7 |
| B4-2 | 3,3',4,4'-Biphenyltetracarboxylic dianhydride | | | 518 | 482 | 65.5 | 98.5 | 45.1 |
| B4-3 | 2,3,3',4'-Biphenyltetracarboxylic dianhydride | | | 518 | 482 | 67.2 | 98.4 | 42.8 |
| B4-4 | 3,3',4,4'-Benzophenone tetracarboxylic dianhydride | | | 541 | 459 | 65.6 | 99.4 | 50.9 |
| B4-5 | 4,4'-Oxydiphthalic anhydride | | | 531 | 469 | 68.5 | 98.5 | 45.8 |
| B4-6 | 4,4'-(Hexafluoroisopropyridene) diphthalic anhydride | | | 619 | 381 | 67.8 | 98.2 | 50.9 |

[Table 13]

| Examples | Raw materials | | Mixing ratio (part by mass) | | Reaction rate (%) | | Average particle size (μm) |
|---|---|---|---|---|---|---|---|
| | Acid component | Amine component | Acid component | Amine component | After mechanochemical treatment | After heat treatment | |
| | | | | | | | |

Diimide tricarboxylic acid-based compound (solid/solid reaction)

| Examples | Acid component | Amine component | Acid component | Amine component | After mechanochemical treatment | After heat treatment | Average particle size (μm) |
|---|---|---|---|---|---|---|---|
| B5-1 | Trimellitic anhydride | 3,4-Diaminobenzoic acid | 716 | 284 | 62.3 | 98.2 | 55.5 |
| B5-2 | | 3,5-Diaminobenzoic acid | 716 | 284 | 61.5 | 97.5 | 56.3 |
| B5-3 | | 2,5-Diaminobenzoic acid | 716 | 284 | 61.7 | 98.8 | 51.6 |
| B5-4 | | 3,5-Bis(4-aminophenoxy) benzoic acid | 533 | 467 | 60.7 | 98.9 | 56.2 |

[Table 14]

| Examples | Raw materials | | Mixing ratio (part by mass) | | Reaction rate (%) | | Average particle size ($\mu$m) |
| | Acid component | Amine component | Acid component | Amine component | After mechanochemical treatment | After heat treatment | |
|---|---|---|---|---|---|---|---|
| | Diimide tetracarboxylic acid-based compound (solid/solid reaction) | | | | | | |
| B6-1 | Pyromellitic dianhydride | 2-Aminoterephthalic acid | 377 | 623 | 63.3 | 98.3 | 54.5 |
| B6-2 | | 5-Aminoisophthalic acid | 377 | 623 | 66.5 | 98.8 | 53.1 |
| B6-3 | | 3-Aminophthalic acid | 377 | 623 | 68.2 | 97.3 | 51.8 |
| B6-4 | | 4-Aminophthalic acid | 377 | 623 | 64.6 | 97.8 | 64.8 |
| B6-5 | 3,3',4,4'-Biphenyltetracarboxylic dianhydride | 2-Aminoterephthalic acid | 449 | 551 | 68.7 | 99.2 | 55.8 |
| B6-6 | | 5-Aminoisophthalic acid | 449 | 551 | 67.6 | 98.9 | 53.6 |
| B6-7 | | 3-Aminophthalic acid | 449 | 551 | 62.2 | 97.9 | 68.5 |
| B6-8 | | 4-Aminophthalic acid | 449 | 551 | 61.9 | 97.6 | 50.3 |

(continued)

| Diimide tetracarboxylic acid-based compound (solid/solid reaction) | | | | | | | |
|---|---|---|---|---|---|---|---|
| Examples | Raw materials | | Mixing ratio (part by mass) | | Reaction rate (%) | | Average particle size (μm) |
| | Acid component | Amine component | Acid component | Amine component | After mechanochemical treatment | After heat treatment | |
| B6-9 | 2,3,3',4'-Biphenyltetracarboxylic dianhydride | 2-Aminoterephthalic acid | 449 | 551 | 62.8 | 99.5 | 50.0 |
| B6-10 | | 5-Aminoisophthalic acid | 449 | 551 | 63.8 | 98.7 | 54.8 |
| B6-11 | | 3-Aminophthalic acid | 449 | 551 | 63.5 | 97.9 | 59.4 |
| B6-12 | | 4-Aminophthalic acid | 449 | 551 | 60.6 | 97.5 | 60.9 |
| B6-13 | 3,3',4,4'-Benzophenone tetracarboxylic dianhydride | 2-Aminoterephthalic acid | 471 | 529 | 62.9 | 98.8 | 54.2 |
| B6-14 | | 5-Aminoisophthalic acid | 471 | 529 | 59.7 | 99.0 | 59.1 |
| B6-15 | | 3-Aminophthalic acid | 471 | 529 | 67.3 | 97.4 | 68.0 |
| B6-16 | | 4-Aminophthalic acid | 471 | 529 | 63.5 | 97.7 | 50.9 |

(continued)

| Diimide tetracarboxylic acid-based compound (solid/solid reaction) | | | | | | | |
|---|---|---|---|---|---|---|---|
| Examples | Raw materials | | Mixing ratio (part by mass) | | Reaction rate (%) | | Average particle size ($\mu$m) |
| | Acid component | Amine component | Acid component | Amine component | After mechanochemical treatment | After heat treatment | |
| B6-17 | 4,4'-Oxydiphthalic anhydride | 2-Aminoterephthalic acid | 462 | 538 | 66.2 | 98.4 | 53.7 |
| B6-18 | | 5-Aminoisophthalic acid | 462 | 538 | 64.6 | 99.4 | 52.2 |
| B6-19 | | 3-Aminophthalic acid | 462 | 538 | 60.7 | 98.0 | 64.4 |
| B6-20 | | 4-Aminophthalic acid | 462 | 538 | 58.8 | 98.1 | 51.8 |
| B6-21 | 4,4'-(Hexafluoroisopropyridene ) diphthalic anhydride | 2-Aminoterephthalic acid | 552 | 448 | 67.3 | 98.6 | 53.6 |
| B6-22 | | 5-Aminoisophthalic acid | 552 | 448 | 60.9 | 99.1 | 68.5 |
| B6-23 | | 3-Aminophthalic acid | 552 | 448 | 66.8 | 97.5 | 54.3 |
| B6-24 | | 4-Aminophthalic acid | 552 | 448 | 67.2 | 97.4 | 50.6 |

[Table 15]

Monoimide dicarboxylic acid-based compound (solid/solid reaction)

| Examples | Raw materials | | Mixing ratio (part by mass) | | Reaction rate (%) | | Average particle size (μm) |
|---|---|---|---|---|---|---|---|
| | Acid component | Amine component | Acid component | Amine component | After mechanochemical treatment | After heat treatment | |
| B7-1 | Trimellitic anhydride | 2-Aminobenzoic acid | 584 | 416 | 65.5 | 98.2 | 48.6 |
| B7-2 | | 3-Aminobenzoic acid | 584 | 416 | 67.9 | 98.8 | 47.9 |
| B7-3 | | 4-Aminobenzoic acid | 584 | 416 | 68.2 | 99.3 | 55.9 |
| B7-4 | | 2-Amino-3-methylbenzoic acid | 560 | 440 | 66.9 | 99.0 | 58.7 |
| B7-5 | | 2-Amino-4-methylbenzoic acid | 560 | 440 | 68.1 | 99.2 | 49.9 |
| B7-6 | | 2-Amino-5-methylbenzoic acid | 560 | 440 | 68.2 | 98.2 | 58.5 |
| B7-7 | | 2-Amino-6-methylbenzoic acid | 560 | 440 | 64.3 | 98.1 | 52.4 |
| B7-8 | | 3-Amino-2-methylbenzoic acid | 560 | 440 | 64.4 | 98.1 | 54.2 |
| B7-9 | | 3-Amino-4-methylbenzoic acid | 560 | 440 | 66.6 | 99.5 | 48.8 |
| B7-10 | | 4-Amino-2-methylbenzoic acid | 560 | 440 | 66.3 | 99.4 | 58.7 |
| B7-11 | | 4-Amino-3-methylbenzoic acid | 560 | 440 | 62.1 | 99.4 | 45.3 |
| B7-12 | | 5-Amino-2-methylbenzoic acid | 560 | 440 | 63.5 | 98.9 | 53.7 |
| B7-13 | | 2-Amino-3,4-dimethylbenzoic acid | 538 | 462 | 65.4 | 98.8 | 46.1 |
| B7-14 | | 2-Amino-4,5-dimethylbenzoic acid | 538 | 462 | 63.8 | 98.7 | 57.2 |

(continued)

| Monoimide dicarboxylic acid-based compound (solid/solid reaction) | | | | | | | |
|---|---|---|---|---|---|---|---|
| | Raw materials | | Mixing ratio (part by mass) | | Reaction rate (%) | | Average particle size ($\mu$m) |
| Examples | Acid component | Amine component | Acid component | Amine component | After mechanochemical treatment | After heat treatment | |
| B7-15 | | 2-Amino-4-methoxybenzoic acid | 535 | 465 | 62.2 | 98.8 | 55.1 |
| B7-16 | | 3-Amino-4-methoxybenzoic acid | 535 | 465 | 63.5 | 98.8 | 55.6 |
| B7-17 | | 4-Amino-2-methoxybenzoic acid | 535 | 465 | 67.3 | 99.3 | 61.0 |

[Table 16]

| Monoimide tricarboxylic acid-based compound (solid/solid reaction) | | | | | | | |
|---|---|---|---|---|---|---|---|
| Examples | Raw materials | | Mixing ratio (part by mass) | | Reaction rate (%) | | Average particle size (μm) |
| | Acid component | Amine component | Acid component | Amine component | After mechanochemical treatment | After heat treatment | |
| B8-1 | Trimellitic anhydride | 2-Aminoterephthalic acid | 515 | 485 | 69.7 | 99.4 | 48.5 |
| B8-2 | | 5-Aminoisophthalic acid | 515 | 485 | 70.9 | 99.8 | 48.8 |
| B8-3 | | 3-Aminophthalic acid | 515 | 485 | 68.3 | 97..9 | 46.0 |
| B8-4 | | 4-Aminophthalic acid | 515 | 485 | 69.9 | 98.1 | 49.8 |

[Table 17]

| Amide group-containing imide-based compound (solid/solid reaction) | | | | | | | |
|---|---|---|---|---|---|---|---|
| Examples | Raw materials | | Mixing ratio (part by mass) | | Reaction rate (%) | | Average particle size ($\mu$m) |
| | Acid component | Amine component | Acid component | Amine component | After mechanochemical treatment | After heat treatment | |
| B9-1[1] | Trimellitic anhydride | 4,4'-Diaminobenzanilide | 628 | 372 | 63.2 | 98.4 | 51.5 |
| B9-2[2] | Trimellitic anhydride chloride | 2-Aminobenzoic acid | 435 | 565 | 71.3 | 98.7 | 67.5 |
| B9-3[2] | | 3-Aminobenzoic acid | 435 | 565 | 73.9 | 99.2 | 60.3 |
| B9-4[2] | | 4-Aminobenzoic acid | 435 | 565 | 73.2 | 99.2 | 61.0 |
| B9-5[3] | | 2-Aminoterephthalic acid | 368 | 632 | 72.2 | 98.2 | 69.3 |
| B9-6[3] | | 5-Aminoisophthalic acid | 368 | 632 | 72.4 | 98.1 | 61.4 |
| (1) Amide group-containing diimide dicarboxylic acid-based compound (2) Amide group-containing monoimide dicarboxylic acid-based compound (3) Amide group-containing monoimide tetracarboxylic acid-based compound | | | | | | | |

[Table 18]

Ester group-containing imide-based compound (ester group-containing monoimide tricarboxylic acid-based compound) (solid/solid reaction)

| Examples | Raw materials | | Mixing ratio (part by mass) | | Reaction rate (%) | | Average particle size (μm) |
|---|---|---|---|---|---|---|---|
| | Acid component | Amine component | Acid component | Amine component | After mechanochemical treatment | After heat treatment | |
| B10-1 | Trimellitic anhydride | 2-Aminophenol | 778 | 222 | 43.3 | 97.3 | 67.0 |
| B10-2 | | 3-Aminophenol | 778 | 222 | 45.1 | 97.6 | 50.2 |
| B10-3 | | 4-Aminophenol | 778 | 222 | 39.8 | 98.1 | 69.8 |
| B10-4 | | 2-Amino-4-methylphenol | 757 | 243 | 42.5 | 97.9 | 61.1 |
| B10-5 | | 2-Amino-5-methylphenol | 757 | 243 | 42.6 | 98.3 | 65.5 |
| B10-6 | | 3-Amino-2-methylphenol | 757 | 243 | 39.9 | 98.3 | 59.5 |
| B10-7 | | 3-Amino-4-methylphenol | 757 | 243 | 41.4 | 97.7 | 67.0 |
| B10-8 | | 4-Amino-2-methylphenol | 757 | 243 | 41.2 | 98.7 | 56.6 |
| B10-9 | | 5-Amino-2-methylphenol | 757 | 243 | 44.3 | 98.4 | 62.4 |
| B10-10 | | 4-Amino-2-methoxyphenol | 734 | 266 | 43.3 | 98.2 | 57.4 |
| B10-11 | | 3-Hydroxy-4-methoxyaniline | 734 | 266 | 42.7 | 98.3 | 66.9 |
| B10-12 | | 4-Amino-3,5-xylenol | 736 | 264 | 50.1 | 98.8 | 69.4 |
| B10-13 | | 2-Aminobenzylalcohol | 757 | 243 | 51.9 | 98.6 | 58.2 |
| B10-14 | | 3-Aminobenzylalcohol | 757 | 243 | 52.0 | 98.8 | 69.7 |
| B10-15 | | 4-Aminobenzylalcohol | 757 | 243 | 51.4 | 98.7 | 61.1 |
| B10-16 | | 2-(4-Aminophenyl)ethanol | 736 | 264 | 53.6 | 98.1 | 65.1 |

[Table 19]

Hydroxyl group-containing imide-based compound (diimide diphenol and diimide dihydroxy-based compounds) (solid/solid reaction)

| Examples | Raw materials | | Mixing ratio (part by mass) | | Reaction rate (%) | | Average particle size (μm) |
|---|---|---|---|---|---|---|---|
| | Acid component | Amine component | Acid component | Amine component | After mechanochemical treatment | After heat treatment | |
| B11-1 | Pyromellitic dianhydride | 3-Aminophenol | 501 | 499 | 66.4 | 99.0 | 52.3 |
| B11-2 | | 4-Aminophenol | 501 | 499 | 66.4 | 98.2 | 42.5 |
| B11-3 | | 4-Aminobenzylalcohol | 470 | 530 | 68.3 | 98.8 | 57.1 |
| B11-4 | | 2-(4-Aminophenyl)ethanol | 444 | 556 | 66.9 | 98.5 | 53.4 |
| B11-5 | 3,3',4,4'-Biphenyltetracarboxylic dianhydride | 3-Aminophenol | 575 | 425 | 68.2 | 99.3 | 53.2 |
| B11-6 | | 4-Aminophenol | 575 | 425 | 65.1 | 99.2 | 47.2 |
| B11-7 | | 4-Aminobenzylalcohol | 545 | 455 | 65.3 | 98.9 | 55.3 |
| B11-8 | | 2-(4-Aminophenyl)ethanol | 518 | 482 | 66.0 | 98.7 | 44.3 |
| B11-9 | 2,3,3',4'-Biphenyltetracarboxylic dianhydride | 3-Aminophenol | 575 | 425 | 67.9 | 98.6 | 50.1 |
| B11-10 | | 4-Aminophenol | 575 | 425 | 68.8 | 99.3 | 45.1 |
| B11-11 | | 4-Aminobenzylalcohol | 545 | 455 | 68.2 | 98.5 | 54.6 |
| B11-12 | | 2-(4-Aminophenyl)ethanol | 518 | 482 | 68.1 | 98.4 | 57.1 |
| B11-13 | 3,3',4,4'-Benzophenone tetracarboxylic dianhydride | 3-Aminophenol | 597 | 403 | 68.3 | 98.7 | 46.5 |
| B11-14 | | 4-Aminophenol | 597 | 403 | 67.7 | 99.4 | 56.1 |
| B11-15 | | 4-Aminobenzylalcohol | 568 | 432 | 67.6 | 99.5 | 46.5 |
| B11-16 | | 2-(4-Aminophenyl)ethanol | 541 | 459 | 67.9 | 99.4 | 57.4 |

(continued)

Hydroxyl group-containing imide-based compound (diimide diphenol and diimide dihydroxy-based compounds) (solid/solid reaction)

| Examples | Raw materials | | Mixing ratio (part by mass) | | Reaction rate (%) | | | Average particle size (μm) |
|---|---|---|---|---|---|---|---|---|
| | Acid component | Amine component | Acid component | Amine component | After mechanochemical treatment | After heat treatment | | |
| B11-17 | 4,4'-Oxydiphthalic anhydride | 3-Aminophenol | 588 | 412 | 66.3 | 98.8 | | 53.4 |
| B11-18 | | 4-Aminophenol | 588 | 412 | 65.4 | 98.9 | | 53.4 |
| B11-19 | | 4-Aminobenzylalcohol | 558 | 442 | 66.7 | 98.8 | | 55.3 |
| B11-20 | | 2-(4-Aminophenyl) ethanol | 531 | 469 | 66.8 | 98.5 | | 42.5 |
| B11-21 | 4,4'-(Hexafluoroisopropyridene ) diphthalic anhydride | 3-Aminophenol | 671 | 329 | 65.1 | 99.4 | | 57.5 |
| B11-22 | | 4-Aminophenol | 671 | 329 | 64.3 | 99.3 | | 53.4 |
| B 11-23 | | 4-Aminobenzylalcohol | 644 | 356 | 64.6 | 99.2 | | 57.1 |
| B11-24 | | 2-(4-Aminophenyl) ethanol | 619 | 381 | 68.0 | 98.7 | | 46.5 |

[Table 20]

| | Raw materials | | Mixing ratio (part by mass) | | Reaction rate (%) | | Average particle size (μm) |
|---|---|---|---|---|---|---|---|
| Amide group-containing carboxylic acid-based compound (solid/solid reaction) | | | | | | | |
| Examples | Acid component | Amine component | Acid component | Amine component | After mechanochemical treatment | After heat treatment | |
| B12-1[1] | Terephthalic acid chloride | 2-Aminobenzoic acid | 426 | 574 | 70.1 | 98.9 | 63.2 |
| B12-2[1] | | 3-Aminobenzoic acid | 426 | 574 | 69.7 | 99.1 | 53.4 |
| B12-3[1] | | 4-Aminobenzoic acid | 426 | 574 | 72.1 | 98.2 | 68.0 |
| B 12-4[2] | | 2-Aminoterephthalic acid | 360 | 640 | 71.1 | 97.9 | 64.3 |
| B12-5[2] | | 5-Aminoisophthalic acid | 360 | 640 | 68.8 | 98.3 | 64.1 |
| B12-6[1] | Isophthalic acid chloride | 2-Aminobenzoic acid | 426 | 574 | 72.1 | 98.8 | 58.1 |
| B12-7[1] | | 3-Aminobenzoic acid | 426 | 574 | 72.2 | 98.9 | 68.2 |
| B12-8[1] | | 4-Aminobenzoic acid | 426 | 574 | 71.4 | 99.4 | 57.2 |
| B 12-9[2] | | 2-Aminoterephthalic acid | 360 | 640 | 70.9 | 97.0 | 60.0 |
| B12-10[2] | | 5-Aminoisophthalic acid | 360 | 640 | 73.0 | 97.8 | 58.0 |
| (1) Diamide dicarboxylic acid-based compound<br>(2) Diamide tetracarboxylic acid-based compound | | | | | | | |

[Table 21]

| Ester group-containing carboxylic acid-based compound (solid/solid reaction) | | | | | | | |
|---|---|---|---|---|---|---|---|
| Examples | Raw materials | | Mixing ratio (part by mass) | | Reaction rate (%) | | Average particle size (μm) |
| | Acid component | Hydroxy component | Acid component | Amine component | After mechanochemical treatment | After heat treatment | |
| B13-1[1] | Terephthalic acid chloride | 4-Hydroxybenzoic acid | 424 | 576 | 66.2 | 97.8 | 54.3 |
| B13-2[1] | | 4-Hydroxy-2-methylbenzoic acid | 401 | 599 | 64.3 | 97.9 | 62.3 |
| B13-3[1] | | 4-Hydroxy-3-methylbenzoic acid | 401 | 599 | 65.1 | 97.6 | 54.1 |
| B13-4[1] | | 4-Hydroxy-3,5,-dimethylbenzoic acid | 380 | 620 | 64.3 | 97.7 | 56.5 |
| B13-5[1] | | 2-Hydroxy-4-methoxybenzoic acid | 377 | 623 | 64.2 | 98.3 | 55.1 |
| B13-6[1] | | 3-Hydroxy-4-methoxybenzoic acid | 377 | 623 | 63.7 | 97.5 | 67.3 |
| B13-7[1] | | 4-Hydroxy-3-methoxybenzoic acid | 377 | 623 | 63.8 | 98.2 | 59.1 |
| B13-8[1] | | 4-Hydroxy-3,5-dimethoxybenzoic acid | 339 | 661 | 62.9 | 98.2 | 66.1 |
| B13-9[1] | | 3,5-Di-tert-butyl-4-hydroxybenzoic acid | 289 | 711 | 60.7 | 98.4 | 51.7 |
| B13-10[2] | | 4-Hydroxyisophthalic acid | 359 | 641 | 62.3 | 97.9 | 67.1 |
| B13-11[2] | | 4-Hydroxyphthalic acid | 359 | 641 | 63.0 | 97.9 | 54.3 |

(continued)

| Ester group-containing carboxylic acid-based compound (solid/solid reaction) | | | | | | | |
|---|---|---|---|---|---|---|---|
| Examples | Raw materials | | Mixing ratio (part by mass) | | Reaction rate (%) | | Average particle size ($\mu$m) |
| | Acid component | Hydroxy component | Acid component | Amine component | After mechanochemical treatment | After heat treatment | |
| B13-12[1] | Isophthalic acid chloride | 4-Hydroxybenzoic acid | 424 | 576 | 64.3 | 98.1 | 66.2 |
| B13-13[1] | | 4-Hydroxy-2-methylbenzoic acid | 401 | 599 | 63.2 | 98.2 | 51.0 |
| B13-14[1] | | 4-Hydroxy-3-methylbenzoic acid | 401 | 599 | 63.7 | 98.1 | 67.2 |
| B13-15[1] | | 4-Hydroxy-3,5,-dimethylbenzoic acid | 380 | 620 | 63.3 | 98.3 | 52.1 |
| B13-16[1] | | 2-Hydroxy-4-methoxybenzoic acid | 377 | 623 | 64.1 | 97.3 | 62.7 |
| B13-17[1] | | 3-Hydroxy-4-methoxybenzoic acid | 377 | 623 | 62.1 | 97.2 | 52.1 |
| B13-18[1] | | 4-Hydroxy-3-methoxybenzoic acid | 377 | 623 | 62.4 | 97.6 | 55.4 |
| B13-19[1] | | 4-Hydroxy-3,5-dimethoxybenzoic acid | 339 | 661 | 62.9 | 97.0 | 57.3 |
| B13-20[1] | | 3,5-Di-tert-butyl-4-hydroxybenzoic acid | 289 | 711 | 59.6 | 98.1 | 69.5 |
| B13-21[2] | | 4-Hydroxyisophthalic acid | 359 | 641 | 61.3 | 97.8 | 53.1 |
| B13-22[2] | | 4-Hydroxyphthalic acid | 359 | 641 | 63.2 | 98.3 | 62.5 |
| (1) Diester dicarboxylic acid-based compound (2) Diester tetracarboxylic acid-based compound | | | | | | | |

[Table 22]

Curable imide-based compound (solid/solid and solid/liquid reactions)

| Examples | Raw materials | | Mixing ratio (part by mass) | | Reaction rate (%) | | Average particle size (μm) |
|---|---|---|---|---|---|---|---|
| | Acid component | Amine component | Acid component | Amine component | After mechanochemical treatment | After heat treatment | |
| B14-1(1) | 4-Phenylethynyl phthalic anhydride | p-Phenylenediamine | 821 | 179 | 64.2 | 98.0 | 45.2 |
| B14-2(1) | | 4,4'-Oxydianiline | 712 | 288 | 68.3 | 98.9 | 43.1 |
| B14-3(1) | | 2,2-Bis[4-(4-aminophenoxy)phenyl]propane | 547 | 453 | 68.1 | 98.0 | 44.0 |
| B14-4(2) | | PRIAMINE 1075 (Croda Japan KK) | 478 | 522 | 73.0 | 99.4 | 47.6 |
| B14-5(1) | 5-Norbornene-2,3-dicarboxylic anhydride | p-Phenylenediamine | 752 | 248 | 64.3 | 97.3 | 46.3 |
| B14-6(1) | | 4,4'-Oxydianiline | 620 | 380 | 66.1 | 97.4 | 48.5 |
| B14-7(1) | | 2,2-Bis[4-(4-aminophenoxy)phenyl]propane | 444 | 556 | 69.0 | 98.1 | 48.3 |
| B14-8(2) | | PRIAMINE 1075 (Croda Japan KK) | 377 | 623 | 72.5 | 98.7 | 45.3 |
| B14-9(1) | Methylmaleic anhydride | p-Phenylenediamine | 674 | 326 | 66.1 | 97.1 | 42.9 |
| B14-10(1) | | 4,4'-Oxydianiline | 527 | 473 | 67.2 | 97.2 | 46.2 |
| B14-11(1) | | 2,2-Bis[4-(4-aminophenoxy)phenyl]propane | 353 | 647 | 68.2 | 97.7 | 45.4 |
| B14-12(2) | | PRIAMINE 1075 (Croda Japan KK) | 292 | 708 | 72.2 | 98.5 | 47.7 |

(1) Solid/solid reaction
(2) Solid/liquid reaction

[Table 23]

| Examples | Raw materials | | Mixing ratio (part by mass) | | Evaluation | | | | | | |
|---|---|---|---|---|---|---|---|---|---|---|---|
| | Acid component | Amine component | Acid component | Amine component | First-step reaction rate (%) | Second-step reaction rate (%) | First-step average particle size ($\mu$m) | Second-step average particle size ($\mu$m) | Final average degree of polymerization | Imidization ratio (%) | Content of solvent (%) |
| C1 | Pyromellitic dianhydride | 4,4'-Diaminodiphenyl ether | 521 | 479 | 78.2 | 90.1 | 48.2 | 14.2 | 10.3 | 0 | 0 |
| C2 | 3,3',4,4'-Biphenyltetracarboxylic dianhydride | | 595 | 405 | 72.8 | 92.2 | 50.2 | 13.4 | 9.5 | 0 | 0 |
| C3 | 3,3',4,4'Benzophenone tetracarboxylic dianhydride | | 617 | 383 | 72.2 | 91.0 | 46.2 | 17.4 | 9.2 | 0 | 0 |
| C4 | 4,4'-Oxydiphthalic anhydride | | 608 | 392 | 71.1 | 89.5 | 47.3 | 15.3 | 8.9 | 0 | 0 |

[Table 24]

| Examples | Raw materials | | Mixing ratio (part by mass) | | Reaction rate (%) | | | First-step average particle size (μm) | Second-step average particle size (μm) |
|---|---|---|---|---|---|---|---|---|---|
| | Acid component | Amine component | Acid component | Amine component | First step | Second step | After heat treatment | | |
| D1 | Trimellitic anhydride | 4,4'-Diaminodiphenyl ether | 657 | 343 | 65.3 | 92.3 | 99.1 | 48.2 | 13.3 |
| D2 | | 4-Aminophenol | 778 | 222 | 39.8 | 87.5 | 98.7 | 69.8 | 15.6 |
| D3 | 3,3',4,4'Benzophenone tetracarboxylic dianhydride | | 597 | 403 | 67.7 | 93.4 | 98.9 | 56.1 | 12.5 |

INDUSTRIAL APPLICABILITY

Since the production method of an organic compound of the present invention allows an organic compound to be produced without using any solvents, it is useful in the field of producing organic compounds from the viewpoints of environmental load, working environment, and the like.

**Claims**

1. A production method of an organic compound, wherein a reaction is performed between functional groups by using a mechanochemical effect.

2. The production method of an organic compound of claim 1, wherein the reaction is a condensation reaction, an addition reaction, or a composite reaction thereof.

3. The production method of an organic compound of claim 1 or 2, wherein the reaction is a reaction between two functional groups selected from the group consisting of a carboxyl group and a halide thereof, an acid anhydride group, an amino group, an isocyanate group, and a hydroxyl group.

4. The production method of an organic compound of any one of claims 1 to 3, wherein the reaction is one or more reactions selected from the group consisting of following reactions:

   (A) a reaction in which an acid anhydride group and an amino group are allowed to react to form (a1) an amide group and a carboxyl group, (a2) an imide group, (a3) an isoimide group or (a4) a mixture thereof;
   (B) a reaction in which an acid anhydride group and an isocyanate group are allowed to react to form an imide group;
   (C) a reaction in which a carboxyl group or a halide thereof and an amino group or an isocyanate group are allowed to react to form an amide group;
   (D) a reaction in which a carboxyl group or a halide thereof and a hydroxyl group are allowed to react to form an ester group;
   (E) a reaction in which an isocyanate group and an amino group are allowed to react to form a urea group;
   (F) a reaction in which an isocyanate group and a hydroxyl group are allowed to react to form a urethane group; and
   (G) a reaction in which an acid anhydride group and a hydroxyl group are allowed to react to form an ester group and a carboxyl group.

5. The production method of an organic compound of any one of claims 1 to 4, wherein the reaction between functional groups occurs between two or more raw material compound molecules.

6. The production method of an organic compound of claim 5, wherein each of the raw material compounds is a compound having a molecular weight of 2000 or less.

7. The production method of an organic compound of any one of claims 1 to 6, wherein the reaction is promoted by heating.

8. The production method of an organic compound of any one of claims 1 to 7, wherein the organic compound is a high-molecular compound containing a repeating unit.

9. The production method of an organic compound of claim 8, wherein the reaction belongs to one or more reactions selected from the group consisting of a sequential polymerization reaction, a condensation polymerization reaction, and a polyaddition reaction.

10. The production method of an organic compound of claim 8 or 9, wherein the high-molecular compound is a polyamic acid-based compound, a polyimide-based compound, a polyamide-based compound, a polyamide-imide-based compound, a polyurethane-based compound, a polyurea-based compound, or a polyester-based compound.

11. The production method of an organic compound of any one of claims 8 to 10, wherein the reaction is performed in a presence of a terminal blocking agent.

12. The production method of an organic compound of any one of claims 1 to 11, wherein a polyamic acid-based compound, a polyimide-based compound, or a mixture thereof is produced as the organic compound by performing a reaction of a tetracarboxylic dianhydride component with a diamine component or a diisocyanate component, as the reaction.

13. The production method of an organic compound of any one of claims 1 to 11, wherein a polyamide-based compound is produced as the organic compound by performing a reaction of a dicarboxylic acid component or its acid halide component with a diamine component or a diisocyanate component, as the reaction.

14. The production method of an organic compound of any one of claims 1 to 11, wherein a polyamide-imide-based compound is produced as the organic compound by performing a reaction of a tricarboxylic acid anhydride component or its acid halide component with a diamine component or a diisocyanate component, as the reaction.

15. The production method of an organic compound of any one of claims 1 to 11, wherein a polyester-based compound is produced as the organic compound by performing a reaction of a dicarboxylic acid component or its acid halide component with a polyhydroxy component, as the reaction.

16. The production method of an organic compound of any one of claims 1 to 11, wherein a polyurea-based compound is produced as the organic compound by performing a reaction of a diisocyanate component with a diamine component as the reaction.

17. The production method of an organic compound of any one of claims 1 to 11, wherein a polyurethane-based compound is produced as the organic compound by performing a reaction of a diisocyanate component with a polyhydroxy component as the reaction.

18. The production method of an organic compound of any one of claims 12 to 17, wherein the components are used in an amount such that one component has a molar amount of 0.8 to 1.2 times that of the other component.

19. The production method of an organic compound of any one of claims 1 to 7, wherein the organic compound is a low-molecular compound containing no repeating unit.

20. The production method of an organic compound of claim 19, wherein a diimide dicarboxylic acid-based compound is produced as the organic compound by performing a reaction of a tricarboxylic acid anhydride component with a diamine component in a molar amount of 0.1 to 0.7 times that of the tricarboxylic acid anhydride component, as the reaction.

21. The production method of an organic compound of claim 19, wherein a diimide dicarboxylic acid-based compound is produced as the organic compound by performing a reaction of a tetracarboxylic dianhydride component with a monoaminomonocarboxylic acid component in a molar amount of 1.5 to 10.0 times that of the tetracarboxylic dianhydride component, as the reaction.

22. The production method of an organic compound of claim 19, wherein a diimide tricarboxylic acid-based compound is produced as the organic compound by performing a reaction of a tricarboxylic acid anhydride component with a diaminomonocarboxylic acid component in a molar amount of 0.1 to 0.7 times that of the tricarboxylic acid anhydride component, as the reaction.

23. The production method of an organic compound of claim 19, wherein a diimide tetracarboxylic acid-based compound is produced as the organic compound by performing a reaction of a tetracarboxylic dianhydride component with a monoaminodicarboxylic acid component in a molar amount of 1.5 to 10.0 times that of the tetracarboxylic dianhydride component, as the reaction.

24. The production method of an organic compound of claim 19, wherein a monoimide dicarboxylic acid-based compound is produced as the organic compound by performing a reaction of a tricarboxylic acid anhydride component with a monoaminomonocarboxylic acid component in a molar amount of 0.5 to 5.0 times that of the tricarboxylic acid anhydride component, as the reaction.

25. The production method of an organic compound of claim 19, wherein a monoimide tricarboxylic acid-based compound is produced as the organic compound by performing a reaction of a tricarboxylic acid anhydride component with a monoaminodicarboxylic acid component in a molar amount of 0.5 to 5.0 times that of the tricarboxylic acid anhydride component, as the reaction.

26. The production method of an organic compound of claim 20, wherein an amide group-containing diimide dicarboxylic acid-based compound is produced by using a diamine component containing an amide group as the diamine component.

27. The production method of an organic compound of claim 19, wherein an amide group-containing monoimide dicarboxylic acid-based compound is produced as the organic compound by performing a reaction of a tricarboxylic acid anhydride halide component with a monoaminomonocarboxylic acid component in a molar amount of 1.5 to 10.0 times that of the tricarboxylic acid anhydride halide component, as the reaction.

28. The production method of an organic compound of claim 19, wherein an amide group-containing monoimide tetracarboxylic acid-based compound is produced as the organic compound by performing a reaction of a tricarboxylic acid anhydride halide component with a monoaminodicarboxylic acid component in a molar amount of 1.5 to 10.0 times that of the tricarboxylic acid anhydride halide component, as the reaction.

29. The production method of an organic compound of claim 19, wherein an ester group-containing monoimide tricarboxylic acid-based compound is produced as the organic compound by performing a reaction of a tricarboxylic acid anhydride component with a monohydroxy monoamine component in a molar amount of 0.1 to 0.7 times that of the tricarboxylic acid anhydride component, as the reaction.

30. The production method of an organic compound of claim 19, wherein a diimide dihydroxy-based compound is produced as the organic compound by performing a reaction of a tetracarboxylic dianhydride component with a monohydroxy monoamine component in a molar amount of 1.5 to 10.0 times that of the tetracarboxylic dianhydride component, as the reaction.

31. The production method of an organic compound of claim 19, wherein a diamide dicarboxylic acid-based compound is produced as the organic compound by performing a reaction of a dicarboxylic acid halide component with a monoaminomonocarboxylic acid component in a molar amount of 1.5 to 10.0 times that of the dicarboxylic acid halide component, as the reaction.

32. The production method of an organic compound of claim 19, wherein a diamide tetracarboxylic acid-based compound is produced as the organic compound by performing a reaction of a dicarboxylic acid halide component with a monoaminodicarboxylic acid component in a molar amount of 1.5 to 10.0 times that of the dicarboxylic acid halide component, as the reaction.

33. The production method of an organic compound of claim 19, wherein a diester dicarboxylic acid-based compound is produced as the organic compound by performing a reaction of a dicarboxylic acid halide component with a monohydroxy monocarboxylic acid component in a molar amount of 1.5 to 10.0 times that of the dicarboxylic acid halide component, as the reaction.

34. The production method of an organic compound of claim 19, wherein a diester tetracarboxylic acid-based compound is produced as the organic compound by performing a reaction of a dicarboxylic acid halide component with a monohydroxy dicarboxylic acid component in a molar amount of 1.5 to 10.0 times that of the dicarboxylic acid halide component, as the reaction.

35. The production method of an organic compound of claim 19, wherein a curable imide-based compound is produced as the organic compound by performing a reaction of an unsaturated dicarboxylic acid anhydride component with a diamine component in a molar amount of 0.1 to 0.7 times that of the unsaturated dicarboxylic acid anhydride component, as the reaction.

36. The production method of an organic compound of any one of claims 1 to 35, wherein the reaction is performed in a presence of a catalyst.

37. The production method of an organic compound of any one of claims 1 to 36, wherein the reaction is performed in a presence of an auxiliary agent.

**38.** The production method of an organic compound of any one of claims 1 to 37, wherein after reaction by the mechanochemical effect, a heat treatment is performed, to thereby increase a reaction rate.

## INTERNATIONAL SEARCH REPORT

| | International application No. |
|---|---|
| | PCT/JP2019/025811 |

A. CLASSIFICATION OF SUBJECT MATTER
Int.Cl. See extra sheet

According to International Patent Classification (IPC) or to both national classification and IPC

B. FIELDS SEARCHED

Minimum documentation searched (classification system followed by classification symbols)
Int.Cl.    C07B61/00, C07C67/00, C07C69/00, C07C231/00, C07C233/00,
               C07C235/00, C07C273/00, C07C275/00, C07D207/00, C08F2/00,
               C08G18/00, C08G63/00, C08G69/00, C08G73/00

Documentation searched other than minimum documentation to the extent that such documents are included in the fields searched
    Published examined utility model applications of Japan          1922–1996
    Published unexamined utility model applications of Japan     1971–2019
    Registered utility model specifications of Japan          1996–2019
    Published registered utility model applications of Japan     1994–2019

Electronic data base consulted during the international search (name of data base and, where practicable, search terms used)
    CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | WO 2006/001076 A1 (AGURI FUTURE JOETSU CO., LTD.) 05 January 2006, claims 1–9, example 1 & US 2008/0306235 A1, claims 1–10, example 1 & EP 1762583 A1 & CN 1972980 A | 1–5,7 |
| X | WO 2003/066548 A2 (IOWA STATE UNIVERSITY RESARCH FOUNDATION, INC.) 14 August 2003, claims 1–13, fig. 1, 4, 5 & US 2003/0176740 A1 | 1,5–6,19,36–38 |
| X | CN 101440080 A (ZHEJIANG UNIVERSITY OF TECHNOLOGY) 27 May 2009, claim 1, pp. 7–8, example 1 (Family: none) | 1,19,36–38 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 22 August 2019 (22.08.2019) | 01 October 2019 (01.10.2019) |

| Name and mailing address of the ISA/ Japan Patent Office 3-4-3, Kasumigaseki, Chiyoda-ku, Tokyo 100-8915, Japan | Authorized officer |
|---|---|
| | Telephone No. |

Form PCT/ISA/210 (second sheet) (January 2015)

**INTERNATIONAL SEARCH REPORT**

| International application No. |
|---|
| PCT/JP2019/025811 |

C (Continuation). DOCUMENTS CONSIDERED TO BE RELEVANT

| Category* | Citation of document, with indication, where appropriate, of the relevant passages | Relevant to claim No. |
|---|---|---|
| X | JP 2004-292760 A (NATIONAL INSTITUTE OF ADVANCED INDUSTRIAL SCIENCE AND TECHNOLOGY) 21 October 2004, claims 1-3, examples 1-3 (Family: none) | 1-5,7 |
| X | WO 92/07884 A1 (EISAI CO., LTD.) 14 May 1992, claims 8-13, examples 1-5 & JP 3180106 B2 & US 5821281 A & EP 506979 A1 | 1-2,5-6,8-9 |
| X Y | US 9006488 B1 (MUHAMMAD, Amin) 14 April 2015, claims 1-5, examples 1-4 (Family: none) | 1-6,19 31-32 |
| X | MIKHAILENKO, M. A. et al., "On the mechanism of mechanochemical synthesis of phthalylsulphathiazole", Journal of Materials Science, 2004, 39(16-17), pp. 5435-5439, abstract | 1-6,19 |
| X | MIKHAILENKO, Mikhail A. et al., "Acylation of sulfathiazole with maleic anhydride under mechanochemical activation", Mendeleev Communications, 2007, 17(6), pp. 315-317, page 315 | 1-6,19 |
| X Y | WOUTERS, Johan et al., "MACHANOCHEMICAL SYNTHESIS OF PHTALIMIDES WITH CRYSTAL STRUCTURES OF INTERMEDIATES AND PRODUCTS", CrystEngComm, 2015, 17(12), pp. 2523-2528, scheme 1 | 1-6,19 20-27,30,35 |
| X Y | YANG, Yang et al., "Mechanochemical synthesis of two-dimensional aromatic polyamides", Chemical Communications, 2017, 53(54), pp. 7481-7484, page 7482, fig. 1 | 1-6,8-10 11-18 |
| Y | 社団法人 高分子学会, 高分子化学の基礎, 第 2 版, 株式会社東京化学同人, 25 March 1994, pp. 284-295, in particular, pp. 285-287, 289, non-official translation (The Society of Polymer Science, Japan, "Basics of polymer chemistry", 2nd edition, TOKYO KAGAKU DOJIN KK) | 12-18 |
| Y | JP 8-325376 A (TORAY INDUSTRIES, INC.) 10 December 1996, claims 1, 5-6 (Family: none) | 12 |
| Y | 後藤邦夫, 高分子のメカノケミカル反応, 工業化学雑誌, 1968, vol. 71, no. 9, pp. 1319-1331, 1319, in particular, pp. 1326-1327, 1329, (GOTO, Kunio, "Mechanochemical Reactions of High Polymers", The Journal of the Society of Chemical Industry, Japan) | 12-18 |
| Y | KONNERT, Laure et al., "Solventless Mechanosynthesis of N-Protected Amino Esters", Journal of Organic Chemistry, 2014, 79(9), pp. 4008-4017, page 4009, scheme 1, table 1 | 33-34 |

Form PCT/ISA/210 (continuation of second sheet) (January 2015)

**INTERNATIONAL SEARCH REPORT**

| International application No. |
| --- |
| PCT/JP2019/025811 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | WO 2016/163412 A1 (UNITIKA LTD.) 13 October 2016, claim 1 & US 2018/0086704 A1, claim 1 & EP 3281938 A1 & CN 107531626 A & KR 10-2017-0132752 A | 20,26 |
| Y | JP 9-77872 A (UBE INDUSTRIES, LTD.) 25 March 1997, page 5, synthesis example 1 (Family: none) | 21 |
| Y | JP 50-4063 A (TEIJIN LTD.) 16 January 1975, claims, example 1 (Family: none) | 22 |
| Y | JP 50-50362 A (SHOWA DENKO KABUSHIKI KAISHA) 06 May 1975, claims, example 4 (Family: none) | 23 |
| Y | JP 59-27921 A (HITACHI CHEMICAL INDUSTRY CO., LTD.) 14 February 1984, claims (Family: none) | 24-25,27 |
| Y | JP 51-125477 A (MITSUBISHI ELECTRIC CORP.) 01 November 1976, page 4, upper left column (Family: none) | 30 |
| Y | JP 9-295961 A (TOSOH CORP.) 18 November 1997, claim 1 (Family: none) | 31-32 |
| Y | JP 2006-71765 A (HITACHI CHEMICAL INDUSTRY CO., LTD.) 16 March 2006, claim 2, example 1 (Family: none) | 33-34 |
| Y | JP 2014-80494 A (MITSUBISHI GAS CHEMICAL CO., INC.) 08 May 2014, claims 1-4, synthesis example 4 (Family: none) | 35 |

Form PCT/ISA/210 (continuation of second sheet) (January 2015)

**INTERNATIONAL SEARCH REPORT**

International application No.

PCT/JP2019/025811

```
CLASSIFICATION OF SUBJECT MATTER
C07B61/00(2006.01)i,         C07C67/14(2006.01)i,            C07C69/80(2006.01)i,
C07C69/82(2006.01)i,         C07C231/02(2006.01)i,           C07C233/67(2006.01)i,
C07C233/77(2006.01)i,        C07C233/81(2006.01)i,           C07C235/84(2006.01)i,
C07C273/18(2006.01)i,        C07C275/40(2006.01)i,          C07D207/448(2006.01)i,
C08F2/00(2006.01)i,          C08G18/32(2006.01)i,            C08G18/34(2006.01)i,
C08G63/16(2006.01)i,         C08G63/78(2006.01)i,            C08G69/26(2006.01)i,
C08G73/10(2006.01)i
```

Form PCT/ISA/210 (extra sheet) (January 2015)

**EP 3 819 283 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11333836 A
- JP 2004191760 A

**Non-patent literature cited in the description**

- *Pharmaceutical Journal,* 2000, vol. 120 (12), 1337
- *Proceedings of the Society of Polymer Science,* 2000, vol. 49 (2), 181
- *Polymer-Plastic Technology and Engineering,* 2001, vol. 40 (2), 183
- Controlling Microstructures, Research and Development at the Nano Level Advances. *Recent Trends in Reactive Processing Technology in Polymer Materials (Sumibe Techno Research, 2003.3)*